# EUROPEAN PATENT APPLICATION

(11) **EP 3 656 857 A1**
(43) Date of publication of application: **27.05.2020**
(21) Application number: 18207629.9
(22) Date of filing: 21.11.2018
(51) Int. Cl.: C12N 9/88, C12N 9/10, C12N 9/90, C12N 9/16, C12N 9/02, C12P 7/42

(54) **UTILIZATION OF PHOSPHOKETOLASE FOR PRODUCTION OF AROMATIC AMINO ACID DERIVED PRODUCTS**

(71) Applicant: Danmarks Tekniske Universitet, 2800 Kgs. Lyngby (DK)
(72) Inventor: NIELSEN, Jens, 41128 Gothenburg (SE); CHEN, Yun, 41510 Gothenburg (SE); LIU, Quanli, 41262 Gothenburg (SE)
(74) Representative: Guardian IP Consulting I/S

(57) **Abstract**

The invention provides a genetically modified microorganism for production of aromatic amino acid derivatives; where the microorganism is capable of expressing enhanced levels of DAHP synthase and chorismate mutase, and further comprises transgenes encoding polypeptides having enzymatic activity of phosphoketolase and phosphate acetyltransferase. The invention further provides a method for producing aromatic amino acid derivatives using the genetically modified microorganism of the invention; as well as the use of the genetically modified microorganism for aromatic amino acid derivatives production.

## Description

### FIELD OF THE INVENTION

The invention relates to a genetically modified microorganism for production of aromatic amino acid derived products; where the microorganism is capable of expressing enhanced levels of DAHP synthase and chorismate mutase, and further comprises transgenes encoding polypeptides having enzymatic activity of phosphoketolase and phosphate acetyltransferase. The invention further relates to a method for producing aromatic amino acid derived products using the genetically modified microorganism of the invention; as well as the use of the genetically modified microorganism for aromatic amino acid derivatives production.

### BACKGROUND OF THE INVENTION

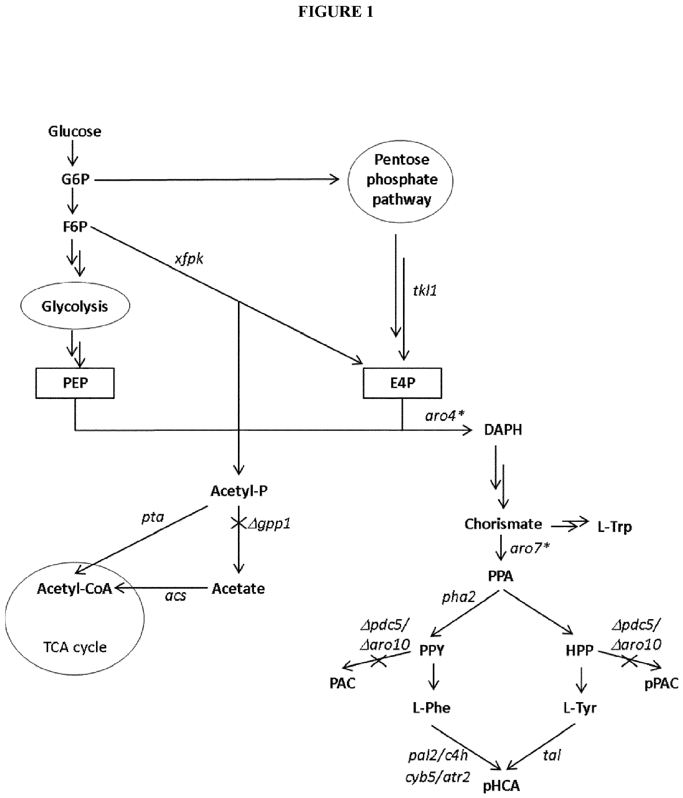

Microbial synthesis of aromatic amino acids proceeds via the shikimate pathway, which consists of several enzymatic reactions leading to the generation of chorismate, the common precursor to all three aromatic amino acids: L-phenylalanine (L-Phe), L-tyrosine (L-Tyr), and L-tryptophan (L-Trp) (Figure 1). The first committed step of the shikimate pathway is the condensation of phosphoenolpyruvate (PEP) and erythrose 4-phosphate (E4P) to form 3-deoxy-D-arabino-heptulosonate-7-phosphate (DAHP). In yeast this reaction is catalyzed by one of two DAHP synthase (E.C.2.5.1.54) isozymes: Aro3 and Aro4, which are allosterically inhibited by phenylalanine and tyrosine, respectively. A single lysine-to-leucine substitution in Aro4 at position 229 results in a deregulated enzyme that is no longer feedback inhibited by tyrosine. Further in yeast, DAHP is consumed by the pentafunctional AROM polypeptide: Aro1, which catalyzes five reactions (E.C.4.2.3.4, E.C.2.5.1.19, E.C.2.7.1.71, E.C.4.2.1.10, and E.C.1.1.1.25), including shikimate synthesis. The last conversion step to chorismate is carried out by chorismate synthase: Aro2 (EC 4.2.3.5). Carbon is diverted away from the tryptophan biosynthesis branch by the activity of chorismate mutase: Aro7 (E.C.5.4.99.5), which catalyzes the conversion of chorismate to prephenate, the last precursor common to both phenylalanine and tyrosine. Aro7 is allosterically inhibited by tyrosine and activated by tryptophan. Substitution of the glycine residue 141 by a serine abolishes the effects of both tyrosine and tryptophan, thus leading to a non-allosterically regulated chorismate mutase.

Many aromatic amino acid derivatives may be produced from L-Phe, L-Tyr, and L-Trp, such as stilbenoids, flavonoids, benzylisoquinoline alkaloids, indole alkaloids, isoprenoids indole alkaloids and coumarins.

p-coumaric acid (pHCA) is an aromatic amino acids derivative. It is a valuable chemical building block and serves as a precursor for biosynthesis of many secondary metabolites, such as polyphenols, flavonoids, and some polyketides.

Tyrosine ammonia-lyase (TAL) can be used to deaminate tyrosine and directly synthesize pHCA from tyrosine. Phenylalanine is similarly deaminated by phenylalanine ammonialyase (PAL), but hydroxylation of the resulting trans-cinnamic acid into p-coumaric acid is further catalyzed by cinnamic acid hydroxylase (C4H), an enzyme which requires cytochrome P450 reductase for efficient electron transfer.

*S*. *cerevisiae* has proven well amenable for genetic engineering and industrial-scale fermentation and is currently used for production of active pharmaceutical ingredients, dietary supplements, chemicals, fuels, etc. *S*. *cerevisiae* has been used as a platform for genetically engineering strains for production of pHCA (Rodriguez et al 2015). However, there remains a need for the development of microorganisms tailor-made for production of a wider range of aromatic amino acid derivatives with enhanced yields and higher carbon conversion efficiency.

### SUMMARY OF THE INVENTION

The invention provides a genetically modified microorganism for production of aromatic amino acid derivatives; wherein said microorganism is capable of expressing enhanced levels of **3-deoxy-D-arabino-heptulosonate-7-phosphate synthase** activity (E.C.2.5.1.54) and **chorismate mutase** activity (E.C.5.4.99.5) compared to a parent microorganism from which said genetically modified microorganism was derived; and wherein said genetically modified microorganism comprises transgenes encoding polypeptides having enzymatic activity of **phosphoketolase** (E.C. 4.1.2.11) and **phosphate acetyltransferase** (E.C.2.3.1.8)

According to a further embodiment, the genetically modified microorganism of the invention is further capable of expressing enhanced levels of **prephenate dehydratase** activity (E.C.4.2.1.51) compared to a parent microorganism from which said genetically modified microorganism was derived.

According to an even further embodiment, one or more genes encoding a polypeptide having enzymatic activity of **glycerol-3-phosphate phosphatase** (E.C.3.1.3.21) are deleted in the genetically modified microorganism of the invention.

The invention further provides a method for producing aromatic amino acid derivatives comprising the steps of (a) introducing a genetically modified microorganism according to any one of claims 1-13 into a growth medium to produce a culture; (b) providing a substrate for the production of an aromatic amino acid derivative to said culture, and culturing said culture; and (c) recovering said aromatic amino acid derivative produced by said culture, and optionally purifying the recovered aromatic amino acid derivative.

The invention further provides for the use of the genetically modified microorganism according to any one embodiment of the invention for the production of aromatic amino acid derivatives.

### DESCRIPTION OF THE INVENTION

### Brief description of the figures:

**Figure 1****:** Schematic map of genetic modifications for pHCA production, wherein G6P= glucose-6-phosphate; F6P= fructose-6-phosphate; PEP= phosphoenolpyruvate; E4P= eryhrose 4-phosphate; DAHP= 3-deoxy-D-arabino-heptulosonic acid; PPA= prephenate; PPY= phenylpyruvate; HPP= para-hydroxy-phenylpyruvate; PAC= phenylacetaldehyde; pPAC= para-hydroxy-acetaldehyde; L-Phe= L-phenylalamine; L-Tyr= L-tyrosine; L-Trp= L-trypophane; pHCA= p-coumaric acid; *xfpk*=xylose-5-phosphate/fructose-6-phosphate phosphoketolase; *pta*= phosphate acetyltransferase; *gpp1*= glycerol-1-phosphatase; *acs*= acetyl-CoA-synthetase; *tkl1*= transketolase; *aro4**=feedback resistant DAHP synthase; *aro7**= feedback resistant choismate mutase; *pha2*= prephenate dehydratase; *pdc5*=pyruvate decarboxylase; *aro10*= phenylpyruvae decarboxylase; *pal2*= phenylalanine ammonia-lyase; *c4h*= cinnamate-4-hydroxylase; *cyb5*= cytochrome 5; *atr2*= cytochrome P450 reductase; *tal*= tyosine ammonia-lyase.
**Figure 2****:** p-Coumaric acid (pHCA) production by genetically modified *S. cerevisias* strains (PAL-branch); demonstrating that xfpk and pta expression in combination with aro4* and aro7' enhances pHCA production.
**Figure 3****:** p-Coumaric acid (pHCA) production by genetically modified *S. cerevisias* strains (TAL-branch); demonstrating that xfpk and pta expression in combination with aro4* and aro7' enhances pHCA production.
**Figure 4****:** p-Coumaric acid (pHCA) production by genetically modified *S. cerevisias* strains; comparing the effect of xfpk and pta expression and tkl1 overexpression.
**Figure 5****:** p-Coumaric acid (pHCA) production by genetically modified *S. cerevisias* strains (PAL-branch); comparing the effect of gpp1 deletion, heterologous acs expression, and expression of a second pta.
**Figure 6****:** p-Coumaric acid (pHCA) production by genetically modified *S. cerevisias* strains (TAL-branch); demonstrating that gpp1 deletion enhances pHCA production.
**Figure 7****:** p-Coumaric acid (pHCA) production by genetically modified *S. cerevisias* strains; demonstrating that Pha2 overexpression enhances pHCA production.
**Figure 8****:** p-Coumaric acid (pHCA) production by genetically modified *S. cerevisias* strains; demonstrating that xfpk and pta expression enhance pHCA production in a benchmark strain.
**Figure 9****:** Schematic map of enzymes involved in production of baicalin, baicalein, and wogonin from phenylalanine, wherein PAL= phenylalanine ammonia lyase; 4CLL= 4-coumaroyl-CoA-like ligase; CHS= chalcone synthase; CHI= chalcone isomerase; FNSII= flavone synthase II; F6H= flavone 6-hydroxylase; UGT= uridine diphosphate (UDP)-dependent glycosyltransferase; F8H= flavone 8-hydroxylase; 8OMT= 8-O-methyltransferase.
**Figure 10****:** Schematic map of enzymes involved in production of daidzein and puerarin from p-coumaric acid, wherein 4CL= 4-coumaroyl-CoA ligase; CHS= chalcone synthase; CHR= chalcone reductase; CHI= chalcone isomerase, IFS= 2-hydroxyisoflavanone synthase; HID= 2-hydroxyisoflavanone dehydratase; 8-C-GT= 8-C-glucosyltransferase.

### Abbreviations, terms and definitions:

**gi number:** (genInfo identifier) is a unique integer which identifies a particular sequence, independent of the database source, which is assigned by NCBI to all sequences processed into Entrez, including nucleotide sequences from DDBJ/EMBL/GenBank, protein sequences from SWISS-PROT, PIR and many others.

**Amino acid sequence identity:** The term "sequence identity" as used herein, indicates a quantitative measure of the degree of homology between two amino acid sequences of substantially equal length. The two sequences to be compared must be aligned to give a best possible fit, by means of the insertion of gaps or alternatively, truncation at the ends of the protein sequences. The sequence identity can be calculated as ((Nref-Ndif)100)/(Nref), wherein Ndif is the total number of non-identical residues in the two sequences when aligned and wherein Nref is the number of residues in one of the sequences. Sequence identity calculations are preferably automated using the BLAST program e.g. the BLASTP program (Pearson W.R and D.J. Lipman (1988)) (www.ncbi.nlm.nih.gov/cgi-bin/BLAST). Multiple sequence alignment is performed with the sequence alignment method ClustalW with default parameters as described by Thompson J., et al 1994, available at http://www2.ebi.ac.uk/clustalw/.

Preferably, the numbers of substitutions, insertions, additions or deletions of one or more amino acid residues in the polypeptide as compared to its comparator polypeptide is limited, i.e. no more than 1, 2, 3, 4, 5, 6, 7, 8, 9, or 10 substitutions, no more than 1, 2, 3, 4, 5, 6, 7, 8, 9, or 10 insertions, no more than 1, 2, 3, 4, 5, 6, 7, 8, 9, or 10 additions, and no more than 1, 2, 3, 4, 5, 6, 7, 8, 9, or 10 deletions. Preferably the substitutions are conservative amino acid substitutions: limited to exchanges within members of group 1: Glycine, Alanine, Valine, Leucine, Isoleucine; group 2: Serine, Cysteine, Selenocysteine, Threonine, Methionine; group 3: proline; group 4: Phenylalanine, Tyrosine, Tryptophan; Group 5: Aspartate, Glutamate, Asparagine, Glutamine.

**Deleted gene:** the deletion of a gene from the genome of a microbial cell leads to a loss of function (knockout) of the gene and hence where the gene encodes a polypeptide the deletion results in a loss of expression of the encoded polypeptide. Where the encoded polypeptide is an enzyme, the gene deletion leads to a loss of detectable enzymatic activity of the respective polypeptide in the microbial cell. A deleted gene in the genome of a microbial cell is characterized by a loss of function due to the deletion of, or substitution of, or addition of, at least one nucleotide leading to a loss of expression of a polypeptide encoded by the gene.

**Genome:** is the genetic material present in a cell or organism; said genome comprising all of the information needed to build and maintain that cell or organism; and includes the genetic material in both chromosome(s) and plasmid(s) present within the cell or organism.

**Native gene:** endogenous gene in a microorganism cell genome, homologous to host microorganism.

**Transgene:** a gene or genetic material that has been transferred naturally or by any of a number of genetic engineering techniques from one organism to another. The transgene that is transferred to the recipient can be from other individuals of the same species or even from unrelated species.

**PAL-branch or PAL-pathway:** Conversion of L-phenylalanine (L-Phe) to pHCA facilitated by phenylalanine ammonia-lyase (Pal2) and cinnamate-4-hydroxylase (C4H).

**TAL-branch or TAL-pathway:** Conversion of L-tyrosine (L-Tyr) to pHCA facilitated by tyrosine ammonia-lysase (TAL).

**Aro4*:** the asterisk denotes that the polypeptide encoded by the aro4* gene is a feedback resistant DAHP synthase, such as *S*. *cerevisiae* ARO4^{K229L}

**Aro7*:** the asterisk denotes that the polypeptide encoded by the aro7* gene is a feedback resistant chorismate mutase, such as S. cerevisiae ARO7^{G141S}

| **Table 1: List of most relevant genes and polypeptides** | | |
|---|---|---|
| **Gene** | **Polypeptide encoded by gene** | **E.C. number** |
| Aro4 | 3-deoxy-D-arabino-heptulosonate-7-phosphate (DAHP) synthase | E.C.2.5.1.54 |
| Aro7 | chorismate mutase | E.C.5.4.99.5 |
| Xfpk | xylulose-5-phosphate/fructose-6-phosphate phosphoketolase | E.C.4.1.2.22 |
| Pta | phosphate acetyltransferase | E.C.2.3.1.8 |
| Pal2 | phenylalanine ammonia-lyase | E.C.4.3.1.24 |
| C4h | cinnamate-4-hydroxylase | E.C.1.14.13.11 |
| Atr2 | P450 reductase | E.C.1.6.2.4 |
| Cyb5 | cytochrome b5 | |
| Pha2 | prephenate dehydratase | E.C.4.2.1.51 |
| Gpp1 | glycerol-1-phosphatase | E.C.3.1.3.21 |
| Tal | tyrosine ammonia-lyase | E.C.4.3.1.25 |
| Tkl1 | transketolase | E.C.2.2.1.1 |
| Acs | acetyl-CoA synthetase | E.C.6.2.1.1 |
| aroL | Shikimate kinase | E.C.2.7.1.7 |
| Aro10 | Phenylpyruvate decarboxylase | E.C.4.1.1.43 |
| Pdc5 | Pyruvate decarboxylase | E.C.4.1.1.1 |
| Gal7 | Galactose-1-phosphate uridyl transferase | EC:2.7.7.12 |
| GallO | UDP-glucose-4-epimerase | EC:5.1.3.2 |
| Gall | Galactokinase | EC:2.7.1.6 |

### Detailed description of the invention:

The present invention provides a two-fold approach to the adaptation of a microorganism for use as a cell factory for production of aromatic amino acid derivatives: (i) modification of the existing aromatic amino acid production pathway wherein phosphoenolpyruvate (PEP) and erythrose 4-phosphate (E4P) are converted to aromatic amino acids, and (ii) expression of transgenes encoding enzymes capable of increasing the supply of E4P.

The second approach, which has not previously been described, involves the expression of recombinant enzymes phosphoketolase (xfpk) and phosphate acetyltransferase (pta).

### I. A genetically modified microorganism for production of aromatic amino acid derivatives

The present invention provides a genetically modified microorganism capable of producing enhanced levels of aromatic amino acid derivatives.

In one embodiment, the present invention provides a genetically modified microorganism for use as a platform strain for enhanced production of aromatic amino acid derivatives.

In a further embodiment, the present invention provides a genetically modified microorganism as a platform for producing aromatic amino acid derivatives selected from among stilbenoids (such as resveratrol, combretastatin A, resveratrol oligomers, piceatannol oligomers, oxyresveratrol oligomers), flavonoids (such as lunularine, marchantins A and C, hirchinol, luteolin, apigenin, tangeritin, tageretin, nobiletin, sinensetin, baicalein, kaempferol, quercetin, myricetin, fisetin, hesperitin, naringenin, eriodictyol, genistein, daidzein, puerarin, calophyllolide, catechin, epicatechin, epigallocatechin, epicatechin gallate, cyanidin, delphinidin, malvidin, pelargonidin, peonidin, phloridzin, arbutin, phloretin, chalconaringenin), benzylisoquinoline alkaloids (such as papaverine, scoulerine, corydaline, protopine, magnoflorine, sanguinarine, noscapine, salutaridine, cularine, rhoeadine, morphine, dauricine, eschsholtzidine), and coumarins (such as p-coumeric acid, umbelliferone, aesculetin, herniarin, psoralen, imperatorin, 4-hydroxycoumarin, warfarin, dicoumarol, acenocoumarol, ethyl biscoumacetate, phenprocoumon). Production of specific aromatic amino acid derivatives may necessitate addition of relevant pathway genes to the platform strain of the present invention.

In another embodiment, the present invention provides a genetically modified microorganism for enhanced production of aromatic amino acid intermediates, selected from prephenate and phenylpyruvate.

In yet another embodiment, the present invention provides a genetically modified microorganism for enhanced production of aromatic amino acids selected from L-Phe and L-Tyr.

### I.i Modifications to the aromatic amino acid pathway

The microorganism of the invention is capable of expressing enhanced levels of 3-deoxy-D-arabino-heptulosonate-7-phosphate (DAHP) synthase activity (E.C.2.5.1.54) and chorismate mutase activity (E.C. 5.4.99.5). The native aromatic amino acid production pathway of the microorganism is thereby modified to obtain an improved conversion of PEP and E4P into aromatic amino acids.

The microorganism of the invention is capable of expressing **enhanced levels of DAHP synthase activity (E.C.2.5.1.54)** for conversion of phosphoenolpyruvate (PEP) and D-erythrose 4-phosphate (E4P) to 3-deoxy-D-arabino-hept-2-ulosonate-7-phosphate (DAHP). In *S*. *cerevisiae,* DAHP synthase is encoded by the gene aro4.

Enhanced levels of DAHP synthase activity (E.C.2.5.1.54) in the microorganism of the invention may be facilitated by standard methods of enhancing gene expression recognized by a person skilled in the art; such as but not limited to the methods described in Section III.

In one embodiment, the amino acid sequence of the expressed polypeptide having DAHP synthase activity (E.C.2.5.1.54) has at least 70, 72, 74, 76, 78, 80, 82, 84, 86, 88, 90, 92, 94, 96, 98, 99 or 100% sequence identity to the amino acid sequence of the DAHP synthase encoded by the *S. cerevisiae* gene aro4 (SEQ ID NO: 1). Alternatively, the amino acid sequence of the expressed polypeptide having DAHP synthase activity (E.C.2.5.1.54), has at least 70, 72, 74, 76, 78, 80, 82, 84, 86, 88, 90, 92, 94, 96, 98, 99 or 100% sequence identity to an amino acid sequence selected from among SEQ ID NO: 2 (derived from *Galactomyces candidus*), SEQ ID NO: 3 (derived from *Saccharomyces eubayanus*), SEQ ID NO: 4 (derived from *Kazachstania saulgeensis*), and SEQ ID NO: 5 (derived from *Komagataella phaffii* GS115).

In a preferred embodiment, enhanced levels of DAHP synthase activity are facilitated by expression of a mutant gene encoding a feedback resistant DAHP synthase polypeptide. Feedback resistant DAHP synthase is in S. *cerevisiae* encoded by gene aro4*.

In a preferred embodiment, the amino acid sequence of the feedback resistant DAHP synthase polypeptide has at least 70, 72, 74, 76, 78, 80, 82, 84, 86, 88, 90, 92, 94, 96, 98, 99 or 100% sequence identity to the amino acid sequence of the DAHP synthase encoded by the *S. cerevisiae* gene aro4 (SEQ ID NO: 1) wherein the amino acid residue lysine at position Lys229 is substituted by any amino acid other than lysine, preferably by a non-polar amino acid, such as by an amino acid selected from the group consisting of leucine, glycine, alanine, valine, cysteine, isoleucine, and methionine, most preferably substituted by leucine.

Alternatively, the amino acid sequence of the feedback resistant DAHP synthase polypeptide has at least 70, 72, 74, 76, 78, 80, 82, 84, 86, 88, 90, 92, 94, 96, 98, 99 or 100% sequence identity to the amino acid sequence selected from among SEQ ID NO: 2 (derived from *Galactomyces candidus*), SEQ ID NO: 3 (derived from *Saccharomyces eubayanus*), SEQ ID NO: 4 (derived from *Kazachstania saulgeensis*), and SEQ ID NO: 5 (derived from *Komagataella phaffii* GS115), wherein amino acid residue lysine at position Lys232, Lys229, Lys224, and Lys119, respectively, is substituted by any amino acid other than lysine, preferably substituted by an non-polar amino acid, such as by an amino acid selected from the group consisting of leucine, glycine, alanine, valine, cysteine, isoleucine, and methionine, most preferably substituted by leucine.

In another embodiment, the amino acid sequence of the feedback resistant DAHP synthase polypeptide has at least 70, 72, 74, 76, 78, 80, 82, 84, 86, 88, 90, 92, 94, 96, 98, 99 or 100% sequence identity to the amino acid sequence of the DAHP synthase encoded by the *S. cerevisiae* gene aro4 (SEQ ID NO: 1 ARO4) wherein one or more amino acid residues at positions 162, 166, 193, 195, 226, 229, or 236 is substituted by an amino acid different from the native amino acid residue.

The aro4* gene encoding the feedback resistant DAHP synthase polypeptide may be present in one or more copies in the genome of the microorganism, such as present in two copies, such as present in three, four, five or more copies. The aro4* gene copies may be identical copies of the same gene or copies of different genes encoding polypeptides having feedback resistant DAHP synthase activity.

The microorganism of the invention is further capable of expressing **enhanced levels of chorismate mutase activity (E.C.5.4.99.5)** for conversion of chorismate to prephenate. In *S. cerevisiae,* chorismate mutase is encoded by the gene aro7.

Enhanced levels of chorismate mutase activity in the microorganism of the invention may be facilitated by standard methods of enhancing gene expression recognized by a person skilled in the art; such as, but not limited to, the methods described in Section III.

In one embodiment, the amino acid sequence of the polypeptide having chorismate mutase activity (E.C.5.4.99.5) has at least 70, 72, 74, 76, 78, 80, 82, 84, 86, 88, 90, 92, 94, 96, 98, 99 or 100% sequence identity to the amino acid sequence of the chorismate mutase encoded by the *S. cerevisiae* gene aro7 (SEQ ID NO: 6). Alternatively, the amino acid sequence of the polypeptide having chorismate mutase activity (E.C.5.4.99.5), has at least 70, 72, 74, 76, 78, 80, 82, 84, 86, 88, 90, 92, 94, 96, 98, 99 or 100% sequence identity to an amino acid sequence selected from among SEQ ID NO: 7 (derived from *Hanseniaspora osmophila*), SEQ ID NO: 8 (derived from *Saccharomyces arboricola*), SEQ ID NO: 9 (derived from *Kluyveromyces marxianus* DMKU3-1042), and SEQ ID NO: 10 (derived from *Candida glabrata*)*.*

In a preferred embodiment, enhanced levels of chorismate mutase activity are facilitated by expression of a mutant gene encoding a feedback resistant chorismate mutase polypeptide. In *S. cerevisiae,* feedback resistant chorismate mutase is encoded by gene aro7*.

In a preferred embodiment, the amino acid sequence of the feedback resistant chorismate mutase polypeptide has at least 70, 72, 74, 76, 78, 80, 82, 84, 86, 88, 90, 92, 94, 96, 98, 99 or 100% sequence identity to the amino acid sequence of the chorismate mutase encoded by the *S. cerevisiae* gene aro7 (SEQ ID NO: 6) wherein the amino acid residue glycine at position Gly141 is substituted by any amino acid other than glycine, such as by a polar amino acid, preferably by an amino acid selected from the group consisting of glutamine, asparagine, histidine, serine, threonine, tyrosine, cysteine, and tryptophan, more preferably by threonine or serine, and most preferably by serine.

Alternatively, the amino acid sequence of the feedback resistant chorismate mutase polypeptide has at least 70, 72, 74, 76, 78, 80, 82, 84, 86, 88, 90, 92, 94, 96, 98, 99 or 100% sequence identity to the amino acid sequence selected from among SEQ ID NO: 7 (derived from *Hanseniaspora osmophila*), SEQ ID NO: 8 (derived from *Saccharomyces arboricola*), SEQ ID NO: 9 (derived from *Kluyveromyces marxianus* DMKU3-1042), and SEQ ID NO: 10 (derived from *Candida glabrata*), wherein amino acid residue glycine at position Gly141, Gly141, Gly141, and Gly142, respectively, is substituted by a polar amino acid, such as by an amino acid selected from the group consisting of glutamine, asparagine, histidine, serine, threonine, tyrosine, cysteine, and tryptophan, preferably by threonine or serine, most preferably by serine.

The aro7* gene encoding the feedback resistant chorismate mutase polypeptide may be present in one or more copies in the genome of the microorganism, such as present in two copies, or present in three, four, five or more copies. The aro7* gene copies may be identical copies of the same gene or copies of different genes encoding polypeptides having feedback resistant chorismate mutase activity.

### I.ii Expression of transgenes for increasing E4P

The microorganism of the invention comprises transgenes encoding polypeptides having phosphoketolase activity (E.C.4.1.2.22) and phosphate acyltransferase activity (E.C.2.3.1.8). Expression of transgenes encoding these enzymes facilitates an increased supply of E4P for aromatic amino acid production.

The microorganism of the invention comprises a transgene encoding a polypeptide having **phosphoketolase** activity (E.C.4.1.2.22) that converts D-fructose 6-phosphate (F6P) to D-erythrose 4-phosphate (E4P) and acetyl phosphate (AcP). In one embodiment, the amino acid sequence of the polypeptide encoded by the phosphoketolase transgene has at least 70, 72, 74, 76, 78, 80, 82, 84, 86, 88, 90, 92, 94, 96, 98, 99 or 100% sequence identity to the amino acid sequence of the xylulose-5-phosphate/fructose-6-phosphate phosphoketolase encoded by the *Bifidobacterium breve* xfpk gene (SEQ ID NO: 12). Alternatively, the amino acid sequence of the polypeptide having phosphoketolase activity (E.C.4.1.2.22), has at least 70, 72, 74, 76, 78, 80, 82, 84, 86, 88, 90, 92, 94, 96, 98, 99 or 100% sequence identity to an amino acid sequence selected from among SEQ ID NO: 12 (derived from *Bifidobacterium cuniculi*), SEQ ID NO: 14 (derived from *Bifidobacterium pseudolongum*) and SEQ ID NO: 15 (derived from *Bifidobacterium thermophilum*)*.*

The transgene encoding phosphoketolase (E.C.4.1.2.22) may be present in one or more copies in the genome of the microorganism, such as present in two copies, or in three, four, five or more copies. The transgene copies may be identical copies of the same gene or copies of different genes encoding polypeptides having phosphoketolase activity.

The microorganism of the invention further comprises a transgene encoding a polypeptide having **phosphate acyltransferase** activity (E.C.2.3.1.8) that converts acetyl phosphate to acetyl-CoA. In one embodiment, the amino acid sequence of the polypeptide encoded by the phosphate acyltransferase gene has at least 70, 72, 74, 76, 78, 80, 82, 84, 86, 88, 90, 92, 94, 96, 98, 99 or 100% sequence identity to the amino acid sequence of the phosphate acyltransferase encoded by the *Clostridium kluveri* pta gene (SEQ ID NO: 17). Alternatively, the amino acid sequence of the polypeptide having phosphate acyltransferase activity (E.C.2.3.1.8), has at least 70, 72, 74, 76, 78, 80, 82, 84, 86, 88, 90, 92, 94, 96, 98, 99 or 100% sequence identity to an amino acid sequence selected from among SEQ ID NO: 18 (derived from *Clostridium beijerinckii*), SEQ ID NO: 19 (derived from *Clostridium cochlearium*), and SEQ ID NO: 20 (derived from *Clostridium magnum*)*.*

The transgene encoding phosphate acyltransferase (E.C.2.3.1.8) may be present in one or more copies in the genome of the microorganism, such as present in two copies, such as present in three, four, five or more copies. The transgene copies may be identical copies of the same gene or copies of different genes encoding polypeptides having phosphate acyltransferase activity.

A genetically modified microorganism according to one embodiment of the invention, that is capable of expressing enhanced levels of 3-deoxy-D-arabino-heptulosonate-7-phosphate (DAHP) synthase activity (E.C.2.5.1.54) and chorismate mutase activity (E.C. 5.4.99.5) and further expressing transgenes encoding polypeptides having phosphoketolase activity (E.C.4.1.2.11) and phosphate acyltransferase activity (E.C.2.3.1.8), is shown to produce enhanced levels of aromatic amino acid derivatives in Example 2. Importantly, this genetically modified microorganism provides a platform strain; which may be tailored for production of a desired aromatic amino acid derivative by introducing further appropriate transgenes required for production of the desired derivative. Measuring increased flux to aromatic amino acid derivatives cannot be done by direct measurement of aromatic amino acids as they are consumed by the microorganism. Instead, the aromatic amino acid derivative, pHCA, was used as a "marker" for determining the efficiency of aromatic amino acid derivative production by the microorganism. For this purpose, additional transgenes (PAL or TAL pathway, as explained in greater detail later) needed for the conversion of phenylalanine or tyrosine to pHCA were introduced into the platform strain. Increased levels of the "marker" pHCA provide an indirect measure of an increase in flux towards phenylalanine and/or tyrosine synthesis and their derivatives. An increased flux towards aromatic amino acid derivatives, reflected by the levels of pHCA in a microorganism of the invention (Example 2), can be expected to give rise to a corresponding increase in production of other aromatic amino acid derivatives in cells of the platform microorganism additionally expressing transgenes required for their production.

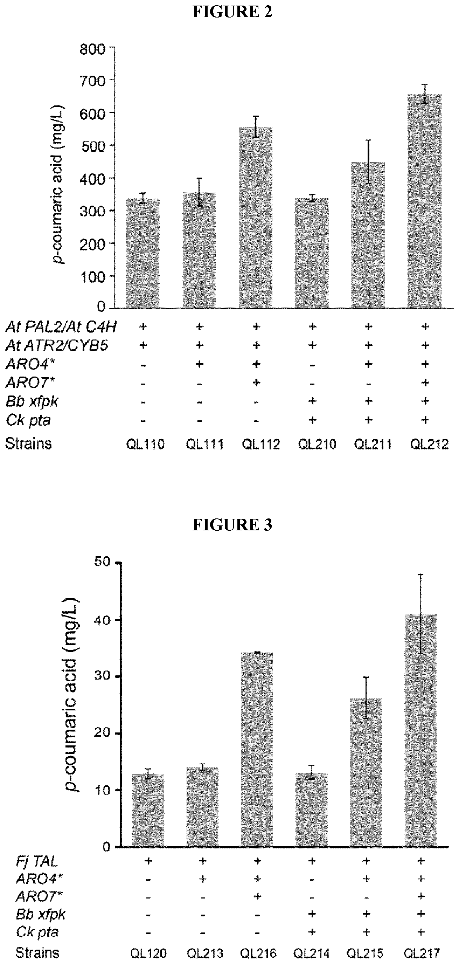

The surprising effect of combining expression of transgenes encoding polypeptides having phosphoketolase and phosphate acyltransferase activity with enhanced levels of 3-deoxy-D-arabino-heptulosonate-7-phosphate (DAHP) synthase and chorismate mutase activity is reported in Figures 2 and 3, showing enhanced titers of pHCA production.

### I.iii Increasing flux to phenylalanine

The microorganism of the invention may further be capable of expressing enhanced levels of **prephenate dehydratase** activity (E.C.4.2.1.51) for conversion of prephanate to phenylpyruvate (a precursor of phenylalanine in the phenylalanine biosynthesis pathway), as shown in Figure 1. Prephenic acid is the branching point in the synthesis of phenylalanine and tyrosine. In *S. cerevisiae,* prephenate dehydratase is encoded by the gene pha2. Overexpression (or other means of enhanced activity) of the pha2 gene has not previously been reported; only limited information on the gene product of pha2 is available.

In one embodiment of the invention, enhanced levels of prephenate dehydratase activity (E.C.4.2.1.51) may be facilitated by standard methods of enhancing gene expression recognized by a person skilled in the art; such as but not limited to the methods described in Section III.

In one embodiment, the amino acid sequence of the polypeptide having prephenate dehydratase activity (E.C.5.4.99.5) has at least 70, 72, 74, 76, 78, 80, 82, 84, 86, 88, 90, 92, 94, 96, 98, 99 or 100% sequence identity to the amino acid sequence of the prephenate dehydratase encoded by the *S. cerevisiae* gene pha2 (SEQ ID NO: 22). Alternatively, the amino acid sequence of the polypeptide having prephenate dehydratase activity (E.C.5.4.99.5), has at least 70, 72, 74, 76, 78, 80, 82, 84, 86, 88, 90, 92, 94, 96, 98, 99 or 100% sequence identity to an amino acid sequence selected from among SEQ ID NO: 23 (derived from *Saccharomyces arboricola* H-6), SEQ ID NO: 24 (derived from *Saccharomyces cerevisiae* Kyokai no. 7), and SEQ ID NO: 25 (derived from *Saccharomyces cerevisiae x Saccharomyces kudriavzevii* VIN7).

A microorganism capable of expressing enhanced levels of prephenate dehydratase activity as provided in one embodiment of the invention is demonstrated in Example 5. As explained previously, pHCA was used as a "marker" for production of aromatic amino acid derivatives. Additional transgenes (PAL pathway, as explained in greater detail later) were introduced for conversion of phenylalanine to pHCA. Increased levels of pHCA detected in the microorganism of the invention reflects an increased flux towards phenylalanine, which can be expected to give rise to a corresponding increase in the production of other Phe-derivatives in cells of the platform microorganism additionally expressing transgenes required for their production.

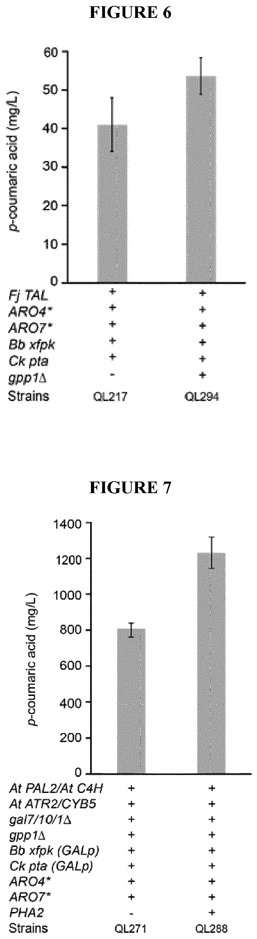

The advantageous effect of enhancing the levels of prephenate dehydratase activity is reported in Figure 7, showing enhanced titers of pHCA production as compared to a parent strain wherein the levels of prephenate dehydratase have not been enhanced gene.

### I.iv Deletion of endogenous gene(s) to enhance the conversion of Acetyl-P to Acetyl-CoA to fuel the TCA cycle while reducing acetate accumulation

The microorganism of the invention may be deficient of **glycerol-3-phosphatase** (E.C.3.1.3.21) activity, such as by having one or more endogenous native genes encoding a polypeptide having glycerol-3-phosphatase activity knocked out, causing a block in the conversion of acetyl-P to acetate in the microorganism. Deletion of glycerol-3-phosphatase enzyme (E.C.3.1.3.21) provides a microorganism of the invention with enhanced conversion of Acetyl-P to Acetyl-CoA by the phosphate acetyltransferase enzyme to fuel the TCA cycle, rather than conversion of the acetyl-P to acetate, accumulation of which can have adverse effects on cell growth. For example, the deleted gene may be an endogenous gene encoding a polypeptide having glycerol-3-phosphatase activity in the respective microorganism. Preferably the amino acid sequence of the polypeptide having glycerol-3-phosphatase activity (E.C.3.1.3.21) has at least 70, 72, 74, 76, 78, 80, 82, 84, 86, 88, 90, 92, 94, 96, 98, 99 or 100% amino acid sequence identity to a sequence selected from among: SEQ ID NO: 27 in *S. cerevisiae,* SEQ ID NO: 28 in *Kazachstania africana* CBS 2517, SEQ ID NO: 29 in *Saccharomyces arboricola* H-6, and SEQ ID NO: 30 in *Zygosaccharomyces parabailii.*

In one embodiment, an endogenous gene encoding a polypeptide having glycerol-3-phosphatase enzymatic activity (E.C.3.1.3.21) is deleted from the genome of the microorganism of the invention. For example, where the microorganism of the invention belongs to a species of *Saccharomyces,* the deleted glycerol-3-phosphatase gene encodes a polypeptide whose sequence has at least 70, 72, 74, 76, 78, 80, 82, 84, 86, 88, 90, 92, 94, 96, 98, 99 or 100% amino acid sequence identity to SEQ ID NO: 27.

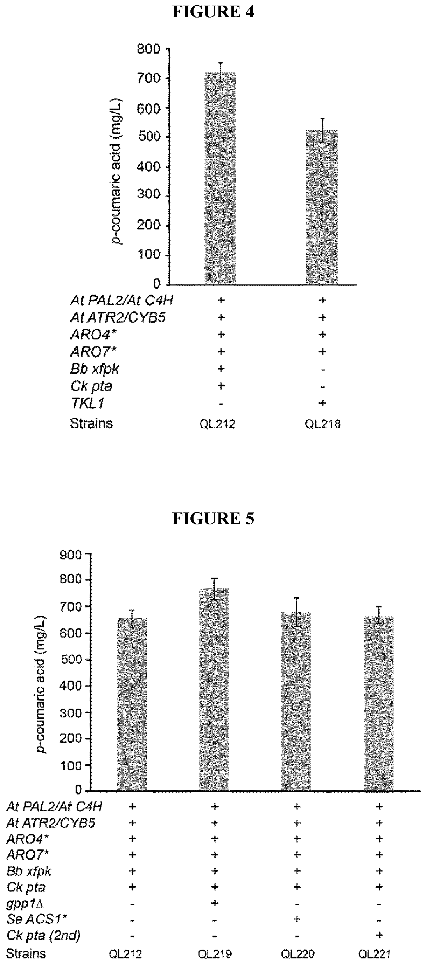

Deletion of an endogenous gene encoding a polypeptide having glycerol-3-phosphatase (ggp1) enzymatic activity as provided in one embodiment of the invention is demonstrated in Example 4. As explained previously, pHCA was used as a "marker" for production of aromatic amino acid derivatives, whereby transgenes (PAL or TAL pathway, as explained in greater detail later) required for conversion of phenylalanine or tyrosine to pHCA were introduced into the tested stain. Increased levels of pHCA must inherently be due to an increased flux towards phenylalanine and/or tyrosine, which can be expected to give rise to a corresponding increase in the production of other Phe-Tyr derivatives in cells of the platform microorganism additionally expressing transgenes required for their production.. The advantageous effect of deleting the ggp1 gene is reported in Figure 5 and 6, showing enhanced titers of pHCA production as compared to a parent strain comprising the native ggp1 gene.

### I.v Expression of transgenes for conversion of L-Phe and/or L-Tyr to pHCA

According to one embodiment of the invention, the microorganism of the invention may further comprise transgenes encoding polypeptides having phenylalanine ammonia-lyase activity (E.C.4.3.1.24) and cinnamate-4-hydroxylase activity (E.C.1.14.13.11). Expression of transgenes encoding these enzymes facilitates conversion of L-Phe to pHCA by a cytochrome P450 reductase mediated pathway. This is herein referred to as the PAL-pathway.

The microorganism of the invention may comprise a transgene encoding a polypeptide having **phenylalanine ammonia-lyase** activity (E.C.4.3.1.24) that converts L-phenylalanine to ammonia and trans-cinnamate. The amino acid sequence of the polypeptide encoded by the phenylalanine ammonia-lyase gene has at least 70, 72, 74, 76, 78, 80, 82, 84, 86, 88, 90, 92, 94, 96, 98, 99 or 100% sequence identity to the amino acid sequence of the phenylalanine ammonia-lyase encoded by the *Arabidopsis thaliana* pal2 gene (SEQ ID NO: 32). Alternatively, the amino acid sequence of the polypeptide having phenylalanine ammonia-lyase activity (E.C.4.3.1.24), has at least 70, 72, 74, 76, 78, 80, 82, 84, 86, 88, 90, 92, 94, 96, 98, 99 or 100% sequence identity to an amino acid sequence selected from among SEQ ID NO: 33 (derived from *Isoetes lacustris*), SEQ ID NO: 34 (derived from *Arabidopsis lyrata* subsp. lyrata), and SEQ ID NO: 35 (derived from *Parrya nudicaulis*)*.*

The transgene encoding phenylalanine ammonia-lyase (E.C.4.3.1.24) may be present in one or more copies in the genome of the microorganisml, such as present in two copies, such as present in three, four, five or more copies. The transgene copies may be identical copies of the same gene or copies of different genes encoding polypeptides having phenylalanine ammonia-lyase activity.

The microorganism of the invention may further comprise a transgene encoding a polypeptide having **cinnamate-4-hydroxylase** activity (E.C.1.14.13.11) that converts trans-cinnamate to 4-hydroxycinnamate (also known as p-coumaric acid (pHCA)). The amino acid sequence of the polypeptide encoded by the cinnamate-4-hydroxylase gene has at least 70, 72, 74, 76, 78, 80, 82, 84, 86, 88, 90, 92, 94, 96, 98, 99 or 100% sequence identity to the amino acid sequence of the cinnamate-4-hydroxylase encoded by the *Arabidopsis thaliana* c4h gene (SEQ ID NO: 37). Alternatively, the amino acid sequence of the polypeptide having cinnamate-4-hydroxylase activity (E.C.1.14.13.11), has at least 70, 72, 74, 76, 78, 80, 82, 84, 86, 88, 90, 92, 94, 96, 98, 99 or 100% sequence identity to an amino acid sequence selected from among SEQ ID NO: 38 (derived from *Daucus carota* subsp. sativus), SEQ ID NO: 39 (derived from *Capsella rubella*)*,* and SEQ ID NO: 40 (derived from *Ginkgo biloba*)*.*

The transgene encoding cinnamate-4-hydroxylase (E.C.1.14.13.11) may be present in one or more copies in the genome of the microorganism, such as present in two copies, such as present in three, four, five or more copies. The transgene copies may be identical copies of the same gene or copies of different genes encoding polypeptides having phenylalanine ammonia-lyase activity.

According to a further embodiment of the invention, the microorganism of the invention may further be capable of expressing (i) enhanced amounts of electron transporter protein **cytochrome b5** and (ii) enhanced levels of **cytochrome P450 reductase** activity (E.C.1.6.2.4).

In one embodiment of the invention, enhanced amounts of electron transporter protein **cytochrome b5** may be facilitated by standard methods of enhancing gene expression recognized by a person skilled in the art; such as but not limited to the methods described in Section III.

In one embodiment, the amino acid sequence of the cytochrome b5 polypeptide has at least 70, 72, 74, 76, 78, 80, 82, 84, 86, 88, 90, 92, 94, 96, 98, 99 or 100% sequence identity to the amino acid sequence of the cytochrome b5 encoded by the *S. cerevisiae* gene cyb5 (SEQ ID NO: 42). Alternatively, the amino acid sequence of the cytochrome b5 polypeptide has at least 70, 72, 74, 76, 78, 80, 82, 84, 86, 88, 90, 92, 94, 96, 98, 99 or 100% sequence identity to an amino acid sequence selected from among SEQ ID NO: 43 (derived from Saccharomyces eubayanus), SEQ ID NO: 44 (derived from *Saccharomyces cerevisiae* YJM627), and SEQ ID NO: 45 (derived from *Saccharomyces arboricola* H-6).

In one embodiment of the invention, enhanced levels of **cytochrome P450 reductase** activity (E.C.1.6.2.4) may be facilitated by standard methods of enhancing gene expression recognized by a person skilled in the art; such as but not limited to the methods described in Section III.

In one embodiment, the amino acid sequence of the polypeptide having cytochrome P450 reductase activity (E.C.1.6.2.4) has at least 70, 72, 74, 76, 78, 80, 82, 84, 86, 88, 90, 92, 94, 96, 98, 99 or 100% sequence identity to the amino acid sequence of the cytochrome P450 reductase encoded by the *Arabidopsis thaliana* gene atr2 (SEQ ID NO: 47). Alternatively, the amino acid sequence of the polypeptide having cytochrome P450 reductase activity (E.C.1.6.2.4) has at least 70, 72, 74, 76, 78, 80, 82, 84, 86, 88, 90, 92, 94, 96, 98, 99 or 100% sequence identity to an amino acid sequence selected from among SEQ ID NO: 48 (derived from *Ananas comosus*), SEQ ID NO: 49 (derived from *Capsella rubella*), and SEQ ID NO: 50 (derived from *Eutrema salsugineum*)*.*

According to another embodiment of the invention, the microorganism of the invention may comprise a transgene encoding a polypeptide having **tyrosine ammonia-lyase** activity (E.C.4.3.1.25) for conversion of L-Tyr to pHCA. This is herein referred to as the TAL-pathway.

In one embodiment, the amino acid sequence of the polypeptide encoded by the tyrosine ammonia-lyase gene has at least 70, 72, 74, 76, 78, 80, 82, 84, 86, 88, 90, 92, 94, 96, 98, 99 or 100% sequence identity to the amino acid sequence of the tyrosine ammonia-lyase encoded by the *Flavobacerium johnsoniaeu* tal gene (SEQ ID NO: 52). Alternatively, the amino acid sequence of the polypeptide having tyrosine ammonia-lyase activity (E.C.4.3.1.24), has at least 70, 72, 74, 76, 78, 80, 82, 84, 86, 88, 90, 92, 94, 96, 98, 99 or 100% sequence identity to an amino acid sequence selected from among SEQ ID NO: 53 (derived from *Chryseobacterium piscium*), SEQ ID NO: 54 (derived from *Flavobacterium denitrificans*), and SEQ ID NO: 55 (derived from *Flavobacterium swingsii).*

The transgene encoding tyrosine ammonia-lyase (E.C.4.3.1.25) may be present in one or more copies in the genome of the microorganism, such as present in two copies, such as present in three, four, five or more copies. The transgene copies may be identical copies of the same gene or copies of different genes encoding polypeptides having tyrosine ammonia-lyase activity.

According to a further embodiment of the invention, the microorganism of the invention may be characterized by comprising both the PAL-pathway and the TAL-pathway for production of pHCA, as described above.

A microorganism expressing phenylalanine ammonia-lyase (pal2) and cinnamate-4-hydroxylase (c4h) while having enhanced amounts of electron transporter protein cytochrome b5 (cyb5) and enhanced levels of cytochrome P450 reductase activity (atr2) for conversion of L-Phe to pHCA (PAL pathway) as provided in one embodiment of the invention is demonstrated in Example 2. A microorganism expressing tyrosine ammonia-lyase for conversion of L-Tyr to pHCA (TAL pathway) as provided in another embodiment of the invention is also demonstrated in Example 2. As mentioned previous, pHCA was used as a marker for production of aromatic amino acid derivative production; the PAL or TAL pathway was therefore introduced for conversion of phenylalanine or tyrosine to pHCA.

The advantageous effect of producing pHCA by conversion of L-Phe to pHCA (pal-pathway) as compared to the conversion of L-Tyr to pHCA by tyrosine ammonia-lyase (tal-pathway) is reported in Figure 2 and 3, showing enhanced titers of pHCA production for the pal-pathway.

### I.vi Deletion of endogenous gene(s) to avoiding formation of aromatic alcohol but rather direct the pathway flux to aromatic amino acids

The microorganism of the invention may be deficient of **phenylpyruvate decarboxylase** (E.C.4.1.1.43) activity, such as by having one or more endogenous native genes encoding a polypeptide having phenylpyruvate decarboxylase activity knocked out, causing a block in the conversion of phenyl pyruvate (PPY) to phenylacetaldehyde (PAC) in the microorganism (see figure 1). Deletion of phenylpyruvate decarboxylase (E.C.4.1.1.43) provides a microorganism of the invention with enhanced flux to aromatic amino acids, rather than formation of aromatic alcohols. For example, the deleted endogenous gene is one encoding a polypeptide having phenylpyruvate decarboxylase activity in the respective microorganism. Preferably the amino acid sequence of the polypeptide having phenylpyruvate decarboxylase activity (E.C.4.1.1.43) has at least 70, 72, 74, 76, 78, 80, 82, 84, 86, 88, 90, 92, 94, 96, 98, 99 or 100% amino acid sequence identity to a sequence selected from among: SEQ ID NO: 57 in *S. cerevisiae,* SEQ ID NO: 58 in *Saccharomyces eubayanus,* SEQ ID NO: 59 in *Saccharomyces arboricola* H-6, and SEQ ID NO: 60 in *Saccharomyces kudriavzevii* IFO 1802.

In one embodiment, an endogenous gene encoding a polypeptide having phenylpyruvate decarboxylase activity (E.C.4.1.1.43) is deleted from the microorganism of the invention. For example, where the microorganism of the invention belongs to a species of *Saccharomyces,* the deleted phenylpyruvate decarboxylase gene encodes a polypeptide whose sequence has at least 70, 72, 74, 76, 78, 80, 82, 84, 86, 88, 90, 92, 94, 96, 98, 99 or 100% amino acid sequence identity to SEQ ID NO: 57.

The microorganism of the invention may further be deficient of **pyruvate decarboxylase** (E.C.4.1.1.1) activity, such as by having one or more endogenous native genes encoding a polypeptide having pyruvate decarboxylase activity knocked out, causing a block in the conversion of pyruvate to acetaldehyde in the microorganism. Deletion of pyruvate decarboxylase (E.C.4.1.1.1) provides a microorganism of the invention with enhanced flux to aromatic amino acids, rather than formation of aromatic alcohols. For example, the deleted endogenous gene is one encoding a polypeptide having pyruvate decarboxylase activity in the respective microorganism. Preferably the amino acid sequence of the polypeptide having pyruvate decarboxylase activity (E.C.4.1.1.1) has at least 70, 72, 74, 76, 78, 80, 82, 84, 86, 88, 90, 92, 94, 96, 98, 99 or 100% amino acid sequence identity to a sequence selected from among: SEQ ID NO: 62 in *S. cerevisiae,* SEQ ID NO: 63 in *Hanseniaspora guilliermondii,* SEQ ID NO: 64 in *Candida glabrata,* and SEQ ID NO 65 in *Kluyveromyces marxianus* DMKU3-1042.

In one embodiment, an endogenous gene encoding a polypeptide having pyruvate decarboxylase activity (E.C.4.1.1.1) is deleted from the microorganism of the invention. For example, where the microorganism of the invention belongs to a species of *Saccharomyces,* the deleted pyruvate decarboxylase gene encodes a polypeptide whose sequence has at least 70, 72, 74, 76, 78, 80, 82, 84, 86, 88, 90, 92, 94, 96, 98, 99 or 100% amino acid sequence identity to SEQ ID NO: 62.

According to a further preferred embodiment of the invention, the microorganism of the invention may be deficient in both phenylpyruvate decarboxylase (E.C.4.1.1.43) and pyruvate decarboxylase (E.C.4.1.1.1) activity, as described above.

### II. A genetically modified microorganism for producing aromatic amino acid derivatives

The genetically modified microorganism according to the invention for producing aromatic amino acid derivatives may be any eukaryotic microorganism such as yeast or fungi.

Preferably, the genetically modified microorganism of the invention for producing aromatic amino acid derivatives is a yeast strain, such as a member of the genus *Saccharomyces.* The microorganism of the invention may for example be a *Saccharomyces* species selected from the group consisting of *S*. *arboricolus, S. bayanus, S. boulardii, S. bulderi, S. cariocanus, S. cariocus, S. cerevisiae, S. chevalieri, S. dairenensis, S. ellipsoideus, S. eubayanus, S. exiguous, S. florentinus, S. fragilis, S. kluyveri, S. kudriavzevii, S. martiniae, S. mikatae, S. monacensis, S. norbensis, S. paradoxus, S. pastorianus, S. spencerorum, S. turicensis, S. unisporus, S. uvarum, S. zonatus.*

Most preferably the microorganism of the invention is *Saccharomyces cerevisiae.*

### III Methods for producing a genetically modified microorganism for production of aromatic amino acid derivatives

### III i. Enhancing levels of enzyme activity

Enhancing the levels of different enzyme activities in a microorganism may be engineered at different regulatory levels as a person skilled in the art would recognize. Illustrative, but non-exhaustive, examples of such regulatory measures are described herein.

Methods of transcriptional/translational regulation may be used for enhancing the levels of enzyme activity by enhancing the amount of a polypeptide of interest that is produced. Enhanced expression of a gene encoding a polypeptide of interest may be engineered by (i) coupling the gene to a strong promoter, such as a constitutive or inducible promoter; (ii) engineering promoters such as stitching transcription factor binding sites or manipulating upstream activating sequence elements, or (iii) expressing extra or multiple copies of the genes in the microorganism.

Enzyme activity may further be affected by regulatory mechanisms such as feedback inhibition. Alleviating such inhibition, e.g. by engineering the enzyme amino acid sequence to be feedback resistant, can enhance the activity of the respective enzyme. Enzyme activity may further be affected by regulatory mechanisms such as posttranslational modifications in protein phosphorylation or acetylation. For example, phosphorylation of a protein may lead to a decrease or increase its enzyme activity; hence enzyme activity can be enhanced by engineering the phosphorylated amino acid residue to remove or mimic its phosphorylation.

### III ii. Introduction of gene constructs

A nucleic acid molecule, that encodes one or more polypeptide(s) having an enzymatic activity associated with aromatic amino acid derivative synthesis according to the invention, can be introduced into a host cell and integrated into the host cell genome by techniques that are standard in the art. Optionally methods employing a self-replicating vector may be used as a means of introducing such nucleic acid molecules into a host cell. Nucleic acid molecules can be introduced into the genome of a microorganism by standard protocols such as transformation including chemical transformation and electroporation, transduction, particle bombardment, etc. Expressing the nucleic acid molecule encoding the enzymes of the claimed invention is accomplished by integrating the nucleic acid molecule into a chromosome the genome or introduced on self-replicating episome.

Genomic integrations may be accomplished by CRISPR/Cas9-mediated DNA editing tools which are commercially available and known to those skilled in the art (see, e.g. Mans et al., 2015). As an illustrative example hereof, the host cell harbors an integrated Cas9 expression cassette under the control of a constitutive promoter. DNA integration constructs are produced and integrated at selected genomic loci via the CRISPR/cas9 system. Such integration constructs may be generated by overlapping extension PCR (OE-PCR) procedure or any other method of DNA assembly known to those skilled in the art. Some constructs or fragments thereof may be synthetic fragments, manufactured and directly used.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. In particular, such 5' non-transcribed regulatory sequences will include a promoter region which includes a promoter sequence for transcriptional control of the operably joined gene. Expression of the nucleic acid molecule(s) encoding the polypeptide(s) of the claimed invention may be inducible, such as promoters controlled by the presence or absence of a molecule, or constitutive i.e. the promoter is unregulated allowing for continual transcription of its associated gene. The promoter can be a native promoter, i.e., the promoter of the gene in its endogenous context, which provides normal regulation of expression of the gene. In some embodiments the promoter can be constitutive. Regulatory sequences may also include enhancer sequences or upstream activator sequences as desired. The vectors of the invention may optionally include 5' leader or signal sequences. The choice and design of an appropriate expression system is within the ability and discretion of one of ordinary skill in the art.

Construction of microorganism cells expressing genes encoding one or more polypeptide(s) having an enzymatic activity associated with aromatic amino acid derivate synthesis in a microorganism of the invention is demonstrated in the Example 1.

### III iii. Deletion of endogenous gene(s)

Deletion of endogenous genes in a host microorganism cell can be achieved by a variety of methods; for example by transformation of the host cell with linear DNA fragments containing a locus for resistance to an antibiotic, or any other gene allowing for rapid phenotypic selection, flanked by sequences homologous to closely spaced regions on the cell chromosome on either side of the gene to be deleted.

More preferably, gene deletion may be accomplished by CRISPR/Cas9-mediated DNA editing tools, where a double stranded DNA fragment consisting of sequences homologous to up- and down-stream regions of the chromosomal target site (e.g. two 60 bp sequences) may be used for the homologous repair of genome double-strand break introduced by the cleavage of Cas9 nuclease.

Construction of microorganism cells deleted for gene(s) encoding one or more polypeptide(s) having an enzymatic activity otherwise negatively affecting the yields of aromatic amino acid derivative production by a microorganism of the invention is demonstrated in the Example 1.

### IV. A method for producing aromatic amino acid derivatives using a genetically modified microorganism of the invention

As illustrated in the examples, aromatic amino acids and their derivatives can be produced using a microorganism of the invention by introducing the cells into a culture medium comprising a carbon source for biosynthesis of one or more aromatic amino acids; culturing the cells for a desired period of time for sufficient production of aromatic amino acid derivatives, and finally recovering (and optionally purifying) the aromatic amino acid derivatives produced by the culture.

The microorganism of the invention will produce aromatic amino acid derivatives when supplied with a suitable carbon source including glucose, maltose, galactose, fructose, sucrose, arabinose, xylose, raffinose, mannose, and lactose.

### V. A method of detecting aromatic amino acid derivatives produced by the genetically modified microorganism

Methods for detecting and quantifying aromatic amino acid derivatives produced by a microorganism of the invention include high performance liquid chromatography (HPLC) combined with refractive index detection to identify and quantify the product compared to standards, as one of ordinary skill in the art would be familiar with. Example 2 comprises the outline of one method of detection and quantification of pHCA.

### EXAMPLES

### Example 1: Genetically modified S. cerevisiae strains engineered for enhanced production of aromatic amino acids

### 1.1 Cloning strategy

*Saccharomyces cerevisiae* strain CEN.PK113-5D-derivative IMX581 (MATa ura3-52 can1Δ::cas9-natNT2 TRP1 LEU2 HIS3) was used as parent strain. IMX581 harbors an integrated Cas9 expression cassette under the control of constitutive TEF1p promoter. All genomic integrations and gene deletion were accomplished by CRISPR/Cas9-mediated DNA editing tools (Mans et al., 2015).

For gene overexpression, DNA integration constructs were produced and integrated at selected genomic loci via the CRISPR/cas9 system. All native promoters, genes and terminators were PCR amplified using CEN.PK113-5D genomic DNA as template. For optimized heterologous genes, synthetic fragments obtained from GenScript (www.genscript.com) were used for PCR amplification. High-fidelity Phusion DNA polymerase was utilized throughout the entire molecular cloning procedures, except PrimeSTAR HS polymerase was employed for in-vitro DNA part fusion and module generation. Functional modules (integration constructs) were generated according to described overlapping extension PCR (OE-PCR) procedure (Zhou et al., 2012).

For gene deletion, a double-stranded DNA fragment consisting of two 60 bp sequences homologous to up- and down-stream regions of the chromosomal target site was used for the homologous repair of the genome double-strand break introduced by the cleavage of Cas9 nuclease.

Table 1 provides a list of all integration constructs used, while table 2 provides a list of the relevant characteristics of each plasmid used. Genetic transformation of exogenous DNA into *S. cerevisiae* was carried out using the standard lithium acetate protocol (Gietz et al., 2002).

| Table 1. Overview of DNA fragments in the different integrations constructs used in construction of pHCA producing strains. | |
|---|---|
| **Integration construct ID** | **DNA fragments in the different integrations constructs** |
| M3 | XII-2us - GPM1p-**AtPAL2**-FBA1t - CYC1t-**AtC4H**-TDH3p - **tHXT7p-AtATR2-pYX212t** - ADH1t-**CYB5**-PGK1p - XII-2ds |
| M2 | XI-3us - TEF1p-**FjTAL**-TDH2t - XI-3ds |
| M210 | X-3us - PGK1p-**ARO4^{K229L}**-CYC1t - X-3ds |
| M211 | X-3us - ADH1t-**ARO7^{G141S}**-TEF1p - PGK1p-**ARO4^{K229L}**-CYC1t - X-3ds |
| M196 | XII-5us - ADH1t-**Bbxfpk**-TDH3p - tHXT7p-**Ckpta**-CYC1t - XII-5ds |
| M214 | XII-5us - tHXT7p-**TKL1**-FAB1t - XII-5ds |
| M213 | XI-2us - tHXT7p**-Seacs^{L641P}**-CYC1t - XI-2ds |
| M212 | XI-2us - tHXT7p-**Ckpta**-CYC1t - XI-2ds |
| M136 | XII-5us - ADH1t-**Bbxfpk**-GAL1p - GAL2p-**Ckpta**-CYC1t - XII-5ds |
| M204 | GPP1us - PGK1p-**ARO4^{K229L}**-CYC1t - GPP1ds |
| M205 | GPP1us - ADH1t-**ARO7^{G141S}**-TEF1p - PGK1p-**ARO4^{K229L}**-CYC1t - GPP1ds |
| M50 | X-2us - GPM1p-**PHA2**-CYC1t - X-2ds |
| M1 | X-3us - TPIp-**EcaroL**-pYX212t - ADH1t-**ARO7^{G141S}**-TEF1p - PGK1p-**ARO4^{K229L}**-CYC1t - X-3ds |

Bold font indicates genes expressed; p indicates promoter; t indicates terminator; underline indicates up-stream (us) and down-stream (ds) sequences Cas9-targeting locus for homologous recombination;

| Table 2. Overview of relevant characteristics of plasmids used | | |
|---|---|---|
| **Plasmid ID** | **Relevant characteristics** | **Reference** |
| pQC005 | 2µm ampR KIURA3 gRNA-X-3 | This study |
| pQC006 | 2µm ampR KIURA3 gRNA-XI-3 | This study |
| pQC007 | 2µm ampR KIURA3 gRNA-XII-2 | This study |
| pQC029 | 2µm ampR URA3 gRNA-X-2 | This study |
| pQC033 | 2µm ampR URA3 gRNA-XII-5 | This study |
| pQC127 | 2µm ampR URA3 gRNA-GAL7/10/1 | This study |
| pQC130 | 2µm ampR URA3 gRNA-XI-2 | This study |
| pQC157 | 2µm ampR URA3 gRNA-GPP1 | This study |
| pQC162 | 2µm ampR URA3 gRNA-GPP1 gRNA-XII-5 | This study |
| pQC190 | 2µm ampR URA3 gRNA-X-3 gRNA-XII-5 | This study |
| pQC184 | 2µm ampR URA3 gRNA-PDC5 gRNA-ARO10 | This study |

Synthetic complete (SC) medium as well as drop-out media (SC-Ura) and agar plates were prepared using pre-mixed powders from Sigma-Aldrich (cat# Y1501). All transformations and selections were performed on SC-Ura plates. Six single clones were grown and subject to diagnostic PCR analysis to confirm the integration of modules or gene deletions. Table 3 provides a list of the genotype of all strains constructed.

| Table 3. Overview of strains | | |
|---|---|---|
| **Strain ID** | **Genotype** | **Reference** |
| IMX581 | MATa ura3-52 can1Δ::cas9-natNT2 TRP1 LEU2 HIS3 | Mans et al., 2015 |
| QL110 | MATa ura3-52 can1Δ::cas9-natNT2 TRP1 LEU2 HIS3 XII-2:: (GPM1p-**AtPAL2**-FBA1t)+(TDH3p-**AtC4H-**CYC1t)+(tHXT7p-**AtATR2**-pYX212t)+(PGK1p-**CYB5-**ADH1t) | This study |
| QL111 | MATa ura3-52 can1Δ::cas9-natNT2 TRP1 LEU2 HIS3 XII-2:: (GPM1p**-AtPAL2**-FBA1t)+(TDH3p-**AtC4H-**CYC1t)+(tHXT7p-**AtATR2**-pYX212t)+(PGK1p-**CYB5-**ADH1t) X-3: :(PGK1p-**ARO4^{K229L}**-CYC1t) | This study |
| QL112 | MATa ura3-52 can1Δ::cas9-natNT2 TRP1 LEU2 HIS3 XII-2::(GPM1p-**AtPAL2**-FBA1t)+(TDH3p-**AtC4H-**CYC1t)+(tHXT7p-**AtATR2**-pYX212t)+(PGK1p-**CYB5-**ADH1t) X-3::(TEF1p-**ARO7^{S141G}**-ADH1t)+(PGK1p-**ARO4^{K229L}**-CYC1t) | This study |
| QL120 | MATa ura3-52 can1Δ::cas9-natNT2 TRP1 LEU2 HIS3 XI-3::(TEF1p-**FjTAL**-TDH2t) | This study |
| QL124 | MATa ura3-52 can1Δ::cas9-natNT2 TRP1 LEU2 HIS3 XI-3::(TEF1p-**FjTAL**-TDH2t) X-3::(TPIp-**EcaroL-**pYX212t)+(TEF1p-**ARO7^{S141G}**-ADH1t)+(PGK1p-**ARO4^{K229L}**-CYC1t) | This study |
| QL210 | MATa ura3-52 can1Δ::cas9-natNT2 TRP1 LEU2 HIS3 XII-2:: (GPM1p-**AtPAL2**-FBA1t)+(TDH3p-**AtC4H-**CYC1t)+(tHXT7p-**AtATR2**-pYX212t)+(PGK1p-**CYB5-**ADH1t) XII-5::(TDH3p-**Bbxfpk**-ADH1t)+(tHXT7p-**Ckpta**-CYC1t) | This study |
| QL211 | MATa ura3-52 can1Δ::cas9-natNT2 TRP1 LEU2 HIS3 XII-2:: (GPM1p-**AtPAL2**-FBA1t)+(TDH3p-**AtC4H-**CYC1t)+(tHXT7p-**AtATR2**-pYX212t)+(PGK1p-**CYB5-**ADH1t) X-3::(PGK1p-**ARO4^{K229L}**-CYC1t) XII-5::(TDH3p-**Bbxfpk**-ADH1t)+(tHXT7p-**Ckpta**-CYC1t) | This study |
| QL212 | MATa ura3-52 can1Δ::cas9-natNT2 TRP1 LEU2 HIS3 XII-2::(GPM1p-**AtPAL2**-FBA1t)+(TDH3p-**AtC4H-**CYC1t)+(tHXT7p-**AtATR2**-pYX212t)+(PGK1p-**CYB5-**ADH1t) X-3::(TEF1p-**ARO7^{S141G}**-ADH1t)+(PGK1p-**ARO4^{K229L}**-CYC1t) XII-5::(TDH3p-**Bbxfpk-**ADH1t)+(tHXT7p-**Ckpta**-CYC1t) | This study |
| QL213 | MATa ura3-52 can1Δ::cas9-natNT2 TRP1 LEU2 HIS3 XI-3: :(TEF1p-**FjTAL**-TDH2t) X-3::(PGK1p-**ARO4^{K229L}**-CYC1t) | This study |
| QL214 | MATa ura3-52 can1Δ::cas9-natNT2 TRP1 LEU2 HIS3 XI-3: :(TEF1p-**FjTAL**-TDH2t) XII-5::(TDH3p-**Bbxfpk-**ADH 1t)+(tHXT7p-**Ckpta**-CYC1t) | This study |
| QL215 | MATa ura3-52 can1Δ::cas9-natNT2 TRP1 LEU2 HIS3 XI-3::(TEF1p-**FjTAL**-TDH2t) X-3::(PGK1p-**ARO4^{K229L}**-CYC1t) XII-5: :(TDH3p-Bbxfpk-ADH1t)+(tHXT7p-**Ckpta**-CYC1t) | This study |
| QL216 | MATa ura3-52 can1Δ::cas9-natNT2 TRP1 LEU2 HIS3 XI-3::(TEF1p-**FjTAL**-TDH2t) X-3::(TEF1p-**ARO7^{S141G}**-ADH 1t)+(PGK1p-**ARO4^{K229L}**-CYC1t) | This study |
| QL217 | MATa ura3-52 can1Δ::cas9-natNT2 TRP1 LEU2 HIS3 XI-3::(TEF1p-**FjTAL**-TDH2t) X-3::(TEF1p-**ARO7^{S141G}**-ADH1t)+(PGK1p-**ARO4^{K229L}**-CYC1t) XII-5::(TDH3p-**Bbxfpk**-ADH 1t)+(tHXT7p-**Ckpta**-CYC1t) | This study |
| QL218 | MATa ura3-52 can1Δ::cas9-natNT2 TRP1 LEU2 HIS3 XII-2::(GPM1p-**AtPAL2**-FBA1t)+(TDH3p-**AtC4H-**CYC1t)+(tHXT7p-**AtATR2**-pYX212t)+(PGK1p-**CYB5-**ADH1t) X-3::(TEF1p-**ARO7^{S141G}**-ADH1t)+(PGK1p-**ARO4^{K229L}**-CYC1t) XII-5::(tHXT7p-**TKL1**-FAB1t) | This study |
| QL219 | MATa ura3-52 can1Δ::cas9-natNT2 TRP1 LEU2 HIS3 **gpp1Δ** XII-2::(GPM1p-**AtPAL2**-FBA1t)+(TDH3p-**AtC4H**-CYC1t)+(tHXT7p-**AtATR2**-pYX212t)+(PGK1p-**CYB5**-ADH1t) X-3::(TEF1p-**ARO7^{S141G}**-ADH1t)+(PGK1p-**ARO4^{K229L}**-CYC1t) XII-5::(TDH3p-**Bbxfpk**-ADH 1t)+(tHXT7p-**Ckpta**-CYC1t) | This study |
| QL220 | MATa ura3-52 can1Δ::cas9-natNT2 TRP1 LEU2 HIS3 XII-2::(GPM1p-**AtPAL2**-FBA1t)+(TDH3p-**AtC4H-**CYC1t)+(tHXT7p-**AtATR2**-pYX212t)+(PGK1p-**CYB5-**ADH1t) X-3::(TEF1p-**ARO7^{S141G}**-ADH1t)+(PGK1p-**ARO4^{K229L}**-CYC1t) XII-5:**:**(TDH3p**-Bbxfpk-**ADH1t)+(tHXT7p-**Ckpta**-CYC1t) XI-2::(tHXT7p-**Seacs^{L641P}**-CYC1t) | This study |
| QL221 | MATa ura3-52 can1Δ::cas9-natNT2 TRP1 LEU2 HIS3 XII-2::(GPM1p-**AtPAL2**-FBA1t)+(TDH3p-**AtC4H-**CYC1t)+(tHXT7p-**AtATR2**-pYX212t)+(PGK1p-**CYB5-**ADH1t) X-3::(TEF1p-**ARO7^{S141G}**-ADH1t)+(PGK1p-**ARO4^{K229L}**-CYC1t) XII-5::(TDH3p-**Bbxfpk-**ADH1t)+(tHXT7p-**Ckpta**-CYC1t) XI-2::(tHXT7p-**Ckpta**-CYC1t) | This study |
| QL263 | MATa ura3-52 can1Δ::cas9-natNT2 TRP1 LEU2 HIS3 **gal7/10/1Δ** XII-2::(GPM1p-**AtPAL2-**FBA1t)+(TDH3p-**AtC4H**-CYC1t)+(tHXT7p-**AtATR2-**pYX212t)+(PGK1p-**CYB5**-ADH1t) | This study |
| QL264 | MATa ura3-52 can1Δ::cas9-natNT2 TRP1 LEU2 HIS3 **pdc5Δ aro10Δ** XI-3::(TEF1p-**FjTAL**-TDH2t) X-3::(TPIp-**EcaroL**-pYX212t)+(TEF1p-**ARO7^{S141G}**-ADH 1t)+(PGK1p-**ARO4^{K229L}**-CYC1t) | This study |
| QL270 | MATa ura3-52 can1Δ::cas9-natNT2 TRP1 LEU2 HIS3 **gal7/10/1Δ gpp1Δ**: :(PGK1p-**ARO4^{K229L}**-CYC1t) XII-2::(GPM1p-**AtPAL2**-FBA1t)+(TDH3p-**AtC4H-**CYC1t)+(tHXT7p-**AtATR2**-pYX212t)+(PGK1p-**CYB5-**ADH1t) XII-5::(GAL1p-**Bbxfpk**-ADH1t)+(GAL2p-**Ckpta**-CYC1t) | This study |
| QL271 | MATa ura3-52 can1Δ::cas9-natNT2 TRP1 LEU2 HIS3 **gal7/10/1Δ gpp1Δ:** :(TEF1p-**ARO7^{S141G}**-ADH1t)+(PGK1p-**ARO4^{K229L}**-CYC1t) XII-2::(GPM1p-**AtPAL2**-FBA1t)+(TDH3p-**AtC4H**-CYC1t)+(tHXT7p-**AtATR2**-pYX212t)+(PGK1p-**CYB5**-ADH1t) XII-5::(GAL1p-**Bbxfpk**-ADH1t)+(GAL2p-**Ckpta**-CYC1t) | This study |
| QL286 | MATa ura3-52 can1Δ::cas9-natNT2 TRP1 LEU2 HIS3 **pdc5Δ aro10Δ** XI-3::(TEF1p-**FjTAL**-TDH2t) X-3::(TPIp-**EcaroL**-pYX212t)+(TEF1p-**ARO7^{S141G}**-ADH1t)+(PGK1p-**ARO4^{K229L}**-CYC1t) XII-5::(TDH3p-**Bbxfpk**-ADH 1t)+(tHXT7p-**Ckpta**-CYC1t) | This study |
| QL287 | MATa ura3-52 can1Δ::cas9-natNT2 TRP1 LEU2 HIS3 **gal7/10/1Δ gpp1Δ:** :(PGK1p-**ARO4^{K229L}**-CYC1t) XII-2:: (GPM1p-**AtPAL2**-FBA1t)+(TDH3p-**AtC4H-**CYC1t)+(tHXT7p-**AtATR2-**pYX212t)+(PGK1p-**CYB5-**ADH1t) XII-5::(GAL1p-**Bbxfpk**-ADH1t)+(GAL2p-**Ckpta**-CYC1t) X-2::(GPM1p-**PHA2**-CYC1t) | This study |
| QL288 | MATa ura3-52 can1Δ::cas9-natNT2 TRP1 LEU2 HIS3 **gal7/10/1Δ gpp1Δ:** :(TEF1p-**ARO7^{S141G}**-ADH1t)+(PGK1p-**ARO4^{K229L}**-CYC1t) XII-2::(GPM1p-**AtPAL2**-FBA1t)+(TDH3p-**AtC4H**-CYC1t)+(tHXT7p-**AtATR2**-pYX212t)+(PGK1p-**CYB5**-ADH1t) XII-5::(GAL1p-**Bbxfpk**-ADH1t)+(GAL2p-**Ckpta**-CYC1t) X-2::(GPM1p-**PHA2**-CYC1t) | This study |
| QL294 | MATa ura3-52 can1Δ::cas9-natNT2 TRP1 LEU2 HIS3 **gpplΔ::**(TEF1p-**ARO7^{S141G}**-ADH1t)+(PGK1p-**ARO4^{K229L}**-CYC1t) XI-3::(TEF1p-**FjTAL**-TDH2t) XII-5::(TDH3p-Bbxfpk-ADH 1t)+(tHXT7p-**Ckpta**-CYC1t) | This study |
| QL295 | MATa ura3-52 can1Δ::cas9-natNT2 TRP1 LEU2 HIS3 **pdc5Δ aro10Δ gpp1Δ** XI-3::(TEF1p-**FjTAL**-TDH2t) X-3::(TPIp-**EcaroL**-pYX212t)+(TEF1p-**ARO7^{S141G}**-ADH 1t)+(PGK1p-**ARO4^{K229L}**-CYC1t) | This study |

### 1.2 Construction of a S. cerevisiae benchmark strain.

Strain **QL264** was generated through construct integration and gene deletion in strain QL120. First, integration construct M1 (table 1) was introduced into strain QL120.

M1 consists of (i) one copy of yeast optimized *Escherichia coli* shikimate kinase aroL gene (SEQ ID No 66) under the control of TPI promoter (SEQ ID No 68 consisting of 578 basepairs upstream of the native *S. cerevisiae* TPI coding region) and pYX212 terminator (SEQ ID No 69 consisting of 559 bp from yeast expression plasmid pYX212); (ii) the *S. cerevisiae* feedback-resistant mutant gene ARO7^{G141S} (SEQ ID No 70) under the control of the TEF1 promoter (SEQ ID No 72 consisting 412 basepairs upstream of the native S. cerevisiae TEF1 coding region) and the ADH1 terminator (SEQ ID No 73 consisting 243 basepairs downstream of the native S. cerevisiae ADH1 coding region); and (iii) the *S. cerevisiae* feedback-resistant mutant gene ARO4^{K229L} (SEQ ID No 74) under the control of the PGK1 promoter (SEQ ID No 76 consisting 750 basepairs upstream of the native S. cerevisiae PGK1 coding region) and the CYC1 terminator (SEQ ID No 77 consisting 277 basepairs downstream of the native *S. cerevisiae* CYC1 coding region). These components are flanked by upstream and downstream sequences of the Cas9-targeting locus X-3. The construct was in vitro assembled using the previously described overlapping-extension PCR method. The construct was co-transformed with X-3 specific gRNA expression vector pQC005 into QL120, and transformants were selected on SC-URA plates with 2% glucose and identified by diagnostic PCR. Correct transformants were streaked on 5-FOA plates and the resulting strain was QL124.

Next, the coding regions of native genes pyruvate decarboxylase pdc5 (SEQ ID No 61) and phenylpyruvae decarboxylase aro10 (SEQ ID No 56) were deleted in QL124. gRNA expression vector pQC184 and double-strand 120 bp oligos consisting of 60 bp sequences homologous to up- and downstream regions of these two genes ((SEQ ID No 78 and SEQ ID No 79) and (SEQ ID No 80 and SEQ ID No 81), respectively) were co-transformed into QL124. Transformants were selected on SC-URA plates with 2% glucose and identified by diagnostic PCR. Correct transformants were streaked on 5-FOA plates and the resulting strain QL264. This benchmark strain corresponds to the *S. cerevisiae* strain QL264 described by Rodriguez et al 2015.

### 1.3 Construction of pHCA producing S. cerevisiae basic strains

As noted previously, pHCA may be produced from L-Phe and/or L-Tyr, utilizing the Pal- and/or Tal-pathway, respectively (Figure 1). Strains comprising each of these pathways were created.

Pal-pathway: **Strain QL110** was generated by introducing integration construct M3 (see table 1) into strain IMX581. M3 consists of four gene fragments: (i) yeast codon-optimized phenylalanine ammonia-lyase gene from *Arabidopsis thaliana* (AtPAL2) (SEQ ID No 31) under the control of the GPM1 promoter (SEQ ID No 82 consisting of 760 basepairs upstream of the native S. cerevisiae GPM1 coding region) and the FBA1 terminator (SEQ ID No 83 consisting of 401 basepairs downstream of the native S. cerevisiae FBA1 coding region); (ii) yeast codon-optimized cinnamate-4-hydroxylase gene from *Arabidopsis thaliana* (AtC4H) (SEQ ID No 36) under the control of the TDH3 promoter (SEQ ID No 84 consisting of 675 basepairs upstream of the native S. cerevisiae TDH3 coding region) and the CYC1 terminator (SEQ ID No 77 consisting of 277 basepairs downstream of the native S. cerevisiae CYC1 coding region); and (iii) yeast codon-optimized cytochrome P450 reductase gene from *Arabidopsis thaliana* (AtATR2) (SEQ ID No 46) under the control of the tHXT7 promoter (SEQ ID No 85 consisting of 391 basepairs upstream of the native S. cerevisiae TDH3 coding region) and the pYX212 terminator (SEQ ID No 69 consisting of 559 basepairs from yeast expression plasmid pYX212); and (iv) native *S. cerevisiae* cytochrome 5 gene (cyb5) (SEQ ID No 41) under the control of the PGK1 promoter (SEQ ID No 76 consisting of 750 basepairs upstream of the native S. cerevisiae PGK1 coding region) and the ADH1 terminator (SEQ ID No 73 consisting of 243 basepairs downstream of the native S. cerevisiae ADH1 coding region). These four components are flanked by upstream and downstream sequences of the Cas9-targeting locus XII-2 (SEQ ID No 86 and SEQ ID No 87, respectively). The construct was in vitro assembled using the previously described overlapping-extension PCR method. Upon co-transformation with XII-2 specific gRNA expression vector pQC007 (table 2) into *S. cerevisiae* IMX581, this construct integrated by Cas9-mediated mark-free homologous recombination into the host cell genome. Transformants were selected on SC-URA plates with 2% glucose and identified by diagnostic PCR. Corrected transformants were streaked on 5-FOA plates and the resulting strain was QL110.

Tal-pathway: **Strain QL120** was generated by introducing integration construct M2 (see table 1) into strain IMX581. M2 comprises a yeast codon-optimized tyrosine ammonia-lyase gene from *Flavobacterium johnsoniaeu* (FjTAL) (SEQ ID No 51) under control of the TEF1 promoter (SEQ ID No 72 consisting of 412 basepairs upstream of the native S. cerevisiae TEF1 coding region) and the TDH2 terminator (SEQ ID No 88 consisting of 400 basepairs downstream of the native S. cerevisiae TDH2 coding region). These components are flanked by upstream and downstream sequences of the Cas9-targeting locus XI-3 (SEQ ID No 89 and SEQ ID No 90, respectively). The construct was in vitro assembled using the previously described overlapping-extension PCR method. Upon co-transformation with XI-3 specific gRNA expression vector pQC006 (table 2) into *S. cerevisiae* IMX581, this construct integrated by Cas9-mediated mark-free homologous recombination into the host cell genome. Transformants were selected on SC-URA plates with 2% glucose and identified by diagnostic PCR. Correct transformants were streaked on 5-FOA plates and the resulting strain was QL120.

### 1.4 Construction of S. cerevisiae strains expressing feedback resistant aro4* and aro7*.

As noted previously, PEP and E4P are converted to the aromatic acids L-Phe and L-Tyr via the DAHP pathway. The pathway comprises several genes, including aro4 and aro7 (Figure 1). Strains comprising feedback resistant mutants of genes Aro4 and Aro7 were created.

Strain **QL111** was generated by introducing integration construct M210 (see table 1) into strain QL110. M210 comprises a sequences of *S. cerevisiae* encoding the feedback-resistant mutant gene ARO4^{K229L} (SEQ ID No 74) under the control of the PGK1 promoter (SEQ ID No 76 consisting of 750 basepairs upstream of the native S. cerevisiae PGK1 coding region) and the CYC1 terminator (SEQ ID No 77 consisting of 277 basepairs downstream of the native S. cerevisiae CYC1 coding region), flanked by upstream and downstream sequences of the Cas9-targeting locus X-3 (SEQ ID No 91 and SEQ ID No 92, respectively). The construct was in vitro assembled using the previously described overlapping-extension PCR method. The construct was co-transformed with X-3 specific gRNA expression vector pQC005 (table 2) into QL110, and transformants were selected on SC-URA plates with 2% glucose and identified by diagnostic PCR. Correct transformants were streaked on 5-FOA plates and the resulting strain was QL111.

Strain **QL213** was similarly generated by introducing integration construct M210 (described above and summarized in table 1) into strain QL120.

Strain **QL112** was generated by introducing integration construct M211 (see table 1) into QL110 strain. M211 consists of two *S. cerevisiae* genes encoding (i) the feedback-resistant mutant gene ARO7^{G141S} (SEQ ID No 70) under the control of the TEF1 promoter (SEQ ID No 72 consisting of 412 basepairs upstream of the native S. cerevisiae TEF1 coding region) and the ADH1 terminator (SEQ ID No 73 consisting of 243 basepairs downstream of the native S. cerevisiae ADH1 coding region) and (ii) the feedback-resistant mutant gene ARO4^{K229L} (SEQ ID No 74) under the control of the PGK1 promoter (SEQ ID No 76 consisting of 750 basepairs upstream of the native S. cerevisiae PGK1 coding region) and the CYC1 terminator (SEQ ID No 77 consisting of 277 basepairs downstream of the native S. cerevisiae CYC1 coding region). These components are flanked by upstream and downstream sequences of the Cas9-targeting locus X-3 (SEQ ID No 91 and SEQ ID No 92, respectively). The construct was in vitro assembled using the previously described overlapping-extension PCR method. The construct was co-transformed with X-3 specific gRNA expression vector pQC005 (see Table 2) into QL110, and transformants were selected on SC-URA plates with 2% glucose and identified by diagnostic PCR. Correct transformants were streaked on 5-FOA plates and the resulting strain was QL112.

Strain **QL216** was similarly generated by introducing integration construct M211 (described above and summarized in table 1) into strain QL120.

### 1.5 Construction of S. cerevisiae strains expressing xfpk and pta.

Strain **QL210** was generated by introducing integration construct M196 (see table 1) into strain QL110. M196 consists of (i) one copy of yeast codon-optimized xylulose-5-phosphate/fructose-6-phosphate phosphoketolase from *Bifidobacterium breve* (Bbxfpk) (SEQ ID No 11) under the control of the TDH3 promoter (SEQ ID No 84 consisting of 675 basepairs upstream of the native S. cerevisiae TDH3 coding region) and the ADH1 terminator (SEQ ID No 73 consisting of 243 basepairs downstream of the native S. cerevisiae ADH1 coding region); and (ii) one copy of yeast codon-optimized phosphate acetyltransferase from *Clostridium kluyveri* (Ckpta) (SEQ ID No 16) under the control of the tHXT7 promoter (SEQ ID No 85 consisting of 391 basepairs upstream of the native S. cerevisiae HXT7 coding region) and the CYC1 terminator (SEQ ID No 77 consisting of 277 basepairs downstream of the native S. cerevisiae CYC1 coding region). These components are flanked by upstream and downstream sequences of the Cas9-targeting locus XII-5 (SEQ ID No 93 and SEQ ID No 94, respectively). The construct was in vitro assembled using the previously described overlapping-extension PCR method. The construct was co-transformed with XII-5 specific gRNA expression vector pQC033 (table 2) into QL110, and transformants were selected on SC-URA plates with 2% glucose and identified by diagnostic PCR. Correct transformants were streaked on 5-FOA plates and the resulting strain was QL210.

Strain **QL214** was similarly generated by introducing integration construct M196 (described above and summarized in table 1) into strain QL120.

Strain **QL286** was similarly generated by introducing integration construct M196 (described above and summarized in table 1) into strain QL264.

Strain **QL211** was generated by introducing integration constructs M211 and M196 (previously described and summarized in table 1) into strain QL110. The two constructs were co-transformed with X-3/XII-5 specific gRNA expression vector pQC190 (table 2) into QL110, and transformants were selected on SC-URA plates with 2% glucose and identified by diagnostic PCR. Correct transformants were streaked on 5-FOA plates and the resulting strain was QL211.

Strain **QL215** was similarly generated by introducing integration constructs M211 and M196 (previously described and summarized in table 1) into strain QL120 strain.

Strain **QL212** was generated by introducing integration constructs M210 and M196 (previously described and summarized in table 1) into QL110 strain. The two constructs were co-transformed with X-3/XII-5 specific gRNA expression vector pQC190 (table 2) into QL110, and transformants were selected on SC-URA plates with 2% glucose and identified by diagnostic PCR. Correct transformants were streaked on 5-FOA plates and the resulting strain was QL211.

Strain **QL217** was similarly generated by introducing integration constructs M210 and M196 (previously described and summarized in table 1) into strain QL120.

### 1.6 Construction of S. cerevisiae strain expressing tkl1.

Strain **QL218** was generated by introducing integration construct M214 (see table 1) into strain QL212. M214 consists of the yeast native sequence encoding transketolase TKL1 (SEQ ID No 95) under the control of the tHXT7 promoter (SEQ ID No 85 consisting of 391 basepairs upstream of the native *S. cerevisiae* HXT7 coding region) and the FBA1 terminator (SEQ ID No 83 consisting of 401 basepairs downstream of the native *S. cerevisiae* FBA1 coding region). These components are flanked by upstream and downstream sequences of the Cas9-targeting locus XII-5 (SEQ ID No 93 and SEQ ID No 94, respectively). The construct was in vitro assembled using the previously described overlapping-extension PCR method. The construct was co-transformed with XII-5 specific gRNA expression vector pQC033 (table 2) into QL212, and transformants were selected on SC-URA plates with 2% glucose and identified by diagnostic PCR. Correct transformants were streaked on 5-FOA plates and the resulting strain was QL218.

### 1.7 Construction of S. cerevisiae Δgpp1 strain.

Strain **QL219** was generated by deleting the coding region of native glycerol-1-phosphatase gene gpp1 (SEQ ID No 26) in strain QL212. gRNA expression vector pQC157 (table 2) and double-strand 120 bp oligos consisting of two 60 bp sequences homologous to up- and downstream regions of GPP1(SEQ ID No 97 and SEQ ID No 98), respectively, were co-transformed into QL212. Genomic double strand break was repaired by the 120 bp oligos resulting in the removal of GPP1 coding region. Transformants were selected on SC-URA plates with 2% glucose and identified by diagnostic PCR. Correct transformants were streaked on 5-FOA plates and the resulting strain was QL219.

Strain **QL294** was similarly generated by deleting the coding region of native glycerol-1-phosphatase gene gpp1 (SEQ ID No 26) in strain QL217.

Strain **QL295** was similarly generated by deleting the coding region of native glycerol-1-phosphatase gene gpp1 (SEQ ID No 26) in strain QL286

### 1.8 Construction of S. cerevisiae strain expressing SeACS1.

Strain **QL220** was generated by introducing integration construct M213 (see table 1) into QL212 strain. M213 consists of one copy of yeast codon-optimized acetyl-CoA synthetase from *Salmonella enterica* (Seacs) (SEQ ID No 99) under the control of the tHXT7 promoter (SEQ ID No 85 consisting of 391 basepairs upstream of the native *S. cerevisiae* HXT7 coding region) and the CYC1 terminator (SEQ ID No 77 consisting of 277 basepairs downstream of the native *S. cerevisiae* CYC1 coding region). These components are flanked by upstream and downstream sequences of the Cas9-targeting locus XI-2 (SEQ ID No 101 and SEQ ID No 102, respectively). The construct was in vitro assembled using the previously described overlapping-extension PCR method. The construct was co-transformed with XI-2 specific gRNA expression vector pQC130 (table 2) into QL212, and transformants were selected on SC-URA plates with 2% glucose and identified by diagnostic PCR. Correct transformants were streaked on 5-FOA plates and the resulting strain was QL220.

### 1.9 Construction of S. cerevisiae strain expressing a second pta.

Strain **QL221** was generated by introducing integration construct M212 (see table 1) into QL212 strain. M212 consists of one copy of yeast codon-optimized phosphate acetyltransferase from *Clostridium kluyveri* (Ckpta) (SEQ ID No 16) under the control of the tHXT7 promoter (SEQ ID No 85 consisting of 391 basepairs upstream of the native *S. cerevisiae* HXT7 coding region) and the CYC1 terminator (SEQ ID No 77 consisting of 277 basepairs downstream of the native *S. cerevisiae* CYC1 coding region). These components are flanked by upstream and downstream sequences of the Cas9-targeting locus XI-2 (SEQ ID No 101 and SEQ ID No 102, respectively). The construct was in vitro assembled using the previously described overlapping-extension PCR method. The construct was co-transformed with XI-2 specific gRNA expression vector pQC130 (table 2) into QL212, and transformants were selected on SC-URA plates with 2% glucose and identified by diagnostic PCR. Correct transformants were streaked on 5-FOA plates and the resulting strain was QL221.

### 1.10 Construction of S. cerevisiae strains expressing GAL-induced xfpk and pta.

Strain **QL263** was generated by deleting the coding regions of native GAL7/10/1 genes in QL110. gRNA expression vector pQC127 (table 2) and double-strand 120 bp oligos consisting of 60 bp sequences homologous to up- and downstream regions of GAL7/10/1 genes, respectively, were co-transformed into QL110. Genomic double strand break was repaired by the 120 bp oligos resulting in the removal of GAL7/10/1 genes. Transformants were selected on SC-URA plates with 2% glucose and identified by diagnostic PCR. Correct transformants were streaked on 5-FOA plates and the resulting strain was QL263.

Strain **QL270** was generated by introducing integration constructs M136 and M204 (see table 1) into strain QL263. M136 consists (i) one copy of xylulose-5-phosphate/fructose-6-phosphate phosphoketolase from *Bifidobacterium breve* (SEQ ID No 11) under the control of the GAL1 promoter (SEQ ID No 103 consisting of 442 basepairs upstream of the native *S. cerevisiae* GAL1 coding region) and the ADH1 terminator (SEQ ID No 73 consisting of 243 basepairs downstream of the native *S. cerevisiae* ADH1 coding region); and (ii) one copy of yeast codon-optimized phosphate acetyltransferase from *Clostridium kluyveri* (SEQ ID No 16) under the control of the GAL2 promoter (SEQ ID No 104 consisting of 743 basepairs upstream of the native S. *cerevisiae* GAL2 coding region) and the CYC1 terminator (SEQ ID No 77 consisting of 277 basepairs downstream of the native S. cerevisiae CYC1 coding region). These components are flanked by upstream and downstream sequences of the Cas9-targeting locus XII-5 (SEQ ID No 93 and SEQ ID No 94, respectively). M204 consists of the feedback-resistant mutant gene ARO4^{K229L} (SEQ ID No 74) under the control of the PGK1 promoter (SEQ ID No 76 consisting of 750 basepairs upstream of the native *S. cerevisiae* PGK1 coding region) and the CYC1 terminator (SEQ ID No 77 consisting of 277 basepairs downstream of the native *S. cerevisiae* CYC1 coding region), flanked by homologous sequences corresponding to upstream and downstream nucleotide sequences of the GPP1 locus (SEQ ID No 97 and SEQ ID No 98, respectively). Both constructs were in vitro assembled using the previously described overlapping-extension PCR method. These constructs were co-transformed with XII-5/GPP1 specific gRNA expression vector pQC162 (table 2) into QL263, and transformants were selected on SC-URA plates with 2% glucose and identified by diagnostic PCR. Correct transformants were streaked on 5-FOA plates and the resulting strain was QL270.

Strain **QL271** was generated by introducing integration constructs M136 (described above and summarized in table 1) and M205 (see table 1) into QL263 strain.

M205 consists of the two *S. cerevisiae* feedback-resistant mutant genes (i) ARO7^{G141S} (SEQ ID No 70) under the control of the TEF1 promoter (SEQ ID No 72 consisting of 412 basepairs upstream of the native *S. cerevisiae* TEF1 coding region) and the ADH1 terminator (SEQ ID No 73 consisting of 243 basepairs downstream of the native *S. cerevisiae* ADH1 coding region); and (ii) ARO4^{K229L} (SEQ ID No 74) under the control of the PGK1 promoter (SEQ ID No 76 consisting of 750 basepairs upstream of the native *S. cerevisiae* PGK1 coding region) and the CYC1 terminator (SEQ ID No 77 consisting of 277 basepairs downstream of the native S. cerevisiae CYC1 coding region); flanked by homologous sequences corresponding to upstream and downstream nucleotide sequences of the GPP1 locus (SEQ ID No 97 and SEQ ID No 98, respectively). Both constructs were in vitro assembled using the previously described overlapping-extension PCR method. These constructs were co-transformed with XII-5/GPP1 specific gRNA expression vector pQC162 (table 2) into QL263, and transformants were selected on SC-URA plates with 2% glucose and identified by diagnostic PCR. Correct transformants were streaked on 5-FOA plates and the resulting strain was QL271.

### 1.11 Construction of S. cerevisiae strain expressing Pha2.

Strain **QL287** was generated by introducing integration construct M50 (see table 1) into strain QL270. M50 consists of the yeast native prephenate dehydratase PHA2 (SEQ ID No 21) under control of the GPM1 promoter (SEQ ID No 82 consisting of 760 basepairs upstream of the native *S. cerevisiae* GPM1 coding region) and the CYC1 terminator (SEQ ID No 77 consisting of 277 basepairs downstream of the native *S. cerevisiae* CYC1 coding region). These components are flanked by upstream and downstream sequences of the Cas9-targeting locus X-2 (SEQ ID No 105 and SEQ ID No 106, respectively). The construct was in vitro assembled using the previously described overlapping-extension PCR method. The construct was co-transformed with X-2 specific gRNA expression vector pQC029 (table 2) into QL270, and transformants were selected on SC-URA plates with 2% glucose and identified by diagnostic PCR. Correct transformants were streaked on 5-FOA plates and the resulting strain was QL287.

Strain **QL288** was similarly generated by introducing integration construct M50 (described above and summarized in table 1) into QL271 strain.

### EXAMPLE 2: pHCA production is enhanced in genetically modified S. cerevisiae having feedback resistant aro4* and aro7* combined with expression of Xfpk and Pta.

p-Coumaric acid (pHCA) is an aromatic amino acids derivative. It is a valuable chemical building block and serves as precursor for biosynthesis of many secondary metabolites, such as polyphenols, flavonoids, and some polyketides.

### 2.1 Culture conditions

Three independent single colonies with designed genetic modifications were inoculated into 14 ml tubes with 1.5 ml fresh minimal medium (7.5 g/l (NH4)2SO4, 14.4 g/l KH2PO4, 0.5 g/l MgSO4·7H2O, 60 mg/l URA, 20 g/l glucose, 2 ml/l trace metal (3.0 g/l FeSO4•7H2O, 4.5 g/l ZnSO4•7H2O, 4.5 g/l CaCl2•2H2O, 0.84 g/l MnCl2•2H2O, 0.3 g/l CoCl2•6H2O, 0.3 g/l CuSO4•5H2O, 0.4 g/l Na2MoO4•2H2O, 1.0 g/l H3BO3, 0.1 g/l KI and 19.0 g/l Na₂EDTA•2H20) and 1 ml/l vitamin solutions (0.05 g/l D-Biotin, 1.0 g/l D-Pantothenic acid hemicalcium salt, 1.0 g/l Thiamin-HCI, 1.0 g/l Pyridoxin-HCI, 1.0 g/l Nicotinic acid, 0.2 g/l 4-aminobenzoic acid, 25.0 g/l myo-Inositol) (Verduyn et al. 1992)). The tubes were incubated at 30 °C with 200 rpm agitation overnight. For production of p-Coumaric acid (p-HCA), precultures were then inoculated into a 125 ml non-baffled flask carrying 20 ml minimal media, and 1% galactose was supplemented for the induction of GAL promoters, if necessary. The initial OD600 value was kept at 0.05, and 6 tablets of Feedbeads (SMFB08001, Kuhner Shaker, Basel, Switzerland) corresponding to 20 g/l glucose were used as the sole carbon source. Fermentation was carried out for 96 h at 30 °C with 200 rpm agitation.

### 2.2 Quantification of p-HCA

One milliliter of culture was mixed with equal volume of absolute ethanol, vortexed thoroughly and centrifuged at 12,000 RPM for 5 min. The supernatant was used to analyze p-HCA concentration by HPLC. p-HCA was quantified on HPLC (Thermo) equipped with a Discovery HS F5 150 mm x 4.6 mm column (particle size 5 µm). The eluent flow rate was 1.5 ml/min. Linear gradient from 5% to 60% of solvent A over 0.5-14 min was used. Solvent A was 10 mM ammonium formate (pH 3.0, adjusted by formic acid). Solvent B was acetonitrile. p-HCA was detected by absorbance at 277 nm with a retention time of 6.2 min. p-HCA concentration was calculated from the standard curves, and p-HCA standard was purchased from Sigma-Aldrich.

### 2.3 p-coumaric acid production by TAL-branch

Conversion of L-Tyr to pHCA in *S. cerevisiae* is facilitated by codon-optimized *Flavobacterium johnsoniaeu* tyrosine ammonia-lysase (FjTAL). This route of pHCA formation is herein referred to as the TAL-branch.

Figure 3 shows data for production of pHCA though the TAL-branch. Different strain modifications are tested individually and combined, including introducing feedback resistant DAHP synthase (aro4*) and/or chlorismate mutase (aro7*), and expression of phosphoketolase (Bbxfpk) and phosphate acetyltransferase (Ckpta).

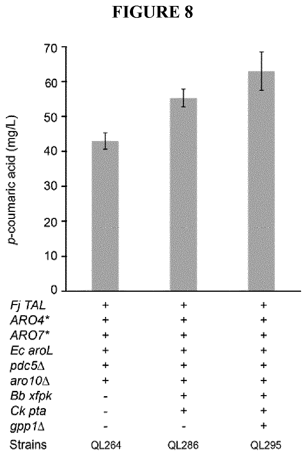

The expression of aro4* and aro7* (QL216) has a significant effect on pHCA production. Meanwhile, expression of Bbxfpk and Ckpta alone (QL214) does not seem to have any effect on the production of pHCA. Surprisingly, the combination of Bbxfpk and Ckpta expression with aro4* and aro7* expression (QL217) significantly enhanced the production titers of QL216. pHCA production titers of ∼41 mg/l were obtained for strain QL217, resembling those obtained for the benchmark strain QL264 (see figure 8), despite the fact that the benchmark strain additionally has gene deletions (Δpdc5/Δaro10) removing enzymes needed for the conversion of para-hydroxy-phenylpyruvate (HPP) to phenylacetaldehyde (PAC); thereby enhancing flux towards L-tyrosine .

### 2.4 p-coumaric acid production by PAL-branch

Conversion of L-Phe to pHCA in *S. cerevisiae* is facilitated by codon-optimized *Arabidopsis thaliana* phenylalanine ammonia-lyase (AtPal2) and cinnamate-4-hydroxylase (AtC4H). This route of pHCA formation is herein referred to as the PAL-branch.

Figure 2 shows data for production of pHCA though the PAL-branch. Different strain modifications are tested individually and combined, including introducing feedback resistant DAHP synthase (aro4*) and/or chlorismate mutase (aro7*), and expression of phosphoketolase (Bbxfpk) and phosphate acetyltransferase (Ckpta).

A similar trend for the PAL-branch is observed, as described for the TAL-branch, namely the expression of aro4* and aro7* (QL112) has a significant effect on pHCA production. Meanwhile, expression of Bbxfpk and Ckpta alone (QL210) does not seem to have any effect on the production of pHCA. Surprisingly, the combination of Bbxfpk and Ckpta expression with aro4* and aro7* expression (QL212) significantly enhanced the production titers of QL212. pHCA production titers of 656 mg/l were obtained for strain QL212.

It is noted that the PAL-branch provides a more efficient route for pHCA production with much higher titers than the TAL-branch.

### EXAMPLE 3: Alternative route for increasing E4P

Overexpression of transketolase activity (tkl1) (E.C.2.2.1.1) represents an alternative route for increasing E4P levels for enhanced production of aromatic amino acid derivatives (see Figure 1). Figure 4 compares the pHCA production by strain QL212 and QL218 engineered to express the PAL-pathway. Both strains express feedback resistant DAHP synthase (aro4*) and chlorismate mutase (aro7*), but strain QL212 further expresses phosphoketolase (Bbxfpk) and phosphate acetyltransferase (Ckpta), while strain QL218 overexpresses transketolase (tkl1). It is shown that expression of transgenes xfpk and pta is much more efficient than overexpression of the native tkl1 gene for production of aromatic amino acid derivatives. In fact, QL218 performs no better than to QL112 which merely expresses the aro4* and aro7* (Figure 2); in other words, overexpression of tkl1 has no effect on pHCA yields mediated via the PAL pathway in yeast.

### EXAMPLE 4: Deletion of gpp1 enhances pHCA production

Other approaches of further enhancing aromatic amino acid derivative production by genetic modifications were explored, including strain QL219, wherein the native glycerol-phosphatase (gpp1) was deleted, strain QL220 wherein transgene acetyl-coA-synthetase (Seacs) was expressed, and strain QL221 wherein a second phosphate acetyltransferase (Ckpta) was expressed. Expression of phosphoketolase leads to formation of both E4P and acetyl-P. The produced acetyl-P can be degraded by nonspecific phosphatases Gpp1 and Gpp2 to form acetate, accumulation of which can have an adverse effect on cell growth (Meadows et al, 2016; Bergman et al, 2016). Hence, the modifications provided in strains QL291, QL220, and QL221 (i) reduce conversion of acetyl-P to acetate (QL219), (ii) reduce accumulation of acetate (QL220), and (iii) achieve an efficient conversion of acetyl-P to acetyl-CoA (QL221). Figure 5 shows that deletion of native gpp1 (QL219) has a positive effect on pHCA titers, while introducing transgene Seacs (QL220) or expressing a second Ckpta (QL221) has no significant effect. While all the strains presented in Figure 5 have the PAL-pathway for production of pHCA, Figure 6 further confirms the results in Figure 5 in TAL-branch strain QL294, again showing increased pHCA titers by deletion of gpp1.

### EXAMPLE 5: pha2 overexpression enhances pHCA production

Overexpression of prephenate dehydratase activity (pha2) (E.C.4.2.1.51) facilitates increased conversion of prephanate to phenylpyruvate, which is a precursor of phenylalanine in the phenylalanine biosynthesis pathway. Figure 7 shows a significant increase in pHCA titers in the strain QL288 overexpressing Pha2.

### EXAMPLE 6: Expression of Xfpk and pta enhance pHCA production in benchmark strain

The effect of introducing transgenes xfpk and pta in the benchmark strain QL264 is shown in figure 8. It is clearly shown that benchmark strain QL264 benefits from introduction of these transgenes, as the pHCA production of strain QL286 is increased by 29% compared to QL264. The pHCA production titers are even further enhanced by deletion of gpp1 (strain QL295).

### EXAMPLE 7: Production of baicalin, baicalein, and wogonin

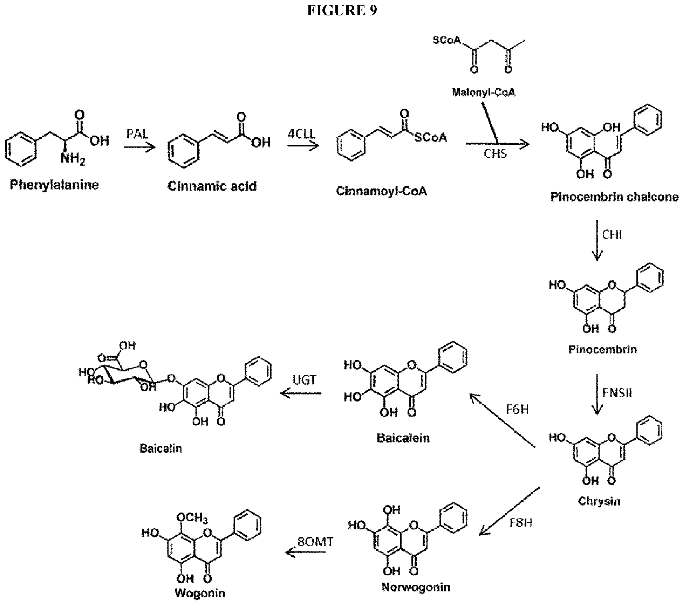

Baicalin, baicalein, and wogonin are valuable bioactive compounds that can be derived from phenylalanine (see Figure 9).

For production of baicalin, a microorganism is prepared expressing transgenes encoding polypeptides having enzyme activities of xfpk, pta, aro4* and aro7* in combination with transgenes encoding polypeptides having enzyme activities of phenylalanine ammonia lyase (PAL, EC 4.3.1.24), 4-coumaroyl-CoA-like ligase (4CLL, EC 6.2.1.12), chalcone synthase (CHS, EC 2.3.1.74), chalcone isomerase (CHI, EC 5.5.1.6), flavone synthase II (FNSII, EC 1.14.11.22), and flavone 6-hydroxylase (F6H, EC 1.14.13.-).

Specifically, yeast codon-optimized xylulose-5-phosphate/fructose-6-phosphate phosphoketolase gene BbXfpk from *Bifidobacterium breve* (SEQ ID No 11), yeast codon-optimized phosphate acetyltransferase CkPta from *Clostridium kluyveri* (SEQ ID No 16), feedback-resistant mutant aro4* gene from *S. cerevisiae* (SEQ ID No 74), feedback-resistant mutant aro7* gene from *S. cerevisiae* (SEQ ID No 70) yeast codon-optimized phenylalanine ammonia-lyase gene AtPal2 from *Arabidopsis thaliana* (SEQ ID No 31), 4-coumaroyl-CoA-like ligase gene from *Scutellaria baicalensis* (SEQ ID No 107), chalcone synthase gene from *Scutellaria baicalensis* (SEQ ID No 109), chalcone isomerase gene from *Scutellaria baicalensis* (SEQ ID No 111), flavone synthase II gene from *Scutellaria baicalensis* (SEQ ID No 113), and flavone 6-hydroxylase gene from *Scutellaria baicalensis* (SEQ ID No 115) are expressed in *S. cerevisiae* strain IMX581.

For production of baicalin, the baicalein producing strain described above is further modified, expressing the additional transgenes uridine diphosphate (UDP)-glucose dehydrogenase (UGD, EC1.1.1.22) and UDP-dependent glycosyltransferase (UGT, EC 2.4.1.-).

Specifically, uridine diphosphate (UDP)-glucose dehydrogenase gene from *Arabidopsis thaliana* (SEQ ID No 117) and UDP-dependent glycosyltransferase gene from *Antirrhinum majus* (SEQ ID No 119) are expressed in the baicalein producing strain described above.

For production of wogonin, a strain is prepared expressing transgenes encoding polypeptides having activities of xfpk, pta, aro4* and aro7* in combination with transgenes encoding polypeptides having activities of phenylalanine ammonia lyase (PAL), 4-coumaroyl-CoA-like ligase (4CL), chalcone synthase (CHS), chalcone isomerase (CHI), flavone synthase II (FNSII), flavone 8-hydroxylase (F8H, EC 1.14.13.-) and 8-O-methyltransferase (8OMT, EC 2.1.1.88).

Specifically, yeast codon-optimized xylulose-5-phosphate/fructose-6-phosphate phosphoketolase gene BbXfpk from *Bifidobacterium breve* (SEQ ID No 11), yeast codon-optimized phosphate acetyltransferase CkPta from *Clostridium kluyveri* (SEQ ID No 16), feedback-resistant mutant aro4* gene from *S. cerevisiae* (SEQ ID No 74), feedback-resistant mutant aro7* gene from *S. cerevisiae* (SEQ ID No 70), yeast codon-optimized phenylalanine ammonia-lyase gene AtPal2 from *Arabidopsis thaliana* (SEQ ID No 31), 4-coumaroyl-CoA-like ligase gene from *Scutellaria baicalensis* (SEQ ID No 107), chalcone synthase gene from *Scutellaria baicalensis* (SEQ ID No 109), chalcone isomerase gene from *Scutellaria baicalensis* (SEQ ID No 111), flavone synthase II gene from *Scutellaria baicalensis* (SEQ ID NOo 113), flavone 8-hydroxylase gene from *Scutellaria baicalensis* (SEQ ID No 121), and 8-O-methyltransferase gene from *Cucurbita maxima* (SEQ ID No 123) are expressed in *S. cerevisiae* strain IMX581.

Baicalin, baicalein, and wogonin are detected by HPLC and LC/MS. Separation is carried out on a 100*2-mm 3µLuna C18 column using 0.1% formic acid in water (A) versus 1:1 CAN/MeOH +0.1% formic acid (B) and run at 260 µl min⁻¹ with the following gradient: 0-3 min, 20% B; 20 min, 50% B; 20-30 min, 50% B; 36 min, 30% B; 37 min, 20% B, and 37-43 min, 20% B; the column is maintained at 35 degrees and absorption detected at 280 nm with diode array detector. Metabolites and products are measured by comparing the area of the individual peaks with standard curves obtained from standard compounds.

### EXAMPLE 8: Production of daidzein and puerarin

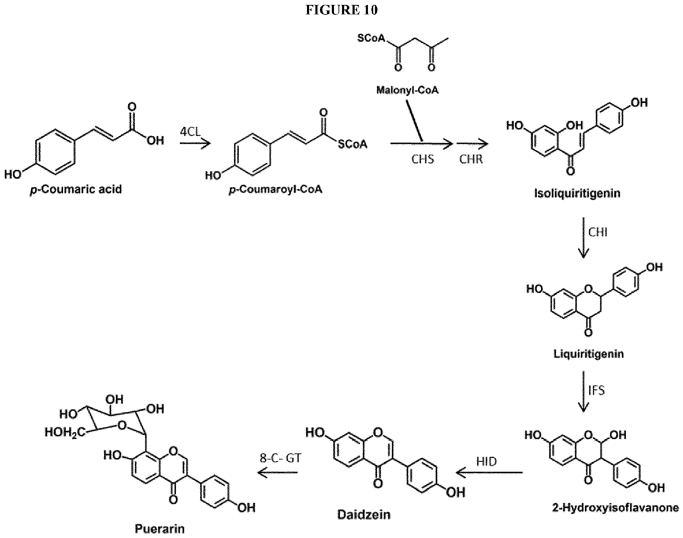

Daidzein and puerarin are valuable bioactive compounds that can be derived from pHCA (see Figure 10).

For production of daidzein from p-coumaric acid, a microorganism is prepared as described in any of examples 1-6, further expressing transgenes encoding polypeptides having enzyme activities of 4-coumaroyl-CoA ligase (4CL, EC 6.2.1.12), chalcone synthase (CHS, 2.3.1.74), chalcone reductase (CHR, EC 1.1.1.285), chalcone isomerase (CHI, EC 5.5.1.6), 2-hydroxyisoflavanone synthase (IFS, EC 1.14.14.87), and 2-hydroxyisoflavanone dehydratase (HID, EC 4.2.1.105).

Specifically, 4-coumaroyl-CoA ligase gene from *Pueraria montana var. lobata* (SEQ ID No 125), chalcone synthase gene from *Pueraria montana var. lobata* (SEQ ID No 127), chalcone reductase gene from *Glycine max* (SEQ ID No 129), chalcone isomerase gene from *Pueraria montana var. lobata* (SEQ ID No 131), 2-hydroxyisoflavanone synthase gene from *Pueraria montana var. lobata* (SEQ ID No 133), and 2-hydroxyisoflavanone dehydratase gene from *Pueraria montana var. lobata* (SEQ ID No 135) are expressed in strains QL212, QL219, and QL288.

For production of puerarin, the daidzein producing strain described above is further modified, expressing the additional transgene isoflavone 8-C-glucosyltransferase (8-C-GT, EC 2.4.1.170).

Specifically, isoflavone 8-C-glucosyltransferase gene from *Pueraria montana var. lobata* (SEQ ID No 137) is expressed in the daidzein producing strains described above.

HPLC and LC/MS is used to detect daidzein and puerarin and/or any intermediates. Separation is carried out on a Discovery HS F5 150 mm* 4.6 mm column using 10 mM ammonium formate pH 3.0 (A) versus 1:10 acetonitrile (B) and run at 1.5 ml min⁻¹ with the following gradient: 0-0.5 min, 5% B; 0.5-7 min, linear increase from 5% B to 60% B; 7-9.5 min, decrease from 60% B to 5% B; 9.6-12 min, 5% B; the column is maintained at 35 degrees and absorption detected at 277, 290, 333, 370 nm with UV detector. Metabolites and products are measured by comparing the area of the individual peaks with standard curves obtained from standard compounds.

### References

Rodriguez et al (2015) Establishment of a yeast platform strain for production of p-coumaic acid through metabolic engineering of aromatic amino acid biosynthesis. Metabolic Engineering 31: 181-188.
Mans et al (2015) CRISPR/Cas9: a molecular Swiss army knife for simultaneous introduction of multiple genetic modifications in Saccharomyces cerevisiae. FEMS Yeast Res 15(2). pii: fov004.
Zhou et al (2012) Modular pathway engineering of diterpenoid synthases and the mevalonic acid pathway for miltiradiene production. J Am Chem Soc. 134(6):3234-3241.
Gietz & Woods (2002) Transformation of yeast by lithium acetate/single-stranded carrier DNA/polyethylene glycol method. Methods Enzymol 350: 87-96.
Verduyn et al (1992) Effect of benzoic acid on metabolic fluxes in yeasts: a continuous-culture study on the regulation of respiration and alcoholic fermentation. Yeast 8(7):501-517.
Meadows et al (2016) Rewriting yeast central carbon metabolism for industrial isoprenoid production. Nature 537(7622):694-697
Bergman et al (2016) Functional expression and evaluation of heterologous phosphoketolases in Saccharomyces cerevisiae. Amb Express 6:115.

## Claims

1. A genetically modified microorganism for enhanced production of aromatic amino acid derivatives, wherein said microorganism is capable of expressing enhanced levels of
a. 3-deoxy-D-arabino-heptulosonate-7-phosphate synthase activity (E.C.2.5.1.54) and
b. chorismate mutase activity (E.C.5.4.99.5)
compared to a parent microorganism from which said genetically modified microorganism was derived; and wherein said genetically modified microorganism comprises transgenes encoding polypeptides having enzymatic activity of
c. phosphoketolase (E.C. 4.1.2.22) and
d. phosphate acetyltransferase (E.C.2.3.1.8);

2. The genetically modified microorganism according to claim 1, wherein said genetically modified microorganism is further capable of expressing enhanced levels of prephenate dehydratase activity (E.C.4.2.1.51) compared to a parent microorganism from which said genetically modified microorganism was derived.

3. The genetically modified microorganism according to claims 1 or 2, wherein one or more genes encoding a polypeptide having enzymatic activity of glycerol-3-phosphate phosphatase (E.C.3.1.3.21) are deleted in said genetically modified microorganism.

4. The genetically modified microorganism according to any one of claims 1 to 3, wherein said genetically modified microorganism further comprises transgenes encoding polypeptides having enzymatic activity of phenylalanine ammonia-lyase (E.C.4.3.1.24) and cinnamate-4-hydroxylase (E.C.1.14.13.11).

5. The genetically modified microorganism according to claim 4, wherein said genetically modified microorganism is further capable of expressing (i) enhanced amounts of electron transporter protein cytochrome b5 and (ii) enhanced levels of cytochrome P450 reductase activity (E.C.1.6.2.4), compared to a parent microorganism from which said genetically modified microorganism was derived.

6. The genetically modified microorganism according to any one of claims 1 to 5, wherein one or more genes encoding polypeptides having phenylpyruvate decarboxylase (E.C.4.1.1.43) activity or pyruvate decarboxylase (E.C.4.1.1.1) activity are deleted in said genetically modified microorganism.

7. The genetically modified microorganism according to any one of claims 1 to 6, wherein said genetically modified microorganism comprises a transgene encoding a polypeptide having feedback resistant 3-deoxy-D-arabino-heptulosonate-7-phosphate synthase activity.

8. The genetically modified microorganism according to any one of claims 1 to 7, wherein said genetically modified microorganism comprises a transgene encoding a polypeptide having feedback resistant chorismate mutase activity.

9. The genetically modified microorganism according to any one of claims 1 to 8, wherein the amino acid sequence of said polypeptide having phosphoketolase (E.C. 4.1.2.22) activity has at least 80% sequence identity to SEQ ID NO: 12; and wherein the amino acid sequence of said polypeptide having phosphate acetyltransferase (E.C.2.3.1.8) activity has at least 80% sequence identity to SEQ ID NO: 17.

10. The genetically modified microorganism according to any one of claims 2 to 9, wherein said genetically modified microorganism expresses a transgene encoding a polypeptide having prephenate dehydratase activity, wherein said transgene has at least 80% sequence identity to SEQ ID NO: 22.

11. The genetically modified microorganism according to any one of claims 1 to 10, wherein the microorganism is a species of *Saccharomyces.*

12. Use of the genetically modified microorganism according to any one of claims 1-11 for the production of an aromatic amino acid derivative.

13. The use according to claim 12, wherein said aromatic amino acid derivative is selected from the group comprising p-coumaric acid, baicalin, baicalein, wogonin, daidzein, and puerarin.

14. A method for producing an aromatic amino acid derivative comprising the steps of:
a. introducing a genetically modified microorganism according to any one of claims 1-13 into a growth medium to produce a culture;
b. providing a substrate for the production of an aromatic amino acid derivative to said culture, and culturing said culture;
c. recovering said aromatic amino acid derivative produced by said culture, and optionally purifying the recovered aromatic amino acid derivative.

15. The method according to claim 14, wherein said aromatic amino acid derivative is selected from the group comprising p-coumaric acid, baicalin, baicalein, wogonin, daidzein, and puerarin.


SEQUENCE LISTING

<110>  Technical University of Denmark

<120>  Utilization of phosphoketolase for production of aromatic amino
       acid derived products

<130>  P2493EP00

<160>  138

<170>  PatentIn version 3.5

<210>  1
<211>  370
<212>  PRT
<213>  Saccharomyces cerevisiae


<220>
<221>  POLYPEPTIDE
<222>  (1)..(370)
<223>  DAHP synthase (ARO4)

<300>
<308>  NCBI Reference Sequence: NP_009808.1
<309>  2018-04-04
<313>  (1)..(370)

<400>  1

Met Ser Glu Ser Pro Met Phe Ala Ala Asn Gly Met Pro Lys Val Asn
1               5                   10                  15


Gln Gly Ala Glu Glu Asp Val Arg Ile Leu Gly Tyr Asp Pro Leu Ala
            20                  25                  30


Ser Pro Ala Leu Leu Gln Val Gln Ile Pro Ala Thr Pro Thr Ser Leu
        35                  40                  45


Glu Thr Ala Lys Arg Gly Arg Arg Glu Ala Ile Asp Ile Ile Thr Gly
    50                  55                  60


Lys Asp Asp Arg Val Leu Val Ile Val Gly Pro Cys Ser Ile His Asp
65                  70                  75                  80


Leu Glu Ala Ala Gln Glu Tyr Ala Leu Arg Leu Lys Lys Leu Ser Asp
                85                  90                  95


Glu Leu Lys Gly Asp Leu Ser Ile Ile Met Arg Ala Tyr Leu Glu Lys
            100                 105                 110


Pro Arg Thr Thr Val Gly Trp Lys Gly Leu Ile Asn Asp Pro Asp Val
        115                 120                 125

Asn Asn Thr Phe Asn Ile Asn Lys Gly Leu Gln Ser Ala Arg Gln Leu
    130                 135                 140

Phe Val Asn Leu Thr Asn Ile Gly Leu Pro Ile Gly Ser Glu Met Leu
145                 150                 155                 160

Asp Thr Ile Ser Pro Gln Tyr Leu Ala Asp Leu Val Ser Phe Gly Ala
                165                 170                 175

Ile Gly Ala Arg Thr Thr Glu Ser Gln Leu His Arg Glu Leu Ala Ser
            180                 185                 190

Gly Leu Ser Phe Pro Val Gly Phe Lys Asn Gly Thr Asp Gly Thr Leu
        195                 200                 205

Asn Val Ala Val Asp Ala Cys Gln Ala Ala Ala His Ser His His Phe
    210                 215                 220

Met Gly Val Thr Lys His Gly Val Ala Ala Ile Thr Thr Thr Lys Gly
225                 230                 235                 240

Asn Glu His Cys Phe Val Ile Leu Arg Gly Gly Lys Lys Gly Thr Asn
                245                 250                 255

Tyr Asp Ala Lys Ser Val Ala Glu Ala Lys Ala Gln Leu Pro Ala Gly
            260                 265                 270

Ser Asn Gly Leu Met Ile Asp Tyr Ser His Gly Asn Ser Asn Lys Asp
        275                 280                 285

Phe Arg Asn Gln Pro Lys Val Asn Asp Val Val Cys Glu Gln Ile Ala
    290                 295                 300

Asn Gly Glu Asn Ala Ile Thr Gly Val Met Ile Glu Ser Asn Ile Asn
305                 310                 315                 320

Glu Gly Asn Gln Gly Ile Pro Ala Glu Gly Lys Ala Gly Leu Lys Tyr
                325                 330                 335

Gly Val Ser Ile Thr Asp Ala Cys Ile Gly Trp Glu Thr Thr Glu Asp
            340                 345                 350

Val Leu Arg Lys Leu Ala Ala Ala Val Arg Gln Arg Arg Glu Val Asn
        355                 360                 365

Lys Lys
    370

<210>  2
<211>  374
<212>  PRT
<213>  Galactomyces candidus

<220>
<221>  POLYPEPTIDE
<222>  (1)..(374)
<223>  DAHP synthase

<300>
<308>  GenBank: CDO53356.1
<309>  2015-09-30
<313>  (1)..(374)

<400>  2

Met Ser Ala Ser Pro Met Phe Gly Pro Asp Gly Ile Ser Arg His Gln
1               5                   10                  15

Thr Pro Ile Ala Glu Asp Ser Glu Asp Val Arg Ile Leu Gly Tyr Asp
            20                  25                  30

Pro Leu Val Pro Pro Ala Leu Leu Gln Thr Glu Val Pro Ala Ser Glu
        35                  40                  45

Lys Ala Val Lys Val Val Ala Lys Gly Arg Lys Asp Ser Ile Asn Val
    50                  55                  60

Ile Ser Gly Ala Ser Asp Lys Val Leu Cys Ile Val Gly Pro Cys Ser
65                  70                  75                  80

Ile His Asp Ala Val Ala Ala Arg Glu Tyr Ala Asn Arg Leu Ile Asn
                85                  90                  95

Leu Ala Ala Glu Leu Asp Asn Glu Leu Val Val Ile Met Arg Ala Tyr
            100                 105                 110

Leu Glu Lys Pro Arg Thr Thr Val Gly Trp Lys Gly Leu Ile Asn Asp
        115                 120                 125

Pro Asp Val Asp Glu Ser Phe Asn Ile Asn Lys Gly Leu Arg Ile Ser
    130                 135                 140

Arg Ser Leu Phe Ala Glu Leu Thr Asp Met Gly Ile Pro Ile Ala Ser
145                 150                 155                 160

Glu Met Leu Asp Thr Ile Ser Pro Gln Phe Leu Ala Asp Leu Leu Ser
                165                 170                 175

Leu Gly Ala Ile Gly Ala Arg Thr Thr Glu Ser Gln Leu His Arg Glu
            180                 185                 190

Leu Ala Ser Gly Leu Ser Phe Pro Ile Gly Phe Lys Asn Gly Thr Asp
        195                 200                 205

Gly Thr Val Gly Val Ala Leu Asp Ala Val Gln Ala Ala Ser Phe Pro
    210                 215                 220

His His Phe Met Gly Val Thr Lys Gln Gly Val Ala Ala Ile Thr Thr
225                 230                 235                 240

Thr Lys Gly Asn Glu Asn Cys Phe Ile Ile Leu Arg Gly Gly Thr Ser
                245                 250                 255

Gly Thr Asn Tyr Asp Glu Ala Ser Val Lys Ala Val Lys Glu Lys Leu
            260                 265                 270

Ala Lys Ala Asn Ser Ser Thr Pro Ile Met Ile Asp Cys Ser His Gly
        275                 280                 285

Asn Ser Asn Lys Asp Tyr Arg Asn Gln Pro Lys Val Leu Ser Glu Val
    290                 295                 300

Ala Arg Gln Ile Ala Asn Gly Glu Asp Lys Ile Ile Gly Val Met Ile
305                 310                 315                 320

Glu Ser His Leu Asn Glu Gly Lys Gln Ala Ala Gly Thr Thr Gly Lys
                325                 330                 335

Asp Gly Leu Lys Tyr Gly Val Ser Ile Thr Asp Ala Cys Val Gly Trp
            340                 345                 350

Asp Thr Thr Glu Val Met Leu Arg Glu Leu Ala Asp Ala Val Lys Thr
        355                 360                 365

Arg Arg Ala Asn Lys Asn
    370

<210>  3
<211>  370
<212>  PRT
<213>  Saccharomyces eubayanus

<220>
<221>  POLYPEPTIDE
<222>  (1)..(370)
<223>  DAHP synthase

<300>
<308>  NCBI Reference Sequence: XP_018223276.1
<309>  2017-09-27
<313>  (1)..(370)

<400>  3

Met Ser Glu Ser Pro Met Phe Gly Ala Asn Gly Thr Pro Asn Val Asn
1               5                   10                  15

Glu Ala Ala Glu Glu Asp Val Arg Ile Leu Gly Tyr Asp Pro Leu Ala
            20                  25                  30

Ser Pro Ala Leu Leu Gln Val Gln Ile Pro Ala Thr Gln Thr Ser Leu
        35                  40                  45

Glu Thr Ala Lys Arg Gly Arg Arg Glu Ala Ile Asp Ile Ile Thr Gly
    50                  55                  60

Lys Asp Asp Arg Val Leu Val Ile Val Gly Pro Cys Ser Ile His Asp
65                  70                  75                  80

Leu Asp Ala Ala Gln Glu Tyr Ala Leu Arg Leu Lys Lys Leu Ala Glu
                85                  90                  95

Glu Leu Lys Gly Asp Leu Ser Ile Ile Met Arg Ala Tyr Leu Glu Lys
            100                 105                 110

Pro Arg Thr Thr Val Gly Trp Lys Gly Leu Ile Asn Asp Pro Asp Val
        115                 120                 125

Asn Asn Thr Phe Asn Ile Asn Lys Gly Leu Gln Ser Ala Arg Gln Leu
    130                 135                 140

Phe Val Asn Leu Thr Asn Ile Gly Leu Pro Ile Gly Ser Glu Met Leu
145                 150                 155                 160

Asp Thr Ile Ser Pro Gln Tyr Leu Ala Asp Leu Val Ser Phe Gly Ala
                165                 170                 175

Ile Gly Ala Arg Thr Thr Glu Ser Gln Leu His Arg Glu Leu Ala Ser
            180                 185                 190

Gly Leu Ser Phe Pro Val Gly Phe Lys Asn Gly Thr Asp Gly Thr Leu
        195                 200                 205

Asn Val Ala Val Asp Ala Cys Gln Ala Ala Ala His Ser His His Phe
    210                 215                 220

Met Gly Val Thr Lys His Gly Val Ala Ala Ile Thr Thr Thr Lys Gly
225                 230                 235                 240

Asn Glu His Cys Phe Val Ile Leu Arg Gly Gly Lys Lys Gly Thr Asn
                245                 250                 255

Tyr Asp Ala Lys Ser Val Ala Glu Ala Lys Ala Gln Leu Pro Ala Gly
            260                 265                 270

Ser Asn Gly Leu Met Ile Asp Tyr Ser His Gly Asn Ser Asn Lys Asp
        275                 280                 285

Tyr Arg Asn Gln Pro Lys Val Asn Asp Val Val Cys Glu Gln Ile Ala
    290                 295                 300

Lys Gly Asp Asn Ala Ile Thr Gly Val Met Ile Glu Ser Asn Ile Asn
305                 310                 315                 320

Glu Gly Asn Gln Gly Ile Pro Thr Glu Gly Lys Ala Gly Leu Lys Tyr
                325                 330                 335

Gly Val Ser Ile Thr Asp Ala Cys Ile Gly Trp Ser Thr Thr Glu Asp
            340                 345                 350

Val Leu Arg Lys Leu Ala Ser Ala Val Arg Gln Arg Arg Glu Leu Asn
        355                 360                 365

Lys Lys
    370

<210>  4
<211>  365
<212>  PRT
<213>  Kazachstania saulgeensis

<220>
<221>  POLYPEPTIDE
<222>  (1)..(365)
<223>  DAHP synthase

<300>
<308>  GenBank: SMN22584.1
<309>  2017-04-28
<313>  (1)..(365)

<400>  4

Met Ser Ser Pro Met Phe Asn Ala Asp Gly Thr Thr Asn Gln Ser Glu
1               5                   10                  15

Asp Val Arg Ile Leu Gly Tyr Asp Pro Leu Val Ser Pro Ala Leu Leu

20                  25                  30

Gln Val Gln Ile Pro Ala Thr Gln Lys Cys Leu Glu Thr Ala Gln His
        35                  40                  45

Gly Arg Lys Asp Ser Ile Glu Ile Ile Ser Gly Arg Asp Asp Arg Val
    50                  55                  60

Leu Val Ile Val Gly Pro Cys Ser Ile His Asp Leu Asp Ala Ala Gln
65                  70                  75                  80

Glu Tyr Ala Leu Lys Leu Lys Lys Leu Ala Asp Glu Leu Lys Gly Asp
                85                  90                  95

Leu Leu Ile Val Met Arg Ala Tyr Leu Glu Lys Pro Arg Thr Thr Val
            100                 105                 110

Gly Trp Lys Gly Leu Ile Asn Asp Pro Asp Val Asn Asn Ser Phe Asn
        115                 120                 125

Ile Asn Lys Gly Leu Gln Ala Ala Arg Gln Leu Phe Val Asn Leu Thr
    130                 135                 140

Ser Ile Gly Leu Pro Ile Gly Ser Glu Met Leu Asp Thr Ile Ser Pro
145                 150                 155                 160

Gln Tyr Leu Thr Asp Leu Leu Ser Phe Gly Ala Ile Gly Ala Arg Thr
                165                 170                 175

Thr Glu Ser Gln Leu His Arg Glu Leu Ala Ser Gly Leu Ser Phe Pro
            180                 185                 190

Ile Gly Phe Lys Asn Gly Thr Asp Gly Thr Leu Asp Val Ala Val Ala
        195                 200                 205

Ala Cys Gln Ala Ala Ser His Pro His His Phe Leu Gly Val Thr Lys
    210                 215                 220

His Gly Val Ala Ala Ile Thr Thr Thr Lys Gly Asn Glu His Cys Phe
225                 230                 235                 240

Val Ile Leu Arg Gly Gly Lys Lys Gly Thr Asn Tyr Asp Ala Lys Ser
                245                 250                 255

Val Ala Glu Ala Lys Ala Gln Leu Pro Ala Gly Ser Asn Gly Ile Met
            260                 265                 270

Ile Asp Tyr Ser His Gly Asn Ser Asn Lys Asp Tyr Arg Asn Gln Pro
        275                 280                 285

Lys Val Asn Asp Val Val Cys Glu Gln Ile Ala Ala Gly Glu Lys Ser
    290                 295                 300

Ile Ile Gly Val Met Ile Glu Ser Asn Ile Asn Glu Gly Arg Gln Asp
305                 310                 315                 320

Val Pro Pro Glu Gly Lys Ala Gly Leu Lys Tyr Gly Val Ser Ile Thr
                325                 330                 335

Asp Ala Cys Ile Ser Trp Glu Thr Thr Asp Ala Val Leu Arg Lys Leu
            340                 345                 350

Ala Ala Ala Val Gln Gln Arg Arg Ala Val Asn Lys Asn
        355                 360                 365

<210>  5
<211>  360
<212>  PRT
<213>  Komagataella phaffii GS115

<220>
<221>  POLYPEPTIDE
<222>  (1)..(360)
<223>  DAHP synthase

<300>
<308>  NCBI Reference Sequence: XP_002490334.1
<309>  2017-10-11
<313>  (1)..(360)

<400>  5

Met Thr Ser Thr Pro Val Gln Glu Glu Tyr Asp Asp Val Arg Ile Ala
1               5                   10                  15

Gly Tyr Asp Pro Leu Val Ser Pro Ala Leu Leu Gln Gln Glu Ile Pro
            20                  25                  30

Ala Thr Lys Val Ala Leu Gln Thr Val Val Lys Gly Arg Lys Asp Ala
        35                  40                  45

Met Asn Val Val Ser Leu Lys Asp Asp Arg Leu Leu Val Val Val Gly
    50                  55                  60

Pro Cys Ser Ile His Asp Ser Asp Ala Ala Leu Glu Tyr Val Ala Arg
65                  70                  75                  80

Leu Lys Ala Leu Ser Glu Glu Leu Lys Asp Glu Leu Val Ile Ile Met

                85                  90                  95

Arg Ala Tyr Leu Glu Lys Pro Arg Thr Thr Val Gly Trp Lys Gly Leu
            100                 105                 110

Ile Asn Asp Pro Asp Leu Asp Asn Ser Phe Ser Ile Asn Lys Gly Leu
        115                 120                 125

Lys Ile Ser Arg Lys Leu Phe Val Asp Leu Thr Asn Ser Gly Met Pro
    130                 135                 140

Ile Ala Ser Glu Met Leu Asp Thr Ile Ser Pro Gln Tyr Leu Ala Asp
145                 150                 155                 160

Leu Leu Ser Phe Gly Ala Ile Gly Ala Arg Thr Thr Glu Ser Gln Leu
                165                 170                 175

His Arg Glu Leu Ala Ser Gly Leu Ser Phe Pro Ile Gly Phe Lys Asn
            180                 185                 190

Gly Thr Asp Gly Thr Leu Asp Val Ala Leu Asp Ala Val Gln Ala Ala
        195                 200                 205

Ser His Ala His His Phe Met Gly Val Thr Lys His Gly Ile Ala Ala
    210                 215                 220

Ile Thr Thr Thr Lys Gly Asn Glu Tyr Cys Phe Val Ile Leu Arg Gly
225                 230                 235                 240

Gly Lys Lys Gly Thr Asn Tyr Asp Pro Glu Ser Val Ala Glu Ala Lys
                245                 250                 255

Ala Lys Leu Pro Ala Asp Gly Val Leu Met Ile Asp Cys Ser His Gly
            260                 265                 270

Asn Ser Asn Lys Asp Tyr Arg Asn Gln Pro Lys Val Ser Lys Val Val
        275                 280                 285

Ala Asp Gln Val Ala Ala Gly Glu Asp Arg Ile Ile Gly Val Met Ile
    290                 295                 300

Glu Ser Asn Ile Asn Glu Gly Lys Gln Ser Ile Pro Ala Glu Gly Arg
305                 310                 315                 320

Lys Ala Leu Lys Tyr Gly Val Ser Val Thr Asp Gly Cys Val Ser Trp
                325                 330                 335

Glu Thr Thr Asp Ala Met Leu Arg Glu Leu Ala Glu Ala Val Gln Lys
            340                 345                 350

Arg Arg Ala Leu Lys Asn Ala Ala
        355                 360

<210>  6
<211>  256
<212>  PRT
<213>  Saccharomyces cerevisiae

<220>
<221>  POLYPEPTIDE
<222>  (1)..(256)
<223>  Chorismate mutase ARO7

<300>
<308>  NCBI Reference Sequence: NP_015385.1
<309>  2018-04-04
<313>  (1)..(256)

<400>  6

Met Asp Phe Thr Lys Pro Glu Thr Val Leu Asn Leu Gln Asn Ile Arg
1               5                   10                  15

Asp Glu Leu Val Arg Met Glu Asp Ser Ile Ile Phe Lys Phe Ile Glu
            20                  25                  30

Arg Ser His Phe Ala Thr Cys Pro Ser Val Tyr Glu Ala Asn His Pro
        35                  40                  45

Gly Leu Glu Ile Pro Asn Phe Lys Gly Ser Phe Leu Asp Trp Ala Leu
    50                  55                  60

Ser Asn Leu Glu Ile Ala His Ser Arg Ile Arg Arg Phe Glu Ser Pro
65                  70                  75                  80

Asp Glu Thr Pro Phe Phe Pro Asp Lys Ile Gln Lys Ser Phe Leu Pro
                85                  90                  95

Ser Ile Asn Tyr Pro Gln Ile Leu Ala Pro Tyr Ala Pro Glu Val Asn
            100                 105                 110

Tyr Asn Asp Lys Ile Lys Lys Val Tyr Ile Glu Lys Ile Ile Pro Leu
        115                 120                 125

Ile Ser Lys Arg Asp Gly Asp Asp Lys Asn Asn Phe Gly Ser Val Ala
    130                 135                 140

Thr Arg Asp Ile Glu Cys Leu Gln Ser Leu Ser Arg Arg Ile His Phe

145                 150                 155                 160

Gly Lys Phe Val Ala Glu Ala Lys Phe Gln Ser Asp Ile Pro Leu Tyr
                165                 170                 175

Thr Lys Leu Ile Lys Ser Lys Asp Val Glu Gly Ile Met Lys Asn Ile
            180                 185                 190

Thr Asn Ser Ala Val Glu Glu Lys Ile Leu Glu Arg Leu Thr Lys Lys
        195                 200                 205

Ala Glu Val Tyr Gly Val Asp Pro Thr Asn Glu Ser Gly Glu Arg Arg
    210                 215                 220

Ile Thr Pro Glu Tyr Leu Val Lys Ile Tyr Lys Glu Ile Val Ile Pro
225                 230                 235                 240

Ile Thr Lys Glu Val Glu Val Glu Tyr Leu Leu Arg Arg Leu Glu Glu
                245                 250                 255

<210>  7
<211>  258
<212>  PRT
<213>  Hanseniaspora osmophila

<220>
<221>  POLYPEPTIDE
<222>  (1)..(258)
<223>  Chorismate mutase

<300>
<308>  GenBank: OEJ85185.1
<309>  2016-09-27
<313>  (1)..(258)

<400>  7

Met Asn Phe Leu Glu Pro Ser Thr Val Leu Asp Leu Gln Asn Ile Arg
1               5                   10                  15

Asp Glu Leu Val Lys Met Glu Asp Thr Ile Ile Phe Asp Phe Ile Glu
            20                  25                  30

Arg Ser His Phe Ala Arg Cys Ser Gly Val Tyr Gln Ala Asn Asn Ala
        35                  40                  45

Glu Val Asp Ile Pro Asn Phe Asp Gly Ser Phe Leu Asp Trp Ser Leu
    50                  55                  60

Met Gln Ile Glu Ile Val His Ser Gln Leu Arg Arg Phe Glu Ser Pro
65                  70                  75                  80

Asp Gln Thr Pro Phe Phe Pro Asn His Ile Leu Lys Pro Ile Leu Pro
                85                  90                  95

Ser Ile Asn Tyr Pro Lys Val Leu Ala Ser Tyr Asp Ala Glu Ile Asn
            100                 105                 110

Tyr Asn Thr Lys Ile Lys Asn Leu Tyr Ile Asp His Ile Val Pro Met
        115                 120                 125

Ile Ala Lys Arg Ser Gly Glu Gln Lys Glu Gln Met Gly Ser Val Thr
    130                 135                 140

Met Ile Asp Ile Glu Thr Leu Gln Asn Leu Ser Arg Arg Ile His Phe
145                 150                 155                 160

Gly Lys Phe Val Ala Glu Ala Lys Tyr Gln Ser Asn Lys Glu Leu Tyr
                165                 170                 175

Lys Lys Leu Ile Leu Glu Lys Asp Val Asn Gly Ile Met Glu Asn Ile
            180                 185                 190

Thr Asn Ser Gln Val Glu Glu Lys Ile Leu Glu Arg Leu Arg Val Lys
        195                 200                 205

Ala Gln Val Tyr Gly Val Asp Pro Thr Asn Lys Glu Gly Glu Arg Lys
    210                 215                 220

Ile Thr Pro Glu Tyr Leu Val Arg Ile Tyr Arg Asp Phe Val Ile Pro
225                 230                 235                 240

Met Thr Lys Glu Val Glu Val Glu Tyr Leu Leu Arg Arg Leu Glu Asp
                245                 250                 255

Glu Lys

<210>  8
<211>  256
<212>  PRT
<213>  Saccharomyces arboricola

<220>
<221>  POLYPEPTIDE
<222>  (1)..(256)
<223>  Chorismate mutase

<300>
<308>  GenBank: EJS41344.1

<309>  2012-08-16
<313>  (1)..(256)

<400>  8

Met Asp Phe Thr Lys Pro Glu Thr Val Leu Asn Leu Gln Asn Ile Arg
1               5                   10                  15

Asp Glu Leu Val Arg Met Glu Asp Ser Ile Ile Phe Lys Phe Ile Glu
            20                  25                  30

Arg Ser His Phe Ala Thr Cys Pro Ser Val Tyr Lys Glu Asn Tyr Pro
        35                  40                  45

Gly Leu Ala Ile Pro Asn Phe Lys Gly Ser Phe Leu Asp Trp Ala Leu
    50                  55                  60

Ser Asn Leu Glu Ile Ala His Ser Arg Ile Arg Arg Phe Glu Ser Pro
65                  70                  75                  80

Asp Glu Thr Pro Phe Phe Pro Asp Lys Ile Leu Lys Ser Phe Leu Pro
                85                  90                  95

Ser Val Asn Tyr Pro Gln Ile Leu Ala Pro Tyr Ala Pro Glu Val Asn
            100                 105                 110

Tyr Asn Asp Lys Ile Lys Lys Val Tyr Ile Glu Lys Ile Ile Pro Leu
        115                 120                 125

Ile Ser Lys Lys Asp Gly Asp Asp Lys Asn Asn Phe Gly Ser Val Ala
    130                 135                 140

Thr Arg Asp Ile Glu Cys Leu Gln Ser Leu Ser Arg Arg Ile His Phe
145                 150                 155                 160

Gly Lys Phe Val Ala Glu Ala Lys Phe Leu Ser Asp Ile Pro Leu Tyr
                165                 170                 175

Thr Lys Leu Ile Glu Asp Asn Asn Val Glu Gly Ile Met Lys Asn Ile
            180                 185                 190

Thr Asn Ser Ala Val Glu Glu Lys Ile Leu Glu Arg Leu Thr Lys Lys
        195                 200                 205

Ala Glu Val Tyr Gly Val Asp Pro Thr Asn Lys Ile Asp Glu Arg Arg
    210                 215                 220

Ile Thr Ser Glu Tyr Leu Val Lys Ile Tyr Lys Glu Ile Val Ile Pro
225                 230                 235                 240

Ile Thr Lys Glu Val Glu Val Glu Tyr Leu Leu Arg Arg Leu Glu Lys
                245                 250                 255

<210>  9
<211>  259
<212>  PRT
<213>  Kluyveromyces marxianus DMKU3-1042

<220>
<221>  POLYPEPTIDE
<222>  (1)..(259)
<223>  Chorismate mutase

<300>
<308>  NCBI Reference Sequence: XP_022678381.1
<309>  2017-10-23
<313>  (1)..(259)

<400>  9

Met Asp Phe Phe Lys Pro Glu Thr Val Leu Asn Leu Gln Asn Ile Arg
1               5                   10                  15

Asp Glu Leu Val Lys Met Glu Asp Thr Ile Ile Phe Asn Phe Ile Glu
            20                  25                  30

Arg Ser His Phe Ala Thr Cys Pro Ser Val Tyr Gln Asn Lys Asp Pro
        35                  40                  45

Leu Val Asn Leu Pro Asp Phe Asp Gly Ser Phe Leu Asp Trp Ala Leu
    50                  55                  60

Met His Leu Glu Ile Val His Ser Gln Leu Arg Arg Phe Glu Ser Pro
65                  70                  75                  80

Asp Glu Thr Pro Phe Phe Pro Asp Lys Ile Leu Lys Pro Ile Ile Pro
                85                  90                  95

Ser Leu Asn Tyr Pro Lys Ile Leu Ala Ser Tyr Ser Ser Gln Ile Asn
            100                 105                 110

Tyr Asn Asp Lys Ile Lys Lys Ile Tyr Ile Glu Thr Ile Val Pro Leu
        115                 120                 125

Ile Ser Lys Arg Glu Gly Asn Thr Ser Glu Asn Phe Gly Ser Val Ala
    130                 135                 140

Thr Arg Asp Ile Glu Thr Leu His Ala Leu Ser Arg Arg Ile His Phe
145                 150                 155                 160

Gly Lys Phe Val Ala Glu Ala Lys Phe Gln Ser Glu Lys Glu Lys Tyr
                165                 170                 175

Thr Glu Leu Ile Arg Asn Lys Asp Thr Glu Gly Ile Met Lys Ala Ile
            180                 185                 190

Thr Asn Ser Glu Val Glu Glu Lys Ile Leu Lys Arg Leu Gln Val Lys
        195                 200                 205

Ala Glu Val Tyr Gly Val Asp Pro Thr Asn Ala Gln Gly Asp Lys Lys
    210                 215                 220

Ile Thr Pro Glu Tyr Leu Val Arg Ile Tyr Lys Glu Ile Val Ile Pro
225                 230                 235                 240

Ile Thr Lys Glu Val Glu Val Glu Tyr Leu Leu Arg Arg Leu Glu Asp
                245                 250                 255

Glu Glu Lys

<210>  10
<211>  260
<212>  PRT
<213>  Candida glabrata

<220>
<221>  POLYPEPTIDE
<222>  (1)..(260)
<223>  Chorismate mutase

<300>
<308>  GenBank: KTB02179.1
<309>  2016-02-09
<313>  (1)..(260)

<400>  10

Met Asp Phe Thr Lys Pro Glu Thr Val Leu Asn Leu Gln Asn Ile Arg
1               5                   10                  15

Asp Glu Leu Val Lys Met Glu Asp Ser Ile Ile Phe Lys Phe Ile Glu
            20                  25                  30

Arg Ser His Phe Pro Val Cys Ala Pro Val Tyr Ala Pro Asn His Pro
        35                  40                  45

Asp Leu Asn Ile Pro Asp Phe Thr Gly Ser Phe Leu Asp Trp Ala Leu
    50                  55                  60

Leu Gln Met Glu Ile Thr His Ser Lys Leu Arg Arg Phe Asp Ser Pro
65                  70                  75                  80

Asp Glu Thr Pro Phe Phe Pro Asn Asp Ile Gln Glu Pro Ile Leu Pro
                85                  90                  95

Arg Ile Asn Tyr Pro Lys Ile Leu His Glu Lys Ala Ser Lys Pro Val
            100                 105                 110

Asn Tyr Asn Glu Lys Ile Lys Ser Ile Tyr Ile Lys Glu Ile Val Pro
        115                 120                 125

Leu Ile Ser Asn Ser Asp Gly Asp Ser Lys Glu Asn Tyr Gly Ser Val
    130                 135                 140

Ala Thr Arg Asp Met Glu Cys Leu Gln Ser Leu Ser Arg Arg Ile His
145                 150                 155                 160

Phe Gly Lys Phe Val Ala Glu Ala Lys Phe Gln Ser Asp Ile Asp Leu
                165                 170                 175

Tyr Thr Lys Met Ile Arg Glu Lys Asp Val Asp Gly Ile Met Ser Ser
            180                 185                 190

Ile Thr Asn Ala Ala Val Glu Glu Lys Ile Leu Glu Arg Leu Val Arg
        195                 200                 205

Lys Ala Asp Val Tyr Gly Val Asp Pro Thr Thr Pro Val Asp Ser Thr
    210                 215                 220

Glu Lys Val Arg Arg Ile Thr Pro Glu Tyr Leu Val Lys Ile Tyr Lys
225                 230                 235                 240

Glu Ile Val Ile Pro Ile Thr Lys Glu Val Glu Val Asp Tyr Leu Leu
                245                 250                 255

Arg Arg Leu Asp
            260

<210>  11
<211>  2478
<212>  DNA
<213>  Bifidobacterium breve

<220>
<221>  CDS
<222>  (1)..(2478)
<223>  Gene encoding phosphoketolase from Bifidobacterium breve codon
       opimized for yeast

<400> 11
atg act aac cct gta atc ggt act cct tgg caa aag ttg gat aga cca 48
Met Thr Asn Pro Val Ile Gly Thr Pro Trp Gln Lys Leu Asp Arg Pro
1               5                   10                  15

gtt tca gaa gaa gca atc gaa ggt atg gat aaa tat tgg aga gtt acc 96
Val Ser Glu Glu Ala Ile Glu Gly Met Asp Lys Tyr Trp Arg Val Thr
            20                  25                  30

aac tat atg tcc ata ggt caa atc tac ttg aga agt aac cca ttg atg 144
Asn Tyr Met Ser Ile Gly Gln Ile Tyr Leu Arg Ser Asn Pro Leu Met
        35                  40                  45

aag gaa cct ttt act aga gat gac gtt aag cat aga tta gtc ggt cac 192
Lys Glu Pro Phe Thr Arg Asp Asp Val Lys His Arg Leu Val Gly His
    50                  55                  60

tgg ggt act aca cca ggt ttg aac ttc ttg ttg gcc cat atc aac aga 240
Trp Gly Thr Thr Pro Gly Leu Asn Phe Leu Leu Ala His Ile Asn Arg
65                  70                  75                  80

ttg atc gct gat cac caa caa aac acc gtt ttt ata atg ggt cca ggt 288
Leu Ile Ala Asp His Gln Gln Asn Thr Val Phe Ile Met Gly Pro Gly
                85                  90                  95

cat ggt ggt cca gct ggt act tcc caa agt tat gtt gac ggt act tac 336
His Gly Gly Pro Ala Gly Thr Ser Gln Ser Tyr Val Asp Gly Thr Tyr
            100                 105                 110

act gaa tac tac cca aac ata aca aaa gat gaa gct ggt ttg caa aag 384
Thr Glu Tyr Tyr Pro Asn Ile Thr Lys Asp Glu Ala Gly Leu Gln Lys
        115                 120                 125

ttt ttc aga caa ttc tcc tat cca ggt ggt atc cct agt cat ttt gca 432
Phe Phe Arg Gln Phe Ser Tyr Pro Gly Gly Ile Pro Ser His Phe Ala
    130                 135                 140

cca gaa acc cct ggt tca att cac gaa ggt ggt gaa ttg ggt tat gct 480
Pro Glu Thr Pro Gly Ser Ile His Glu Gly Gly Glu Leu Gly Tyr Ala
145                 150                 155                 160

tta tct cat gct tac ggt gca gta atg aat aac cca tca ttg ttt gtt 528
Leu Ser His Ala Tyr Gly Ala Val Met Asn Asn Pro Ser Leu Phe Val
                165                 170                 175

cct tgt att ata ggt gac ggt gaa gcc gaa aca ggt cca tta gct acc 576
Pro Cys Ile Ile Gly Asp Gly Glu Ala Glu Thr Gly Pro Leu Ala Thr
            180                 185                 190

ggt tgg caa tct aac aaa ttg gtc aat cca aga act gat ggt atc gta 624
Gly Trp Gln Ser Asn Lys Leu Val Asn Pro Arg Thr Asp Gly Ile Val
        195                 200                 205

ttg cct atc ttg cat ttg aac ggt tac aag att gca aat cca aca atc 672
Leu Pro Ile Leu His Leu Asn Gly Tyr Lys Ile Ala Asn Pro Thr Ile
    210                 215                 220

ttg gcc aga ata tct gat gaa gaa tta cat gac ttt ttc cgt ggt atg 720
Leu Ala Arg Ile Ser Asp Glu Glu Leu His Asp Phe Phe Arg Gly Met
225                 230                 235                 240

ggt tat cac cct tac gaa ttt gtt gcc ggt ttc gat aat gaa gac cac 768

Gly Tyr His Pro Tyr Glu Phe Val Ala Gly Phe Asp Asn Glu Asp His
                245                 250                 255

atg tct atc cac aga aga ttc gct gaa ttg ttc gaa act atc ttc gat      816
Met Ser Ile His Arg Arg Phe Ala Glu Leu Phe Glu Thr Ile Phe Asp
            260                 265                 270

gaa att tgt gac ata aaa gct gct gct caa acc gat gac atg act aga      864
Glu Ile Cys Asp Ile Lys Ala Ala Ala Gln Thr Asp Asp Met Thr Arg
        275                 280                 285

cca ttc tac cct atg ttg att ttt aga act cca aag ggt tgg aca tgc      912
Pro Phe Tyr Pro Met Leu Ile Phe Arg Thr Pro Lys Gly Trp Thr Cys
    290                 295                 300

cct aag ttc atc gat ggt aaa aag aca gaa ggt tcc tgg aga gca cat      960
Pro Lys Phe Ile Asp Gly Lys Lys Thr Glu Gly Ser Trp Arg Ala His
305                 310                 315                 320

caa gtt cca tta gct agt gca aga gat acc gaa gaa cac ttt gaa gtc      1008
Gln Val Pro Leu Ala Ser Ala Arg Asp Thr Glu Glu His Phe Glu Val
                325                 330                 335

ttg aaa ggt tgg atg gaa tct tac aag cct gaa gaa tta ttc aat gca      1056
Leu Lys Gly Trp Met Glu Ser Tyr Lys Pro Glu Glu Leu Phe Asn Ala
            340                 345                 350

gat ggt tca att aaa gat gac gtt aca gcc ttt atg cca aag ggt gaa      1104
Asp Gly Ser Ile Lys Asp Asp Val Thr Ala Phe Met Pro Lys Gly Glu
        355                 360                 365

ttg aga ata ggt gcc aat cct aac gct aat ggt ggt gtt atc aga gaa      1152
Leu Arg Ile Gly Ala Asn Pro Asn Ala Asn Gly Gly Val Ile Arg Glu
    370                 375                 380

gat ttg aaa ttg cca gaa tta gac caa tat gaa gta act ggt gtt aag      1200
Asp Leu Lys Leu Pro Glu Leu Asp Gln Tyr Glu Val Thr Gly Val Lys
385                 390                 395                 400

gaa tac ggt cat ggt tgg ggt caa gtt gaa gcc cct aga gct ttg ggt      1248
Glu Tyr Gly His Gly Trp Gly Gln Val Glu Ala Pro Arg Ala Leu Gly
                405                 410                 415

gca tat tgt aga gat atc att aaa aat aac cca gac tcc ttt aga ata      1296
Ala Tyr Cys Arg Asp Ile Ile Lys Asn Asn Pro Asp Ser Phe Arg Ile
            420                 425                 430

ttc ggt cct gat gaa aca gct agt aac aga ttg aac gca act tat gaa      1344
Phe Gly Pro Asp Glu Thr Ala Ser Asn Arg Leu Asn Ala Thr Tyr Glu
        435                 440                 445

gta acc gat aag caa tgg gac aat ggt tac ttg tct ggt tta gtt gat      1392
Val Thr Asp Lys Gln Trp Asp Asn Gly Tyr Leu Ser Gly Leu Val Asp
    450                 455                 460

gaa cac atg gca gtc act ggt caa gta aca gaa caa tta tca gaa cac      1440
Glu His Met Ala Val Thr Gly Gln Val Thr Glu Gln Leu Ser Glu His
465                 470                 475                 480

caa tgc gaa ggt ttc ttg gaa gca tat ttg tta aca ggt aga cat ggt      1488
Gln Cys Glu Gly Phe Leu Glu Ala Tyr Leu Leu Thr Gly Arg His Gly
                485                 490                 495

att tgg tct tca tac gaa tct ttt gta cat gtt atc gat tca atg ttg 1536
Ile Trp Ser Ser Tyr Glu Ser Phe Val His Val Ile Asp Ser Met Leu
            500                 505                 510

aac caa cac gcc aaa tgg tta gaa gct act gtt aga gaa ata cct tgg 1584
Asn Gln His Ala Lys Trp Leu Glu Ala Thr Val Arg Glu Ile Pro Trp
        515                 520                 525

aga aag cct atc tcc agt gtt aac ttg tta gtc tct tca cat gta tgg 1632
Arg Lys Pro Ile Ser Ser Val Asn Leu Leu Val Ser Ser His Val Trp
    530                 535                 540

aga caa gat cat aat ggt ttt tct cac caa gac cca ggt gtc aca tca 1680
Arg Gln Asp His Asn Gly Phe Ser His Gln Asp Pro Gly Val Thr Ser
545                 550                 555                 560

ttg ttg att aat aag acc ttc aat aac gat cac gtt acc aac atc tat 1728
Leu Leu Ile Asn Lys Thr Phe Asn Asn Asp His Val Thr Asn Ile Tyr
                565                 570                 575

ttt gcc act gac gct aac atg ttg ttg gct atc tct gaa aag tgc ttc 1776
Phe Ala Thr Asp Ala Asn Met Leu Leu Ala Ile Ser Glu Lys Cys Phe
            580                 585                 590

aag tca act aac aaa atc aat gca ata ttc gcc ggt aaa caa cca gca 1824
Lys Ser Thr Asn Lys Ile Asn Ala Ile Phe Ala Gly Lys Gln Pro Ala
        595                 600                 605

cct aca tgg gtt acc ttg gat gaa gcc aga gct gaa tta gaa gct ggt 1872
Pro Thr Trp Val Thr Leu Asp Glu Ala Arg Ala Glu Leu Glu Ala Gly
    610                 615                 620

gct gct gaa tgg aaa tgg gct tct aat gca gaa aat aac gat gaa gtt 1920
Ala Ala Glu Trp Lys Trp Ala Ser Asn Ala Glu Asn Asn Asp Glu Val
625                 630                 635                 640

caa gtt gtc ttg gca tcc gcc ggt gac gtc cca aca caa gaa ttg atg 1968
Gln Val Val Leu Ala Ser Ala Gly Asp Val Pro Thr Gln Glu Leu Met
                645                 650                 655

gcc gct agt gat gct ttg aac aaa atg ggt att aag ttt aaa gta gtt 2016
Ala Ala Ser Asp Ala Leu Asn Lys Met Gly Ile Lys Phe Lys Val Val
            660                 665                 670

aac gtc gta gat ttg ttg aag tta caa tca aga gaa aac aac gat gaa 2064
Asn Val Val Asp Leu Leu Lys Leu Gln Ser Arg Glu Asn Asn Asp Glu
        675                 680                 685

gca ttg act gac gaa gag ttt act gaa ttg ttt act gct gat aaa cca 2112
Ala Leu Thr Asp Glu Glu Phe Thr Glu Leu Phe Thr Ala Asp Lys Pro
    690                 695                 700

gta ttg ttt gca tat cat tcc tac gcc caa gat gtt aga ggt ttg atc 2160
Val Leu Phe Ala Tyr His Ser Tyr Ala Gln Asp Val Arg Gly Leu Ile
705                 710                 715                 720

tat gat aga cca aac cat gac aat ttc cac gtt gtc ggt tac aaa gaa 2208
Tyr Asp Arg Pro Asn His Asp Asn Phe His Val Val Gly Tyr Lys Glu
                725                 730                 735

caa ggt tca acc act aca cct ttt gat atg gtc aga gta aat gat atg 2256
Gln Gly Ser Thr Thr Thr Pro Phe Asp Met Val Arg Val Asn Asp Met
            740                 745                 750

gac aga tat gca ttg caa gca gcc gct ttg aag tta att gat gca gac 2304
Asp Arg Tyr Ala Leu Gln Ala Ala Ala Leu Lys Leu Ile Asp Ala Asp
        755                 760                 765

aaa tac gcc gat aag atc gac gaa tta aac gct ttt aga aag aaa gca 2352
Lys Tyr Ala Asp Lys Ile Asp Glu Leu Asn Ala Phe Arg Lys Lys Ala
    770                 775                 780

ttt caa ttc gca gtc gat aat ggt tat gac att cca gag ttt act gat 2400
Phe Gln Phe Ala Val Asp Asn Gly Tyr Asp Ile Pro Glu Phe Thr Asp
785                 790                 795                 800

tgg gta tac cct gat gtt aag gtt gat gaa aca caa atg ttg tct gct 2448
Trp Val Tyr Pro Asp Val Lys Val Asp Glu Thr Gln Met Leu Ser Ala
                805                 810                 815

act gct gct act gct ggt gac aat gaa taa 2478
Thr Ala Ala Thr Ala Gly Asp Asn Glu
            820                 825

<210> 12
<211> 825
<212> PRT
<213> Bifidobacterium breve

<400> 12

Met Thr Asn Pro Val Ile Gly Thr Pro Trp Gln Lys Leu Asp Arg Pro
1               5                   10                  15

Val Ser Glu Glu Ala Ile Glu Gly Met Asp Lys Tyr Trp Arg Val Thr
            20                  25                  30

Asn Tyr Met Ser Ile Gly Gln Ile Tyr Leu Arg Ser Asn Pro Leu Met
        35                  40                  45

Lys Glu Pro Phe Thr Arg Asp Asp Val Lys His Arg Leu Val Gly His
    50                  55                  60

Trp Gly Thr Thr Pro Gly Leu Asn Phe Leu Leu Ala His Ile Asn Arg
65                  70                  75                  80

Leu Ile Ala Asp His Gln Gln Asn Thr Val Phe Ile Met Gly Pro Gly
                85                  90                  95

His Gly Gly Pro Ala Gly Thr Ser Gln Ser Tyr Val Asp Gly Thr Tyr
            100                 105                 110

Thr Glu Tyr Tyr Pro Asn Ile Thr Lys Asp Glu Ala Gly Leu Gln Lys
        115                 120                 125

Phe Phe Arg Gln Phe Ser Tyr Pro Gly Gly Ile Pro Ser His Phe Ala
    130                 135                 140

Pro Glu Thr Pro Gly Ser Ile His Glu Gly Gly Glu Leu Gly Tyr Ala
145                 150                 155                 160

Leu Ser His Ala Tyr Gly Ala Val Met Asn Asn Pro Ser Leu Phe Val
                165                 170                 175

Pro Cys Ile Ile Gly Asp Gly Glu Ala Glu Thr Gly Pro Leu Ala Thr
            180                 185                 190

Gly Trp Gln Ser Asn Lys Leu Val Asn Pro Arg Thr Asp Gly Ile Val
        195                 200                 205

Leu Pro Ile Leu His Leu Asn Gly Tyr Lys Ile Ala Asn Pro Thr Ile
    210                 215                 220

Leu Ala Arg Ile Ser Asp Glu Glu Leu His Asp Phe Phe Arg Gly Met
225                 230                 235                 240

Gly Tyr His Pro Tyr Glu Phe Val Ala Gly Phe Asp Asn Glu Asp His
                245                 250                 255

Met Ser Ile His Arg Arg Phe Ala Glu Leu Phe Glu Thr Ile Phe Asp
            260                 265                 270

Glu Ile Cys Asp Ile Lys Ala Ala Ala Gln Thr Asp Asp Met Thr Arg
        275                 280                 285

Pro Phe Tyr Pro Met Leu Ile Phe Arg Thr Pro Lys Gly Trp Thr Cys
    290                 295                 300

Pro Lys Phe Ile Asp Gly Lys Lys Thr Glu Gly Ser Trp Arg Ala His
305                 310                 315                 320

Gln Val Pro Leu Ala Ser Ala Arg Asp Thr Glu Glu His Phe Glu Val
                325                 330                 335

Leu Lys Gly Trp Met Glu Ser Tyr Lys Pro Glu Glu Leu Phe Asn Ala
            340                 345                 350

Asp Gly Ser Ile Lys Asp Asp Val Thr Ala Phe Met Pro Lys Gly Glu
        355                 360                 365

Leu Arg Ile Gly Ala Asn Pro Asn Ala Asn Gly Gly Val Ile Arg Glu
    370                 375                 380

Asp Leu Lys Leu Pro Glu Leu Asp Gln Tyr Glu Val Thr Gly Val Lys

385                 390                 395                 400

Glu Tyr Gly His Gly Trp Gly Gln Val Glu Ala Pro Arg Ala Leu Gly
                405                 410                 415

Ala Tyr Cys Arg Asp Ile Ile Lys Asn Asn Pro Asp Ser Phe Arg Ile
            420                 425                 430

Phe Gly Pro Asp Glu Thr Ala Ser Asn Arg Leu Asn Ala Thr Tyr Glu
        435                 440                 445

Val Thr Asp Lys Gln Trp Asp Asn Gly Tyr Leu Ser Gly Leu Val Asp
    450                 455                 460

Glu His Met Ala Val Thr Gly Gln Val Thr Glu Gln Leu Ser Glu His
465                 470                 475                 480

Gln Cys Glu Gly Phe Leu Glu Ala Tyr Leu Leu Thr Gly Arg His Gly
                485                 490                 495

Ile Trp Ser Ser Tyr Glu Ser Phe Val His Val Ile Asp Ser Met Leu
            500                 505                 510

Asn Gln His Ala Lys Trp Leu Glu Ala Thr Val Arg Glu Ile Pro Trp
        515                 520                 525

Arg Lys Pro Ile Ser Ser Val Asn Leu Leu Val Ser Ser His Val Trp
    530                 535                 540

Arg Gln Asp His Asn Gly Phe Ser His Gln Asp Pro Gly Val Thr Ser
545                 550                 555                 560

Leu Leu Ile Asn Lys Thr Phe Asn Asn Asp His Val Thr Asn Ile Tyr
                565                 570                 575

Phe Ala Thr Asp Ala Asn Met Leu Leu Ala Ile Ser Glu Lys Cys Phe
            580                 585                 590

Lys Ser Thr Asn Lys Ile Asn Ala Ile Phe Ala Gly Lys Gln Pro Ala
        595                 600                 605

Pro Thr Trp Val Thr Leu Asp Glu Ala Arg Ala Glu Leu Glu Ala Gly
    610                 615                 620

Ala Ala Glu Trp Lys Trp Ala Ser Asn Ala Glu Asn Asn Asp Glu Val
625                 630                 635                 640

Gln Val Val Leu Ala Ser Ala Gly Asp Val Pro Thr Gln Glu Leu Met
                645                 650                 655

Ala Ala Ser Asp Ala Leu Asn Lys Met Gly Ile Lys Phe Lys Val Val
            660                 665                 670

Asn Val Val Asp Leu Leu Lys Leu Gln Ser Arg Glu Asn Asn Asp Glu
        675                 680                 685

Ala Leu Thr Asp Glu Glu Phe Thr Glu Leu Phe Thr Ala Asp Lys Pro
    690                 695                 700

Val Leu Phe Ala Tyr His Ser Tyr Ala Gln Asp Val Arg Gly Leu Ile
705                 710                 715                 720

Tyr Asp Arg Pro Asn His Asp Asn Phe His Val Val Gly Tyr Lys Glu
                725                 730                 735

Gln Gly Ser Thr Thr Thr Pro Phe Asp Met Val Arg Val Asn Asp Met
            740                 745                 750

Asp Arg Tyr Ala Leu Gln Ala Ala Ala Leu Lys Leu Ile Asp Ala Asp
        755                 760                 765

Lys Tyr Ala Asp Lys Ile Asp Glu Leu Asn Ala Phe Arg Lys Lys Ala
    770                 775                 780

Phe Gln Phe Ala Val Asp Asn Gly Tyr Asp Ile Pro Glu Phe Thr Asp
785                 790                 795                 800

Trp Val Tyr Pro Asp Val Lys Val Asp Glu Thr Gln Met Leu Ser Ala
                805                 810                 815

Thr Ala Ala Thr Ala Gly Asp Asn Glu
            820                 825

<210>  13
<211>  825
<212>  PRT
<213>  Bifidobacterium cuniculi

<220>
<221>  POLYPEPTIDE
<222>  (1)..(825)
<223>  Phosphoketolase

<300>
<308>  NCBI Reference Sequence: WP_033516398.1
<309>  2018-08-20
<313>  (1)..(825)

<400>  13

Met Thr Asn Pro Val Ile Gly Thr Pro Trp Ala Lys Leu Asp Arg Pro
1               5                   10                  15

Val Ser Glu Glu Ala Ile Glu Gly Met Asp Lys Tyr Trp Arg Val Thr
            20                  25                  30

Asn Tyr Met Ser Ile Gly Gln Ile Tyr Leu Arg Ser Asn Pro Leu Met
        35                  40                  45

Lys Glu Pro Phe Thr Arg Asp Asp Val Lys His Arg Leu Val Gly His
    50                  55                  60

Trp Gly Thr Thr Pro Gly Leu Asn Phe Leu Leu Ala His Ile Asn Arg
65                  70                  75                  80

Leu Ile Ala Asp His Gln Gln Asn Thr Val Phe Ile Met Gly Pro Gly
                85                  90                  95

His Gly Gly Pro Ala Gly Thr Ala Gln Ser Tyr Val Asp Gly Thr Tyr
            100                 105                 110

Thr Glu Tyr Tyr Pro Asn Ile Thr Lys Asp Glu Ala Gly Leu Gln Lys
        115                 120                 125

Phe Phe Arg Gln Phe Ser Tyr Pro Gly Gly Ile Pro Ser His Phe Ala
    130                 135                 140

Pro Glu Thr Pro Gly Ser Ile His Glu Gly Gly Glu Leu Gly Tyr Ala
145                 150                 155                 160

Leu Ser His Ala Tyr Gly Ala Val Met Asn Asn Pro Ser Leu Phe Val
                165                 170                 175

Pro Cys Ile Ile Gly Asp Gly Glu Ala Glu Thr Gly Pro Leu Ala Thr
            180                 185                 190

Gly Trp Gln Ser Asn Lys Leu Ile Asn Pro Arg Thr Asp Gly Ile Val
        195                 200                 205

Leu Pro Ile Leu His Leu Asn Gly Tyr Lys Ile Ala Asn Pro Thr Ile
    210                 215                 220

Leu Ala Arg Ile Ser Asp Glu Glu Leu His Asp Phe Phe Arg Gly Met
225                 230                 235                 240

Gly Tyr His Pro Tyr Glu Phe Val Ala Gly Phe Asp Asn Glu Asp His
                245                 250                 255

Leu Ser Ile His Arg Arg Phe Ala Glu Leu Phe Glu Thr Val Phe Asp
            260                 265                 270

Glu Ile Cys Asp Ile Lys Ala Ala Ala Gln Thr Asp Asp Met Ser Arg
        275                 280                 285

Pro Phe Tyr Pro Met Ile Ile Phe Arg Thr Pro Lys Gly Trp Thr Cys
    290                 295                 300

Pro Lys Phe Ile Asp Gly Lys Lys Thr Glu Gly Ser Trp Arg Ala His
305                 310                 315                 320

Gln Val Pro Leu Ala Ser Ala Arg Asp Thr Glu Glu His Phe Glu Val
                325                 330                 335

Leu Lys Glu Trp Met Glu Ser Tyr Lys Pro Glu Glu Leu Phe Asn Glu
            340                 345                 350

Asp Gly Ser Ile Lys Glu Asp Val Thr Glu Phe Met Pro Lys Gly Glu
        355                 360                 365

Leu Arg Ile Gly Ala Asn Pro Asn Ala Asn Gly Gly Val Ile Arg Glu
    370                 375                 380

Asp Leu Lys Leu Pro Asn Leu Asp Asp Tyr Glu Val Thr Gly Val Lys
385                 390                 395                 400

Glu Tyr Gly His Gly Trp Gly Gln Val Glu Ala Pro Arg Ser Leu Gly
                405                 410                 415

Ala Tyr Cys Arg Asp Ile Ile Lys Asn Asn Pro Asp Ser Phe Arg Ile
            420                 425                 430

Phe Gly Pro Asp Glu Thr Ala Ser Asn Arg Leu Asn Ala Thr Tyr Glu
        435                 440                 445

Val Thr Lys Lys Gln Trp Asp Asn Gly Tyr Leu Ser Ala Leu Val Asp
    450                 455                 460

Glu Asn Met Ala Val Thr Gly Gln Val Thr Glu Gln Leu Ser Glu His
465                 470                 475                 480

Gln Cys Glu Gly Phe Leu Glu Ala Tyr Leu Leu Thr Gly Arg His Gly
                485                 490                 495

Ile Trp Ser Ser Tyr Glu Ser Phe Val His Val Ile Asp Ser Met Leu
        500                 505                 510

Asn Gln His Ala Lys Trp Leu Glu Ala Thr Val Arg Glu Ile Pro Trp
    515                 520                 525

Arg Lys Pro Ile Ser Ser Met Asn Leu Leu Val Ser Ser His Val Trp
    530                 535                 540

Arg Gln Asp His Asn Gly Phe Ser His Gln Asp Pro Gly Val Thr Ser
545                 550                 555                 560

Leu Leu Ile Asn Lys Thr Phe Asn Asn Asp His Val Thr Asn Ile Tyr
                565                 570                 575

Phe Ala Thr Asp Ala Asn Met Leu Leu Ala Ile Ser Glu Lys Cys Phe
            580                 585                 590

Lys Ser Thr Asn Ala Ile Asn Ala Ile Phe Ala Gly Lys Gln Pro Ala
        595                 600                 605

Pro Thr Trp Val Thr Leu Asp Glu Ala Arg Ala Glu Leu Glu Ala Gly
    610                 615                 620

Ala Ala Glu Trp Lys Trp Ala Ser Asn Ala Asp Asn Asn Asp Glu Val
625                 630                 635                 640

Gln Val Val Leu Ala Ala Ala Gly Asp Val Pro Thr Gln Glu Thr Met
                645                 650                 655

Ala Ala Ala Asp Ala Leu Asn Gln Met Gly Ile Lys Phe Lys Val Val
            660                 665                 670

Asn Val Val Asp Leu Leu Lys Leu Gln Ser Gln Glu Asn Asn Asp Glu
        675                 680                 685

Ala Met Ser Asp Glu Asp Phe Ala Glu Leu Phe Thr Ala Asp Lys Pro
    690                 695                 700

Val Leu Phe Ala Tyr His Ser Tyr Ala Gln Asp Val Arg Gly Leu Ile
705                 710                 715                 720

Tyr Asp Arg Pro Asn His Asp Asn Phe His Val Val Gly Tyr Lys Glu
                725                 730                 735

Gln Gly Ser Thr Thr Thr Pro Phe Asp Met Val Arg Val Asn Asp Met
            740                 745                 750

Asp Arg Tyr Ala Leu Gln Ala Glu Ala Leu Arg Leu Ile Asp Ala Asp
        755                 760                 765

Lys Tyr Ala Asp Lys Ile Asp Glu Leu Asn Ala Phe Arg Lys Lys Ala
    770                 775                 780

Phe Gln Phe Ala Val Asp Asn Gly Tyr Asp Ile Pro Glu Phe Thr Asp
785                 790                 795                 800

Trp Val Trp Pro Gly Val Asn Thr Asp Lys Gln Gly Ala Ile Ser Ala
                805                 810                 815

Thr Ala Ala Thr Ala Gly Asp Asn Glu
            820                 825

<210>  14
<211>  825
<212>  PRT
<213>  Bifidobacterium pseudolongum

<220>
<221>  POLYPEPTIDE
<222>  (1)..(825)
<223>  phosphoketolase

<300>
<308>  NCBI Reference Sequence: WP_101428637.1
<309>  2018-08-20
<313>  (1)..(825)

<400>  14

Met Thr Asn Pro Val Ile Gly Thr Pro Trp Gln Lys Leu Asp Arg Pro
1               5                   10                  15

Val Ser Glu Glu Ala Ile Glu Gly Met Asp Lys Tyr Trp Arg Val Thr
            20                  25                  30

Asn Tyr Met Ser Ile Gly Gln Ile Tyr Leu Arg Ser Asn Pro Leu Met
        35                  40                  45

Lys Glu Pro Phe Thr Arg Asp Asp Val Lys His Arg Leu Val Gly His
    50                  55                  60

Trp Gly Thr Thr Pro Gly Leu Asn Phe Leu Leu Ala His Ile Asn Arg
65                  70                  75                  80

Leu Ile Ala Asp His Gln Gln Asn Thr Val Phe Ile Met Gly Pro Gly
                85                  90                  95

His Gly Gly Pro Ala Gly Thr Ser Gln Ser Tyr Val Asp Gly Thr Tyr
            100                 105                 110

Thr Glu Tyr Tyr Pro Asn Ile Thr Lys Asp Glu Ala Gly Leu Gln Lys
        115                 120                 125

Phe Phe Arg Gln Phe Ser Tyr Pro Gly Gly Ile Pro Ser His Phe Ala
    130                 135                 140

Pro Glu Thr Pro Gly Ser Ile His Glu Gly Gly Glu Leu Gly Tyr Ala
145                 150                 155                 160

Leu Ser His Ala Tyr Gly Ala Val Met Asn Asn Pro Ser Leu Phe Val
                165                 170                 175

Pro Cys Ile Ile Gly Asp Gly Glu Ala Glu Thr Gly Pro Leu Ala Thr
            180                 185                 190

Gly Trp Gln Ser Asn Lys Leu Val Asn Pro Arg Thr Asp Gly Ile Val
        195                 200                 205

Leu Pro Ile Leu His Leu Asn Gly Tyr Lys Ile Ala Asn Pro Thr Ile
    210                 215                 220

Leu Ala Arg Ile Ser Asp Glu Glu Leu His Asp Phe Phe Arg Gly Met
225                 230                 235                 240

Gly Tyr His Pro Tyr Glu Phe Val Ala Gly Phe Asp Asn Glu Asp His
                245                 250                 255

Met Ser Ile His Arg Arg Phe Ala Glu Leu Phe Glu Thr Ile Phe Asp
            260                 265                 270

Glu Ile Cys Asp Ile Lys Ala Ala Ala Gln Thr Asp Asp Met Thr Arg
        275                 280                 285

Pro Phe Tyr Pro Met Leu Ile Phe Arg Thr Pro Lys Gly Trp Thr Cys
    290                 295                 300

Pro Lys Phe Ile Asp Gly Lys Lys Thr Glu Gly Ser Trp Arg Ala His
305                 310                 315                 320

Gln Val Pro Leu Ala Ser Ala Arg Asp Thr Glu Glu His Phe Glu Val
                325                 330                 335

Leu Lys Gly Trp Met Glu Ser Tyr Lys Pro Glu Glu Leu Phe Asn Ala
            340                 345                 350

Asp Gly Ser Ile Lys Asp Asp Val Thr Ala Phe Met Pro Lys Gly Glu
        355                 360                 365

Leu Arg Ile Gly Ala Asn Pro Asn Ala Asn Gly Gly Val Ile Arg Glu
    370                 375                 380

Asp Leu Lys Leu Pro Glu Leu Asp Gln Tyr Glu Val Thr Gly Val Lys
385                 390                 395                 400

Glu Tyr Gly His Gly Trp Gly Gln Val Glu Ala Pro Arg Ala Leu Gly
                405                 410                 415

Ala Tyr Cys Arg Asp Ile Ile Lys Asn Asn Pro Asp Ser Phe Arg Ile
            420                 425                 430

Phe Gly Pro Asp Glu Thr Ala Ser Asn Arg Leu Asn Ala Thr Tyr Glu
        435                 440                 445

Val Thr Asp Lys Gln Trp Asp Asn Gly Tyr Leu Ser Gly Leu Val Asp
    450                 455                 460

Glu His Met Ala Val Thr Gly Gln Val Thr Glu Gln Leu Ser Glu His
465                 470                 475                 480

Gln Cys Glu Gly Phe Leu Glu Ala Tyr Leu Leu Thr Gly Arg His Gly
                485                 490                 495

Ile Trp Ser Ser Tyr Glu Ser Phe Val His Val Ile Asp Ser Met Leu
            500                 505                 510

Asn Gln His Ala Lys Trp Leu Glu Ala Thr Val Arg Glu Ile Pro Trp
        515                 520                 525

Arg Lys Pro Ile Ser Ser Val Asn Leu Leu Val Ser Ser His Val Trp
    530                 535                 540

Arg Gln Asp His Asn Gly Phe Ser His Gln Asp Pro Gly Val Thr Ser
545                 550                 555                 560

Leu Leu Ile Asn Lys Thr Phe Asn Asn Asp His Val Thr Asn Ile Tyr
                565                 570                 575

Phe Ala Thr Asp Ala Asn Met Leu Leu Ala Ile Ser Glu Lys Cys Phe
            580                 585                 590

Lys Ser Thr Asn Lys Ile Asn Ala Ile Phe Ala Gly Lys Gln Pro Ala

595                 600                 605

Pro Thr Trp Val Thr Leu Asp Glu Ala Arg Ala Glu Leu Glu Ala Gly
    610                 615                 620

Ala Ala Glu Trp Lys Trp Ala Ser Asn Ala Glu Asn Asn Asp Glu Val
625                 630                 635                 640

Gln Val Val Leu Ala Ser Ala Gly Asp Val Pro Thr Gln Glu Leu Met
                645                 650                 655

Ala Ala Ser Asp Ala Leu Asn Lys Met Gly Ile Lys Phe Lys Val Val
            660                 665                 670

Asn Val Val Asp Leu Leu Lys Leu Gln Ser Arg Glu Asn Asn Asp Glu
        675                 680                 685

Ala Leu Thr Asp Glu Glu Phe Thr Glu Leu Phe Thr Ala Asp Lys Pro
    690                 695                 700

Val Leu Phe Ala Tyr His Ser Tyr Ala Gln Asp Val Arg Gly Leu Ile
705                 710                 715                 720

Tyr Asp Arg Pro Asn His Asp Asn Phe His Val Val Gly Tyr Lys Glu
                725                 730                 735

Gln Gly Ser Thr Thr Thr Pro Phe Asp Met Val Arg Val Asn Asp Met
            740                 745                 750

Asp Arg Tyr Ala Leu Gln Ala Ala Ala Leu Lys Leu Ile Asp Ala Asp
        755                 760                 765

Lys Tyr Ala Asp Lys Ile Asp Glu Leu Asn Ala Phe Arg Lys Lys Ala
    770                 775                 780

Phe Gln Phe Ala Val Asp Asn Gly Tyr Asp Ile Pro Glu Phe Thr Asp
785                 790                 795                 800

Trp Val Tyr Pro Asp Val Lys Val Asp Glu Thr Gln Met Leu Ser Ala
                805                 810                 815

Thr Ala Ala Thr Ala Gly Asp Asn Glu
            820                 825

<210>  15
<211>  825
<212>  PRT
<213>  Bifidobacterium thermophilum

<220>
<221>  POLYPEPTIDE
<222>  (1)..(825)
<223>  phosphoketolase

<300>
<308>  NCBI Reference Sequence: WP_101452099.1
<309>  2018-08-20
<313>  (1)..(825)

<400>  15

Met Thr Asn Pro Val Ile Gly Thr Pro Trp Lys Lys Leu Asp Ala Pro
1               5                   10                  15

Val Ser Glu Glu Ala Leu Glu Gly Val Asp Lys Tyr Trp Arg Thr Ala
            20                  25                  30

Asn Tyr Leu Ser Ile Gly Gln Ile Tyr Leu Arg Ser Asn Pro Leu Met
        35                  40                  45

Lys Glu Pro Phe Thr Arg Glu Asp Val Lys His Arg Leu Val Gly His
    50                  55                  60

Trp Gly Thr Thr Pro Gly Leu Asn Phe Leu Ile Gly His Ile Asn Arg
65                  70                  75                  80

Leu Ile Ala Asp His Gln Gln Asn Thr Val Ile Ile Met Gly Pro Gly
                85                  90                  95

His Gly Gly Pro Ala Gly Thr Ala Gln Ser Tyr Leu Asp Gly Thr Tyr
            100                 105                 110

Thr Glu Tyr Tyr Pro Asn Ile Thr Lys Asp Glu Ala Gly Leu Gln Lys
        115                 120                 125

Phe Phe Arg Gln Phe Ser Tyr Pro Gly Gly Ile Pro Ser His Phe Ala
    130                 135                 140

Pro Glu Thr Pro Gly Ser Ile His Glu Gly Gly Glu Leu Gly Tyr Ala
145                 150                 155                 160

Leu Ser His Ala Tyr Gly Ala Val Met Asn Asn Pro Ser Leu Phe Val
                165                 170                 175

Pro Ala Ile Val Gly Asp Gly Glu Ala Glu Thr Gly Pro Leu Ala Thr
            180                 185                 190

Gly Trp Gln Ser Asn Lys Leu Ile Asn Pro Arg Thr Asp Gly Ile Val

195                 200                 205

Leu Pro Ile Leu His Leu Asn Gly Tyr Lys Ile Ala Asn Pro Thr Ile
    210                 215                 220

Leu Ser Arg Ile Ser Asp Glu Glu Leu His Glu Phe Phe His Gly Met
225                 230                 235                 240

Gly Tyr Glu Pro Tyr Glu Phe Val Ala Gly Phe Asp Asp Glu Asp His
                245                 250                 255

Met Ser Ile His Arg Arg Phe Ala Asp Leu Phe Glu Ser Val Phe Asp
            260                 265                 270

Glu Ile Cys Asp Ile Lys Ala Ala Ala Gln Thr Asp Asp Met Thr Arg
        275                 280                 285

Pro Phe Tyr Pro Met Ile Ile Phe Arg Thr Pro Lys Gly Trp Thr Cys
    290                 295                 300

Pro Lys Phe Ile Asp Gly Lys Lys Thr Glu Gly Ser Trp Arg Ala His
305                 310                 315                 320

Gln Val Pro Leu Ala Ser Ala Arg Asp Thr Glu Glu His Phe Gln Val
                325                 330                 335

Leu Lys Asn Trp Leu Glu Ser Tyr Lys Pro Glu Glu Leu Phe Asp Asp
            340                 345                 350

Lys Gly Ala Val Arg Pro Glu Val Thr Ala Phe Met Pro Lys Gly Glu
        355                 360                 365

Leu Arg Ile Gly Gln Asn Pro Asn Ala Asn Gly Gly Arg Ile Arg Glu
    370                 375                 380

Glu Leu Asn Leu Pro Lys Leu Glu Asp Tyr Glu Val Lys Glu Val Ser
385                 390                 395                 400

Glu Tyr Gly His Gly Trp Gly Gln Leu Glu Ala Thr Arg Arg Leu Gly
                405                 410                 415

Val Tyr Cys Arg Asp Ile Ile Lys Asn Asn Pro Asp Ser Phe Arg Ile
            420                 425                 430

Phe Gly Pro Asp Glu Thr Ala Ser Asn Arg Leu Ala Ala Ala Tyr Glu
        435                 440                 445

Val Thr Asn Lys Gln Trp Asp Ala Gly Tyr Leu Ser Ala Gln Val Asp
    450                 455                 460

Glu His Met Ala Val Thr Gly Gln Val Thr Glu Gln Leu Ser Glu His
465                 470                 475                 480

Gln Met Glu Gly Phe Leu Glu Gly Tyr Leu Leu Thr Gly Arg His Gly
                485                 490                 495

Ile Trp Ser Ser Tyr Glu Ser Phe Val His Val Ile Asp Ser Met Leu
            500                 505                 510

Asn Gln His Ala Lys Trp Leu Glu Ala Thr Val Arg Glu Ile Pro Trp
        515                 520                 525

Arg Lys Pro Ile Ser Ser Met Asn Leu Leu Val Ser Ser His Val Trp
    530                 535                 540

Arg Gln Asp His Asn Gly Phe Ser His Gln Asp Pro Gly Val Thr Ser
545                 550                 555                 560

Val Leu Leu Asn Lys Cys Phe Asn Asn Asp His Val Ile Gly Ile Tyr
                565                 570                 575

Phe Pro Val Asp Ser Asn Met Leu Leu Ala Val Val Glu Lys Cys Phe
            580                 585                 590

Lys Ser Thr Asn Met Ile Asn Ala Ile Ile Ala Gly Lys Gln Pro Ala
        595                 600                 605

Ala Thr Trp Leu Ser Leu Asp Glu Ala Arg Ala Glu Leu Glu Lys Gly
    610                 615                 620

Ala Ala Glu Trp Lys Trp Ala Ser Asn Val Lys Ser Asn Asp Glu Ala
625                 630                 635                 640

Asp Ile Val Leu Ala Ser Val Gly Asp Val Pro Thr Gln Glu Ile Met
                645                 650                 655

Ala Ala Ala Asp Lys Leu Asn Lys Leu Gly Ile Lys Phe Lys Val Val
            660                 665                 670

Asn Val Val Asp Leu Val Lys Leu Gln Ser Ala Lys Glu Asn Asn Glu
        675                 680                 685

Ala Leu Thr Asp Glu Glu Phe Ala Asp Leu Phe Thr Thr Asp Lys Pro
    690                 695                 700

Val Leu Phe Ala Tyr His Ser Tyr Ala Arg Asp Val Arg Gly Leu Ile
705                 710                 715                 720

Tyr Asp Arg Pro Asn His Asp Asn Phe Asn Val His Gly Tyr Glu Glu
                725                 730                 735

Gln Gly Ser Thr Thr Thr Pro Tyr Asp Met Val Arg Val Asn Asn Ile
            740                 745                 750

Asp Arg Tyr Glu Leu Thr Ala Glu Ala Leu Arg Met Val Asp Ala Asp
        755                 760                 765

Lys Tyr Ala Ala Glu Ile Asp Lys Leu Glu Ala Phe Arg Asp Glu Ala
    770                 775                 780

Phe Gln Phe Ala Val Asp Lys Gly Tyr Asp His Pro Asp Tyr Thr Asp
785                 790                 795                 800

Trp Val Trp Pro Gly Val Asn Thr Ala Lys Gln Gly Ala Val Thr Ala
                805                 810                 815

Thr Ala Ala Thr Ala Gly Asp Asn Glu
            820                 825

<210>  16
<211>  1002
<212>  DNA
<213>  Clostridiun kluveri

<220>
<221>  CDS
<222>  (1)..(1002)
<223>  Gene encoding phosphate acyltransferase from Clostridiun kluveri
       codon optimized for yeast

<400>  16
atg aag ttg atg gaa aac atc ttc ggt ttg gcc aag gct gat aaa aag      48
Met Lys Leu Met Glu Asn Ile Phe Gly Leu Ala Lys Ala Asp Lys Lys
1               5                   10                  15

aaa ata gtt ttg gct gaa ggt gaa gaa gaa aga aac atc aga gca tcc      96
Lys Ile Val Leu Ala Glu Gly Glu Glu Glu Arg Asn Ile Arg Ala Ser
            20                  25                  30

gaa gaa att ata aga gac ggt att gcc gat atc att ttg gtt ggt tcc      144
Glu Glu Ile Ile Arg Asp Gly Ile Ala Asp Ile Ile Leu Val Gly Ser
        35                  40                  45

gaa agt gtc atc aag gaa aat gct gca aaa ttc ggt gtt aac tta gct      192
Glu Ser Val Ile Lys Glu Asn Ala Ala Lys Phe Gly Val Asn Leu Ala
    50                  55                  60

ggt gtt gaa ata gtc gat cca gaa act tct tca aaa aca gca ggt tac      240

Gly Val Glu Ile Val Asp Pro Glu Thr Ser Ser Lys Thr Ala Gly Tyr
65                  70                  75                  80

gca aac gcc ttc tac gaa atc aga aag aac aaa ggt gtt aca tta gaa      288
Ala Asn Ala Phe Tyr Glu Ile Arg Lys Asn Lys Gly Val Thr Leu Glu
                85                  90                  95

aag gcc gac aaa ata gtc aga gat cct atc tat ttc gca acc atg atg      336
Lys Ala Asp Lys Ile Val Arg Asp Pro Ile Tyr Phe Ala Thr Met Met
            100                 105                 110

gtt aag ttg ggt gac gcc gac ggt tta gtt tca ggt gct att cat act      384
Val Lys Leu Gly Asp Ala Asp Gly Leu Val Ser Gly Ala Ile His Thr
        115                 120                 125

act ggt gac ttg tta aga cca ggt ttg caa ata gta aaa act gtt cct      432
Thr Gly Asp Leu Leu Arg Pro Gly Leu Gln Ile Val Lys Thr Val Pro
    130                 135                 140

ggt gct tcc gtt gtc tcc agt gta ttt tta atg agt gtt cca gac tgt      480
Gly Ala Ser Val Val Ser Ser Val Phe Leu Met Ser Val Pro Asp Cys
145                 150                 155                 160

gaa tac ggt gaa gat ggt ttc ttg tta ttc gcc gat tgt gct gtc aat      528
Glu Tyr Gly Glu Asp Gly Phe Leu Leu Phe Ala Asp Cys Ala Val Asn
                165                 170                 175

gta tgc cct acc gca gaa gaa ttg tct tca ata gcc atc acc act gct      576
Val Cys Pro Thr Ala Glu Glu Leu Ser Ser Ile Ala Ile Thr Thr Ala
            180                 185                 190

gaa act gcc aag aac ttg tgc aaa att gaa cca aga gtt gct atg tta      624
Glu Thr Ala Lys Asn Leu Cys Lys Ile Glu Pro Arg Val Ala Met Leu
        195                 200                 205

tct ttc tca aca atg ggt tcc gca agt cac gaa tta gtc gat aaa gta      672
Ser Phe Ser Thr Met Gly Ser Ala Ser His Glu Leu Val Asp Lys Val
    210                 215                 220

aca aaa gct acc aag ttg gct aaa gaa gca aga cca gat ttg gat att      720
Thr Lys Ala Thr Lys Leu Ala Lys Glu Ala Arg Pro Asp Leu Asp Ile
225                 230                 235                 240

gat ggt gaa ttg caa tta gac gca tct ttg gtt aag aaa gtc gct gat      768
Asp Gly Glu Leu Gln Leu Asp Ala Ser Leu Val Lys Lys Val Ala Asp
                245                 250                 255

ttg aaa gca cca ggt tca aag gtc gcc ggt aaa gct aat gta ttg atc      816
Leu Lys Ala Pro Gly Ser Lys Val Ala Gly Lys Ala Asn Val Leu Ile
            260                 265                 270

ttt cct gac att caa gct ggt aac atc ggt tac aag tta gtt caa aga      864
Phe Pro Asp Ile Gln Ala Gly Asn Ile Gly Tyr Lys Leu Val Gln Arg
        275                 280                 285

ttc gct aaa gca gaa gcc att ggt cca ata tgt caa ggt ttc gct aag      912
Phe Ala Lys Ala Glu Ala Ile Gly Pro Ile Cys Gln Gly Phe Ala Lys
    290                 295                 300

cct ata aat gat ttg tca aga ggt tgt tct gtc gat gac atc gta aaa      960
Pro Ile Asn Asp Leu Ser Arg Gly Cys Ser Val Asp Asp Ile Val Lys
305                 310                 315                 320

gta gtt gca gta act gcc gtt caa gct caa gca caa ggt taa                    1002
Val Val Ala Val Thr Ala Val Gln Ala Gln Ala Gln Gly
                325                 330

<210>  17
<211>  333
<212>  PRT
<213>  Clostridiun kluveri

<400>  17

Met Lys Leu Met Glu Asn Ile Phe Gly Leu Ala Lys Ala Asp Lys Lys
1               5                   10                  15

Lys Ile Val Leu Ala Glu Gly Glu Glu Glu Arg Asn Ile Arg Ala Ser
            20                  25                  30

Glu Glu Ile Ile Arg Asp Gly Ile Ala Asp Ile Ile Leu Val Gly Ser
        35                  40                  45

Glu Ser Val Ile Lys Glu Asn Ala Ala Lys Phe Gly Val Asn Leu Ala
    50                  55                  60

Gly Val Glu Ile Val Asp Pro Glu Thr Ser Ser Lys Thr Ala Gly Tyr
65                  70                  75                  80

Ala Asn Ala Phe Tyr Glu Ile Arg Lys Asn Lys Gly Val Thr Leu Glu
                85                  90                  95

Lys Ala Asp Lys Ile Val Arg Asp Pro Ile Tyr Phe Ala Thr Met Met
            100                 105                 110

Val Lys Leu Gly Asp Ala Asp Gly Leu Val Ser Gly Ala Ile His Thr
        115                 120                 125

Thr Gly Asp Leu Leu Arg Pro Gly Leu Gln Ile Val Lys Thr Val Pro
    130                 135                 140

Gly Ala Ser Val Val Ser Ser Val Phe Leu Met Ser Val Pro Asp Cys
145                 150                 155                 160

Glu Tyr Gly Glu Asp Gly Phe Leu Leu Phe Ala Asp Cys Ala Val Asn
                165                 170                 175

Val Cys Pro Thr Ala Glu Glu Leu Ser Ser Ile Ala Ile Thr Thr Ala
            180                 185                 190

Glu Thr Ala Lys Asn Leu Cys Lys Ile Glu Pro Arg Val Ala Met Leu
        195                 200                 205

Ser Phe Ser Thr Met Gly Ser Ala Ser His Glu Leu Val Asp Lys Val
    210                 215                 220

Thr Lys Ala Thr Lys Leu Ala Lys Glu Ala Arg Pro Asp Leu Asp Ile
225                 230                 235                 240

Asp Gly Glu Leu Gln Leu Asp Ala Ser Leu Val Lys Lys Val Ala Asp
                245                 250                 255

Leu Lys Ala Pro Gly Ser Lys Val Ala Gly Lys Ala Asn Val Leu Ile
            260                 265                 270

Phe Pro Asp Ile Gln Ala Gly Asn Ile Gly Tyr Lys Leu Val Gln Arg
        275                 280                 285

Phe Ala Lys Ala Glu Ala Ile Gly Pro Ile Cys Gln Gly Phe Ala Lys
    290                 295                 300

Pro Ile Asn Asp Leu Ser Arg Gly Cys Ser Val Asp Asp Ile Val Lys
305                 310                 315                 320

Val Val Ala Val Thr Ala Val Gln Ala Gln Ala Gln Gly
                325                 330

<210>  18
<211>  333
<212>  PRT
<213>  Clostridium beijerinckii

<220>
<221>  POLYPEPTIDE
<222>  (1)..(333)
<223>  phosphate acetyltransferase

<300>
<308>  GenBank: AWK51335.1
<309>  2018-05-16
<313>  (1)..(333)

<400>  18

Met Asp Leu Met Lys Lys Ile Trp Thr Asp Ala Gln Ala Asn Lys Lys
1               5                   10                  15

Lys Ile Val Leu Pro Glu Gly Asn Glu Glu Arg Thr Ile Ala Ala Ala
            20                  25                  30

Glu Lys Ile His Lys Leu Gly Leu Ala Tyr Pro Ile Leu Val Gly Asn
        35                  40                  45

Lys Glu Glu Ile Met Asn Lys Ser Lys Asp Leu Asp Val Asp Leu Ser
    50                  55                  60

Gly Val Glu Ile Ile Asp Pro Asn Glu Ala Asp Asn Leu Asn Lys Tyr
65                  70                  75                  80

Ile Thr Ala Phe Tyr Asp Leu Arg Lys Lys Lys Gly Met Thr Met Glu
                85                  90                  95

Lys Ala Asp Lys Ile Val Arg Asp Pro Leu Tyr Phe Gly Thr Met Met
            100                 105                 110

Val Lys Leu Asp Asp Ala Asp Gly Met Val Ser Gly Ala Val His Thr
        115                 120                 125

Thr Gly Asp Leu Leu Arg Pro Gly Leu Gln Ile Ile Lys Thr Ala Pro
    130                 135                 140

Gly Val Ser Val Val Ser Ser Phe Phe Ile Met Gln Val Pro Gly Ser
145                 150                 155                 160

Asn Tyr Gly Glu Glu Gly Leu Leu Leu Phe Gly Asp Cys Ala Val Asn
                165                 170                 175

Pro Asn Pro Asn Glu Asp Gln Leu Ala Ala Ile Ala Ile Ala Thr Ala
            180                 185                 190

Glu Thr Ala Lys Thr Leu Cys Lys Met Glu Pro Lys Val Ala Met Leu
        195                 200                 205

Ser Phe Ser Thr Met Gly Ser Ala Asp Asn Glu Leu Val Asp Lys Val
    210                 215                 220

Arg Asn Ala Thr Gln Lys Ala Lys Glu Leu Arg Pro Asp Leu Asp Ile
225                 230                 235                 240

Asp Gly Glu Leu Gln Leu Asp Ala Ala Ile Val Lys Lys Val Ala Asp
                245                 250                 255

Gln Lys Ala Pro Asn Ser Lys Val Ala Gly Arg Ala Asn Val Leu Val
            260                 265                 270

Phe Pro Asp Leu Gln Ala Gly Asn Ile Gly Tyr Lys Leu Val Gln Arg
        275                 280                 285

Phe Ala Asn Ala Asp Ala Ile Gly Pro Val Cys Gln Gly Phe Ala Lys
    290                 295                 300

Pro Ile Asn Asp Leu Ser Arg Gly Cys Ser Ser Asp Asp Ile Val Asn
305                 310                 315                 320

Val Val Ala Leu Thr Ala Val Gln Ala Gln Thr Asn Lys
                325                 330

<210>  19
<211>  332
<212>  PRT
<213>  Clostridium cochlearium

<220>
<221>  POLYPEPTIDE
<222>  (1)..(322)
<223>  phosphate acetyltransferase

<300>
<308>  NCBI Reference Sequence: WP_095177662.1
<309>  2017-08-28
<313>  (1)..(332)

<400>  19

Met Lys Leu Met Glu Glu Ile Trp Ala Lys Ala Lys Ala Asn Lys Lys
1               5                   10                  15

Lys Ile Val Leu Pro Glu Gly Glu Glu Glu Arg Asn Ile Glu Ala Cys
            20                  25                  30

Ser Lys Ile Ile Lys Asn Ala Ile Ala Asp Val Val Leu Val Gly Asn
        35                  40                  45

Ala Glu Lys Ile Lys Glu Met Ala Asn Lys Leu Gly Val Ser Val Glu
    50                  55                  60

Gly Ala Glu Ile Val Asp Pro Glu Asn Ser Asp Lys Thr Ala Asp Tyr
65                  70                  75                  80

Ala Lys Ala Leu Tyr Glu Leu Arg Lys His Lys Gly Val Thr Leu Glu
                85                  90                  95

Lys Ala Glu Lys Ile Ile Lys Asp Pro Leu Tyr Phe Gly Thr Met Met
            100                 105                 110

Val Gln Lys Asn Asp Val Asp Gly Met Val Ser Gly Ala Ile His Thr
        115                 120                 125

Thr Gly Asp Leu Leu Arg Pro Gly Leu Gln Ile Val Lys Thr Ala Pro
    130                 135                 140

Gly Ala Ser Val Val Ser Ser Ser Phe Ile Met Met Val Pro Asp Ser
145                 150                 155                 160

Lys Phe Gly Asn Glu Gly Leu Leu Leu Phe Ala Asp Cys Ala Val Asn
                165                 170                 175

Pro Asn Pro Thr Ser Glu Asp Leu Ala Ser Ile Ala Ile Ser Thr Ala
            180                 185                 190

Glu Thr Ala Lys Asn Leu Cys Gly Leu Asp Pro Lys Val Ala Leu Leu
        195                 200                 205

Ser Phe Ser Thr Gln Gly Ser Ala Ser His Glu Leu Val Asp Lys Val
    210                 215                 220

Val Asn Ala Thr Lys Leu Ala Lys Glu Lys Arg Pro Asp Leu Lys Ile
225                 230                 235                 240

Asp Gly Glu Leu Gln Leu Asp Ala Ala Ile Val Lys Ser Val Ala Asp
                245                 250                 255

Leu Lys Ala Pro Asn Ser Glu Val Ala Gly Lys Ala Asn Val Leu Val
            260                 265                 270

Phe Pro Asp Leu Gln Ala Gly Asn Ile Gly Tyr Lys Leu Val Gln Arg
        275                 280                 285

Phe Ala Asn Ala Glu Ala Ile Gly Pro Ile Thr Gln Gly Phe Ala Lys
    290                 295                 300

Pro Ile Asn Asp Leu Ser Arg Gly Cys Thr Ser Asp Asp Ile Val Asn
305                 310                 315                 320

Val Val Ala Val Thr Val Val Gln Ala Gln Asn Gln
                325                 330

<210>  20
<211>  334
<212>  PRT
<213>  Clostridium magnum

<220>
<221>  POLYPEPTIDE
<222>  (1)..(334)
<223>  phosphate acetyltransferase

<300>
<308>  NCBI Reference Sequence: WP_066619999.1
<309>  2016-08-18
<313>  (1)..(334)

<400>  20

Met Glu Leu Met Arg Gln Ile Trp Glu Lys Ala Lys Ala Asp Lys Arg
1               5                   10                  15

Arg Ile Val Leu Pro Glu Gly Glu Glu Glu Arg Asn Leu Gln Ala Ala
            20                  25                  30

Gly Lys Ile Leu Lys Asn Glu Leu Ala Asn Val Ile Leu Val Gly Ser
        35                  40                  45

Glu Lys Val Ile Arg Glu Lys Ala Thr Ser Leu Gly Val Asn Leu Glu
    50                  55                  60

Gly Ala Glu Ile Val Asp Pro Glu Thr Ser Asp Lys Thr Ala Ser Tyr
65                  70                  75                  80

Ala Asn Glu Phe Tyr Glu Leu Arg Lys Lys Lys Gly Met Thr Leu Glu
                85                  90                  95

Lys Ala Asp Lys Val Val Arg Asp Pro Leu Tyr Tyr Ala Thr Met Met
            100                 105                 110

Val Lys Leu Gly Asp Ala Asp Gly Met Val Ser Gly Ala Ile His Thr
        115                 120                 125

Thr Gly Asp Leu Leu Arg Pro Gly Leu Gln Ile Ile Lys Thr Ala Pro
    130                 135                 140

Gly Met Ser Val Val Ser Ser Thr Phe Val Met Asn Val Pro Asn Ser
145                 150                 155                 160

Gln Tyr Gly Glu Ser Gly Leu Leu Leu Phe Ala Asp Cys Ala Val Asn
                165                 170                 175

Pro Cys Pro Thr Ala Glu Glu Leu Ala Ser Ile Ala Ile Ser Thr Ala
            180                 185                 190

Glu Thr Ala Lys Ala Leu Cys Gly Ile Glu Pro Lys Val Ala Met Leu
        195                 200                 205

Ser Phe Ser Thr Met Gly Ser Ala Gln His Glu Leu Val Asp Lys Val
    210                 215                 220

Arg Lys Ala Thr Glu Leu Ala Lys Ser Met Arg Pro Asp Leu Glu Ile
225                 230                 235                 240

Asp Gly Glu Leu Gln Leu Asp Ala Ala Ile Val Lys Lys Val Gly Asp
                245                 250                 255

Leu Lys Ala Pro Asn Ser Thr Val Ala Gly Lys Ala Asn Val Leu Val
            260                 265                 270

Phe Pro Asp Ile Gln Ala Gly Asn Ile Gly Tyr Lys Leu Val Gln Arg
        275                 280                 285

Phe Ala Asn Ala Glu Ala Ile Gly Pro Ile Cys Gln Gly Phe Ala Lys
    290                 295                 300

Pro Ile Asn Asp Leu Ser Arg Gly Cys Ser Asp Asp Asp Ile Val Asn
305                 310                 315                 320

Val Val Ala Val Thr Ala Val Gln Ala Gln Ala Gln Ala Lys
                325                 330

<210>  21
<211>  1005
<212>  DNA
<213>  Saccharomyces cerevisiae

<220>
<221>  CDS
<222>  (1)..(1005)
<223>  Gene encoding prephenate dehydratase

<300>
<308>  NCBI Reference Sequence: NM_001183154.1
<309>  2018-04-04
<313>  (1)..(1005)

<400>  21
atg gcc agc aag act ttg agg gtt ctt ttt ctg ggt ccc aaa ggt acg        48
Met Ala Ser Lys Thr Leu Arg Val Leu Phe Leu Gly Pro Lys Gly Thr
1               5                   10                  15

tat tcc cat caa gct gca tta caa caa ttt caa tca aca tct gat gtt        96
Tyr Ser His Gln Ala Ala Leu Gln Gln Phe Gln Ser Thr Ser Asp Val
            20                  25                  30

gag tac ctc cca gca gcc tct atc ccc caa tgt ttt aac caa ttg gag       144
Glu Tyr Leu Pro Ala Ala Ser Ile Pro Gln Cys Phe Asn Gln Leu Glu
        35                  40                  45

aac gac act agt ata gat tat tca gtg gta ccg ttg gaa aat tcc acc       192
Asn Asp Thr Ser Ile Asp Tyr Ser Val Val Pro Leu Glu Asn Ser Thr
    50                  55                  60

aat gga caa gta gtt ttt tcc tat gat ctc ttg cgt gat agg atg atc       240
Asn Gly Gln Val Val Phe Ser Tyr Asp Leu Leu Arg Asp Arg Met Ile
65                  70                  75                  80

aaa aaa gcc cta tcc tta cct gct cca gca gat act aat aga att aca       288
Lys Lys Ala Leu Ser Leu Pro Ala Pro Ala Asp Thr Asn Arg Ile Thr

                85                  90                  95

cca gat ata gaa gtt ata gcg gag caa tat gta ccc att acc cat tgt        336
Pro Asp Ile Glu Val Ile Ala Glu Gln Tyr Val Pro Ile Thr His Cys
            100                 105                 110

cta atc agc cca atc caa cta cca aat ggt att gca tcc ctt gga aat        384
Leu Ile Ser Pro Ile Gln Leu Pro Asn Gly Ile Ala Ser Leu Gly Asn
        115                 120                 125

ttt gaa gaa gtc ata ata cac tca cat ccg caa gta tgg ggc cag gtt        432
Phe Glu Glu Val Ile Ile His Ser His Pro Gln Val Trp Gly Gln Val
    130                 135                 140

gaa tgt tac tta agg tcc atg gca gaa aaa ttt ccg cag gtc acc ttt        480
Glu Cys Tyr Leu Arg Ser Met Ala Glu Lys Phe Pro Gln Val Thr Phe
145                 150                 155                 160

ata aga ttg gat tgt tct tcc aca tct gaa tca gtg aac caa tgc att        528
Ile Arg Leu Asp Cys Ser Ser Thr Ser Glu Ser Val Asn Gln Cys Ile
                165                 170                 175

cgg tca tca acg gcc gat tgc gac aac att ctg cat tta gcc att gct        576
Arg Ser Ser Thr Ala Asp Cys Asp Asn Ile Leu His Leu Ala Ile Ala
            180                 185                 190

agt gaa aca gct gcc caa ttg cat aag gcg tac atc att gaa cat tcg        624
Ser Glu Thr Ala Ala Gln Leu His Lys Ala Tyr Ile Ile Glu His Ser
        195                 200                 205

ata aat gat aag cta gga aat aca aca aga ttt tta gta ttg aag aga        672
Ile Asn Asp Lys Leu Gly Asn Thr Thr Arg Phe Leu Val Leu Lys Arg
    210                 215                 220

agg gag aac gca ggc gac aat gaa gta gaa gac act gga tta cta cgg        720
Arg Glu Asn Ala Gly Asp Asn Glu Val Glu Asp Thr Gly Leu Leu Arg
225                 230                 235                 240

gtt aac cta ctc acc ttt act act cgt caa gat gac cct ggt tct ttg        768
Val Asn Leu Leu Thr Phe Thr Thr Arg Gln Asp Asp Pro Gly Ser Leu
                245                 250                 255

gta gat gtt ttg aac ata cta aaa atc cat tca ctc aac atg tgt tct        816
Val Asp Val Leu Asn Ile Leu Lys Ile His Ser Leu Asn Met Cys Ser
            260                 265                 270

ata aac tct aga cca ttc cat ttg gac gaa cat gat aga aac tgg cga        864
Ile Asn Ser Arg Pro Phe His Leu Asp Glu His Asp Arg Asn Trp Arg
        275                 280                 285

tat tta ttt ttc att gaa tat tac acc gag aag aat acc cca aag aat        912
Tyr Leu Phe Phe Ile Glu Tyr Tyr Thr Glu Lys Asn Thr Pro Lys Asn
    290                 295                 300

aaa gaa aaa ttc tat gaa gat atc agc gac aaa agt aaa cag tgg tgc        960
Lys Glu Lys Phe Tyr Glu Asp Ile Ser Asp Lys Ser Lys Gln Trp Cys
305                 310                 315                 320

ctg tgg ggt aca ttc ccc aga aat gag aga tat tat cac aaa taa           1005
Leu Trp Gly Thr Phe Pro Arg Asn Glu Arg Tyr Tyr His Lys
                325                 330

<210>  22
<211>  334
<212>  PRT
<213>  Saccharomyces cerevisiae

<400>  22

Met Ala Ser Lys Thr Leu Arg Val Leu Phe Leu Gly Pro Lys Gly Thr
1               5                   10                  15

Tyr Ser His Gln Ala Ala Leu Gln Gln Phe Gln Ser Thr Ser Asp Val
            20                  25                  30

Glu Tyr Leu Pro Ala Ala Ser Ile Pro Gln Cys Phe Asn Gln Leu Glu
        35                  40                  45

Asn Asp Thr Ser Ile Asp Tyr Ser Val Val Pro Leu Glu Asn Ser Thr
    50                  55                  60

Asn Gly Gln Val Val Phe Ser Tyr Asp Leu Leu Arg Asp Arg Met Ile
65                  70                  75                  80

Lys Lys Ala Leu Ser Leu Pro Ala Pro Ala Asp Thr Asn Arg Ile Thr
                85                  90                  95

Pro Asp Ile Glu Val Ile Ala Glu Gln Tyr Val Pro Ile Thr His Cys
            100                 105                 110

Leu Ile Ser Pro Ile Gln Leu Pro Asn Gly Ile Ala Ser Leu Gly Asn
        115                 120                 125

Phe Glu Glu Val Ile Ile His Ser His Pro Gln Val Trp Gly Gln Val
    130                 135                 140

Glu Cys Tyr Leu Arg Ser Met Ala Glu Lys Phe Pro Gln Val Thr Phe
145                 150                 155                 160

Ile Arg Leu Asp Cys Ser Ser Thr Ser Glu Ser Val Asn Gln Cys Ile
                165                 170                 175

Arg Ser Ser Thr Ala Asp Cys Asp Asn Ile Leu His Leu Ala Ile Ala
            180                 185                 190

Ser Glu Thr Ala Ala Gln Leu His Lys Ala Tyr Ile Ile Glu His Ser
        195                 200                 205

Ile Asn Asp Lys Leu Gly Asn Thr Thr Arg Phe Leu Val Leu Lys Arg
    210                 215                 220

Arg Glu Asn Ala Gly Asp Asn Glu Val Glu Asp Thr Gly Leu Leu Arg
225                 230                 235                 240

Val Asn Leu Leu Thr Phe Thr Thr Arg Gln Asp Asp Pro Gly Ser Leu
                245                 250                 255

Val Asp Val Leu Asn Ile Leu Lys Ile His Ser Leu Asn Met Cys Ser
            260                 265                 270

Ile Asn Ser Arg Pro Phe His Leu Asp Glu His Asp Arg Asn Trp Arg
        275                 280                 285

Tyr Leu Phe Phe Ile Glu Tyr Tyr Thr Glu Lys Asn Thr Pro Lys Asn
    290                 295                 300

Lys Glu Lys Phe Tyr Glu Asp Ile Ser Asp Lys Ser Lys Gln Trp Cys
305                 310                 315                 320

Leu Trp Gly Thr Phe Pro Arg Asn Glu Arg Tyr Tyr His Lys
                325                 330

<210>  23
<211>  335
<212>  PRT
<213>  Saccharomyces arboricola H-6

<220>
<221>  POLYPEPTIDE
<222>  (1)..(335)
<223>  Prephenate dehydratase

<300>
<308>  GenBank: EJS42101.1
<309>  2012-08-16
<313>  (1)..(335)

<400>  23

Met Asn Glu Arg Gly Leu Lys Val Leu Phe Leu Gly Pro Glu Gly Thr
1               5                   10                  15

Tyr Ser His Gln Ala Ala Leu Gln Gln Phe Gln Cys Met Pro Asn Val
            20                  25                  30

Glu Tyr Leu Pro Ala Thr Ser Ile Pro Gln Cys Phe Asp Lys Leu Glu
        35                  40                  45

Lys Asp Ala Ser Ile Asn Tyr Ser Val Val Pro Leu Glu Asn Ser Thr
    50                  55                  60

Asn Gly Gln Val Val Phe Ser Tyr Asp Leu Leu Arg Asp Arg Met Met

65                  70                  75                  80

Pro Lys Ala Leu Ser Phe Thr Ala Ser Thr Glu Thr Asn Asn Arg Ile
                85                  90                  95

Gln Ala Asp Ile Glu Val Val Ala Glu Gln Tyr Val Ser Ile Thr His
            100                 105                 110

Cys Leu Ile Ser Pro Ile Lys Leu Ser Asn Gly Ile Glu Ser Leu Gly
        115                 120                 125

Lys Phe Gln Glu Val Ile Ile Tyr Ser His Pro Gln Val Trp Gly Gln
    130                 135                 140

Val Asp Gly Tyr Leu Lys Ser Leu Glu Glu Lys Phe Pro Gln Leu Thr
145                 150                 155                 160

Phe Thr Arg Thr Asp Cys Ser Ser Thr Ser Ala Ser Val Asn Gln Cys
                165                 170                 175

Leu Gln Ser Gln Thr Ala Cys Pro Asp Ser Ile Leu His Leu Ala Ile
            180                 185                 190

Ala Ser Glu Thr Ala Ala Arg Leu Tyr Lys Ala His Ile Ile Gly His
        195                 200                 205

Ser Ile Asn Asp Lys Leu Gly Asn Thr Thr Arg Phe Leu Val Leu Ala
    210                 215                 220

Arg Arg Glu Asp Met Arg Asn Asn Met Phe Lys His Ser Glu Leu Pro
225                 230                 235                 240

Gln Val Asn Leu Leu Thr Phe Thr Ile Arg Gln Asp Asp Pro Gly Ser
                245                 250                 255

Leu Val Asp Val Leu Asn Val Leu Lys Ile His Ser Leu Asn Met Cys
            260                 265                 270

Ser Ile Ser Ser Arg Pro Phe His Leu Asn Glu Asp Asp Arg Asn Trp
        275                 280                 285

Arg Tyr Leu Phe Phe Ile Glu Tyr Tyr Thr Asp Lys Asn Ala Pro Glu
    290                 295                 300

Asp Asn Glu Thr Phe Tyr Lys Asp Ile Ser Glu Lys Ser Lys Gln Trp
305                 310                 315                 320

Cys Leu Trp Gly Thr Phe Ser Arg Asn Lys Met Tyr Tyr His Lys
                325                 330                 335

<210>  24
<211>  334
<212>  PRT
<213>  Saccharomyces cerevisiae Kyokai no. 7

<220>
<221>  POLYPEPIDE
<222>  (1)..(334)
<223>  Prephente dehydratase

<300>
<308>  GenBank: GAA25795.1
<309>  2013-08-02
<313>  (1)..(334)

<400>  24

Met Ala Ser Lys Thr Leu Arg Val Leu Phe Leu Gly Pro Lys Gly Thr
1               5                   10                  15

Tyr Ser His Gln Ala Ala Leu Gln Gln Phe Gln Ser Thr Ser Asp Val
            20                  25                  30

Glu Tyr Leu Pro Ala Ala Ser Ile Pro Gln Cys Phe Asn Gln Leu Glu
        35                  40                  45

Asn Asp Thr Ser Ile Asp Tyr Ser Val Val Pro Leu Glu Asn Ser Thr
    50                  55                  60

Asn Gly Gln Val Val Phe Ser Tyr Asp Leu Leu Arg Asp Arg Met Ile
65                  70                  75                  80

Lys Lys Ala Leu Ser Leu Pro Ala Pro Ala Asp Thr Asn Arg Ile Thr
                85                  90                  95

Pro Asp Ile Glu Val Ile Ala Glu Gln Tyr Val Pro Ile Thr His Cys
            100                 105                 110

Leu Ile Ser Pro Ile Gln Leu Pro Asn Gly Ile Ala Ser Leu Gly Asn
        115                 120                 125

Phe Glu Glu Val Ile Ile His Ser His Pro Gln Val Trp Gly Gln Val
    130                 135                 140

Glu Cys Tyr Leu Arg Ser Met Ala Glu Lys Phe Pro Gln Val Thr Phe
145                 150                 155                 160

Lys Arg Leu Asp Cys Ser Ser Thr Ser Glu Ser Val Asn Gln Cys Ile

                165                 170                 175

Arg Ser Ser Thr Ala Asp Cys Asp Asn Ile Gln His Leu Ala Ile Ala
            180                 185                 190

Ser Glu Thr Ala Ala Gln Leu His Lys Ala Tyr Ile Ile Glu His Ser
        195                 200                 205

Ile Asn Asp Lys Leu Gly Asn Thr Thr Arg Phe Leu Val Leu Lys Arg
    210                 215                 220

Arg Glu Asn Ala Gly Asp Asn Glu Val Glu Asp Thr Gly Leu Leu Arg
225                 230                 235                 240

Val Asn Leu Leu Thr Phe Thr Thr Arg Gln Asp Asp Pro Gly Ser Leu
                245                 250                 255

Val Asp Val Leu Asn Ile Leu Lys Ile His Ser Leu Asn Met Cys Ser
            260                 265                 270

Ile Asn Ser Arg Pro Phe His Leu Asp Glu His Asp Arg Asn Trp Arg
        275                 280                 285

Tyr Leu Phe Phe Ile Glu Tyr Tyr Thr Glu Lys Asn Thr Pro Lys Asn
    290                 295                 300

Lys Glu Lys Phe Tyr Glu Asp Ile Ser Asp Lys Ser Lys Gln Trp Cys
305                 310                 315                 320

Leu Trp Gly Thr Phe Pro Arg Asn Glu Arg Tyr Tyr His Lys
                325                 330

<210> 25
<211> 334
<212> PRT
<213> Saccharomyces cerevisiae x Saccharomyces kudriavzevii VIN7

<220>
<221> POLYPEPTIDE
<222> (1)..(334)
<223> Prephente dehydratase

<300>
<308> GenBank: EHN00663.1
<309> 2011-12-23
<313> (1)..(334)

<400> 25

Met Asn Gly Lys Ser Leu Lys Val Leu Phe Leu Gly Pro Gln Gly Thr
1               5                   10                  15

Tyr Ser His Gln Ala Val Leu Gln Gln Phe Gln Ser Ile Pro Asn Val
            20                  25                  30

Glu Tyr Leu Pro Ala Thr Ser Ile Pro Gln Cys Phe Asp Glu Leu Glu
        35                  40                  45

Glu Asp Val Thr Val Asp Tyr Ser Val Val Pro Leu Glu Asn Ser Thr
    50                  55                  60

Asn Gly Gln Val Val Phe Ser Tyr Asp Leu Leu Arg Asp Arg Met Ile
65                  70                  75                  80

Gln Lys Ala Gln Ser Ser Thr Thr Ser Ala Lys Thr Asn Met Ile Thr
                85                  90                  95

Ser Asp Ile Glu Val Ile Ala Glu Gln Tyr Val Ser Ile Thr His Cys
            100                 105                 110

Leu Ile Ser Pro Ile Glu Leu Pro Asn Gly Val Glu Ser Leu Glu Arg
        115                 120                 125

Phe Glu Glu Val Val Ile His Ser His Pro Gln Val Trp Gly Gln Val
    130                 135                 140

Asp Gly Tyr Leu Lys Ser Leu Glu Glu Lys Phe Pro Ser Leu Thr Phe
145                 150                 155                 160

Ser Arg Ser Asp Cys Ser Ser Thr Ser Glu Ser Val Tyr Gln Cys Met
                165                 170                 175

Arg Ser His Ala Ala Ser Asp Asp Asn Ile Leu His Leu Ala Ile Ala
            180                 185                 190

Ser Glu Thr Ala Ala Gln Leu His Lys Ala His Ile Ile Glu His Ser
        195                 200                 205

Ile Asn Asp Lys Leu Gly Asn Thr Thr Arg Phe Leu Val Leu Lys Arg
    210                 215                 220

Arg Glu Asn Thr Ser Arg Asn Glu Ile Glu Ser Thr Glu Leu Pro Gln
225                 230                 235                 240

Val Asn Leu Leu Thr Phe Thr Thr Arg Gln Asp Asp Pro Gly Ser Leu
                245                 250                 255

Val Asp Val Leu Asn Val Leu Lys Ile His Ser Leu Asn Met Cys Ser

260                 265                 270

Ile Asn Ser Arg Pro Phe His Met Asp Glu Asn Asp Arg Asn Trp Arg
        275                 280                 285

Tyr Leu Phe Phe Ile Glu Tyr Tyr Thr Asp Gly Asn Ala Pro Arg Asn
    290                 295                 300

Lys Glu Thr Phe Tyr Glu Asp Ile Ser Glu Lys Ser Lys Gln Trp Cys
305                 310                 315                 320

Leu Trp Gly Thr Phe Pro Arg Asn Gly Lys Tyr Tyr His Lys
                325                 330

<210>  26
<211>  753
<212>  DNA
<213>  Saccharomyces cerevisiae

<220>
<221>  CDS
<222>  (1)..(735)
<223>  Gene encoding glycerol-1-phosphatase (GPP1)

<300>
<308>  NCBI Reference Sequence: NM_001179403.1
<309>  2018-04-04
<313>  (1)..(753)

<400>  26
atg cct ttg acc aca aaa cct tta tct ttg aaa atc aac gcc gct cta      48
Met Pro Leu Thr Thr Lys Pro Leu Ser Leu Lys Ile Asn Ala Ala Leu
1               5                   10                  15

ttc gat gtt gac ggt acc atc atc atc tct caa cca gcc att gct gct      96
Phe Asp Val Asp Gly Thr Ile Ile Ile Ser Gln Pro Ala Ile Ala Ala
            20                  25                  30

ttc tgg aga gat ttc ggt aaa gac aag cct tac ttc gat gcc gaa cac      144
Phe Trp Arg Asp Phe Gly Lys Asp Lys Pro Tyr Phe Asp Ala Glu His
        35                  40                  45

gtt att cac atc tct cac ggt tgg aga act tac gat gcc att gcc aag      192
Val Ile His Ile Ser His Gly Trp Arg Thr Tyr Asp Ala Ile Ala Lys
    50                  55                  60

ttc gct cca gac ttt gct gat gaa gaa tac gtt aac aag cta gaa ggt      240
Phe Ala Pro Asp Phe Ala Asp Glu Glu Tyr Val Asn Lys Leu Glu Gly
65                  70                  75                  80

gaa atc cca gaa aag tac ggt gaa cac tcc atc gaa gtt cca ggt gct      288
Glu Ile Pro Glu Lys Tyr Gly Glu His Ser Ile Glu Val Pro Gly Ala
                85                  90                  95

gtc aag ttg tgt aat gct ttg aac gcc ttg cca aag gaa aaa tgg gct      336
Val Lys Leu Cys Asn Ala Leu Asn Ala Leu Pro Lys Glu Lys Trp Ala
            100                 105                 110

gtc gcc acc tct ggt acc cgt gac atg gcc aag aaa tgg ttc gac att     384
Val Ala Thr Ser Gly Thr Arg Asp Met Ala Lys Lys Trp Phe Asp Ile
        115                 120                 125

ttg aag atc aag aga cca gaa tac ttc atc acc gcc aat gat gtc aag     432
Leu Lys Ile Lys Arg Pro Glu Tyr Phe Ile Thr Ala Asn Asp Val Lys
    130                 135                 140

caa ggt aag cct cac cca gaa cca tac tta aag ggt aga aac ggt ttg     480
Gln Gly Lys Pro His Pro Glu Pro Tyr Leu Lys Gly Arg Asn Gly Leu
145                 150                 155                 160

ggt ttc cca att aat gaa caa gac cca tcc aaa tct aag gtt gtt gtc     528
Gly Phe Pro Ile Asn Glu Gln Asp Pro Ser Lys Ser Lys Val Val Val
                165                 170                 175

ttt gaa gac gca cca gct ggt att gct gct ggt aag gct gct ggc tgt     576
Phe Glu Asp Ala Pro Ala Gly Ile Ala Ala Gly Lys Ala Ala Gly Cys
            180                 185                 190

aaa atc gtt ggt att gct acc act ttc gat ttg gac ttc ttg aag gaa     624
Lys Ile Val Gly Ile Ala Thr Thr Phe Asp Leu Asp Phe Leu Lys Glu
        195                 200                 205

aag ggt tgt gac atc att gtc aag aac cac gaa tct atc aga gtc ggt     672
Lys Gly Cys Asp Ile Ile Val Lys Asn His Glu Ser Ile Arg Val Gly
    210                 215                 220

gaa tac aac gct gaa acc gat gaa gtc gaa ttg atc ttt gat gac tac     720
Glu Tyr Asn Ala Glu Thr Asp Glu Val Glu Leu Ile Phe Asp Asp Tyr
225                 230                 235                 240

tta tac gct aag gat gacttgttga aatggtaa                             753
Leu Tyr Ala Lys Asp
                245

<210>  27
<211>  245
<212>  PRT
<213>  Saccharomyces cerevisiae

<400>  27

Met Pro Leu Thr Thr Lys Pro Leu Ser Leu Lys Ile Asn Ala Ala Leu
1               5                   10                  15

Phe Asp Val Asp Gly Thr Ile Ile Ile Ser Gln Pro Ala Ile Ala Ala
            20                  25                  30

Phe Trp Arg Asp Phe Gly Lys Asp Lys Pro Tyr Phe Asp Ala Glu His
        35                  40                  45

Val Ile His Ile Ser His Gly Trp Arg Thr Tyr Asp Ala Ile Ala Lys
    50                  55                  60

Phe Ala Pro Asp Phe Ala Asp Glu Glu Tyr Val Asn Lys Leu Glu Gly
65                  70                  75                  80

Glu Ile Pro Glu Lys Tyr Gly Glu His Ser Ile Glu Val Pro Gly Ala
                85                  90                  95

Val Lys Leu Cys Asn Ala Leu Asn Ala Leu Pro Lys Glu Lys Trp Ala
            100                 105                 110

Val Ala Thr Ser Gly Thr Arg Asp Met Ala Lys Lys Trp Phe Asp Ile
        115                 120                 125

Leu Lys Ile Lys Arg Pro Glu Tyr Phe Ile Thr Ala Asn Asp Val Lys
    130                 135                 140

Gln Gly Lys Pro His Pro Glu Pro Tyr Leu Lys Gly Arg Asn Gly Leu
145                 150                 155                 160

Gly Phe Pro Ile Asn Glu Gln Asp Pro Ser Lys Ser Lys Val Val Val
                165                 170                 175

Phe Glu Asp Ala Pro Ala Gly Ile Ala Ala Gly Lys Ala Ala Gly Cys
            180                 185                 190

Lys Ile Val Gly Ile Ala Thr Thr Phe Asp Leu Asp Phe Leu Lys Glu
        195                 200                 205

Lys Gly Cys Asp Ile Ile Val Lys Asn His Glu Ser Ile Arg Val Gly
    210                 215                 220

Glu Tyr Asn Ala Glu Thr Asp Glu Val Glu Leu Ile Phe Asp Asp Tyr
225                 230                 235                 240

Leu Tyr Ala Lys Asp
                245

<210>  28
<211>  251
<212>  PRT
<213>  Kazachstania africana CBS 2517

<220>
<221>  POLYPEPTIDE
<222>  (1)..(251)
<223>  phosphatase

<300>
<308>  NCBI Reference Sequence: XP_003959834.1
<309>  2017-06-02
<313>  (1)..(251)

<400>  28

Met Thr Ala Ser Thr Lys Thr Pro Ile Ile Leu Lys Val Asn Ala Ala
1               5                   10                  15

Leu Phe Asp Val Asp Gly Thr Ile Ile Ile Ser Gln Pro Ala Ile Ala
            20                  25                  30

Glu Met Trp Arg Asp Phe Gly Lys Asp Lys Pro Tyr Phe Asp Ser Glu
        35                  40                  45

Phe Val Ile Lys Ile Ser His Gly Ala Arg Thr Tyr Asp Met Ile Ala
    50                  55                  60

Lys Tyr Ala Pro Asp Phe Ala Asn Glu Lys Phe Val Arg Gln Leu Glu
65                  70                  75                  80

Asp Lys Ile Pro Asp Lys Phe Gly Asp His Ser Tyr Glu Val Pro Gly
                85                  90                  95

Ala Thr Lys Leu Cys Asn Glu Phe Asn Lys Leu Pro Lys Glu Lys Trp
            100                 105                 110

Ala Val Ala Thr Ser Gly Thr Phe Glu Met Ala His Lys Trp Phe Asp
        115                 120                 125

Ile Leu Gly Ile Lys Arg Pro Thr Tyr Phe Ile Thr Ala Asn Asp Val
    130                 135                 140

Lys Asn Gly Lys Pro His Pro Glu Pro Tyr Leu Lys Gly Arg Ile Gly
145                 150                 155                 160

Leu Gly Tyr Pro Val Asn Glu Gln Lys Pro Ser His Ser Lys Val Val
                165                 170                 175

Val Phe Glu Asp Ala Pro Asn Gly Val Lys Ser Gly Gln Ala Ala Gly
            180                 185                 190

Cys Lys Ile Ile Gly Val Ala Thr Thr Phe Asp Gln Asp Phe Leu Lys
        195                 200                 205

Glu Lys Gly Cys Asp Leu Ile Ile Lys Asp Phe Thr Gln Val Lys Leu
    210                 215                 220

Asn Gly Tyr Asp Pro Ile Lys Asp Glu Val Glu Leu Gln Phe Asn Asp
225                 230                 235                 240

Tyr Ile Tyr Ala Lys Pro Asp Leu Met Val Trp
                245                 250

<210>  29
<211>  250
<212>  PRT
<213>  Saccharomyces arboricola H-6

<220>
<221>  POLYPEPTIDE
<222>  (1)..(250)
<223>  phosphatase

<300>
<308>  GenBank: EJS43287.1
<309>  2012-08-16
<313>  (1)..(250)

<400>  29

Met Pro Leu Thr Thr Lys Pro Leu Ser Leu Lys Ile Asn Ala Ala Leu
1               5                   10                  15

Phe Asp Val Asp Gly Thr Ile Ile Ile Ser Gln Pro Ala Ile Ala Ala
            20                  25                  30

Phe Trp Arg Asp Phe Gly Lys Asp Lys Pro Tyr Phe Asp Ala Glu His
        35                  40                  45

Val Ile His Ile Ser His Gly Trp Arg Thr Phe Asp Ala Ile Ala Lys
    50                  55                  60

Phe Ala Pro Asp Phe Ala Gln Glu Glu Tyr Val Asn Lys Leu Glu Gly
65                  70                  75                  80

Glu Ile Pro Glu Lys Tyr Gly Glu His Ser Ile Glu Val Pro Gly Ala
                85                  90                  95

Val Lys Leu Cys Asn Ala Leu Asn Ala Leu Pro Lys Glu Lys Trp Ala
            100                 105                 110

Val Ala Thr Ser Gly Thr Arg Asp Met Ala Lys Lys Trp Phe Asp Ile
        115                 120                 125

Leu Lys Ile Lys Arg Pro Glu Tyr Phe Ile Thr Ala Asn Asp Val Lys
    130                 135                 140

Gln Gly Lys Pro His Pro Glu Pro Tyr Leu Lys Gly Arg Asn Gly Leu
145                 150                 155                 160

Gly Phe Pro Ile Asn Glu Gln Asp Pro Ser Lys Ser Lys Val Val Val
                165                 170                 175

Phe Glu Asp Ala Pro Ala Gly Ile Ala Ala Gly Lys Ala Ala Gly Cys
            180                 185                 190

Lys Ile Val Gly Ile Ala Thr Thr Phe Asp Leu Asp Phe Leu Lys Glu
        195                 200                 205

Lys Gly Cys Asp Ile Ile Val Lys Asn His Glu Ser Ile Arg Val Gly
    210                 215                 220

Glu Tyr Asn Ala Glu Thr Asp Glu Val Glu Leu Ile Phe Asp Asp Tyr
225                 230                 235                 240

Leu Tyr Ala Lys Asp Asp Leu Leu Lys Trp
                245                 250

<210> 30
<211> 250
<212> PRT
<213> Zygosaccharomyces parabailii

<220>
<221> POLYPEPTIDE
<222> (1)..(250)
<223> phosphatase

<300>
<308> GenBank: AQZ10237.1
<309> 2017-07-05
<313> (1)..(250)

<400> 30

Met Pro Leu Thr Gln Lys Pro Leu Ser Val Lys Val Asn Ala Cys Leu
1               5                   10                  15

Phe Asp Val Asp Gly Thr Ile Ile Ile Ser Gln Pro Ala Ile Ala Glu
            20                  25                  30

Phe Trp Arg Asp Phe Gly Lys Asp Lys Pro Tyr Phe Asp Ala Glu His
        35                  40                  45

Val Ile Asn Ile Ser His Gly Trp Arg Thr Tyr Asp Ala Ile Ala Lys
    50                  55                  60

Phe Ala Pro Asp Tyr Ala Asp Glu Asp Tyr Ala Thr Lys Leu Glu Ala
65                  70                  75                  80

Glu Ile Pro Asp Lys Tyr Gly Lys Ala Ala Ile Glu Val Pro Gly Ala
                85                  90                  95

Lys Lys Leu Cys Ala Ala Phe Asn Ala Leu Pro Lys Glu Lys Trp Ala
            100                 105                 110

Val Ala Thr Ser Gly Thr Lys Ile Met Ala Arg Lys Trp Phe Glu Ile
        115                 120                 125

Leu Gly Ile Lys Arg Pro Ser Leu Phe Ile Thr Ala Asn Asp Val Lys
    130                 135                 140

Gln Gly Lys Pro Phe Pro Glu Pro Tyr Leu Lys Gly Arg Asp Gly Leu
145                 150                 155                 160

Gly Tyr Pro Ile Asn Lys Glu His Pro Glu Lys Ser Lys Val Phe Val
                165                 170                 175

Phe Glu Asp Ala Pro Ala Gly Ile Ala Ala Gly Lys Ala Ala Gly Cys
            180                 185                 190

Lys Ile Val Gly Ile Gln Thr Thr Phe Pro Leu Glu Phe Leu Ile Glu
        195                 200                 205

Lys Gly Cys Asp Ile Ile Val Lys Asn His Glu Ser Ile Glu Leu Ala
    210                 215                 220

Gly Tyr Asp Glu Lys Thr Glu Glu Val Glu Leu Val Phe Lys Asp Tyr
225                 230                 235                 240

Leu Tyr Ala Lys Asp Asp Leu Leu Lys Trp
                245                 250

<210>  31
<211>  2154
<212>  DNA
<213>  Arabidopsis thaliana

<220>
<221>  CDS
<222>  (1)..(2154)
<223>  Gene encoding phenylalanine ammonia-lyase from Arabidopsis
       thaliana codon optimized for yeast

<400>  31
atg gat caa atc gaa gct atg ttg tgt ggt ggt ggt gaa aaa aca aaa        48
Met Asp Gln Ile Glu Ala Met Leu Cys Gly Gly Gly Glu Lys Thr Lys
1               5                   10                  15

gtt gct gtt act act aag acc ttg gcc gat cca ttg aat tgg ggt ttg        96
Val Ala Val Thr Thr Lys Thr Leu Ala Asp Pro Leu Asn Trp Gly Leu
            20                  25                  30

gct gct gat caa atg aag ggt tct cat ttg gat gaa gtc aag aag atg       144
Ala Ala Asp Gln Met Lys Gly Ser His Leu Asp Glu Val Lys Lys Met

35                  40                  45

gtc gaa gaa tac aga aga cca gtt gtt aat ttg ggt ggt gaa act ttg          192
Val Glu Glu Tyr Arg Arg Pro Val Val Asn Leu Gly Gly Glu Thr Leu
    50                  55                  60

act att ggt caa gtt gct gct att tct act gtt ggt ggt tct gtt aag          240
Thr Ile Gly Gln Val Ala Ala Ile Ser Thr Val Gly Gly Ser Val Lys
65                  70                  75                  80

gtt gaa ttg gct gaa act tct aga gct ggt gtt aag gct tct tct gat          288
Val Glu Leu Ala Glu Thr Ser Arg Ala Gly Val Lys Ala Ser Ser Asp
                85                  90                  95

tgg gtt atg gaa tct atg aac aag ggt act gat tct tac ggt gtt act          336
Trp Val Met Glu Ser Met Asn Lys Gly Thr Asp Ser Tyr Gly Val Thr
            100                 105                 110

aca ggt ttt ggt gct act tct cat aga aga act aag aat ggt act gcc          384
Thr Gly Phe Gly Ala Thr Ser His Arg Arg Thr Lys Asn Gly Thr Ala
        115                 120                 125

ttg caa acc gaa ttg atc aga ttt ttg aac gcc ggt att ttc ggt aac          432
Leu Gln Thr Glu Leu Ile Arg Phe Leu Asn Ala Gly Ile Phe Gly Asn
    130                 135                 140

acc aaa gaa act tgt cat acc ttg cca caa tct gct act aga gct gct          480
Thr Lys Glu Thr Cys His Thr Leu Pro Gln Ser Ala Thr Arg Ala Ala
145                 150                 155                 160

atg ttg gtt aga gtt aac act ttg ttg caa ggt tac tcc ggt atc aga          528
Met Leu Val Arg Val Asn Thr Leu Leu Gln Gly Tyr Ser Gly Ile Arg
                165                 170                 175

ttc gaa att ttg gaa gct atc acc tcc ttg ttg aac cat aac att tct          576
Phe Glu Ile Leu Glu Ala Ile Thr Ser Leu Leu Asn His Asn Ile Ser
            180                 185                 190

cca tct ttg cca ttg aga ggt act att act gct tct ggt gat ttg gtt          624
Pro Ser Leu Pro Leu Arg Gly Thr Ile Thr Ala Ser Gly Asp Leu Val
        195                 200                 205

cca ttg tct tat att gct ggt ttg ttg act ggt aga cca aac tct aaa          672
Pro Leu Ser Tyr Ile Ala Gly Leu Leu Thr Gly Arg Pro Asn Ser Lys
    210                 215                 220

gct act ggt cca gat ggt gaa tca ttg act gct aaa gaa gct ttt gaa          720
Ala Thr Gly Pro Asp Gly Glu Ser Leu Thr Ala Lys Glu Ala Phe Glu
225                 230                 235                 240

aag gct ggt atc tct act ggt ttt ttc gac ttg caa cct aaa gaa ggt          768
Lys Ala Gly Ile Ser Thr Gly Phe Phe Asp Leu Gln Pro Lys Glu Gly
                245                 250                 255

ttg gct ttg gtt aat ggt aca gct gtt ggt tct ggt atg gct tct atg          816
Leu Ala Leu Val Asn Gly Thr Ala Val Gly Ser Gly Met Ala Ser Met
            260                 265                 270

gtt ttg ttt gaa gct aac gtt caa gct gtt ttg gcc gaa gtt ttg tct          864
Val Leu Phe Glu Ala Asn Val Gln Ala Val Leu Ala Glu Val Leu Ser
        275                 280                 285

gct att ttt gct gaa gtt atg tcc ggt aag cca gaa ttc act gat cat          912

Ala Ile Phe Ala Glu Val Met Ser Gly Lys Pro Glu Phe Thr Asp His
    290                 295                 300

ttg acc cat aga ttg aaa cat cac cca ggt caa att gaa gct gct gca      960
Leu Thr His Arg Leu Lys His His Pro Gly Gln Ile Glu Ala Ala Ala
305                 310                 315                 320

att atg gaa cat atc ttg gat ggt tcc tct tac atg aag ttg gct caa      1008
Ile Met Glu His Ile Leu Asp Gly Ser Ser Tyr Met Lys Leu Ala Gln
                325                 330                 335

aaa gtt cac gaa atg gac cca ttg caa aag cca aaa caa gat aga tac      1056
Lys Val His Glu Met Asp Pro Leu Gln Lys Pro Lys Gln Asp Arg Tyr
            340                 345                 350

gct ttg aga act tct cca caa tgg ttg ggt cca caa ata gaa gtt att      1104
Ala Leu Arg Thr Ser Pro Gln Trp Leu Gly Pro Gln Ile Glu Val Ile
        355                 360                 365

aga caa gcc acc aag tcc atc gaa aga gaa atc aat tct gtt aac gac      1152
Arg Gln Ala Thr Lys Ser Ile Glu Arg Glu Ile Asn Ser Val Asn Asp
    370                 375                 380

aac cca ttg atc gac gtc agt aga aac aaa gct att cat ggt ggt aac      1200
Asn Pro Leu Ile Asp Val Ser Arg Asn Lys Ala Ile His Gly Gly Asn
385                 390                 395                 400

ttc caa ggt act cca att ggt gtt tct atg gac aac act aga ttg gct      1248
Phe Gln Gly Thr Pro Ile Gly Val Ser Met Asp Asn Thr Arg Leu Ala
                405                 410                 415

att gct gcc att ggt aaa ttg atg ttc gct caa ttc tcc gaa ttg gtc      1296
Ile Ala Ala Ile Gly Lys Leu Met Phe Ala Gln Phe Ser Glu Leu Val
            420                 425                 430

aac gat ttt tac aac aac ggt ttg cct tct aac ttg acc gct tct tct      1344
Asn Asp Phe Tyr Asn Asn Gly Leu Pro Ser Asn Leu Thr Ala Ser Ser
        435                 440                 445

aat cca tca ttg gat tac ggt ttt aag ggt gct gaa att gct atg gct      1392
Asn Pro Ser Leu Asp Tyr Gly Phe Lys Gly Ala Glu Ile Ala Met Ala
    450                 455                 460

tca tac tgt tct gaa ttg caa tac ttg gct aac cca gtt acc tct cat      1440
Ser Tyr Cys Ser Glu Leu Gln Tyr Leu Ala Asn Pro Val Thr Ser His
465                 470                 475                 480

gtt caa tct gct gaa caa cac aat caa gac gtt aac tcc ttg ggt ttg      1488
Val Gln Ser Ala Glu Gln His Asn Gln Asp Val Asn Ser Leu Gly Leu
                485                 490                 495

atc tct tct aga aag act tct gaa gcc gtt gac atc ttg aag ttg atg      1536
Ile Ser Ser Arg Lys Thr Ser Glu Ala Val Asp Ile Leu Lys Leu Met
            500                 505                 510

tct act aca ttc ttg gtc ggt att tgc caa gct gtt gat ttg aga cat      1584
Ser Thr Thr Phe Leu Val Gly Ile Cys Gln Ala Val Asp Leu Arg His
        515                 520                 525

ttg gaa gaa aac ttg aga caa acc gtc aag aac acc gtt tca caa gtt      1632
Leu Glu Glu Asn Leu Arg Gln Thr Val Lys Asn Thr Val Ser Gln Val
    530                 535                 540

gct aag aaa gtt ttg acc acc ggt att aac ggt gaa ttg cat cca tct 1680
Ala Lys Lys Val Leu Thr Thr Gly Ile Asn Gly Glu Leu His Pro Ser
545                 550                 555                 560

aga ttc tgc gaa aag gat ttg ttg aag gtc gtt gat aga gaa caa gtt 1728
Arg Phe Cys Glu Lys Asp Leu Leu Lys Val Val Asp Arg Glu Gln Val
                565                 570                 575

ttc acc tac gtt gat gat cca tgt tct gct act tat cca ttg atg caa 1776
Phe Thr Tyr Val Asp Asp Pro Cys Ser Ala Thr Tyr Pro Leu Met Gln
            580                 585                 590

aga ttg aga caa gtc atc gtt gat cat gct ttg tct aat ggt gaa acc 1824
Arg Leu Arg Gln Val Ile Val Asp His Ala Leu Ser Asn Gly Glu Thr
        595                 600                 605

gaa aag aac gct gtt acc tcc att ttc caa aag att ggt gct ttc gaa 1872
Glu Lys Asn Ala Val Thr Ser Ile Phe Gln Lys Ile Gly Ala Phe Glu
    610                 615                 620

gaa gaa ttg aag gcc gtt ttg cca aaa gaa gtt gaa gca gct aga gca 1920
Glu Glu Leu Lys Ala Val Leu Pro Lys Glu Val Glu Ala Ala Arg Ala
625                 630                 635                 640

gct tac ggt aac ggt act gct cca att cca aat aga atc aaa gaa tgc 1968
Ala Tyr Gly Asn Gly Thr Ala Pro Ile Pro Asn Arg Ile Lys Glu Cys
                645                 650                 655

aga tcc tac cca tta tac aga ttc gtt aga gaa gaa tta ggt act aag 2016
Arg Ser Tyr Pro Leu Tyr Arg Phe Val Arg Glu Glu Leu Gly Thr Lys
            660                 665                 670

ttg ttg acc ggt gaa aag gtt gtt tct cca ggt gaa gaa ttc gat aag 2064
Leu Leu Thr Gly Glu Lys Val Val Ser Pro Gly Glu Glu Phe Asp Lys
        675                 680                 685

gtt ttc act gct atg tgc gaa ggt aaa ttg atc gat cca ttg atg gac 2112
Val Phe Thr Ala Met Cys Glu Gly Lys Leu Ile Asp Pro Leu Met Asp
    690                 695                 700

tgc ttg aaa gaa tgg aat ggt gct cct att cct atc tgc tga 2154
Cys Leu Lys Glu Trp Asn Gly Ala Pro Ile Pro Ile Cys
705                 710                 715

<210> 32
<211> 717
<212> PRT
<213> Arabidopsis thaliana

<400> 32

Met Asp Gln Ile Glu Ala Met Leu Cys Gly Gly Gly Glu Lys Thr Lys
1               5                   10                  15

Val Ala Val Thr Thr Lys Thr Leu Ala Asp Pro Leu Asn Trp Gly Leu
            20                  25                  30

Ala Ala Asp Gln Met Lys Gly Ser His Leu Asp Glu Val Lys Lys Met
        35                  40                  45

Val Glu Glu Tyr Arg Arg Pro Val Val Asn Leu Gly Gly Glu Thr Leu
    50                  55                  60

Thr Ile Gly Gln Val Ala Ala Ile Ser Thr Val Gly Gly Ser Val Lys
65                  70                  75                  80

Val Glu Leu Ala Glu Thr Ser Arg Ala Gly Val Lys Ala Ser Ser Asp
                85                  90                  95

Trp Val Met Glu Ser Met Asn Lys Gly Thr Asp Ser Tyr Gly Val Thr
            100                 105                 110

Thr Gly Phe Gly Ala Thr Ser His Arg Arg Thr Lys Asn Gly Thr Ala
        115                 120                 125

Leu Gln Thr Glu Leu Ile Arg Phe Leu Asn Ala Gly Ile Phe Gly Asn
    130                 135                 140

Thr Lys Glu Thr Cys His Thr Leu Pro Gln Ser Ala Thr Arg Ala Ala
145                 150                 155                 160

Met Leu Val Arg Val Asn Thr Leu Leu Gln Gly Tyr Ser Gly Ile Arg
                165                 170                 175

Phe Glu Ile Leu Glu Ala Ile Thr Ser Leu Leu Asn His Asn Ile Ser
            180                 185                 190

Pro Ser Leu Pro Leu Arg Gly Thr Ile Thr Ala Ser Gly Asp Leu Val
        195                 200                 205

Pro Leu Ser Tyr Ile Ala Gly Leu Leu Thr Gly Arg Pro Asn Ser Lys
    210                 215                 220

Ala Thr Gly Pro Asp Gly Glu Ser Leu Thr Ala Lys Glu Ala Phe Glu
225                 230                 235                 240

Lys Ala Gly Ile Ser Thr Gly Phe Phe Asp Leu Gln Pro Lys Glu Gly
                245                 250                 255

Leu Ala Leu Val Asn Gly Thr Ala Val Gly Ser Gly Met Ala Ser Met
            260                 265                 270

Val Leu Phe Glu Ala Asn Val Gln Ala Val Leu Ala Glu Val Leu Ser
        275                 280                 285

Ala Ile Phe Ala Glu Val Met Ser Gly Lys Pro Glu Phe Thr Asp His
    290                 295                 300

Leu Thr His Arg Leu Lys His His Pro Gly Gln Ile Glu Ala Ala Ala
305                 310                 315                 320

Ile Met Glu His Ile Leu Asp Gly Ser Ser Tyr Met Lys Leu Ala Gln
                325                 330                 335

Lys Val His Glu Met Asp Pro Leu Gln Lys Pro Lys Gln Asp Arg Tyr
            340                 345                 350

Ala Leu Arg Thr Ser Pro Gln Trp Leu Gly Pro Gln Ile Glu Val Ile
        355                 360                 365

Arg Gln Ala Thr Lys Ser Ile Glu Arg Glu Ile Asn Ser Val Asn Asp
    370                 375                 380

Asn Pro Leu Ile Asp Val Ser Arg Asn Lys Ala Ile His Gly Gly Asn
385                 390                 395                 400

Phe Gln Gly Thr Pro Ile Gly Val Ser Met Asp Asn Thr Arg Leu Ala
                405                 410                 415

Ile Ala Ala Ile Gly Lys Leu Met Phe Ala Gln Phe Ser Glu Leu Val
            420                 425                 430

Asn Asp Phe Tyr Asn Asn Gly Leu Pro Ser Asn Leu Thr Ala Ser Ser
        435                 440                 445

Asn Pro Ser Leu Asp Tyr Gly Phe Lys Gly Ala Glu Ile Ala Met Ala
    450                 455                 460

Ser Tyr Cys Ser Glu Leu Gln Tyr Leu Ala Asn Pro Val Thr Ser His
465                 470                 475                 480

Val Gln Ser Ala Glu Gln His Asn Gln Asp Val Asn Ser Leu Gly Leu
                485                 490                 495

Ile Ser Ser Arg Lys Thr Ser Glu Ala Val Asp Ile Leu Lys Leu Met
            500                 505                 510

Ser Thr Thr Phe Leu Val Gly Ile Cys Gln Ala Val Asp Leu Arg His
        515                 520                 525

Leu Glu Glu Asn Leu Arg Gln Thr Val Lys Asn Thr Val Ser Gln Val
    530                 535                 540

Ala Lys Lys Val Leu Thr Thr Gly Ile Asn Gly Glu Leu His Pro Ser

545                 550                 555                 560

Arg Phe Cys Glu Lys Asp Leu Leu Lys Val Val Asp Arg Glu Gln Val
                565                 570                 575

Phe Thr Tyr Val Asp Asp Pro Cys Ser Ala Thr Tyr Pro Leu Met Gln
            580                 585                 590

Arg Leu Arg Gln Val Ile Val Asp His Ala Leu Ser Asn Gly Glu Thr
        595                 600                 605

Glu Lys Asn Ala Val Thr Ser Ile Phe Gln Lys Ile Gly Ala Phe Glu
    610                 615                 620

Glu Glu Leu Lys Ala Val Leu Pro Lys Glu Val Glu Ala Ala Arg Ala
625                 630                 635                 640

Ala Tyr Gly Asn Gly Thr Ala Pro Ile Pro Asn Arg Ile Lys Glu Cys
                645                 650                 655

Arg Ser Tyr Pro Leu Tyr Arg Phe Val Arg Glu Glu Leu Gly Thr Lys
            660                 665                 670

Leu Leu Thr Gly Glu Lys Val Val Ser Pro Gly Glu Glu Phe Asp Lys
        675                 680                 685

Val Phe Thr Ala Met Cys Glu Gly Lys Leu Ile Asp Pro Leu Met Asp
    690                 695                 700

Cys Leu Lys Glu Trp Asn Gly Ala Pro Ile Pro Ile Cys
705                 710                 715

<210>  33
<211>  718
<212>  PRT
<213>  Isoetes lacustris

<220>
<221>  POLYPEPTIDE
<222>  (1)..(718)
<223>  phenylalanine ammonia-lyase

<300>
<308>  GenBank: AAW80637.1
<309>  2016-07-26
<313>  (1)..(718)

<400>  33

Met Val Ala Asn Glu Asn Pro Ala Ala Tyr Ile Pro Asn Gly Ile Ser
1               5                   10                  15

Leu Cys Ile Thr Pro Pro Ala Ala Met Lys Lys Ser Asp Pro Leu Asn
            20                  25                  30

Trp Gly Lys Ala Gly Glu Ala Leu Gln Arg Ser His Leu Glu Glu Val
        35                  40                  45

Lys Glu Met Ile Lys Thr Val Tyr Ser Ser Lys Lys Val Ser Ile Glu
    50                  55                  60

Gly Thr Lys Leu Thr Ile Ala Gln Val Ala Ala Ile Ala Arg Arg Ala
65                  70                  75                  80

Glu Val Glu Val His Leu Asp Ala Glu Ala Ala Lys Lys Arg Val Glu
                85                  90                  95

Glu Ser Ser Asn Trp Val Leu Gln Asn Ala Met Lys Gly Thr Asp Thr
            100                 105                 110

Tyr Gly Val Thr Thr Gly Phe Gly Ala Thr Ser His Arg Arg Thr Asp
        115                 120                 125

Gln Gly Val Glu Leu Gln Lys Glu Leu Ile Arg Phe Leu Asn Ala Gly
    130                 135                 140

Ile Phe His Ser Pro Glu Gly Cys Asp Asn Val Leu Pro Ser Ser Thr
145                 150                 155                 160

Ala Arg Ala Ala Met Leu Val Arg Thr Asn Thr Leu Met Gln Gly Tyr
                165                 170                 175

Ser Gly Ile Arg Trp Glu Ile Leu Ala Thr Met Glu Lys Leu Leu Asn
            180                 185                 190

Ala Asn Ile Thr Pro Lys Leu Pro Leu Arg Gly Thr Ile Thr Ala Ser
        195                 200                 205

Gly Asp Leu Val Pro Leu Ser Tyr Ile Ala Gly Leu Leu Thr Gly Arg
    210                 215                 220

Pro Asn Ser Arg Ala Val Thr Cys Asp Gly Lys Ile Ile Thr Gly Ala
225                 230                 235                 240

Glu Ala Leu Ala Met Val Gly Val Glu Lys Pro Phe Glu Leu Gln Pro
                245                 250                 255

Lys Glu Gly Leu Ala Leu Val Asn Gly Thr Ala Val Gly Ser Gly Leu

260                 265                 270

Ala Ser Ile Val Cys Phe Glu Ala Asn Val Leu Ala Val Leu Ala Glu
        275                 280                 285

Ile Leu Ser Ala Phe Phe Cys Glu Val Met Gln Gly Lys Ala Glu Phe
    290                 295                 300

Thr Asp Pro Leu Thr His Lys Leu Lys His His Pro Gly Gln Ile Glu
305                 310                 315                 320

Ala Ala Ala Val Met Glu Tyr Val Leu Glu Gly Ser Ala Tyr Met Lys
                325                 330                 335

Ala Ala Ala Lys Leu His Glu Thr Asp Ser Leu Lys Lys Pro Lys Gln
            340                 345                 350

Asp Arg Tyr Ala Leu Arg Thr Ser Pro Gln Trp Leu Gly Pro Gln Ile
        355                 360                 365

Glu Val Ile Arg Met Ala Ser His Ser Ile Glu Arg Glu Ile Asn Ser
    370                 375                 380

Val Asn Asp Asn Pro Ile Ile Asp Val Ser Arg Asp Lys Ala Leu His
385                 390                 395                 400

Gly Gly Asn Phe Gln Gly Thr Pro Ile Gly Val Ser Met Asp Asn Val
                405                 410                 415

Arg Leu Ser Ile Ala Ala Ile Gly Lys Leu Met Phe Ala Gln Phe Ser
            420                 425                 430

Glu Leu Val Cys Asp Phe Tyr Asn Asn Gly Leu Pro Ser Asn Leu Ser
        435                 440                 445

Gly Gly Pro Asn Pro Ser Leu Asp Tyr Gly Phe Lys Gly Ala Glu Ile
    450                 455                 460

Ala Met Ala Ser Tyr Cys Ser Glu Leu Gln Phe Leu Ala Asn Pro Val
465                 470                 475                 480

Thr Asn His Val Gln Ser Ala Glu Gln His Asn Gln Asp Val Asn Ser
                485                 490                 495

Leu Gly Leu Ile Ser Ala Arg Lys Thr Ala Glu Ala Val Glu Ile Leu
            500                 505                 510

Lys Leu Met Ser Thr Ser Phe Leu Val Ala Leu Cys Gln Ala Val Asp
        515                 520                 525

Leu Arg His Leu Glu Glu Ile Met Gln Ser Thr Val Lys Ser Val Val
    530                 535                 540

Ser His Ala Ala Lys Lys Thr Leu Thr Thr Gly Leu Gly Gly Val Leu
545                 550                 555                 560

Leu Pro Ser Arg Phe Cys Glu Lys Glu Leu Leu Gln Val Val Asp Asn
                565                 570                 575

Val His Val Phe Thr Tyr Val Asp Asp Pro Ala Ser Ala Ala Tyr Pro
            580                 585                 590

Leu Met Gln Lys Leu Arg Gln Val Leu Val Glu His Ser Leu Lys Asn
        595                 600                 605

Ile His Glu Glu Gly Asp Glu Ser Thr Ser Val Phe Lys Lys Ile Thr
    610                 615                 620

Leu Phe Glu Glu Glu Leu Lys Lys Gln Leu Val Thr Glu Val Pro Leu
625                 630                 635                 640

Ala Arg Asp Ala Tyr Asp Lys Gly Gln Phe Ser Ile Ala Asn Lys Ile
                645                 650                 655

Gln Glu Cys Arg Ser Tyr Pro Leu Tyr Glu Phe Val Arg Lys Glu Ala
            660                 665                 670

Gly Thr Thr Leu Leu Ser Gly Thr Arg Val Leu Ser Pro Gly Glu Asp
        675                 680                 685

Phe Asp Lys Val Tyr Ala Ala Met Ser Ala Gly Lys Leu Val Thr Pro
    690                 695                 700

Leu Leu Lys Cys Val Asp Gly Trp Ser Gly Ala Pro Ser Phe
705                 710                 715

<210>  34
<211>  717
<212>  PRT
<213>  Arabidopsis lyrata subsp. lyrata

<220>
<221>  POLYPEPTIDE
<222>  (1)..(717)
<223>  phenylalanine ammonia-lyase

<300>
<308>  NCBI Reference Sequence: XP_002877909.1
<309>  2017-05-11
<313>  (1)..(717)

<400>  34

Met Asp Gln Ile Glu Ala Met Leu Cys Gly Gly Gly Glu Lys Thr Lys
1               5                   10                  15

Val Ala Val Thr Thr Lys Thr Leu Ala Asp Pro Leu Asn Trp Gly Leu
            20                  25                  30

Ala Ala Asp Gln Met Lys Gly Ser His Leu Asp Glu Val Lys Lys Met
        35                  40                  45

Val Glu Glu Tyr Arg Arg Pro Val Val Asn Leu Gly Gly Glu Thr Leu
    50                  55                  60

Thr Ile Gly Gln Val Ala Ala Ile Ser Ile Val Gly Gly Asn Val Lys
65                  70                  75                  80

Val Glu Leu Ala Glu Ala Ser Arg Ala Gly Val Lys Ala Ser Ser Asp
                85                  90                  95

Trp Val Met Glu Ser Met Asn Lys Gly Thr Asp Ser Tyr Gly Val Thr
            100                 105                 110

Thr Gly Phe Gly Ala Thr Ser His Arg Arg Thr Lys Asn Gly Thr Ala
        115                 120                 125

Leu Gln Thr Glu Leu Ile Arg Phe Leu Asn Ala Gly Ile Phe Gly Asn
    130                 135                 140

Thr Lys Glu Thr Cys His Thr Leu Pro Gln Ser Ala Thr Arg Ala Ala
145                 150                 155                 160

Met Leu Val Arg Val Asn Thr Leu Leu Gln Gly Tyr Ser Gly Ile Arg
                165                 170                 175

Phe Glu Ile Leu Glu Ala Ile Thr Ser Leu Leu Asn His Asn Ile Ser
            180                 185                 190

Pro Ser Leu Pro Leu Arg Gly Thr Ile Thr Ala Ser Gly Asp Leu Val
        195                 200                 205

Pro Leu Ser Tyr Ile Ala Gly Leu Leu Thr Gly Arg Pro Asn Ser Lys
    210                 215                 220

Ala Thr Gly Pro Asp Gly Glu Ser Leu Thr Ala Lys Glu Ala Phe Glu
225                 230                 235                 240

Lys Ala Gly Ile Ser Thr Gly Phe Phe Asp Leu Gln Pro Lys Glu Gly
                245                 250                 255

Leu Ala Leu Val Asn Gly Thr Ala Val Gly Ser Gly Met Ala Ser Met
            260                 265                 270

Val Leu Phe Glu Ala Asn Val Gln Ala Val Leu Ala Glu Val Leu Ser
        275                 280                 285

Ala Ile Phe Ala Glu Val Met Ser Gly Lys Pro Glu Phe Thr Asp His
    290                 295                 300

Leu Thr His Arg Leu Lys His His Pro Gly Gln Ile Glu Ala Ala Ala
305                 310                 315                 320

Ile Met Glu His Ile Leu Asp Gly Ser Ser Tyr Met Lys Leu Ala Gln
                325                 330                 335

Lys Leu His Glu Met Asp Pro Leu Gln Lys Pro Lys Gln Asp Arg Tyr
            340                 345                 350

Ala Leu Arg Thr Ser Pro Gln Trp Leu Gly Pro Gln Ile Glu Val Ile
        355                 360                 365

Arg Gln Ala Thr Lys Ser Ile Glu Arg Glu Ile Asn Ser Val Asn Asp
    370                 375                 380

Asn Pro Leu Ile Asp Val Ser Arg Asn Lys Ala Ile His Gly Gly Asn
385                 390                 395                 400

Phe Gln Gly Thr Pro Ile Gly Val Ser Met Asp Asn Thr Arg Leu Ala
                405                 410                 415

Ile Ala Ala Ile Gly Lys Leu Met Phe Ala Gln Phe Ser Glu Leu Val
            420                 425                 430

Asn Asp Phe Tyr Asn Asn Gly Leu Pro Ser Asn Leu Thr Ala Ser Ser
        435                 440                 445

Asn Pro Ser Leu Asp Tyr Gly Phe Lys Gly Ala Glu Ile Ala Met Ala
    450                 455                 460

Ser Tyr Cys Ser Glu Leu Gln Tyr Leu Ala Asn Pro Val Thr Ser His
465                 470                 475                 480

Val Gln Ser Ala Glu Gln His Asn Gln Asp Val Asn Ser Leu Gly Leu
                485                 490                 495

Ile Ser Ser Arg Lys Thr Ser Glu Ala Val Asp Ile Leu Lys Leu Met
            500                 505                 510

Ser Thr Thr Phe Leu Val Gly Ile Cys Gln Ala Val Asp Leu Arg His
        515                 520                 525

Leu Glu Glu Asn Leu Arg Gln Thr Val Lys Asn Thr Val Ser Gln Val
    530                 535                 540

Ala Lys Lys Val Leu Thr Thr Gly Ile Asn Gly Glu Leu His Pro Ser
545                 550                 555                 560

Arg Phe Cys Glu Lys Asp Leu Leu Lys Val Val Asp Arg Glu Gln Val
                565                 570                 575

Phe Thr Tyr Val Asp Asp Pro Cys Ser Ala Thr Tyr Pro Leu Met Gln
            580                 585                 590

Arg Leu Arg Gln Val Ile Val Asp His Ala Leu Ser Asn Gly Glu Thr
        595                 600                 605

Glu Lys Asn Ala Val Thr Ser Ile Phe His Lys Ile Gly Ala Phe Glu
    610                 615                 620

Glu Glu Leu Lys Ala Val Leu Pro Lys Glu Val Glu Ala Ala Arg Ala
625                 630                 635                 640

Ala Tyr Gly Asn Gly Thr Ala Pro Ile Pro Asn Arg Ile Lys Glu Cys
                645                 650                 655

Arg Ser Tyr Pro Leu Tyr Arg Phe Val Arg Glu Glu Leu Gly Thr Lys
            660                 665                 670

Leu Leu Thr Gly Glu Lys Val Val Ser Pro Gly Glu Glu Phe Asp Lys
        675                 680                 685

Val Phe Thr Ala Met Cys Glu Gly Lys Ile Ile Asp Pro Leu Met Asp
    690                 695                 700

Cys Leu Lys Glu Trp Asn Gly Ala Pro Ile Pro Ile Cys
705                 710                 715

<210>  35
<211>  693

<212>  PRT
<213>  Parrya nudicaulis

<220>
<221>  POLYPEPTIDE
<222>  (1)..(693)
<223>  phenylalanine ammonia-lyase

<300>
<308>  GenBank: ADY02660.1
<309>  2016-07-25
<313>  (1)..(693)

<400>  35

Ser Asp Pro Leu Asn Trp Gly Ala Ala Ala Glu Gln Met Lys Gly Ser
1               5                   10                  15

His Leu Asp Glu Val Lys Arg Met Val Glu Glu Phe Arg Arg Pro Phe
            20                  25                  30

Val Asn Leu Gly Gly Glu Thr Leu Thr Ile Gly Gln Val Ala Ala Ile
        35                  40                  45

Ser Thr Val Gly Asn Gly Val Lys Val Glu Leu Ser Glu Thr Ala Arg
    50                  55                  60

Ala Gly Val Lys Ala Ser Ser Asp Trp Val Met Glu Ser Met Asn Lys
65                  70                  75                  80

Gly Thr Asp Ser Tyr Gly Val Thr Thr Gly Phe Gly Ala Thr Ser His
                85                  90                  95

Arg Arg Thr Lys Asn Gly Val Ala Leu Gln Lys Glu Leu Ile Arg Phe
            100                 105                 110

Leu Asn Ala Gly Ile Phe Gly Ser Thr Lys Glu Thr Cys His Thr Leu
        115                 120                 125

Pro His Ser Ala Thr Arg Ala Ala Met Leu Val Arg Ile Asn Thr Leu
    130                 135                 140

Leu Gln Gly Tyr Ser Gly Ile Arg Phe Glu Ile Leu Glu Ala Ile Thr
145                 150                 155                 160

Ser Phe Leu Asn Asn Asn Ile Thr Pro Ser Leu Pro Leu Arg Gly Thr
                165                 170                 175

Ile Thr Ala Ser Gly Asp Leu Val Pro Leu Ser Tyr Ile Ala Gly Leu
            180                 185                 190

Leu Thr Gly Arg Pro Asn Ser Lys Ala Thr Gly Pro Asn Gly Glu Ala
        195                 200                 205

Leu Asn Ala Glu Glu Ala Phe Lys Met Ala Gly Ile Ser Ser Gly Phe
    210                 215                 220

Phe Asp Leu Gln Pro Lys Glu Gly Leu Ala Leu Val Asn Gly Thr Ala
225                 230                 235                 240

Val Gly Ser Gly Met Ala Ser Met Val Leu Phe Glu Thr Asn Val Leu
                245                 250                 255

Ser Val Leu Ala Glu Ile Leu Ser Ala Val Phe Ala Glu Val Met Ser
            260                 265                 270

Gly Lys Pro Glu Phe Thr Asp His Leu Thr His Arg Leu Lys His His
        275                 280                 285

Pro Gly Gln Ile Glu Ala Ala Ala Ile Met Glu His Ile Leu Asp Gly
    290                 295                 300

Ser Ser Tyr Met Lys Leu Ala Lys Lys Leu His Glu Met Asp Pro Leu
305                 310                 315                 320

Gln Lys Pro Lys Gln Asp Arg Tyr Ala Leu Arg Thr Ser Pro Gln Trp
                325                 330                 335

Leu Gly Pro Gln Ile Glu Val Ile Arg Tyr Ala Thr Lys Ser Ile Glu
            340                 345                 350

Arg Glu Ile Asn Ser Val Asn Asp Asn Pro Leu Ile Asp Val Ser Arg
        355                 360                 365

Asn Lys Ala Ile His Gly Gly Asn Phe Gln Gly Thr Pro Ile Gly Val
    370                 375                 380

Ser Met Asp Asn Thr Arg Leu Ala Ile Ala Ala Ile Gly Lys Leu Met
385                 390                 395                 400

Phe Ala Gln Phe Ser Glu Leu Val Asn Asp Phe Tyr Asn Asn Gly Leu
                405                 410                 415

Pro Ser Asn Leu Thr Ala Ser Arg Tyr Pro Ser Leu Asp Tyr Gly Phe
            420                 425                 430

Lys Gly Ala Glu Ile Ala Met Ala Ser Tyr Cys Ser Glu Leu Gln Tyr
        435                 440                 445

Leu Ala Asn Pro Val Thr Ser His Val Gln Ser Ala Glu Gln His Asn
    450                 455                 460

Gln Asp Val Asn Ser Leu Gly Leu Ile Ser Ser Arg Lys Thr Ala Glu
465                 470                 475                 480

Ala Val Asp Ile Leu Lys Leu Met Ser Thr Thr Phe Leu Val Ala Ile
                485                 490                 495

Cys Gln Ala Val Asp Leu Arg His Leu Glu Glu Asn Leu Lys Gln Thr
            500                 505                 510

Val Lys Asn Thr Val Ser Gln Val Ala Lys Lys Val Leu Thr Thr Gly
        515                 520                 525

Ile Asn Gly Glu Leu His Pro Ser Arg Phe Cys Glu Lys Asp Leu Leu
    530                 535                 540

Lys Val Val Asp Arg Glu Gln Val Tyr Thr Tyr Ala Asp Asp Pro Cys
545                 550                 555                 560

Ser Ala Thr Tyr Pro Leu Ile Gln Lys Leu Arg Gln Val Ile Val Asp
                565                 570                 575

His Ala Leu Val Asn Gly Glu Ser Glu Lys Asn Ala Val Thr Ser Ile
            580                 585                 590

Phe His Lys Ile Gly Ala Phe Glu Glu Glu Leu Lys Ala Val Leu Pro
        595                 600                 605

Lys Glu Val Glu Ala Thr Arg Ala Ala Tyr Asp Asn Gly Thr Ser Ala
    610                 615                 620

Ile Pro Asn Arg Ile Lys Glu Cys Arg Ser Tyr Pro Leu Tyr Arg Phe
625                 630                 635                 640

Val Arg Glu Glu Leu Gly Thr Glu Leu Leu Thr Gly Glu Lys Val Thr
                645                 650                 655

Ser Pro Gly Glu Glu Phe Asp Lys Val Phe Thr Ala Ile Cys Glu Gly
            660                 665                 670

Lys His Ile Asp Pro Met Met Glu Cys Leu Asn Glu Trp Asn Gly Ala
        675                 680                 685

Pro Ile Pro Ile Cys

    690

<210>  36
<211>  1518
<212>  DNA
<213>  Arabidopsis thaliana

<220>
<221>  CDS
<222>  (1)..(1518)

<400>  36
atg gac ttg ttg ttg ttg gaa aag tcc ttg att gct gtt ttc gtt gct      48
Met Asp Leu Leu Leu Leu Glu Lys Ser Leu Ile Ala Val Phe Val Ala
1               5                   10                  15

gtt att ttg gcc acc gtt atc tct aaa ttg aga ggt aag aaa ttg aag      96
Val Ile Leu Ala Thr Val Ile Ser Lys Leu Arg Gly Lys Lys Leu Lys
            20                  25                  30

ttg cca cca ggt cca att cca atc cca att ttt ggt aat tgg ttg caa      144
Leu Pro Pro Gly Pro Ile Pro Ile Pro Ile Phe Gly Asn Trp Leu Gln
        35                  40                  45

gtt ggt gat gac ttg aac cac aga aac ttg gtt gat tac gct aaa aag      192
Val Gly Asp Asp Leu Asn His Arg Asn Leu Val Asp Tyr Ala Lys Lys
    50                  55                  60

ttc ggt gat ttg ttc ttg ttg aga atg ggt caa aga aat ttg gtc gtt      240
Phe Gly Asp Leu Phe Leu Leu Arg Met Gly Gln Arg Asn Leu Val Val
65                  70                  75                  80

gtt tcc tca cca gac ttg acc aaa gaa gtt ttg ttg act caa ggt gtc      288
Val Ser Ser Pro Asp Leu Thr Lys Glu Val Leu Leu Thr Gln Gly Val
                85                  90                  95

gaa ttc ggt tcc aga act aga aat gtt gtt ttc gat atc ttc acc ggt      336
Glu Phe Gly Ser Arg Thr Arg Asn Val Val Phe Asp Ile Phe Thr Gly
            100                 105                 110

aag ggt caa gat atg gtt ttt act gtt tac ggt gaa cat tgg aga aag      384
Lys Gly Gln Asp Met Val Phe Thr Val Tyr Gly Glu His Trp Arg Lys
        115                 120                 125

atg aga aga att atg acc gtt cca ttc ttc acc aac aag gtt gtc caa      432
Met Arg Arg Ile Met Thr Val Pro Phe Phe Thr Asn Lys Val Val Gln
    130                 135                 140

caa aac aga gaa ggt tgg gaa ttt gaa gct gct tct gtt gtt gaa gat      480
Gln Asn Arg Glu Gly Trp Glu Phe Glu Ala Ala Ser Val Val Glu Asp
145                 150                 155                 160

gtc aag aag aat cca gat tct gct act aag ggt atc gtt ttg aga aaa      528
Val Lys Lys Asn Pro Asp Ser Ala Thr Lys Gly Ile Val Leu Arg Lys
                165                 170                 175

aga ttg caa ttg atg atg tac aac aac atg ttc aga atc atg ttc gac      576
Arg Leu Gln Leu Met Met Tyr Asn Asn Met Phe Arg Ile Met Phe Asp
            180                 185                 190

aga aga ttt gaa tcc gaa gat gac cct ttg ttt ttg aga ttg aag gct      624

Arg Arg Phe Glu Ser Glu Asp Asp Pro Leu Phe Leu Arg Leu Lys Ala
        195                 200                 205

ttg aac ggt gaa aga tct aga ttg gct caa tcc ttc gaa tac aac tac      672
Leu Asn Gly Glu Arg Ser Arg Leu Ala Gln Ser Phe Glu Tyr Asn Tyr
    210                 215                 220

ggt gat ttc atc cca atc tta aga cca ttc ttg aga ggt tac ttg aag      720
Gly Asp Phe Ile Pro Ile Leu Arg Pro Phe Leu Arg Gly Tyr Leu Lys
225                 230                 235                 240

atc tgc caa gat gtt aag gat aga aga atc gcc ttg ttc aaa aag tac      768
Ile Cys Gln Asp Val Lys Asp Arg Arg Ile Ala Leu Phe Lys Lys Tyr
                245                 250                 255

ttc gtt gac gaa aga aag caa atc gct tct tct aaa cct act ggt tct      816
Phe Val Asp Glu Arg Lys Gln Ile Ala Ser Ser Lys Pro Thr Gly Ser
            260                 265                 270

gaa ggt ttg aag tgc gcc att gat cat att ttg gaa gct gaa caa aag      864
Glu Gly Leu Lys Cys Ala Ile Asp His Ile Leu Glu Ala Glu Gln Lys
        275                 280                 285

ggt gaa atc aac gaa gat aac gtc ttg tac atc gtc gaa aac att aac      912
Gly Glu Ile Asn Glu Asp Asn Val Leu Tyr Ile Val Glu Asn Ile Asn
    290                 295                 300

gtt gct gct att gaa act acc ttg tgg tct att gaa tgg ggt att gct      960
Val Ala Ala Ile Glu Thr Thr Leu Trp Ser Ile Glu Trp Gly Ile Ala
305                 310                 315                 320

gaa ttg gtt aat cac cca gaa atc caa tcc aag ttg aga aac gaa ttg     1008
Glu Leu Val Asn His Pro Glu Ile Gln Ser Lys Leu Arg Asn Glu Leu
                325                 330                 335

gat act gtt ttg ggt cca ggt gtt caa gtt act gaa cct gac ttg cat     1056
Asp Thr Val Leu Gly Pro Gly Val Gln Val Thr Glu Pro Asp Leu His
            340                 345                 350

aag ttg cca tac ttg caa gct gtt gta aaa gaa acc ttg aga tta aga     1104
Lys Leu Pro Tyr Leu Gln Ala Val Val Lys Glu Thr Leu Arg Leu Arg
        355                 360                 365

atg gcc atc cct ttg ttg gtt cca cat atg aac ttg cat gat gct aaa     1152
Met Ala Ile Pro Leu Leu Val Pro His Met Asn Leu His Asp Ala Lys
    370                 375                 380

ttg gcc ggt tat gat att cca gcc gaa tcc aag att ttg gtt aat gct     1200
Leu Ala Gly Tyr Asp Ile Pro Ala Glu Ser Lys Ile Leu Val Asn Ala
385                 390                 395                 400

tgg tgg ttg gct aac aat cca aat tct tgg aaa aag cca gaa gaa ttc     1248
Trp Trp Leu Ala Asn Asn Pro Asn Ser Trp Lys Lys Pro Glu Glu Phe
                405                 410                 415

aga cca gaa aga ttt ttc gaa gaa gaa agt cac gtt gaa gcc aac ggt     1296
Arg Pro Glu Arg Phe Phe Glu Glu Glu Ser His Val Glu Ala Asn Gly
            420                 425                 430

aat gat ttt aga tac gtt cca ttt ggt gtt ggt aga aga tct tgt cca     1344
Asn Asp Phe Arg Tyr Val Pro Phe Gly Val Gly Arg Arg Ser Cys Pro
        435                 440                 445

ggt att atc ttg gct ttg cca att ttg ggt att acc atc ggt aga atg          1392
Gly Ile Ile Leu Ala Leu Pro Ile Leu Gly Ile Thr Ile Gly Arg Met
    450                 455                 460

gtc caa aac ttc gaa tta ttg cca cca cct ggt caa tct aag gtt gat          1440
Val Gln Asn Phe Glu Leu Leu Pro Pro Pro Gly Gln Ser Lys Val Asp
465                 470                 475                 480

act tct gaa aag ggt ggt caa ttc tcc ttg cat att ttg aac cac tcc          1488
Thr Ser Glu Lys Gly Gly Gln Phe Ser Leu His Ile Leu Asn His Ser
                485                 490                 495

atc atc gtt atg aag cca aga aac tgt tga                                  1518
Ile Ile Val Met Lys Pro Arg Asn Cys
            500                 505


<210>  37
<211>  505
<212>  PRT
<213>  Arabidopsis thaliana

<400>  37

Met Asp Leu Leu Leu Leu Glu Lys Ser Leu Ile Ala Val Phe Val Ala
1               5                   10                  15


Val Ile Leu Ala Thr Val Ile Ser Lys Leu Arg Gly Lys Lys Leu Lys
            20                  25                  30


Leu Pro Pro Gly Pro Ile Pro Ile Pro Ile Phe Gly Asn Trp Leu Gln
        35                  40                  45


Val Gly Asp Asp Leu Asn His Arg Asn Leu Val Asp Tyr Ala Lys Lys
    50                  55                  60


Phe Gly Asp Leu Phe Leu Leu Arg Met Gly Gln Arg Asn Leu Val Val
65                  70                  75                  80


Val Ser Ser Pro Asp Leu Thr Lys Glu Val Leu Leu Thr Gln Gly Val
                85                  90                  95


Glu Phe Gly Ser Arg Thr Arg Asn Val Val Phe Asp Ile Phe Thr Gly
            100                 105                 110


Lys Gly Gln Asp Met Val Phe Thr Val Tyr Gly Glu His Trp Arg Lys
        115                 120                 125


Met Arg Arg Ile Met Thr Val Pro Phe Phe Thr Asn Lys Val Val Gln
    130                 135                 140


Gln Asn Arg Glu Gly Trp Glu Phe Glu Ala Ala Ser Val Val Glu Asp
145                 150                 155                 160

Val Lys Lys Asn Pro Asp Ser Ala Thr Lys Gly Ile Val Leu Arg Lys
                165                 170                 175

Arg Leu Gln Leu Met Met Tyr Asn Asn Met Phe Arg Ile Met Phe Asp
            180                 185                 190

Arg Arg Phe Glu Ser Glu Asp Asp Pro Leu Phe Leu Arg Leu Lys Ala
        195                 200                 205

Leu Asn Gly Glu Arg Ser Arg Leu Ala Gln Ser Phe Glu Tyr Asn Tyr
    210                 215                 220

Gly Asp Phe Ile Pro Ile Leu Arg Pro Phe Leu Arg Gly Tyr Leu Lys
225                 230                 235                 240

Ile Cys Gln Asp Val Lys Asp Arg Arg Ile Ala Leu Phe Lys Lys Tyr
                245                 250                 255

Phe Val Asp Glu Arg Lys Gln Ile Ala Ser Ser Lys Pro Thr Gly Ser
            260                 265                 270

Glu Gly Leu Lys Cys Ala Ile Asp His Ile Leu Glu Ala Glu Gln Lys
        275                 280                 285

Gly Glu Ile Asn Glu Asp Asn Val Leu Tyr Ile Val Glu Asn Ile Asn
    290                 295                 300

Val Ala Ala Ile Glu Thr Thr Leu Trp Ser Ile Glu Trp Gly Ile Ala
305                 310                 315                 320

Glu Leu Val Asn His Pro Glu Ile Gln Ser Lys Leu Arg Asn Glu Leu
                325                 330                 335

Asp Thr Val Leu Gly Pro Gly Val Gln Val Thr Glu Pro Asp Leu His
            340                 345                 350

Lys Leu Pro Tyr Leu Gln Ala Val Val Lys Glu Thr Leu Arg Leu Arg
        355                 360                 365

Met Ala Ile Pro Leu Leu Val Pro His Met Asn Leu His Asp Ala Lys
    370                 375                 380

Leu Ala Gly Tyr Asp Ile Pro Ala Glu Ser Lys Ile Leu Val Asn Ala
385                 390                 395                 400

Trp Trp Leu Ala Asn Asn Pro Asn Ser Trp Lys Lys Pro Glu Glu Phe
                405                 410                 415

Arg Pro Glu Arg Phe Phe Glu Glu Glu Ser His Val Glu Ala Asn Gly
                420                 425                 430

Asn Asp Phe Arg Tyr Val Pro Phe Gly Val Gly Arg Arg Ser Cys Pro
        435                 440                 445

Gly Ile Ile Leu Ala Leu Pro Ile Leu Gly Ile Thr Ile Gly Arg Met
    450                 455                 460

Val Gln Asn Phe Glu Leu Leu Pro Pro Pro Gly Gln Ser Lys Val Asp
465                 470                 475                 480

Thr Ser Glu Lys Gly Gly Gln Phe Ser Leu His Ile Leu Asn His Ser
                485                 490                 495

Ile Ile Val Met Lys Pro Arg Asn Cys
                500                 505

<210>  38
<211>  498
<212>  PRT
<213>  Daucus carota subsp. sativus

<220>
<221>  POLYPEPTIDE
<222>  (1)..(498)
<223>  Cinnamate-4-hydroxylase

<300>
<308>  GenBank: KZM84967.1
<309>  2016-04-20
<313>  (1)..(498)

<400>  38

Met Met Asp Ala Leu Leu Glu Leu Glu Lys Ala Met Leu Gly Ile Phe
1               5                   10                  15

Leu Gly Ile Met Ile Ala Ile Phe Ile Ser Lys Leu Arg Arg Lys Gln
            20                  25                  30

Phe Lys Leu Pro Pro Gly Pro Thr Pro Val Pro Ile Phe Gly Asn Trp
        35                  40                  45

Leu Gln Val Gly Asn Asp Leu Asn His Gly Asn Leu Val Asp Phe Ala
    50                  55                  60

Lys Arg Phe Gly His Leu Phe Leu Leu Arg Met Gly Val Arg Asn Leu
65                  70                  75                  80

Val Val Val Ser Ser Pro Glu Leu Ala Lys Gln Val Leu His Thr Gln
                85                  90                  95

Gly Val Glu Phe Gly Ser Arg Cys Ser Asn Gly Gln Asp Leu Val Phe
            100                 105                 110

Thr Asn Tyr Gly Glu His Trp Arg Lys Met Arg Arg Ile Leu Thr Val
        115                 120                 125

Pro Leu Phe Thr Asn Lys Val Val Gln Arg Tyr Arg Phe Gly Trp Glu
    130                 135                 140

Asp Glu Ala Ala Arg Val Val Ala Asp Val Lys Ala Asn Pro Glu Ser
145                 150                 155                 160

Ser Thr Asn Gly Ile Val Leu Arg Lys Arg Leu Gln Leu Met Met Tyr
                165                 170                 175

Asn Asn Ala Tyr Lys Ile Met Phe Gly Arg Arg Phe Glu Ser Glu Asp
            180                 185                 190

Asp Pro Leu Leu Leu Lys Leu Lys Ala Ser Ser Trp Glu Arg Ile Arg
        195                 200                 205

Leu Thr Gln Ser Leu Asp Tyr Asn Tyr Gly Asp Phe Ile Pro Val Leu
    210                 215                 220

Arg Pro Phe Leu Lys Gly Tyr Leu Arg Leu Cys Lys Gln Val Lys Asp
225                 230                 235                 240

Glu Arg Phe Gln Leu Phe Lys Asp Asn Phe Val Asp Pro Arg Lys Met
                245                 250                 255

Leu Val Asn Thr Arg Arg Val Asp Asn Asp Ser Leu Thr Cys Ala Leu
            260                 265                 270

Asp His Leu Leu Glu Ala Gln Gln Gln Gly Asp Ile Thr Glu Glu Thr
        275                 280                 285

Ile Phe His Ile Val Asp Gly Leu Asp Ile Ala Ala Ile Asp Thr Thr
    290                 295                 300

Leu Arg Ser Ile Glu Trp Ala Ile Ala Glu Leu Ile Asn His Pro Glu
305                 310                 315                 320

Val Gln Lys Lys Leu Arg His Glu Ile Asp Ser Val Leu Gly Val Gly
                325                 330                 335

Val Gln Val Thr Glu Pro Asp Thr His Lys Leu Pro Tyr Leu Glu Ala
            340                 345                 350

Val Ile Lys Glu Thr Leu Arg Leu Arg Met Gly Val Pro Leu Leu Val
        355                 360                 365

Pro His Met Asn Leu His Asp Ala Lys Leu Gly Gly Tyr Asp Ile Pro
    370                 375                 380

Ala Lys Ser Lys Val Val Val Asn Ala Trp Trp Leu Ala Asn Asn Pro
385                 390                 395                 400

Ala His Trp Lys Asn Pro Glu Glu Phe Arg Pro Glu Arg Phe Met Glu
                405                 410                 415

Glu Glu Ser Asn Val Glu Thr Asn Gly Asn Asp Phe Arg Phe Leu Pro
            420                 425                 430

Phe Gly Ala Gly Arg Arg Ser Cys Pro Gly Ile Met Thr Ala Leu Pro
        435                 440                 445

Ile Met Gly Ile Thr Leu Gly Arg Leu Val Gln Asn Phe Glu Leu Leu
    450                 455                 460

Pro Pro Pro Gly Gln Ser Lys Ile Asp Thr Ala Glu Lys Arg Gly Gln
465                 470                 475                 480

Phe Leu Leu Gln Ile Leu Lys His Ser Thr Val Val Ala Lys Pro Arg
                485                 490                 495

Ser Ile

<210>  39
<211>  506
<212>  PRT
<213>  Capsella rubella

<220>
<221>  POLYPEPTIDE
<222>  (1)..(506)
<223>  Cinnamate-4-hydroxylase

<300>
<308>  NCBI Reference Sequence: XP_006294053.1
<309>  2018-02-02
<313>  (1)..(506)

<400>  39

Met Met Asp Leu Leu Leu Leu Glu Lys Ser Leu Ile Ala Val Phe Val
1               5                   10                  15

Ala Val Val Leu Ala Thr Val Val Ser Lys Leu Arg Gly Lys Lys Leu
            20                  25                  30

Lys Leu Pro Pro Gly Pro Met Pro Ile Pro Ile Phe Gly Asn Trp Leu
        35                  40                  45

Gln Val Gly Asp Asp Leu Asn His Arg Asn Leu Val Asp Tyr Ala Lys
    50                  55                  60

Lys Phe Gly Asp Leu Phe Leu Leu Arg Met Gly Gln Arg Asn Leu Val
65                  70                  75                  80

Val Val Ser Ser Pro Asn Leu Thr Lys Glu Val Leu His Thr Gln Gly
                85                  90                  95

Val Glu Phe Gly Ser Arg Thr Arg Asn Val Val Phe Asp Ile Phe Thr
            100                 105                 110

Gly Lys Gly Gln Asp Met Val Phe Thr Val Tyr Gly Glu His Trp Arg
        115                 120                 125

Lys Met Arg Arg Ile Met Thr Val Pro Phe Phe Thr Asn Lys Val Val
    130                 135                 140

Gln Gln Asn Arg Glu Gly Trp Glu Phe Glu Ala Ala Ser Val Val Glu
145                 150                 155                 160

Asp Val Lys Lys Asn Pro Asp Ser Ala Thr Lys Gly Ile Val Leu Arg
                165                 170                 175

Lys Arg Leu Gln Leu Met Met Tyr Asn Asn Met Phe Arg Ile Met Phe
            180                 185                 190

Asp Arg Arg Phe Glu Ser Glu Asp Asp Pro Leu Phe Leu Arg Leu Lys
        195                 200                 205

Ala Leu Asn Gly Glu Arg Ser Arg Leu Ala Gln Ser Phe Glu Tyr Asn
    210                 215                 220

Tyr Gly Asp Phe Ile Pro Ile Leu Arg Pro Phe Leu Arg Gly Tyr Leu
225                 230                 235                 240

Lys Ile Cys Gln Asp Val Lys Asp Arg Arg Ile Ala Leu Phe Lys Lys
                245                 250                 255

Tyr Phe Val Asp Glu Arg Lys Gln Ile Ala Ser Ser Lys Pro Thr Gly
            260                 265                 270

Ser Glu Gly Leu Lys Cys Ala Ile Asp His Ile Leu Glu Ala Glu Gln
        275                 280                 285

Lys Gly Glu Ile Asn Glu Asp Asn Val Leu Tyr Ile Val Glu Asn Ile
    290                 295                 300

Asn Val Ala Ala Ile Glu Thr Thr Leu Trp Ser Ile Glu Trp Gly Ile
305                 310                 315                 320

Ala Glu Leu Val Asn His Pro Glu Ile Gln Ser Lys Leu Arg Asn Glu
                325                 330                 335

Met Asp Thr Val Leu Gly Leu Gly Val Gln Val Thr Glu Pro Glu Leu
            340                 345                 350

His Lys Leu Pro Tyr Leu Gln Ala Val Val Lys Glu Thr Leu Arg Leu
        355                 360                 365

Arg Met Ala Ile Pro Leu Leu Val Pro His Met Asn Leu His Asp Ala
    370                 375                 380

Lys Leu Ala Gly Tyr Asp Ile Pro Ala Glu Ser Lys Ile Leu Val Asn
385                 390                 395                 400

Ala Trp Trp Leu Ala Asn Asn Pro Glu Ser Trp Lys Lys Pro Glu Glu
                405                 410                 415

Phe Arg Pro Glu Arg Phe Phe Glu Glu Glu Ala His Val Glu Ala Asn
            420                 425                 430

Gly Asn Asp Phe Arg Tyr Val Pro Phe Gly Val Gly Arg Arg Ser Cys
        435                 440                 445

Pro Gly Ile Ile Leu Ala Leu Pro Ile Leu Gly Ile Thr Ile Gly Arg
    450                 455                 460

Met Val Gln Asn Phe Glu Leu Leu Pro Pro Pro Gly Gln Ser Lys Ile
465                 470                 475                 480

Asp Thr Ser Glu Lys Gly Gly Gln Phe Ser Leu His Ile Leu Asn His
                485                 490                 495

Ser Thr Ile Val Met Lys Pro Arg Asn Phe

500                 505

<210>  40
<211>  505
<212>  PRT
<213>  Ginkgo biloba

<220>
<221>  POLYPEPTIDE
<222>  (1)..(505)
<223>  Cinnamate-4-hydroxylase

<300>
<308>  GenBank: AAW70021.1
<309>  2006-11-06
<313>  (1)..(505)

<400>  40

Met Leu Glu Ser Met Asn Leu Glu Lys Gly Leu Ile Ala Leu Phe Val
1               5                   10                  15

Val Val Val Ser Ala Ile Phe Val Ser Lys Leu Lys Ser Lys Lys Leu
            20                  25                  30

Lys Leu Pro Pro Gly Pro Phe Ala Leu Pro Ile Phe Gly Asn Trp Leu
        35                  40                  45

Gln Val Gly Asp Asp Leu Asn His Arg Asn Leu Thr Asp Leu Ala Lys
    50                  55                  60

Lys Tyr Gly Glu Ile Phe Leu Leu Lys Met Gly Gln Arg Asn Leu Val
65                  70                  75                  80

Val Val Ser Ser Pro Glu Tyr Ala Lys Glu Val Leu His Thr Gln Gly
                85                  90                  95

Val Glu Phe Gly Ser Arg Thr Arg Asn Val Val Phe Asp Ile Phe Thr
            100                 105                 110

Gly Lys Gly Gln Asp Met Val Phe Thr Val Tyr Gly Glu His Trp Arg
        115                 120                 125

Lys Met Arg Arg Ile Met Thr Val Pro Phe Phe Thr Asn Lys Val Val
    130                 135                 140

Gln Gln Tyr Arg Phe Ala Trp Glu Asp Glu Ile Ser Arg Ala Val Glu
145                 150                 155                 160

Asp Val Lys Asn Arg Pro Glu Ala Ser Thr Thr Gly Ile Val Ile Arg
                165                 170                 175

Arg Arg Leu Gln Leu Met Met Tyr Asn Ile Met Tyr Arg Met Met Phe
            180                 185                 190

Asp Arg Arg Phe Glu Ser Glu Glu Asp Pro Leu Phe Leu Lys Leu Lys
        195                 200                 205

Ala Leu Asn Gly Glu Arg Ser Arg Leu Ala Gln Ser Phe Glu Tyr Asn
    210                 215                 220

Tyr Gly Asp Phe Ile Pro Ile Leu Arg Pro Phe Leu Arg Gly Tyr Leu
225                 230                 235                 240

Lys Ile Cys Lys Glu Val Lys Glu Met Arg Leu Ser Leu Phe Lys Asp
                245                 250                 255

Tyr Phe Ile Asn Glu Arg Lys Lys Leu Ala Ser Thr Lys Gly Ser Ser
            260                 265                 270

Ser Leu Gly Glu Lys Cys Ala Ile Asp His Ile Leu Asp Ala Leu Asp
        275                 280                 285

Lys Gly Glu Ile Asn Glu Asp Asn Val Leu Tyr Ile Val Glu Asn Ile
    290                 295                 300

Asn Val Ala Ala Ile Glu Thr Thr Leu Trp Ser Met Glu Trp Gly Leu
305                 310                 315                 320

Ala Glu Ile Val Asn His Pro Asp Ile Gln Gln Lys Ile Arg Lys Glu
                325                 330                 335

Leu Asp Thr Val Leu Gly Pro Gly Val Glu Ile Thr Glu Pro Asp Thr
            340                 345                 350

Thr Arg Leu Pro Tyr Leu Gln Ala Val Val Lys Glu Thr Leu Arg Leu
        355                 360                 365

His Met Ala Ile Pro Leu Leu Val Pro His Met Asn Leu Asn Gln Ala
    370                 375                 380

Lys Leu Gly Gly Tyr Asp Ile Pro Ala Glu Ser Lys Ile Leu Val Asn
385                 390                 395                 400

Ala Trp Trp Leu Ala Asn Asn Pro Glu Trp Trp Asn Lys Pro Glu Glu
                405                 410                 415

Phe Ile Pro Glu Arg Phe Leu Glu Asp Glu Gln Lys Ile Glu Ala Asn

420                 425                 430

Gly Asn Asp Phe Arg Phe Leu Pro Phe Gly Val Gly Arg Arg Ser Cys
        435                 440                 445

Pro Gly Ile Ile Leu Ala Leu Pro Ile Leu Ala Leu Ser Leu Gly Arg
    450                 455                 460

Leu Val Gln Asn Phe Asp Leu Ser Pro Pro Pro Gly His Ser Lys Val
465                 470                 475                 480

Asp Val Ser Glu Lys Gly Gly Gln Phe Ser Leu His Ile Leu Asn His
                485                 490                 495

Ser Val Val Val Ala Lys Pro Arg Val
            500                 505

<210>  41
<211>  363
<212>  DNA
<213>  Saccharomyces cerevisiae

<220>
<221>  CDS
<222>  (1)..(363)
<223>  Gene encoding cytochrome b5

<300>
<308>  NCBI Reference Sequence: NM_001182949.3
<309>  2018-04-04
<313>  (1)..(363)

<400>  41
atg cct aaa gtt tac agt tac caa gaa gtt gcc gaa cac aat ggc cca      48
Met Pro Lys Val Tyr Ser Tyr Gln Glu Val Ala Glu His Asn Gly Pro
1               5                   10                  15

gaa aat ttc tgg att atc atc gat gac aaa gtt tac gat gtt tct caa      96
Glu Asn Phe Trp Ile Ile Ile Asp Asp Lys Val Tyr Asp Val Ser Gln
            20                  25                  30

ttc aaa gat gaa cat cca ggt ggt gat gaa att ata atg gat ttg ggt      144
Phe Lys Asp Glu His Pro Gly Gly Asp Glu Ile Ile Met Asp Leu Gly
        35                  40                  45

gga caa gat gct aca gaa agc ttt gtc gat atc ggt cat tct gac gaa      192
Gly Gln Asp Ala Thr Glu Ser Phe Val Asp Ile Gly His Ser Asp Glu
    50                  55                  60

gca ttg aga cta ctg aaa ggt tta tac att ggt gac gtt gac aag acc      240
Ala Leu Arg Leu Leu Lys Gly Leu Tyr Ile Gly Asp Val Asp Lys Thr
65                  70                  75                  80

agt gag cgc gtt tct gtg gaa aag gta tct acc tct gaa aac caa agt      288
Ser Glu Arg Val Ser Val Glu Lys Val Ser Thr Ser Glu Asn Gln Ser
                85                  90                  95

aaa ggt agt ggt aca ttg gtt gtc ata ttg gcc att tta atg cta ggt     336
Lys Gly Ser Gly Thr Leu Val Val Ile Leu Ala Ile Leu Met Leu Gly
            100                 105                 110

gtt gct tat tat ttg ttg aac gaa taa                                 363
Val Ala Tyr Tyr Leu Leu Asn Glu
        115                 120

<210>  42
<211>  120
<212>  PRT
<213>  Saccharomyces cerevisiae

<400>  42

Met Pro Lys Val Tyr Ser Tyr Gln Glu Val Ala Glu His Asn Gly Pro
1               5                   10                  15

Glu Asn Phe Trp Ile Ile Ile Asp Asp Lys Val Tyr Asp Val Ser Gln
            20                  25                  30

Phe Lys Asp Glu His Pro Gly Gly Asp Glu Ile Ile Met Asp Leu Gly
        35                  40                  45

Gly Gln Asp Ala Thr Glu Ser Phe Val Asp Ile Gly His Ser Asp Glu
    50                  55                  60

Ala Leu Arg Leu Leu Lys Gly Leu Tyr Ile Gly Asp Val Asp Lys Thr
65                  70                  75                  80

Ser Glu Arg Val Ser Val Glu Lys Val Ser Thr Ser Glu Asn Gln Ser
                85                  90                  95

Lys Gly Ser Gly Thr Leu Val Val Ile Leu Ala Ile Leu Met Leu Gly
            100                 105                 110

Val Ala Tyr Tyr Leu Leu Asn Glu
        115                 120

<210>  43
<211>  120
<212>  PRT
<213>  Saccharomyces eubayanus

<220>
<221>  POLYPEPTIDE
<222>  (1)..(120)
<223>  cytochrome b5

<300>
<308>  NCBI Reference Sequence: XP_018219767.1
<309>  2017-09-27

<313>  (1)..(120)

<400>  43

Met Pro Gln Val Tyr Ser Tyr Gln Glu Val Ala Glu His Asn Gly Pro
1               5                   10                  15

Glu Asn Cys Trp Ile Ile Ile Asp Asp Lys Val Tyr Asp Val Ser Gln
            20                  25                  30

Phe Lys Asp Glu His Pro Gly Gly Asp Glu Ile Ile Met Asp Leu Gly
        35                  40                  45

Gly Gln Asp Ala Thr Asp Ser Phe Val Asp Ile Gly His Ser Asp Glu
    50                  55                  60

Ala Leu Lys Leu Leu Lys Thr Leu Tyr Ile Gly Asp Val Asp Lys Thr
65                  70                  75                  80

Ser Glu Pro Val Val Val Lys Lys Ala Val Thr Ser Glu Ser Gln Ser
                85                  90                  95

Gln Gly Ser Ala Thr Leu Val Met Ile Leu Ala Val Val Met Leu Gly
            100                 105                 110

Val Ala Tyr Tyr Leu Leu Asn Glu
        115                 120


<210>  44
<211>  120
<212>  PRT
<213>  Saccharomyces cerevisiae YJM627


<220>
<221>  POLYPEPTIDE
<222>  (1)..(120)
<223>  cytochrome b5

<300>
<308>  GenBank: AJT08371.1
<309>  2016-05-13
<313>  (1)..(120)

<400>  44

Met Pro Lys Val Tyr Ser Tyr Gln Glu Val Ala Glu His Asn Gly Pro
1               5                   10                  15

Glu Asn Phe Trp Ile Ile Ile Asp Asp Lys Val Tyr Asp Val Ser Gln
            20                  25                  30

Phe Lys Asp Glu His Pro Gly Gly Asp Glu Ile Ile Met Asp Leu Gly

        35                  40                  45

Gly Gln Asp Ala Thr Glu Ser Phe Val Asp Ile Gly His Ser Asp Glu
    50                  55                  60

Ala Leu Arg Leu Leu Lys Gly Leu Tyr Ile Gly Asp Val Asp Lys Thr
65                  70                  75                  80

Ser Glu Arg Val Ser Val Glu Lys Val Ser Thr Ser Gly Asn Gln Ser
                85                  90                  95

Lys Gly Ser Gly Thr Leu Val Val Ile Leu Ala Ile Leu Met Leu Gly
            100                 105                 110

Val Ala Tyr Tyr Leu Leu Asn Glu
        115                 120

<210> 45
<211> 120
<212> PRT
<213> Saccharomyces arboricola H-6

<220>
<221> POLYPEPTIDE
<222> (1)..(120)
<223> cytochrome b5

<300>
<308> GenBank: EJS42027.1
<309> 2012-08-16
<313> (1)..(120)

<400> 45

Met Pro Lys Val Tyr Ser Tyr Gln Glu Val Ala Glu His Asn Gly Pro
1               5                   10                  15

Glu Asn Tyr Trp Ile Ile Ile Asp Asp Lys Val Tyr Asp Val Ser Gln
            20                  25                  30

Phe Lys Asp Glu His Pro Gly Gly Asp Glu Ile Ile Met Asp Leu Gly
        35                  40                  45

Gly Gln Asp Ala Thr Glu Ser Phe Val Asp Ile Gly His Ser Asp Glu
    50                  55                  60

Ala Leu Arg Leu Leu Lys Asp Leu Tyr Ile Gly Asp Val Asp Lys Ala
65                  70                  75                  80

Ser Lys Pro Val Ala Val Glu Lys Thr Ser Ser Ser Asp Asn Gln Ser
                85                  90                  95

Lys Gly Ser Gly Thr Leu Val Leu Ile Leu Ala Ile Val Met Leu Val
            100                 105                 110

Val Ala Tyr Tyr Leu Leu Asn Glu
        115                 120

<210>  46
<211>  2136
<212>  DNA
<213>  Arabidopsis thaliana

<220>
<221>  CDS
<222>  (1)..(2136)
<223>  Gene encoding cytochrime p450 reductase from Arabidopsis thaliana
       codon optimized for yeast

<300>
<308>  GenBank: AF325101.1
<309>  2001-03-11
<313>  (1)..(2136)

<400>  46
atg tcc tcc tct tct tca tca tcc acc tct atg att gat ttg atg gcc        48
Met Ser Ser Ser Ser Ser Ser Ser Thr Ser Met Ile Asp Leu Met Ala
1               5                   10                  15

gct att atc aag ggt gaa cca gtt ata gtt tct gat cca gct aat gct        96
Ala Ile Ile Lys Gly Glu Pro Val Ile Val Ser Asp Pro Ala Asn Ala
            20                  25                  30

tct gcc tat gaa tct gtt gct gct gaa tta tcc tcc atg ttg atc gaa       144
Ser Ala Tyr Glu Ser Val Ala Ala Glu Leu Ser Ser Met Leu Ile Glu
        35                  40                  45

aac aga caa ttc gct atg atc gtc act acc tct att gct gtt ttg att       192
Asn Arg Gln Phe Ala Met Ile Val Thr Thr Ser Ile Ala Val Leu Ile
    50                  55                  60

ggt tgc atc gtt atg ttg gtt tgg aga aga tct ggt tct ggt aac tct       240
Gly Cys Ile Val Met Leu Val Trp Arg Arg Ser Gly Ser Gly Asn Ser
65                  70                  75                  80

aaa aga gtc gaa cca ttg aag cca ttg gtt atc aaa cct aga gaa gaa       288
Lys Arg Val Glu Pro Leu Lys Pro Leu Val Ile Lys Pro Arg Glu Glu
                85                  90                  95

gaa att gac gac ggt aga aag aag gtt acc att ttc ttt ggt act caa       336
Glu Ile Asp Asp Gly Arg Lys Lys Val Thr Ile Phe Phe Gly Thr Gln
            100                 105                 110

acc ggt act gct gaa ggt ttt gct aaa gct ttg ggt gaa gaa gct aaa       384
Thr Gly Thr Ala Glu Gly Phe Ala Lys Ala Leu Gly Glu Glu Ala Lys
        115                 120                 125

gcc aga tac gaa aag act aga ttc aag atc gtt gac ttg gat gat tac       432
Ala Arg Tyr Glu Lys Thr Arg Phe Lys Ile Val Asp Leu Asp Asp Tyr
    130                 135                 140

gct gca gat gat gat gaa tac gaa gaa aag ttg aag aaa gaa gat gtc     480
Ala Ala Asp Asp Asp Glu Tyr Glu Glu Lys Leu Lys Lys Glu Asp Val
145                 150                 155                 160

gcc ttt ttc ttc ttg gct act tat ggt gat ggt gaa cct act gat aat     528
Ala Phe Phe Phe Leu Ala Thr Tyr Gly Asp Gly Glu Pro Thr Asp Asn
                165                 170                 175

gct gct aga ttt tac aag tgg ttc acc gaa ggt aat gat aga ggt gaa     576
Ala Ala Arg Phe Tyr Lys Trp Phe Thr Glu Gly Asn Asp Arg Gly Glu
            180                 185                 190

tgg ttg aaa aac ttg aag tac ggt gtt ttc ggt ttg ggt aat aga caa     624
Trp Leu Lys Asn Leu Lys Tyr Gly Val Phe Gly Leu Gly Asn Arg Gln
        195                 200                 205

tac gaa cac ttc aac aag gtt gcc aag gtt gtt gat gat atc ttg gtt     672
Tyr Glu His Phe Asn Lys Val Ala Lys Val Val Asp Asp Ile Leu Val
    210                 215                 220

gaa caa ggt gcc caa aga ttg gtt caa gtt ggt tta ggt gat gat gac     720
Glu Gln Gly Ala Gln Arg Leu Val Gln Val Gly Leu Gly Asp Asp Asp
225                 230                 235                 240

caa tgc atc gaa gat gat ttt act gct tgg aga gaa gct ttg tgg cca     768
Gln Cys Ile Glu Asp Asp Phe Thr Ala Trp Arg Glu Ala Leu Trp Pro
                245                 250                 255

gaa ttg gat aca atc ttg aga gaa gaa ggt gat act gct gtt gct act     816
Glu Leu Asp Thr Ile Leu Arg Glu Glu Gly Asp Thr Ala Val Ala Thr
            260                 265                 270

cca tat act gct gct gtt tta gaa tac aga gtt tcc atc cac gat tcc     864
Pro Tyr Thr Ala Ala Val Leu Glu Tyr Arg Val Ser Ile His Asp Ser
        275                 280                 285

gaa gat gct aag ttc aac gat att aac atg gct aac ggt aac ggt tac     912
Glu Asp Ala Lys Phe Asn Asp Ile Asn Met Ala Asn Gly Asn Gly Tyr
    290                 295                 300

acc gtt ttt gat gct caa cat cca tac aag gct aac gtt gct gtt aag     960
Thr Val Phe Asp Ala Gln His Pro Tyr Lys Ala Asn Val Ala Val Lys
305                 310                 315                 320

aga gaa ttg cat act cca gaa tct gac aga tcc tgc att cat ttg gaa     1008
Arg Glu Leu His Thr Pro Glu Ser Asp Arg Ser Cys Ile His Leu Glu
                325                 330                 335

ttc gat att gct ggt tcc ggt ttg act tac gaa act ggt gat cat gtt     1056
Phe Asp Ile Ala Gly Ser Gly Leu Thr Tyr Glu Thr Gly Asp His Val
            340                 345                 350

ggt gtt ttg tgc gat aac ttg tct gaa act gtt gat gaa gcc ttg aga     1104
Gly Val Leu Cys Asp Asn Leu Ser Glu Thr Val Asp Glu Ala Leu Arg
        355                 360                 365

tta ttg gat atg tct cca gat acc tac ttc tcc ttg cat gcc gaa aaa     1152
Leu Leu Asp Met Ser Pro Asp Thr Tyr Phe Ser Leu His Ala Glu Lys
    370                 375                 380

gaa gat ggt act cca atc tct tca tct ttg cca cca cca ttt cca cca     1200
Glu Asp Gly Thr Pro Ile Ser Ser Ser Leu Pro Pro Pro Phe Pro Pro

385                 390                 395                 400

tgt aat ttg aga act gct ttg acc aga tac gct tgc ttg ttg tca tct      1248
Cys Asn Leu Arg Thr Ala Leu Thr Arg Tyr Ala Cys Leu Leu Ser Ser
                405                 410                 415

cca aaa aag tct gct ttg gtt gct ttg gct gct cat gct tca gat cca      1296
Pro Lys Lys Ser Ala Leu Val Ala Leu Ala Ala His Ala Ser Asp Pro
            420                 425                 430

act gaa gct gaa aga ttg aaa cat ttg gct tct cca gct ggt aag gat      1344
Thr Glu Ala Glu Arg Leu Lys His Leu Ala Ser Pro Ala Gly Lys Asp
        435                 440                 445

gaa tat tct aaa tgg gtt gtt gaa tcc caa aga tcc ttg ttg gaa gtt      1392
Glu Tyr Ser Lys Trp Val Val Glu Ser Gln Arg Ser Leu Leu Glu Val
    450                 455                 460

atg gct gaa ttt cca tct gct aaa cca cca ttg ggt gtt ttt ttt gct      1440
Met Ala Glu Phe Pro Ser Ala Lys Pro Pro Leu Gly Val Phe Phe Ala
465                 470                 475                 480

ggt gtt gct cca aga ttg caa cct aga ttc tac tct att tcc tcc tcc      1488
Gly Val Ala Pro Arg Leu Gln Pro Arg Phe Tyr Ser Ile Ser Ser Ser
                485                 490                 495

cca aaa att gcc gaa acc aga att cat gtt act tgc gct ttg gtc tac      1536
Pro Lys Ile Ala Glu Thr Arg Ile His Val Thr Cys Ala Leu Val Tyr
            500                 505                 510

gaa aaa atg cca act ggt aga atc cat aag ggt gtt tgt tct acc tgg      1584
Glu Lys Met Pro Thr Gly Arg Ile His Lys Gly Val Cys Ser Thr Trp
        515                 520                 525

atg aag aat gct gtt cct tac gaa aag tcc gaa aac tgt tct tct gct      1632
Met Lys Asn Ala Val Pro Tyr Glu Lys Ser Glu Asn Cys Ser Ser Ala
    530                 535                 540

cca atc ttc gtt aga caa tcc aat ttc aag ttg cca tcc gat tct aag      1680
Pro Ile Phe Val Arg Gln Ser Asn Phe Lys Leu Pro Ser Asp Ser Lys
545                 550                 555                 560

gtt cca att atc atg att ggt cca ggt act ggt ttg gct cct ttt aga      1728
Val Pro Ile Ile Met Ile Gly Pro Gly Thr Gly Leu Ala Pro Phe Arg
                565                 570                 575

ggt ttt tta caa gaa aga ttg gcc ttg gtc gaa tcc ggt gtt gaa ttg      1776
Gly Phe Leu Gln Glu Arg Leu Ala Leu Val Glu Ser Gly Val Glu Leu
            580                 585                 590

ggt cca tct gtt ttg ttt ttc ggt tgc aga aac aga aga atg gac ttc      1824
Gly Pro Ser Val Leu Phe Phe Gly Cys Arg Asn Arg Arg Met Asp Phe
        595                 600                 605

atc tac gaa gaa gaa tta caa aga ttc gtc gaa tca ggt gct ttg gca      1872
Ile Tyr Glu Glu Glu Leu Gln Arg Phe Val Glu Ser Gly Ala Leu Ala
    610                 615                 620

gaa ttg tca gtt gct ttt tct aga gaa ggt cca aca aaa gaa tac gtc      1920
Glu Leu Ser Val Ala Phe Ser Arg Glu Gly Pro Thr Lys Glu Tyr Val
625                 630                 635                 640

caa cac aag atg atg gat aag gct tct gat atc tgg aac atg att tct      1968

Gln His Lys Met Met Asp Lys Ala Ser Asp Ile Trp Asn Met Ile Ser
                645                 650                 655

caa ggt gcc tac ttg tat gtt tgt ggt gat gct aaa ggt atg gcc aga      2016
Gln Gly Ala Tyr Leu Tyr Val Cys Gly Asp Ala Lys Gly Met Ala Arg
            660                 665                 670

gat gtt cat aga tcc ttg cat aca att gcc caa gaa caa ggt tct atg      2064
Asp Val His Arg Ser Leu His Thr Ile Ala Gln Glu Gln Gly Ser Met
        675                 680                 685

gac tct aca aaa gca gaa ggt ttc gtc aag aac ttg caa act tct ggt      2112
Asp Ser Thr Lys Ala Glu Gly Phe Val Lys Asn Leu Gln Thr Ser Gly
    690                 695                 700

aga tac ttg aga gat gtc tgg tga                                      2136
Arg Tyr Leu Arg Asp Val Trp
705                 710

<210>  47
<211>  711
<212>  PRT
<213>  Arabidopsis thaliana

<400>  47

Met Ser Ser Ser Ser Ser Ser Ser Thr Ser Met Ile Asp Leu Met Ala
1               5                   10                  15

Ala Ile Ile Lys Gly Glu Pro Val Ile Val Ser Asp Pro Ala Asn Ala
            20                  25                  30

Ser Ala Tyr Glu Ser Val Ala Ala Glu Leu Ser Ser Met Leu Ile Glu
        35                  40                  45

Asn Arg Gln Phe Ala Met Ile Val Thr Thr Ser Ile Ala Val Leu Ile
    50                  55                  60

Gly Cys Ile Val Met Leu Val Trp Arg Arg Ser Gly Ser Gly Asn Ser
65                  70                  75                  80

Lys Arg Val Glu Pro Leu Lys Pro Leu Val Ile Lys Pro Arg Glu Glu
                85                  90                  95

Glu Ile Asp Asp Gly Arg Lys Lys Val Thr Ile Phe Phe Gly Thr Gln
            100                 105                 110

Thr Gly Thr Ala Glu Gly Phe Ala Lys Ala Leu Gly Glu Glu Ala Lys
        115                 120                 125

Ala Arg Tyr Glu Lys Thr Arg Phe Lys Ile Val Asp Leu Asp Asp Tyr
    130                 135                 140

Ala Ala Asp Asp Asp Glu Tyr Glu Glu Lys Leu Lys Lys Glu Asp Val
145                 150                 155                 160

Ala Phe Phe Phe Leu Ala Thr Tyr Gly Asp Gly Glu Pro Thr Asp Asn
                165                 170                 175

Ala Ala Arg Phe Tyr Lys Trp Phe Thr Glu Gly Asn Asp Arg Gly Glu
            180                 185                 190

Trp Leu Lys Asn Leu Lys Tyr Gly Val Phe Gly Leu Gly Asn Arg Gln
        195                 200                 205

Tyr Glu His Phe Asn Lys Val Ala Lys Val Val Asp Asp Ile Leu Val
    210                 215                 220

Glu Gln Gly Ala Gln Arg Leu Val Gln Val Gly Leu Gly Asp Asp Asp
225                 230                 235                 240

Gln Cys Ile Glu Asp Asp Phe Thr Ala Trp Arg Glu Ala Leu Trp Pro
                245                 250                 255

Glu Leu Asp Thr Ile Leu Arg Glu Glu Gly Asp Thr Ala Val Ala Thr
            260                 265                 270

Pro Tyr Thr Ala Ala Val Leu Glu Tyr Arg Val Ser Ile His Asp Ser
        275                 280                 285

Glu Asp Ala Lys Phe Asn Asp Ile Asn Met Ala Asn Gly Asn Gly Tyr
    290                 295                 300

Thr Val Phe Asp Ala Gln His Pro Tyr Lys Ala Asn Val Ala Val Lys
305                 310                 315                 320

Arg Glu Leu His Thr Pro Glu Ser Asp Arg Ser Cys Ile His Leu Glu
                325                 330                 335

Phe Asp Ile Ala Gly Ser Gly Leu Thr Tyr Glu Thr Gly Asp His Val
            340                 345                 350

Gly Val Leu Cys Asp Asn Leu Ser Glu Thr Val Asp Glu Ala Leu Arg
        355                 360                 365

Leu Leu Asp Met Ser Pro Asp Thr Tyr Phe Ser Leu His Ala Glu Lys
    370                 375                 380

Glu Asp Gly Thr Pro Ile Ser Ser Ser Leu Pro Pro Pro Phe Pro Pro
385                 390                 395                 400

Cys Asn Leu Arg Thr Ala Leu Thr Arg Tyr Ala Cys Leu Leu Ser Ser
                405                 410                 415

Pro Lys Lys Ser Ala Leu Val Ala Leu Ala Ala His Ala Ser Asp Pro
            420                 425                 430

Thr Glu Ala Glu Arg Leu Lys His Leu Ala Ser Pro Ala Gly Lys Asp
        435                 440                 445

Glu Tyr Ser Lys Trp Val Val Glu Ser Gln Arg Ser Leu Leu Glu Val
    450                 455                 460

Met Ala Glu Phe Pro Ser Ala Lys Pro Pro Leu Gly Val Phe Phe Ala
465                 470                 475                 480

Gly Val Ala Pro Arg Leu Gln Pro Arg Phe Tyr Ser Ile Ser Ser Ser
                485                 490                 495

Pro Lys Ile Ala Glu Thr Arg Ile His Val Thr Cys Ala Leu Val Tyr
            500                 505                 510

Glu Lys Met Pro Thr Gly Arg Ile His Lys Gly Val Cys Ser Thr Trp
        515                 520                 525

Met Lys Asn Ala Val Pro Tyr Glu Lys Ser Glu Asn Cys Ser Ser Ala
    530                 535                 540

Pro Ile Phe Val Arg Gln Ser Asn Phe Lys Leu Pro Ser Asp Ser Lys
545                 550                 555                 560

Val Pro Ile Ile Met Ile Gly Pro Gly Thr Gly Leu Ala Pro Phe Arg
                565                 570                 575

Gly Phe Leu Gln Glu Arg Leu Ala Leu Val Glu Ser Gly Val Glu Leu
            580                 585                 590

Gly Pro Ser Val Leu Phe Phe Gly Cys Arg Asn Arg Arg Met Asp Phe
        595                 600                 605

Ile Tyr Glu Glu Glu Leu Gln Arg Phe Val Glu Ser Gly Ala Leu Ala
    610                 615                 620

Glu Leu Ser Val Ala Phe Ser Arg Glu Gly Pro Thr Lys Glu Tyr Val
625                 630                 635                 640

Gln His Lys Met Met Asp Lys Ala Ser Asp Ile Trp Asn Met Ile Ser
                645                 650                 655

Gln Gly Ala Tyr Leu Tyr Val Cys Gly Asp Ala Lys Gly Met Ala Arg
            660                 665                 670

Asp Val His Arg Ser Leu His Thr Ile Ala Gln Glu Gln Gly Ser Met
        675                 680                 685

Asp Ser Thr Lys Ala Glu Gly Phe Val Lys Asn Leu Gln Thr Ser Gly
    690                 695                 700

Arg Tyr Leu Arg Asp Val Trp
705                 710

<210>  48
<211>  705
<212>  PRT
<213>  Ananas comosus

<220>
<221>  POLYPEPTIDE
<222>  (1)..(705)
<223>  cytochrome p450 reductase

<300>
<308>  GenBank: OAY75970.1
<309>  2016-06-10
<313>  (1)..(705)

<400>  48
   Met Glu Ser Gly Ser Met Lys Pro Ser Pro Leu Asp Leu Leu Thr Ala
   1               5                   10                  15
   Ile Leu Thr Gly Lys Arg Leu Gly Gly Gly Gly Val Gly Gly Ala Ala
               20                  25                  30
   Pro Glu Ser Glu Ala Ile Ala Glu Ala Arg Asp Leu Leu Met Ile Leu
           35                  40                  45
   Ala Thr Ser Leu Ala Leu Leu Ala Gly Cys Ala Ala Leu Ile Leu Trp
       50                  55                  60
   Arg Arg Ser Val Ala Ala Gly Gly Lys Lys Lys Gln Ser Lys Ala Val
   65                  70                  75                  80
   Glu Ala Pro Arg Pro Leu Val Val Lys Lys Glu Ala Glu Thr Glu Val
                   85                  90                  95
   Asp Asp Gly Lys Lys Lys Val Thr Val Phe Phe Gly Thr Gln Thr Gly
               100                 105                 110
   Thr Ala Glu Gly Phe Ala Lys Ala Leu Ala Glu Glu Ala Lys Ala Arg
           115                 120                 125
   Tyr Asp Lys Ala Thr Phe Lys Val Val Asp Leu Asp Asp Tyr Ala Ala
       130                 135                 140
   Glu Asp Asp Glu Tyr Glu Asp Lys Leu Lys Lys Glu Ser Val Ala Leu
   145                 150                 155                 160
   Phe Phe Leu Ala Thr Tyr Gly Asp Gly Glu Pro Thr Asp Asn Ala Ala
                   165                 170                 175
   Arg Phe Tyr Lys Trp Phe Ser Glu Glu Asn Glu Arg Gly Asn Trp Leu
               180                 185                 190
   Lys Asn Leu Gln Tyr Ala Val Phe Gly Leu Gly Asn Lys Gln Tyr Glu
           195                 200                 205
   His Phe Asn Lys Val Ala Lys Val Val Asp Glu Leu Leu Ala Gln Gln
       210                 215                 220
   Gly Gly Lys Arg Ile Val Pro Val Gly Leu Gly Asp Asp Asp Gln Cys
   225                 230                 235                 240
   Ile Glu Asp Asp Phe Thr Ala Trp Lys Glu Leu Leu Trp Pro Glu Leu
                   245                 250                 255
   Asp Gln Leu Leu Arg Asp Glu Asp Ala Val Ser Gly Ala Ser Thr Pro
               260                 265                 270
   Tyr Thr Ala Ala Ile Pro Glu Tyr Arg Val Val Phe Ile Asp Ser Glu
           275                 280                 285
   Asp Ala Ala His Leu Glu Lys Ser Trp Ser Leu Ala Asn Gly His Val
       290                 295                 300
   Val His Asp Ile Gln His Pro Cys Arg Ala Asn Val Ala Val Arg Arg
   305                 310                 315                 320
   Glu Leu His Thr Pro Ala Ser Asp Arg Ser Cys Ile His Leu Glu Phe
                   325                 330                 335
   Asp Ile Ala Gly Thr Gly Leu Thr Tyr Glu Thr Gly Asp His Val Gly
               340                 345                 350
   Val Phe Thr Glu Asn Cys Leu Glu Thr Val Glu Glu Ala Glu Lys Leu
           355                 360                 365
   Leu Gly Tyr Ser Pro Asp Thr Phe Phe Ser Ile His Ala Asp Lys Glu
       370                 375                 380
   Asp Gly Thr Pro Leu Gly Gly Gly Ser Met Gly Pro Pro Phe Pro Ser
   385                 390                 395                 400
   Pro Cys Thr Leu Arg Thr Ala Leu Ala Arg Tyr Ala Asp Leu Leu Asn
                   405                 410                 415
   Ser Pro Lys Lys Ser Ala Leu Thr Ala Leu Ala Ala Tyr Ala Ser Asp
               420                 425                 430
   Pro Ser Glu Ala Glu Arg Leu Arg Phe Leu Ala Ser Pro Thr Gly Lys
           435                 440                 445
   Asp Glu Tyr Ser Gln Trp Val Val Ala Ser Gln Arg Ser Leu Leu Glu
       450                 455                 460
   Val Met Ala Glu Phe Pro Ser Ala Lys Pro Pro Leu Gly Val Phe Phe
   465                 470                 475                 480
   Ala Ala Val Ala Pro Arg Leu Gln Pro Arg Tyr Tyr Ser Ile Ser Ser
                   485                 490                 495
   Ser Pro Arg Met Ala Pro Arg Arg Ile His Val Thr Cys Ala Leu Val
               500                 505                 510
   His Gly Pro Thr Pro Thr Gly Arg Ile His Lys Gly Asn Ser Val Pro
           515                 520                 525
   Ser Glu Glu Ser Asn Glu Cys Ser Trp Ala Pro Ile Phe Val Arg Gln
       530                 535                 540
   Ser Asn Phe Lys Leu Pro Ser Asp Pro Ser Val Pro Ile Ile Met Ile
   545                 550                 555                 560
   Gly Pro Gly Thr Gly Leu Ala Pro Phe Arg Gly Phe Leu Gln Glu Arg
                   565                 570                 575
   Leu Ala Leu Lys Glu Ser Gly Val Glu Leu Gly His Ser Val Leu Phe
               580                 585                 590
   Phe Gly Cys Arg Asn Arg Lys Met Asp Phe Ile Tyr Glu Glu Glu Leu
           595                 600                 605
   Ser Lys Phe Val Asp Glu Gly Ala Leu Ser Glu Leu Ile Val Ala Phe
   610                 615                 620
   Ser Arg Glu Gly Pro Thr Lys Glu Tyr Val Gln His Lys Met Ala Glu
   625                 630                 635                 640
   Lys Ala Pro Glu Ile Trp Lys Ile Ile Ser Gln Gly Gly Tyr Ile Tyr
                   645                 650                 655
   Val Cys Gly Asp Ala Lys Gly Met Ala Arg Asp Val His Arg Val Leu
               660                 665                 670
   His Ser Ile Val Gln Glu Gln Gly Ser Leu Asp Ser Ser Lys Thr Glu
           675                 680                 685
   Ser Met Val Lys Ser Leu Gln Met Glu Gly Arg Tyr Leu Arg Asp Val
       690                 695                 700
   Trp
   705

<210>  49
<211>  710
<212>  PRT
<213>  Capsella rubella

<220>
<221>  POLYPEPTIDE
<222>  (1)..(710)
<223>  cytochrome P450 reductase

<300>
<308>  NCBI Reference Sequence: XP_006283224.1
<309>  2018-02-02
<313>  (1)..(710)

<400>  49

Met Ser Ser Ser Ser Ser Ser Thr Ser Met Ile Asp Leu Met Ala Ala
1               5                   10                  15

Ile Ile Lys Gly Val Pro Val Val Val Ser Asp Pro Ala Asn Ala Ser
            20                  25                  30

Ala Tyr Glu Ser Val Ala Ala Glu Leu Ser Ser Met Leu Ile Glu Asn
        35                  40                  45

Arg Gln Phe Ala Met Ile Val Thr Thr Ser Ile Ala Val Leu Ile Gly
    50                  55                  60

Cys Ile Val Met Leu Val Leu Arg Arg Ser Gly Ser Gly Asp Ser Lys
65                  70                  75                  80

Arg Val Glu Pro Leu Lys Pro Leu Val Leu Lys Pro Arg Asp Glu Glu
                85                  90                  95

Ile Asp Asp Gly Arg Lys Lys Val Thr Ile Phe Phe Gly Thr Gln Thr
            100                 105                 110

Gly Thr Ala Glu Gly Phe Ala Lys Ala Leu Gly Glu Glu Ala Lys Ala
        115                 120                 125

Arg Tyr Glu Lys Ile Arg Phe Lys Ile Val Asp Leu Asp Asp Tyr Ala
    130                 135                 140

Ala Asp Asp Asp Glu Tyr Glu Glu Lys Leu Lys Lys Glu Asp Val Ala
145                 150                 155                 160

Phe Phe Phe Leu Ala Thr Tyr Gly Asp Gly Glu Pro Thr Asp Asn Ala
                165                 170                 175

Ala Arg Phe Tyr Lys Trp Phe Thr Glu Gly Asn Glu Arg Gly Glu Trp
            180                 185                 190

Leu Asn Asn Leu Lys Tyr Gly Val Phe Gly Leu Gly Asn Arg Gln Tyr
        195                 200                 205

Glu His Phe Asn Lys Val Ala Lys Val Val Asp Asp Leu Leu Val Glu
    210                 215                 220

Gln Gly Ala His Arg Ile Val Gln Val Gly Leu Gly Asp Asp Asp Gln
225                 230                 235                 240

Cys Ile Glu Asp Asp Phe Thr Ala Trp Arg Glu Ser Leu Trp Pro Glu
                245                 250                 255

Leu Asp Thr Leu Leu Arg Glu Glu Gly Asp Thr Ala Val Ala Thr Pro
            260                 265                 270

Tyr Thr Ala Ala Val Leu Glu Tyr Arg Val Ser Ile His Asp Ser Glu
        275                 280                 285

Asp Ala Lys Phe Asn Gly Lys Asn Met Ala Asn Gly Asn Gly His Thr
    290                 295                 300

Val Phe Asp Ala Gln His Pro Tyr Arg Ala Asn Val Ala Val Lys Lys
305                 310                 315                 320

Glu Leu His Thr Pro Glu Ser Asp Arg Ser Cys Thr His Leu Glu Phe

                325                 330                 335

Asp Ile Ala Gly Ser Gly Leu Thr Tyr Glu Thr Gly Asp His Val Gly
            340                 345                 350

Val Leu Cys Asp Asn Leu Ser Glu Thr Val Asp Glu Ala Leu Lys Leu
        355                 360                 365

Leu Asp Met Ser Pro Asp Thr Tyr Phe Ser Leu His Ala Glu Lys Glu
    370                 375                 380

Asp Gly Thr Pro Ile Ser Ser Ser Leu Pro Pro Pro Phe Pro Pro Cys
385                 390                 395                 400

Asn Leu Arg Thr Ala Leu Thr Gln Tyr Ala Cys Leu Leu Ser Ser Pro
                405                 410                 415

Lys Lys Ser Ala Leu Leu Ala Leu Ser Ala His Ala Ser Asp Pro Thr
            420                 425                 430

Glu Ala Glu Arg Leu Lys His Leu Ala Ser Pro Ala Gly Lys Asp Glu
        435                 440                 445

Tyr Ser Lys Trp Val Val Glu Ser Gln Arg Ser Leu Leu Glu Val Met
    450                 455                 460

Ala Glu Phe Pro Ser Ala Lys Pro Pro Leu Gly Val Phe Phe Ala Gly
465                 470                 475                 480

Val Ala Pro Arg Leu Gln Pro Arg Phe Tyr Ser Ile Ser Ser Ser Pro
                485                 490                 495

Lys Ile Ala Glu Thr Arg Ile His Val Thr Cys Ala Leu Val His Glu
            500                 505                 510

Lys Met Pro Thr Gly Arg Ile His Lys Gly Val Cys Ser Thr Trp Met
        515                 520                 525

Lys Asn Ala Val Pro Ser Glu Lys Ser Glu Asn Cys Ser Ser Ala Pro
    530                 535                 540

Ile Phe Val Arg Gln Ser Asn Phe Lys Leu Pro Ser Asp Ser Lys Val
545                 550                 555                 560

Pro Ile Ile Met Ile Gly Pro Gly Thr Gly Leu Ala Pro Phe Arg Gly
                565                 570                 575

Phe Leu Gln Glu Arg Leu Ala Leu Val Glu Ser Gly Val Glu Leu Gly
            580                 585                 590

Pro Ser Val Leu Phe Phe Gly Cys Arg Asn Arg Arg Met Asp Phe Ile
        595                 600                 605

Tyr Glu Glu Glu Leu Gln Arg Phe Val Glu Ser Gly Ala Leu Ala Glu
    610                 615                 620

Leu Ser Val Ala Phe Ser Arg Glu Gly Pro Thr Lys Glu Tyr Val Gln
625                 630                 635                 640

His Lys Met Met Asp Arg Ala Ser Asp Ile Trp Asn Met Ile Ser Gln
                645                 650                 655

Gly Ala Tyr Val Tyr Val Cys Gly Asp Ala Lys Gly Met Ala Arg Asp
            660                 665                 670

Val His Arg Ser Leu His Thr Ile Ala Gln Glu Gln Gly Ser Met Asp
        675                 680                 685

Ser Thr Lys Ala Glu Gly Phe Val Lys Asn Leu Gln Met Ser Gly Arg
    690                 695                 700

Tyr Leu Arg Asp Val Trp
705                 710

<210>  50
<211>  711
<212>  PRT
<213>  Eutrema salsugineum

<220>
<221>  POLYPEPTIDE
<222>  (1)..(711)
<223>  cytochrome P450 reductase

<300>
<308>  NCBI Reference Sequence: XP_006412733.1
<309>  2018-02-26
<313>  (1)..(711)

<400>  50

Met Ser Ser Ser Ser Ser Thr Ser Met Lys Val Ser Pro Phe Asp Leu
1               5                   10                  15

Met Ser Ala Ile Ile Lys Gly Glu Pro Val Val Val Ser Asp Pro Ala
            20                  25                  30

Asn Ala Ser Ala Tyr Glu Ser Val Ala Ala Glu Leu Ser Ser Met Leu

35                  40                  45

Ile Glu Asn Arg Gln Phe Ala Met Ile Val Thr Thr Ser Ile Ala Val
    50                  55                  60

Leu Ile Gly Cys Ile Val Met Leu Val Trp Arg Arg Ser Gly Thr Gly
65                  70                  75                  80

Asn Ser Lys Arg Val Glu Pro Leu Lys Pro Leu Val Ile Lys Pro Arg
                85                  90                  95

Glu Asp Asp Val Asp Asp Gly Arg Lys Lys Val Thr Ile Phe Phe Gly
            100                 105                 110

Thr Gln Thr Gly Thr Ala Glu Gly Phe Ala Lys Ala Leu Gly Glu Glu
        115                 120                 125

Ala Arg Ala Arg Tyr Glu Lys Thr Arg Phe Lys Ile Val Asp Leu Asp
    130                 135                 140

Asp Tyr Ala Ala Asp Asp Asp Glu Tyr Glu Glu Lys Leu Lys Lys Glu
145                 150                 155                 160

Asp Val Ala Phe Phe Phe Leu Ala Thr Tyr Gly Asp Gly Glu Pro Thr
                165                 170                 175

Asp Asn Ala Ala Arg Phe Tyr Lys Trp Phe Thr Glu Gly Asn Asp Arg
            180                 185                 190

Gly Glu Trp Leu Lys Asn Leu Lys Tyr Gly Val Phe Gly Leu Gly Asn
        195                 200                 205

Arg Gln Tyr Glu His Phe Asn Lys Val Ala Lys Val Val Asp Asp Ile
    210                 215                 220

Leu Val Glu Gln Gly Ala Gln Arg Leu Val His Val Gly Leu Gly Asp
225                 230                 235                 240

Asp Asp Gln Cys Ile Glu Asp Asp Phe Thr Ala Trp Arg Glu Ala Leu
                245                 250                 255

Trp Pro Glu Leu Asp Thr Leu Leu Arg Glu Glu Gly Asp Thr Ala Val
            260                 265                 270

Thr Pro Tyr Thr Ala Ala Val Leu Glu Tyr Arg Val Ser Ile His Asn
        275                 280                 285

Ser Glu Asp Ala Leu Asn Glu Lys Asn Leu Ala Asn Gly Asn Gly His
    290                 295                 300

Val Val Phe Asp Ala Gln His Pro Tyr Arg Ala Asn Val Ala Val Arg
305                 310                 315                 320

Arg Glu Leu His Thr Pro Glu Ser Asp Arg Ser Cys Thr His Leu Glu
                325                 330                 335

Phe Asp Ile Ala Gly Ser Gly Leu Thr Tyr Glu Thr Gly Asp His Val
            340                 345                 350

Gly Val Leu Thr Glu Asn Leu Asn Glu Thr Val Glu Glu Ala Leu Ser
        355                 360                 365

Leu Leu Asp Ile Ser Pro Asp Thr Tyr Phe Ser Leu His Ser Asp Lys
    370                 375                 380

Glu Asp Gly Thr Pro Ile Ser Ser Ser Leu Pro Pro Pro Phe Pro Pro
385                 390                 395                 400

Cys Asn Leu Arg Thr Ala Leu Lys Arg Tyr Ala Cys Leu Leu Ser Ser
                405                 410                 415

Pro Lys Lys Ser Ala Leu Leu Ala Leu Ala Ala His Ala Ser Asp Pro
            420                 425                 430

Ala Glu Ala Glu Arg Leu Arg His Leu Ala Ser Pro Ala Gly Lys Asp
        435                 440                 445

Glu Tyr Ser Lys Trp Val Val Glu Ser Gln Arg Ser Leu Leu Glu Val
    450                 455                 460

Met Ala Glu Phe Pro Ser Ala Lys Pro Pro Leu Gly Val Phe Phe Ala
465                 470                 475                 480

Ala Val Ala Pro Arg Leu Gln Pro Arg Phe Tyr Ser Ile Ser Ser Ser
                485                 490                 495

Pro Lys Ile Ala Glu Thr Arg Ile His Val Thr Cys Ala Leu Val Tyr
            500                 505                 510

Glu Lys Met Pro Thr Gly Arg Ile His Lys Gly Val Cys Ser Thr Trp
        515                 520                 525

Met Lys Ser Ala Val Pro Tyr Glu Lys Asn Glu Asn Cys Cys Ser Ala
    530                 535                 540

Pro Ile Phe Ile Arg Gln Ser Asn Phe Lys Leu Pro Ser Asp Ser Lys
545                 550                 555                 560

Val Pro Ile Ile Met Ile Gly Pro Gly Thr Gly Leu Ala Pro Phe Arg
                565                 570                 575

Gly Phe Leu Gln Glu Arg Leu Ala Leu Val Glu Ser Gly Val Glu Leu
            580                 585                 590

Gly Pro Ser Val Leu Phe Phe Gly Cys Arg Asn Arg Arg Met Asp Phe
        595                 600                 605

Ile Tyr Glu Glu Glu Leu Gln Arg Phe Leu Glu Ser Gly Ala Leu Ser
    610                 615                 620

Glu Leu Ser Leu Ala Phe Ser Arg Glu Gly Pro Thr Lys Glu Tyr Val
625                 630                 635                 640

Gln His Lys Met Met Asp Lys Ala Ser Asp Ile Trp Ser Met Ile Ser
                645                 650                 655

Glu Gly Ala Tyr Val Tyr Val Cys Gly Asp Ala Lys Gly Met Ala Arg
            660                 665                 670

Asp Val His Arg Ser Leu His Thr Ile Ala Gln Glu Gln Gly Pro Met
        675                 680                 685

Asp Ser Thr Lys Ala Glu Ser Phe Val Lys Asn Leu Gln Met Ser Gly
    690                 695                 700

Arg Tyr Leu Arg Asp Val Trp
705                 710

<210>  51
<211>  1521
<212>  DNA
<213>  Flavobacerium johnsoniaeu

<220>
<221>  CDS
<222>  (1)..(1521)
<223>  Gene encoding tyrosine amminia-lyase from Flavobacerium
       johnsoniaeu codon optimized for yeast

<300>
<308>  GenBank: KR095306.1
<309>  2015-07-14
<313>  (1)..(1521)

<400>  51

atg aac acc atc aac gaa tat ctg agc ctg gaa gaa ttt gaa gcc att 48
Met Asn Thr Ile Asn Glu Tyr Leu Ser Leu Glu Glu Phe Glu Ala Ile
1               5                   10                  15

atc ttt ggc aat cag aaa gtg acc att agt gat gtt gtt gtg aat cgc 96
Ile Phe Gly Asn Gln Lys Val Thr Ile Ser Asp Val Val Val Asn Arg
            20                  25                  30

gtt aac gag agc ttt aac ttt ctg aaa gaa ttt agc ggc aac aaa gtg 144
Val Asn Glu Ser Phe Asn Phe Leu Lys Glu Phe Ser Gly Asn Lys Val
        35                  40                  45

atc tat ggt gtg aat acc ggt ttt ggt ccg atg gca cag tat cgt att 192
Ile Tyr Gly Val Asn Thr Gly Phe Gly Pro Met Ala Gln Tyr Arg Ile
    50                  55                  60

aaa gaa agc gat cag att cag ctg cag tat aat ctg att cgt agc cat 240
Lys Glu Ser Asp Gln Ile Gln Leu Gln Tyr Asn Leu Ile Arg Ser His
65                  70                  75                  80

agc agc ggc acc ggt aaa ccg ctg agt ccg gtt tgt gca aaa gca gca 288
Ser Ser Gly Thr Gly Lys Pro Leu Ser Pro Val Cys Ala Lys Ala Ala
                85                  90                  95

att ctg gca cgt ctg aat acc ctg agt ctg ggt aat agc ggt gtt cat 336
Ile Leu Ala Arg Leu Asn Thr Leu Ser Leu Gly Asn Ser Gly Val His
            100                 105                 110

ccg agc gtt att aat ctg atg agc gaa ctg atc aac aaa gat atc aca 384
Pro Ser Val Ile Asn Leu Met Ser Glu Leu Ile Asn Lys Asp Ile Thr
        115                 120                 125

ccg ctg att ttt gaa cat ggt ggt gtt ggt gca agc ggt gat ctg gtt 432
Pro Leu Ile Phe Glu His Gly Gly Val Gly Ala Ser Gly Asp Leu Val
    130                 135                 140

cag ctg agc cat ctg gca ctg gtt ctg att ggt gaa ggt gaa gtt ttc 480
Gln Leu Ser His Leu Ala Leu Val Leu Ile Gly Glu Gly Glu Val Phe
145                 150                 155                 160

tat aaa ggt gaa cgt cgt ccg aca ccg gaa gtt ttt gaa att gaa ggt 528
Tyr Lys Gly Glu Arg Arg Pro Thr Pro Glu Val Phe Glu Ile Glu Gly
                165                 170                 175

ctg aaa ccg atc cag gtg gaa att cgc gaa ggt ctg gcc ctg att aat 576
Leu Lys Pro Ile Gln Val Glu Ile Arg Glu Gly Leu Ala Leu Ile Asn
            180                 185                 190

ggc acc agc gtt atg acc ggt att ggt gtt gtt aat gtg tac cat gca 624
Gly Thr Ser Val Met Thr Gly Ile Gly Val Val Asn Val Tyr His Ala
        195                 200                 205

aaa aaa ctg ctg gat tgg agc ctg aaa agc agc tgt gca att aat gaa 672
Lys Lys Leu Leu Asp Trp Ser Leu Lys Ser Ser Cys Ala Ile Asn Glu
    210                 215                 220

ctg gtt cag gca tat gat gat cac ttt agc gca gaa ctg aat cag acc 720
Leu Val Gln Ala Tyr Asp Asp His Phe Ser Ala Glu Leu Asn Gln Thr
225                 230                 235                 240

aaa cgt cat aaa ggt cag caa gaa att gca ctg aaa atg cgt cag aat 768
Lys Arg His Lys Gly Gln Gln Glu Ile Ala Leu Lys Met Arg Gln Asn
                245                 250                 255

ctg agc gat agc acc ctg att cgc aaa cgt gaa gat cat ctg tat agc 816
Leu Ser Asp Ser Thr Leu Ile Arg Lys Arg Glu Asp His Leu Tyr Ser
            260                 265                 270

ggt gaa aac acc gaa gaa atc ttc aaa gaa aaa gtg caa gag tat tat 864
Gly Glu Asn Thr Glu Glu Ile Phe Lys Glu Lys Val Gln Glu Tyr Tyr
        275                 280                 285

agc ctg cgt tgt gtt ccg cag att ctg ggt ccg gtt ctg gaa acc att 912
Ser Leu Arg Cys Val Pro Gln Ile Leu Gly Pro Val Leu Glu Thr Ile
    290                 295                 300

aac aat gtt gca agc att ctg gaa gat gaa ttt aac agc gca aac gat 960
Asn Asn Val Ala Ser Ile Leu Glu Asp Glu Phe Asn Ser Ala Asn Asp
305                 310                 315                 320

aac ccg atc atc gat gtt aaa aac cag cat gtt tat cac ggt ggc aat 1008
Asn Pro Ile Ile Asp Val Lys Asn Gln His Val Tyr His Gly Gly Asn
                325                 330                 335

ttt cat ggt gat tat atc agc ctg gaa atg gat aaa ctg aaa atc gtg 1056
Phe His Gly Asp Tyr Ile Ser Leu Glu Met Asp Lys Leu Lys Ile Val
            340                 345                 350

att acc aaa ctg acc atg ctg gca gaa cgt cag ctg aat tat ctg ctg 1104
Ile Thr Lys Leu Thr Met Leu Ala Glu Arg Gln Leu Asn Tyr Leu Leu
        355                 360                 365

aat agc aaa att aac gaa ctg ctg cct ccg ttt gtt aat ctg ggc acc 1152
Asn Ser Lys Ile Asn Glu Leu Leu Pro Pro Phe Val Asn Leu Gly Thr
    370                 375                 380

ctg ggt ttt aac ttt ggt atg cag ggt gtt cag ttt acc gca acc agc 1200
Leu Gly Phe Asn Phe Gly Met Gln Gly Val Gln Phe Thr Ala Thr Ser
385                 390                 395                 400

acc acc gca gaa agc cag atg ctg agc aat ccg atg tat gtt cat agc 1248
Thr Thr Ala Glu Ser Gln Met Leu Ser Asn Pro Met Tyr Val His Ser
                405                 410                 415

att ccg aac aat aat gat aac cag gat att gtt agc atg ggc acc aat 1296
Ile Pro Asn Asn Asn Asp Asn Gln Asp Ile Val Ser Met Gly Thr Asn
            420                 425                 430

agc gca gtt att acc agc aaa gtt atc gaa aat gcc ttt gaa gtt ctg 1344
Ser Ala Val Ile Thr Ser Lys Val Ile Glu Asn Ala Phe Glu Val Leu
        435                 440                 445

gcc att gaa atg att acc att gtt cag gcg att gat tat ctg ggc cag 1392
Ala Ile Glu Met Ile Thr Ile Val Gln Ala Ile Asp Tyr Leu Gly Gln
    450                 455                 460

aaa gat aaa atc agc agc gtt agc aaa aaa tgg tat gat gaa atc cgc 1440
Lys Asp Lys Ile Ser Ser Val Ser Lys Lys Trp Tyr Asp Glu Ile Arg
465                 470                 475                 480

aac atc atc ccg acc ttt aaa gaa gat cag gtg atg tat ccg ttc gtg 1488
Asn Ile Ile Pro Thr Phe Lys Glu Asp Gln Val Met Tyr Pro Phe Val
                485                 490                 495

cag aaa gta aaa gac cac ctg att aac aat taa 1521
Gln Lys Val Lys Asp His Leu Ile Asn Asn

500                 505

<210>  52
<211>  506
<212>  PRT
<213>  Flavobacerium johnsoniaeu

<400>  52

Met Asn Thr Ile Asn Glu Tyr Leu Ser Leu Glu Glu Phe Glu Ala Ile
1               5                   10                  15

Ile Phe Gly Asn Gln Lys Val Thr Ile Ser Asp Val Val Val Asn Arg
            20                  25                  30

Val Asn Glu Ser Phe Asn Phe Leu Lys Glu Phe Ser Gly Asn Lys Val
        35                  40                  45

Ile Tyr Gly Val Asn Thr Gly Phe Gly Pro Met Ala Gln Tyr Arg Ile
    50                  55                  60

Lys Glu Ser Asp Gln Ile Gln Leu Gln Tyr Asn Leu Ile Arg Ser His
65                  70                  75                  80

Ser Ser Gly Thr Gly Lys Pro Leu Ser Pro Val Cys Ala Lys Ala Ala
                85                  90                  95

Ile Leu Ala Arg Leu Asn Thr Leu Ser Leu Gly Asn Ser Gly Val His
            100                 105                 110

Pro Ser Val Ile Asn Leu Met Ser Glu Leu Ile Asn Lys Asp Ile Thr
        115                 120                 125

Pro Leu Ile Phe Glu His Gly Gly Val Gly Ala Ser Gly Asp Leu Val
    130                 135                 140

Gln Leu Ser His Leu Ala Leu Val Leu Ile Gly Glu Gly Glu Val Phe
145                 150                 155                 160

Tyr Lys Gly Glu Arg Arg Pro Thr Pro Glu Val Phe Glu Ile Glu Gly
                165                 170                 175

Leu Lys Pro Ile Gln Val Glu Ile Arg Glu Gly Leu Ala Leu Ile Asn
            180                 185                 190

Gly Thr Ser Val Met Thr Gly Ile Gly Val Val Asn Val Tyr His Ala
        195                 200                 205

Lys Lys Leu Leu Asp Trp Ser Leu Lys Ser Ser Cys Ala Ile Asn Glu

210                 215                 220

Leu Val Gln Ala Tyr Asp Asp His Phe Ser Ala Glu Leu Asn Gln Thr
225                 230                 235                 240

Lys Arg His Lys Gly Gln Gln Glu Ile Ala Leu Lys Met Arg Gln Asn
                245                 250                 255

Leu Ser Asp Ser Thr Leu Ile Arg Lys Arg Glu Asp His Leu Tyr Ser
            260                 265                 270

Gly Glu Asn Thr Glu Glu Ile Phe Lys Glu Lys Val Gln Glu Tyr Tyr
        275                 280                 285

Ser Leu Arg Cys Val Pro Gln Ile Leu Gly Pro Val Leu Glu Thr Ile
    290                 295                 300

Asn Asn Val Ala Ser Ile Leu Glu Asp Glu Phe Asn Ser Ala Asn Asp
305                 310                 315                 320

Asn Pro Ile Ile Asp Val Lys Asn Gln His Val Tyr His Gly Gly Asn
                325                 330                 335

Phe His Gly Asp Tyr Ile Ser Leu Glu Met Asp Lys Leu Lys Ile Val
            340                 345                 350

Ile Thr Lys Leu Thr Met Leu Ala Glu Arg Gln Leu Asn Tyr Leu Leu
        355                 360                 365

Asn Ser Lys Ile Asn Glu Leu Leu Pro Pro Phe Val Asn Leu Gly Thr
    370                 375                 380

Leu Gly Phe Asn Phe Gly Met Gln Gly Val Gln Phe Thr Ala Thr Ser
385                 390                 395                 400

Thr Thr Ala Glu Ser Gln Met Leu Ser Asn Pro Met Tyr Val His Ser
                405                 410                 415

Ile Pro Asn Asn Asn Asp Asn Gln Asp Ile Val Ser Met Gly Thr Asn
            420                 425                 430

Ser Ala Val Ile Thr Ser Lys Val Ile Glu Asn Ala Phe Glu Val Leu
        435                 440                 445

Ala Ile Glu Met Ile Thr Ile Val Gln Ala Ile Asp Tyr Leu Gly Gln
    450                 455                 460

Lys Asp Lys Ile Ser Ser Val Ser Lys Lys Trp Tyr Asp Glu Ile Arg
465                 470                 475                 480

Asn Ile Ile Pro Thr Phe Lys Glu Asp Gln Val Met Tyr Pro Phe Val
                485                 490                 495

Gln Lys Val Lys Asp His Leu Ile Asn Asn
            500                 505

<210>  53
<211>  507
<212>  PRT
<213>  Chryseobacterium piscium

<220>
<221>  POLYPEPTIDE
<222>  (1)..(507)
<223>  tyrosine ammonia-lyase

<300>
<308>  NCBI Reference Sequence: WP_115949289.1
<309>  2018-08-16
<313>  (1)..(507)

<400>  53

Met Met Lys Ile Asn Asn Phe Leu Glu Leu Arg Asp Phe Gln Arg Ile
1               5                   10                  15

Ile Ile Glu Asn Glu Ser Ile Glu Leu Asp Pro Ser Leu Leu Gln Arg
            20                  25                  30

Val Asn Glu Ser Phe Ser Phe Leu Lys Glu Phe Ser Lys Asn Lys Val
        35                  40                  45

Ile Tyr Gly Val Asn Thr Gly Phe Gly Pro Met Ala Gln Phe Lys Ile
    50                  55                  60

Ser Asp Glu Asp Thr His Gln Leu Gln Tyr Asn Leu Ile Arg Ser His
65                  70                  75                  80

Ser Ser Gly Ile Gly Asn Pro Leu Pro Ala Asp Glu Val Lys Ala Cys
                85                  90                  95

Met Leu Ala Arg Leu Asn Thr Leu Ser Leu Gly Asn Ser Gly Val His
            100                 105                 110

Glu Ser Val Val Asn Leu Leu Lys Glu Leu Ile Asn Arg Asp Ile Thr
        115                 120                 125

Pro Leu Ile Phe Glu His Gly Gly Val Gly Ala Ser Gly Asp Leu Val

130                 135                 140

Gln Leu Ala His Leu Ala Leu Val Leu Ile Gly Glu Gly Glu Val Phe
145                 150                 155                 160

Tyr Lys Gly Glu Arg Lys Ser Thr Lys Glu Val Phe Glu Ile Glu Gly
                165                 170                 175

Leu Glu Pro Ile Gln Val Glu Ile Arg Glu Gly Leu Ala Leu Met Asn
            180                 185                 190

Gly Thr Ser Val Met Thr Gly Ile Gly Val Val Asn Ala Tyr Lys Ala
        195                 200                 205

Asn Lys Leu Thr Asp Ile Ser Leu Lys Leu Ser Cys Ala Ile Asn Glu
    210                 215                 220

Ile Val Gln Ala Tyr Asp Asp His Phe Ser Glu Val Leu Asn Gly Thr
225                 230                 235                 240

Lys Leu His Ala Gly Gln Gln Lys Ile Ala Glu Lys Met Arg Ala His
                245                 250                 255

Leu Ser Asp Ser Lys Leu Ile Arg Lys Arg Ala Asp His Leu Tyr Thr
            260                 265                 270

His Phe Glu Glu Gln Glu Lys Val Phe Lys Glu Lys Val Gln Glu Tyr
        275                 280                 285

Tyr Ser Leu Arg Cys Val Pro Gln Ile Leu Gly Pro Val Leu Asp Thr
    290                 295                 300

Leu Glu Tyr Thr Glu Lys Val Leu Glu Asn Glu Ile Asn Ser Ala Asn
305                 310                 315                 320

Asp Asn Pro Ile Ile Asn Val Lys Asp Gln His Val Tyr His Gly Gly
                325                 330                 335

Asn Phe His Gly Asp Tyr Ile Ser Leu Glu Met Asp Lys Leu Lys Ile
            340                 345                 350

Val Val Thr Lys Leu Thr Met Leu Ala Glu Arg Gln Leu Asn Tyr Leu
        355                 360                 365

Leu Asn Ser Lys Ile Asn Glu Ile Leu Pro Pro Phe Val Asn Leu Gly
    370                 375                 380

Lys Leu Gly Phe Asn Phe Gly Met Gln Gly Val Gln Phe Thr Ala Thr
385                 390                 395                 400

Ser Thr Thr Ala Glu Ser Gln Met Leu Ser Asn Pro Met Tyr Val His
                405                 410                 415

Ser Ile Pro Asn Asn Asn Asp Asn Gln Asp Ile Val Ser Met Gly Thr
            420                 425                 430

Asn Ala Ala Val Ile Cys Arg Lys Val Ile Glu Asn Ala Phe Glu Val
        435                 440                 445

Leu Ala Ile Glu Ala Ile Thr Val Val Gln Ala Ile Glu Tyr Leu Gly
    450                 455                 460

Tyr Gln Asp Gln Ile Ser Ser Lys Thr Lys Glu Leu Tyr Asp Glu Ile
465                 470                 475                 480

Arg Lys Ile Ile Pro Ala Phe Ser Ser Asp Met Val Met Tyr Pro Tyr
                485                 490                 495

Leu Glu Glu Val Lys Lys Tyr Leu Lys Thr Met
            500                 505

<210>  54
<211>  507
<212>  PRT
<213>  Flavobacterium denitrificans

<220>
<221>  POLYPEPTIDE
<222>  (1)..(507)
<223>  tyrosine ammonia-lyase

<300>
<308>  NCBI Reference Sequence: WP_026727230.1
<309>  2017-07-23
<313>  (1)..(507)

<400>  54

Met Asn Thr Ile Asn Glu Tyr Leu Ser Leu Ala Glu Phe Glu Ser Ile
1               5                   10                  15

Ile Phe Gly Asn Asn Lys Val Ala Ile Ser Asp Val Val Leu Asn Arg
            20                  25                  30

Val Asn Glu Ser Phe Asn Phe Leu Lys Glu Phe Ser Gly Asn Lys Ile
        35                  40                  45

Ile Tyr Gly Val Asn Thr Gly Phe Gly Pro Met Ala Gln Tyr Arg Ile

50                  55                  60

Lys Glu Ser Asp Gln Ile Gln Leu Gln Tyr Asn Leu Ile Arg Ser His
65                  70                  75                  80

Ser Ser Gly Thr Gly Lys Pro Leu Ser Pro Val Cys Ala Lys Ala Ala
                85                  90                  95

Ile Leu Ala Arg Leu Asn Thr Leu Ser Leu Gly Asn Ser Gly Val His
            100                 105                 110

Pro Ser Val Ile His Leu Met Ser Glu Leu Ile Asn Arg Asp Ile Thr
        115                 120                 125

Pro Leu Ile Phe Glu His Gly Gly Val Gly Ala Ser Gly Asp Leu Val
    130                 135                 140

Gln Leu Ser His Leu Ala Leu Val Leu Ile Gly Glu Gly Glu Val Phe
145                 150                 155                 160

Tyr Lys Gly Glu Arg Arg Pro Thr Pro Glu Val Phe Glu Ile Glu Gly
                165                 170                 175

Leu Lys Pro Ile Gln Val Glu Ile Arg Glu Gly Leu Ala Leu Ile Asn
            180                 185                 190

Gly Thr Ser Val Met Thr Gly Ile Gly Val Val Asn Ile His His Ala
        195                 200                 205

Lys Lys Leu Leu Asp Trp Ser Leu Lys Ser Ser Cys Ala Ile Asn Glu
    210                 215                 220

Leu Val Gln Ala Tyr Asp Asp His Phe Ser Ala Glu Leu Asn Gln Thr
225                 230                 235                 240

Lys Arg His Lys Gly Gln Gln Glu Val Ala Glu Arg Met Arg Lys Asn
                245                 250                 255

Leu Ser Asp Ser Thr Leu Ile Arg Lys Arg Glu Asp His Leu Tyr Ser
            260                 265                 270

Gly Glu Asn Thr Glu Glu Ile Phe Lys Glu Lys Val Gln Glu Tyr Tyr
        275                 280                 285

Ser Leu Arg Cys Val Pro Gln Ile Leu Gly Pro Val Leu Glu Thr Ile
    290                 295                 300

Asn Asn Val Ala Ser Ile Leu Glu Asp Glu Phe Asn Ser Ala Asn Asp
305                 310                 315                 320

Asn Pro Ile Ile Asp Val Lys Asn Lys His Val Tyr His Gly Gly Asn
                325                 330                 335

Phe His Gly Asp Tyr Ile Ser Leu Glu Met Asp Lys Leu Lys Ile Val
            340                 345                 350

Ile Thr Lys Leu Thr Met Leu Ala Glu Arg Gln Leu Asn Tyr Leu Leu
        355                 360                 365

Asn Ser Lys Ile Asn Glu Ile Leu Pro Pro Phe Val Asn Leu Gly Thr
    370                 375                 380

Leu Gly Phe Asn Phe Gly Met Gln Gly Val Gln Phe Thr Ala Thr Ser
385                 390                 395                 400

Thr Thr Ala Glu Ser Gln Met Leu Ser Asn Pro Met Tyr Val His Ser
                405                 410                 415

Ile Pro Asn Asn Asn Asp Asn Gln Asp Ile Val Ser Met Gly Thr Asn
            420                 425                 430

Ala Ala Val Ile Thr Ser Lys Val Ile Glu Asn Ala Phe Glu Val Leu
        435                 440                 445

Ala Ile Glu Met Ile Thr Ile Val Gln Ala Ile Asp Tyr Leu Gly Gln
    450                 455                 460

Lys Asp Lys Ile Ser Ser Val Thr Arg Lys Trp Tyr Asp Asp Val Arg
465                 470                 475                 480

Asn Ile Ile Pro Glu Phe Lys Glu Asp Gln Val Met Tyr Pro Phe Val
                485                 490                 495

Gln Lys Val Lys Asp Tyr Leu Ile Asn Asn Leu
            500                 505

<210>  55
<211>  506
<212>  PRT
<213>  Flavobacterium swingsii

<220>
<221>  POLYPEPTIDE
<222>  (1)..(506)
<223>  tyrosine ammonia-lyase

<300>
<308>  NCBI Reference Sequence: WP_091475235.1
<309>  2017-08-01
<313>  (1)..(506)

<400>  55

Met Asn Thr Ile Asn Glu Phe Leu Ser Leu Lys Glu Phe Glu Ala Ile
1               5                   10                  15

Val Phe Asp Asn Asn Lys Val Glu Ile Ser Asp Val Val Leu Asp Arg
            20                  25                  30

Val Asn Lys Ser Phe Glu Phe Leu Lys Glu Phe Ser Gly Asn Lys Val
        35                  40                  45

Ile Tyr Gly Val Asn Thr Gly Phe Gly Pro Met Ala Gln Tyr Arg Ile
    50                  55                  60

Lys Asp Glu Asp Arg Leu Gln Leu Gln Tyr Asn Leu Ile Arg Ser His
65                  70                  75                  80

Ser Ser Gly Thr Gly Lys Pro Leu Asn Pro Leu Cys Val Lys Ala Ala
                85                  90                  95

Ile Leu Ala Arg Leu Asn Thr Leu Ser Leu Gly Asn Ser Gly Val His
            100                 105                 110

Pro Ser Leu Ile His Leu Met Lys Glu Leu Ile Asn Lys Asp Ile Thr
        115                 120                 125

Pro Leu Ile Phe Glu His Gly Gly Val Gly Ala Ser Gly Asp Leu Val
    130                 135                 140

Gln Leu Ala His Leu Ala Leu Val Leu Ile Gly Glu Gly Glu Val Phe
145                 150                 155                 160

Tyr Lys Gly Glu Arg Arg Pro Thr Gln Glu Val Phe Glu Leu Glu Asn
                165                 170                 175

Leu Lys Pro Ile Gln Val Glu Ile Arg Glu Gly Leu Ala Leu Met Asn
            180                 185                 190

Gly Thr Ser Val Met Thr Gly Ile Gly Val Val Asn Val His Asn Ala
        195                 200                 205

Asn Lys Leu Leu Asp Trp Ser Leu Lys Phe Ser Cys Ala Ile Asn Glu
    210                 215                 220

Ile Val Gln Ala Tyr Asp Asp His Tyr Ser Ala Glu Leu Asn Asn Thr
225                 230                 235                 240

Lys Arg His Lys Gly Gln Arg Glu Val Ala Glu Arg Met Arg Thr Asn
                245                 250                 255

Leu Ala Asp Ser Thr Leu Ile Arg Lys Arg Glu Asp His Leu Tyr Thr
            260                 265                 270

Gly Glu Asn Thr Glu Asp Val Phe Lys Glu Lys Val Gln Glu Tyr Tyr
        275                 280                 285

Ser Leu Arg Cys Val Pro Gln Ile Leu Gly Pro Val Leu Glu Thr Ile
    290                 295                 300

Asn Asn Val Ala Ser Ile Leu Glu Asp Glu Phe Asn Ser Ala Asn Asp
305                 310                 315                 320

Asn Pro Ile Ile Asp Val Lys Asn Gln His Val Tyr His Gly Gly Asn
                325                 330                 335

Phe His Gly Asp Tyr Ile Ser Leu Glu Met Asp Lys Leu Lys Val Val
            340                 345                 350

Ile Thr Lys Leu Thr Met Leu Ala Glu Arg Gln Leu Asn Tyr Leu Leu
        355                 360                 365

Asn Ser Lys Ile Asn Glu Ile Leu Pro Pro Phe Val Asn Leu Gly Thr
    370                 375                 380

Leu Gly Phe Asn Phe Gly Met Gln Gly Val Gln Phe Thr Ala Thr Ser
385                 390                 395                 400

Thr Thr Ala Glu Asn Gln Met Leu Ser Asn Pro Met Tyr Ile His Ser
                405                 410                 415

Ile Pro Asn Asn Asn Asp Asn Gln Asp Ile Val Ser Met Gly Thr Asn
            420                 425                 430

Ala Ala Val Ile Thr Ser Lys Val Ile Glu Asn Ser Phe Glu Val Leu
        435                 440                 445

Ala Ile Glu Leu Ile Thr Ile Val Gln Ala Ile Asp Tyr Leu Glu Gln
    450                 455                 460

Lys Asp Ala Ile Ser Ser Val Thr Arg Lys Met Tyr Asp Asp Val Arg
465                 470                 475                 480

Lys Ile Ile Pro Thr Phe Ser Gln Asp Gln Val Met Tyr Pro Phe Val
                485                 490                 495

Gln Asn Val Lys Asp Tyr Leu Ile Asn Asn
            500                 505

<210>  56
<211>  1908
<212>  DNA
<213>  Saccharomyces cerevisiae

<220>
<221>  CDS
<222>  (1)..(1908)
<223>  phenylpyruvate decarboxylase

<300>
<308>  NCBI Reference Sequence: NM_001180688.3
<309>  2018-04-04
<313>  (1)..(1908)

<400>  56
atg gca cct gtt aca att gaa aag ttc gta aat caa gaa gaa cga cac      48
Met Ala Pro Val Thr Ile Glu Lys Phe Val Asn Gln Glu Glu Arg His
1               5                   10                  15

ctt gtt tcc aac cga tca gca aca att ccg ttt ggt gaa tac ata ttt      96
Leu Val Ser Asn Arg Ser Ala Thr Ile Pro Phe Gly Glu Tyr Ile Phe
            20                  25                  30

aaa aga ttg ttg tcc atc gat acg aaa tca gtt ttc ggt gtt cct ggt      144
Lys Arg Leu Leu Ser Ile Asp Thr Lys Ser Val Phe Gly Val Pro Gly
        35                  40                  45

gac ttc aac tta tct cta tta gaa tat ctc tat tca cct agt gtt gaa      192
Asp Phe Asn Leu Ser Leu Leu Glu Tyr Leu Tyr Ser Pro Ser Val Glu
    50                  55                  60

tca gct ggc cta aga tgg gtc ggc acg tgt aat gaa ctg aac gcc gct      240
Ser Ala Gly Leu Arg Trp Val Gly Thr Cys Asn Glu Leu Asn Ala Ala
65                  70                  75                  80

tat gcg gcc gac gga tat tcc cgt tac tct aat aag att ggc tgt tta      288
Tyr Ala Ala Asp Gly Tyr Ser Arg Tyr Ser Asn Lys Ile Gly Cys Leu
                85                  90                  95

ata acc acg tat ggc gtt ggt gaa tta agc gcc ttg aac ggt ata gcc      336
Ile Thr Thr Tyr Gly Val Gly Glu Leu Ser Ala Leu Asn Gly Ile Ala
            100                 105                 110

ggt tcg ttc gct gaa aat gtc aaa gtt ttg cac att gtt ggt gtg gcc      384
Gly Ser Phe Ala Glu Asn Val Lys Val Leu His Ile Val Gly Val Ala
        115                 120                 125

aag tcc ata gat tcg cgt tca agt aac ttt agt gat cgg aac cta cat      432
Lys Ser Ile Asp Ser Arg Ser Ser Asn Phe Ser Asp Arg Asn Leu His
    130                 135                 140

cat ttg gtc cca cag cta cat gat tca aat ttt aaa ggg cca aat cat      480

His Leu Val Pro Gln Leu His Asp Ser Asn Phe Lys Gly Pro Asn His
145                 150                 155                 160

aaa gta tat cat gat atg gta aaa gat aga gtc gct tgc tcg gta gcc      528
Lys Val Tyr His Asp Met Val Lys Asp Arg Val Ala Cys Ser Val Ala
            165                 170                 175

tac ttg gag gat att gaa act gca tgt gac caa gtc gat aat gtt atc      576
Tyr Leu Glu Asp Ile Glu Thr Ala Cys Asp Gln Val Asp Asn Val Ile
        180                 185                 190

cgc gat att tac aag tat tct aaa cct ggt tat att ttt gtt cct gca      624
Arg Asp Ile Tyr Lys Tyr Ser Lys Pro Gly Tyr Ile Phe Val Pro Ala
        195                 200                 205

gat ttt gcg gat atg tct gtt aca tgt gat aat ttg gtt aat gtt cca      672
Asp Phe Ala Asp Met Ser Val Thr Cys Asp Asn Leu Val Asn Val Pro
    210                 215                 220

cgt ata tct caa caa gat tgt ata gta tac cct tct gaa aac caa ttg      720
Arg Ile Ser Gln Gln Asp Cys Ile Val Tyr Pro Ser Glu Asn Gln Leu
225                 230                 235                 240

tct gac ata atc aac aag att act agt tgg ata tat tcc agt aaa aca      768
Ser Asp Ile Ile Asn Lys Ile Thr Ser Trp Ile Tyr Ser Ser Lys Thr
            245                 250                 255

cct gcg atc ctt gga gac gta ctg act gat agg tat ggt gtg agt aac      816
Pro Ala Ile Leu Gly Asp Val Leu Thr Asp Arg Tyr Gly Val Ser Asn
        260                 265                 270

ttt ttg aac aag ctt atc tgc aaa act ggg att tgg aat ttt tcc act      864
Phe Leu Asn Lys Leu Ile Cys Lys Thr Gly Ile Trp Asn Phe Ser Thr
        275                 280                 285

gtt atg gga aaa tct gta att gat gag tca aac cca act tat atg ggt      912
Val Met Gly Lys Ser Val Ile Asp Glu Ser Asn Pro Thr Tyr Met Gly
    290                 295                 300

caa tat aat ggt aaa gaa ggt tta aaa caa gtc tat gaa cat ttt gaa      960
Gln Tyr Asn Gly Lys Glu Gly Leu Lys Gln Val Tyr Glu His Phe Glu
305                 310                 315                 320

ctg tgc gac ttg gtc ttg cat ttt gga gtc gac atc aat gaa att aat     1008
Leu Cys Asp Leu Val Leu His Phe Gly Val Asp Ile Asn Glu Ile Asn
            325                 330                 335

aat ggg cat tat act ttt act tat aaa cca aat gct aaa atc att caa     1056
Asn Gly His Tyr Thr Phe Thr Tyr Lys Pro Asn Ala Lys Ile Ile Gln
        340                 345                 350

ttt cat ccg aat tat att cgc ctt gtg gac act agg cag ggc aat gag     1104
Phe His Pro Asn Tyr Ile Arg Leu Val Asp Thr Arg Gln Gly Asn Glu
        355                 360                 365

caa atg ttc aaa gga atc aat ttt gcc cct att tta aaa gaa cta tac     1152
Gln Met Phe Lys Gly Ile Asn Phe Ala Pro Ile Leu Lys Glu Leu Tyr
    370                 375                 380

aag cgc att gac gtt tct aaa ctt tct ttg caa tat gat tca aat gta     1200
Lys Arg Ile Asp Val Ser Lys Leu Ser Leu Gln Tyr Asp Ser Asn Val
385                 390                 395                 400

act caa tat acg aac gaa aca atg cgg tta gaa gat cct acc aat gga 1248
Thr Gln Tyr Thr Asn Glu Thr Met Arg Leu Glu Asp Pro Thr Asn Gly
                405                 410                 415

caa tca agc att att aca caa gtt cac tta caa aag acg atg cct aaa 1296
Gln Ser Ser Ile Ile Thr Gln Val His Leu Gln Lys Thr Met Pro Lys
            420                 425                 430

ttt ttg aac cct ggt gat gtt gtc gtt tgt gaa aca ggc tct ttt caa 1344
Phe Leu Asn Pro Gly Asp Val Val Val Cys Glu Thr Gly Ser Phe Gln
        435                 440                 445

ttc tct gtt cgt gat ttc gcg ttt cct tcg caa tta aaa tat ata tcg 1392
Phe Ser Val Arg Asp Phe Ala Phe Pro Ser Gln Leu Lys Tyr Ile Ser
    450                 455                 460

caa gga ttt ttc ctt tcc att ggc atg gcc ctt cct gcc gcc cta ggt 1440
Gln Gly Phe Phe Leu Ser Ile Gly Met Ala Leu Pro Ala Ala Leu Gly
465                 470                 475                 480

gtt gga att gcc atg caa gac cac tca aac gct cac atc aat ggt ggc 1488
Val Gly Ile Ala Met Gln Asp His Ser Asn Ala His Ile Asn Gly Gly
                485                 490                 495

aac gta aaa gag gac tat aag cca aga tta att ttg ttt gaa ggt gac 1536
Asn Val Lys Glu Asp Tyr Lys Pro Arg Leu Ile Leu Phe Glu Gly Asp
            500                 505                 510

ggt gca gca cag atg aca atc caa gaa ctg agc acc att ctg aag tgc 1584
Gly Ala Ala Gln Met Thr Ile Gln Glu Leu Ser Thr Ile Leu Lys Cys
        515                 520                 525

aat att cca cta gaa gtt atc att tgg aac aat aac ggc tac act att 1632
Asn Ile Pro Leu Glu Val Ile Ile Trp Asn Asn Asn Gly Tyr Thr Ile
    530                 535                 540

gaa aga gcc atc atg ggc cct acc agg tcg tat aac gac gtt atg tct 1680
Glu Arg Ala Ile Met Gly Pro Thr Arg Ser Tyr Asn Asp Val Met Ser
545                 550                 555                 560

tgg aaa tgg acc aaa cta ttt gaa gca ttc gga gac ttc gac gga aag 1728
Trp Lys Trp Thr Lys Leu Phe Glu Ala Phe Gly Asp Phe Asp Gly Lys
                565                 570                 575

tat act aat agc act ctc att caa tgt ccc tct aaa tta gca ctg aaa 1776
Tyr Thr Asn Ser Thr Leu Ile Gln Cys Pro Ser Lys Leu Ala Leu Lys
            580                 585                 590

ttg gag gag ctt aag aat tca aac aaa aga agc ggg ata gaa ctt tta 1824
Leu Glu Glu Leu Lys Asn Ser Asn Lys Arg Ser Gly Ile Glu Leu Leu
        595                 600                 605

gaa gtc aaa tta ggc gaa ttg gat ttc ccc gaa cag cta aag tgc atg 1872
Glu Val Lys Leu Gly Glu Leu Asp Phe Pro Glu Gln Leu Lys Cys Met
    610                 615                 620

gtt gaa gca gcg gca ctt aaa aga aat aaa aaa tag 1908
Val Glu Ala Ala Ala Leu Lys Arg Asn Lys Lys
625                 630                 635

<210> 57
<211> 635

<212>  PRT
<213>  Saccharomyces cerevisiae

<400>  57

Met Ala Pro Val Thr Ile Glu Lys Phe Val Asn Gln Glu Glu Arg His
1               5                   10                  15

Leu Val Ser Asn Arg Ser Ala Thr Ile Pro Phe Gly Glu Tyr Ile Phe
            20                  25                  30

Lys Arg Leu Leu Ser Ile Asp Thr Lys Ser Val Phe Gly Val Pro Gly
        35                  40                  45

Asp Phe Asn Leu Ser Leu Leu Glu Tyr Leu Tyr Ser Pro Ser Val Glu
    50                  55                  60

Ser Ala Gly Leu Arg Trp Val Gly Thr Cys Asn Glu Leu Asn Ala Ala
65                  70                  75                  80

Tyr Ala Ala Asp Gly Tyr Ser Arg Tyr Ser Asn Lys Ile Gly Cys Leu
                85                  90                  95

Ile Thr Thr Tyr Gly Val Gly Glu Leu Ser Ala Leu Asn Gly Ile Ala
            100                 105                 110

Gly Ser Phe Ala Glu Asn Val Lys Val Leu His Ile Val Gly Val Ala
        115                 120                 125

Lys Ser Ile Asp Ser Arg Ser Ser Asn Phe Ser Asp Arg Asn Leu His
    130                 135                 140

His Leu Val Pro Gln Leu His Asp Ser Asn Phe Lys Gly Pro Asn His
145                 150                 155                 160

Lys Val Tyr His Asp Met Val Lys Asp Arg Val Ala Cys Ser Val Ala
                165                 170                 175

Tyr Leu Glu Asp Ile Glu Thr Ala Cys Asp Gln Val Asp Asn Val Ile
            180                 185                 190

Arg Asp Ile Tyr Lys Tyr Ser Lys Pro Gly Tyr Ile Phe Val Pro Ala
        195                 200                 205

Asp Phe Ala Asp Met Ser Val Thr Cys Asp Asn Leu Val Asn Val Pro
    210                 215                 220

Arg Ile Ser Gln Gln Asp Cys Ile Val Tyr Pro Ser Glu Asn Gln Leu
225                 230                 235                 240

Ser Asp Ile Ile Asn Lys Ile Thr Ser Trp Ile Tyr Ser Ser Lys Thr
                245                 250                 255

Pro Ala Ile Leu Gly Asp Val Leu Thr Asp Arg Tyr Gly Val Ser Asn
            260                 265                 270

Phe Leu Asn Lys Leu Ile Cys Lys Thr Gly Ile Trp Asn Phe Ser Thr
        275                 280                 285

Val Met Gly Lys Ser Val Ile Asp Glu Ser Asn Pro Thr Tyr Met Gly
    290                 295                 300

Gln Tyr Asn Gly Lys Glu Gly Leu Lys Gln Val Tyr Glu His Phe Glu
305                 310                 315                 320

Leu Cys Asp Leu Val Leu His Phe Gly Val Asp Ile Asn Glu Ile Asn
                325                 330                 335

Asn Gly His Tyr Thr Phe Thr Tyr Lys Pro Asn Ala Lys Ile Ile Gln
            340                 345                 350

Phe His Pro Asn Tyr Ile Arg Leu Val Asp Thr Arg Gln Gly Asn Glu
        355                 360                 365

Gln Met Phe Lys Gly Ile Asn Phe Ala Pro Ile Leu Lys Glu Leu Tyr
    370                 375                 380

Lys Arg Ile Asp Val Ser Lys Leu Ser Leu Gln Tyr Asp Ser Asn Val
385                 390                 395                 400

Thr Gln Tyr Thr Asn Glu Thr Met Arg Leu Glu Asp Pro Thr Asn Gly
                405                 410                 415

Gln Ser Ser Ile Ile Thr Gln Val His Leu Gln Lys Thr Met Pro Lys
            420                 425                 430

Phe Leu Asn Pro Gly Asp Val Val Val Cys Glu Thr Gly Ser Phe Gln
        435                 440                 445

Phe Ser Val Arg Asp Phe Ala Phe Pro Ser Gln Leu Lys Tyr Ile Ser
    450                 455                 460

Gln Gly Phe Phe Leu Ser Ile Gly Met Ala Leu Pro Ala Ala Leu Gly
465                 470                 475                 480

Val Gly Ile Ala Met Gln Asp His Ser Asn Ala His Ile Asn Gly Gly

485                 490                 495

Asn Val Lys Glu Asp Tyr Lys Pro Arg Leu Ile Leu Phe Glu Gly Asp
            500                 505                 510

Gly Ala Ala Gln Met Thr Ile Gln Glu Leu Ser Thr Ile Leu Lys Cys
        515                 520                 525

Asn Ile Pro Leu Glu Val Ile Ile Trp Asn Asn Asn Gly Tyr Thr Ile
    530                 535                 540

Glu Arg Ala Ile Met Gly Pro Thr Arg Ser Tyr Asn Asp Val Met Ser
545                 550                 555                 560

Trp Lys Trp Thr Lys Leu Phe Glu Ala Phe Gly Asp Phe Asp Gly Lys
                565                 570                 575

Tyr Thr Asn Ser Thr Leu Ile Gln Cys Pro Ser Lys Leu Ala Leu Lys
            580                 585                 590

Leu Glu Glu Leu Lys Asn Ser Asn Lys Arg Ser Gly Ile Glu Leu Leu
        595                 600                 605

Glu Val Lys Leu Gly Glu Leu Asp Phe Pro Glu Gln Leu Lys Cys Met
    610                 615                 620

Val Glu Ala Ala Ala Leu Lys Arg Asn Lys Lys
625                 630                 635

<210>  58
<211>  635
<212>  PRT
<213>  Saccharomyces eubayanus

<220>
<221>  POLYPEPTIDE
<222>  (1)..(635)
<223>  phenylpyruvate decarboxylase

<300>
<308>  NCBI Reference Sequence: XP_018223917.1
<309>  2017-09-27
<313>  (1)..(635)

<400>  58

Met Ala Pro Val Thr Ile Asp Thr Phe Met Asp Gln Asp Glu Pro His
1               5                   10                  15

Val Val Phe Asn Gln Ser Ala Thr Ile Pro Phe Gly Glu Tyr Ile Phe
            20                  25                  30

Arg Arg Leu Phe Ser Ile Gly Thr Lys Ser Val Phe Gly Val Pro Gly
        35                  40                  45

Asp Phe Asn Leu Ser Leu Leu Glu Tyr Leu Tyr Ala Pro Ser Val Glu
    50                  55                  60

Glu Asp Gly Leu Lys Trp Val Gly Thr Cys Asn Glu Leu Asn Ala Ala
65                  70                  75                  80

Tyr Ala Ala Asp Gly Tyr Ser Arg Tyr Ser Asn Lys Ile Gly Cys Leu
                85                  90                  95

Ile Thr Thr Tyr Gly Val Gly Glu Leu Ser Ala Leu Asn Gly Ile Ala
            100                 105                 110

Gly Ser Phe Ala Glu Asn Val Lys Val Leu His Ile Val Gly Val Ala
        115                 120                 125

Lys Ser Thr Asp Ser Arg Ser Asp Asn Phe Arg Glu Arg Asn Leu His
    130                 135                 140

His Leu Val Pro Arg Leu His Asp Ser Asn Phe Lys Gly Pro Asn His
145                 150                 155                 160

Lys Val Tyr His Asp Met Val Lys Asp Asn Val Ala Cys Ser Val Ala
                165                 170                 175

Tyr Leu Glu Asp Ile Glu Thr Ala Cys Asp Gln Val Asp Ala Val Ile
            180                 185                 190

Arg Asp Ile Tyr Arg Tyr Ser Lys Pro Gly Tyr Val Phe Val Pro Ala
        195                 200                 205

Asp Phe Pro Asp Met Ser Val Arg Leu Asp Asn Leu Ile Ser Val Pro
    210                 215                 220

His Ile Ser Gln Gln Asp Cys Ile Thr Ile Pro Ser Glu Thr His Leu
225                 230                 235                 240

Phe Ser Ile Ile Asp Met Ile Thr Thr Trp Met Tyr Ser Ser Lys Thr
                245                 250                 255

Pro Ala Ile Leu Gly Asp Val Leu Thr Asp Arg Tyr Gly Val Ser Asp
            260                 265                 270

Ile Leu Asn Lys Leu Ile Tyr Ala Ser Lys Ile Trp Asn Phe Ser Thr

275                 280                 285

Val Met Gly Lys Ser Val Ile Asp Glu Thr Asn Pro Ser Tyr Met Gly
    290                 295                 300

Gln Tyr Asn Gly Lys Glu Gly Leu Lys Gln Val Tyr Glu His Phe Glu
305                 310                 315                 320

Ser Cys Asp Leu Ile Leu His Phe Gly Val Asp Ile Asn Glu Ile Asn
                325                 330                 335

Asn Gly His Tyr Ser Phe Thr Tyr Lys Pro Asn Ala Lys Ile Ile Gln
            340                 345                 350

Phe His Pro Asn Tyr Ile Arg Phe Val Asp Thr Arg Tyr Gly Glu Glu
        355                 360                 365

Gln Ile Phe Glu Gly Val Asn Phe Ala Pro Val Leu Gln Glu Leu Tyr
    370                 375                 380

Gln His Ile Asp Val Ser Lys Leu Ser Leu Gln Tyr Asp Pro Ser Val
385                 390                 395                 400

Ile Pro Tyr Thr Asn Glu Thr Met Lys Leu Glu Asp Pro Thr Ser Asp
                405                 410                 415

Gln Ser Thr Asp Ile Thr Gln Val His Leu Gln Lys Val Ile Pro Lys
            420                 425                 430

Phe Leu Asn Pro Gly Asp Val Val Val Cys Glu Thr Gly Ser Phe Gln
        435                 440                 445

Phe Ser Val Arg Asp Phe Ala Phe Pro Thr Gln Leu Lys Tyr Ile Ser
    450                 455                 460

Gln Gly Phe Phe Leu Ser Ile Gly Met Ala Leu Pro Ala Ala Leu Gly
465                 470                 475                 480

Val Gly Ile Ala Met Gln Asp His Pro Asn Ala His Ile Asn Gly Gly
                485                 490                 495

Asn Ile Asn Glu Gly Tyr Lys Pro Arg Leu Ile Leu Phe Glu Gly Asp
            500                 505                 510

Gly Ala Ala Gln Met Thr Ile Gln Glu Leu Ser Thr Ile Leu Lys Phe
        515                 520                 525

His Ile Pro Ile Glu Ile Val Ile Trp Asn Asn Asn Gly Tyr Thr Ile
    530                 535                 540

Glu Arg Ala Ile Met Gly Pro Thr Arg Ser Tyr Asn Asp Ile Met Ser
545                 550                 555                 560

Trp Lys Trp Thr Lys Leu Phe Glu Ala Phe Gly Asp Phe Asp Gly Lys
                565                 570                 575

Tyr Thr Asn Asn Thr Ile Ile Glu Ser Thr Ser Lys Leu Ala Leu Lys
            580                 585                 590

Leu Lys Asp Leu Lys Asn Ser Ser Ala Arg Asp Arg Ile Glu Leu Leu
        595                 600                 605

Glu Val Lys Leu Gly Val Leu Asp Phe Pro Glu Gln Leu Lys Cys Met
    610                 615                 620

Val Glu Ala Ala Ala Leu Lys Arg Asn Lys Lys
625                 630                 635

<210>  59
<211>  635
<212>  PRT
<213>  Saccharomyces arboricola H-6

<220>
<221>  POLYPEPTIDE
<222>  (1)..(635)
<223>  phenylpyruvate decarboxylase

<300>
<308>  GenBank: EJS42243.1
<309>  2012-08-16
<313>  (1)..(635)

<400>  59

Met Ala Pro Val Thr Ile Asp Thr Phe Val Asn Gln Asp Lys Pro His
1               5                   10                  15

Val Val Ser Asn Gln Ser Ala Thr Ile Thr Phe Gly Glu Tyr Ile Phe
            20                  25                  30

Arg Arg Leu Leu Ser Ile Gly Thr Lys Ser Val Phe Gly Val Pro Gly
        35                  40                  45

Asp Phe Asn Leu Ser Leu Leu Glu Tyr Leu Tyr Ala Pro Ser Val Glu
    50                  55                  60

Ser Ala Gly Leu Lys Trp Val Gly Thr Cys Asn Glu Leu Asn Ala Ala

65                  70                  75                  80

Tyr Ala Ala Asp Gly Tyr Ser Arg Tyr Ser Asn Lys Ile Gly Cys Leu
                85                  90                  95

Ile Thr Thr Tyr Gly Val Gly Glu Leu Ser Ala Leu Asn Gly Ile Ala
            100                 105                 110

Gly Ser Phe Ala Glu Asn Val Lys Val Leu His Ile Val Gly Val Ala
        115                 120                 125

Lys Ser Met Asp Ser Arg Ser Asp Asn Phe Lys Asp Arg Asn Leu His
    130                 135                 140

His Leu Val Pro Gln Leu His Asp Ser Asn Phe Lys Ala Pro Asn His
145                 150                 155                 160

Lys Val Tyr His Asp Met Val Lys Asp Arg Val Ala Cys Ser Val Ala
                165                 170                 175

Tyr Leu Glu Asp Ile Glu Thr Ala Cys Asp Gln Val Asp Ala Val Ile
            180                 185                 190

Arg Asp Ile Tyr Lys Tyr Ser Lys Pro Gly Tyr Ile Phe Val Pro Ala
        195                 200                 205

Asp Phe Ala Asp Met Ser Val Ala Pro Ala Asn Leu Ile Asp Val Ser
    210                 215                 220

His Ile Asn Gln Gln Asp Cys Ile Thr Phe Pro Ser Glu Ile Gln Leu
225                 230                 235                 240

Phe Asp Val Ile Asp Arg Ala Thr Lys Trp Ile Tyr Ser Ser Lys Thr
                245                 250                 255

Pro Ala Ile Ile Gly Asp Val Leu Thr Asp Arg Tyr Gly Val Ser Asp
            260                 265                 270

Val Leu Asn Lys Leu Ile Tyr Ala Thr Gly Ile Trp Asn Phe Ser Thr
        275                 280                 285

Val Met Gly Lys Ser Val Leu Asp Glu Ser Asn Pro Thr Tyr Met Gly
    290                 295                 300

Gln Tyr Asn Gly Lys Glu Gly Leu Lys Gln Val Tyr Gln His Phe Glu
305                 310                 315                 320

Asp Cys Asp Leu Val Leu His Phe Gly Val Asp Ile Asn Glu Ile Asn
                325                 330                 335

Asn Gly His Tyr Thr Phe Thr Tyr Lys Pro Asn Ala Lys Ile Ile Gln
            340                 345                 350

Phe His Pro Asn Tyr Val Arg Phe Val Asp Thr Arg Tyr Gly Glu Glu
        355                 360                 365

Gln Met Phe Glu Gly Ile Asn Phe Ala Pro Val Leu Gln Glu Leu Tyr
    370                 375                 380

Lys Arg Ile Asp Val Ser Arg Leu Ser Leu Gln Tyr Asp Pro Ser Val
385                 390                 395                 400

Thr Pro Tyr Thr Asn Glu Thr Met Gln Leu Glu Asp Pro Ile Asn Glu
                405                 410                 415

Gln Ser Asn Asn Ile Thr Gln Ile His Leu Gln Lys Val Met Pro Lys
            420                 425                 430

Phe Leu Asn Pro Gly Asp Val Val Val Cys Glu Thr Gly Ser Phe Gln
        435                 440                 445

Phe Ser Val Arg Asp Phe Ala Phe Pro Thr Gln Leu Lys Tyr Ile Ser
    450                 455                 460

Gln Gly Phe Phe Leu Ser Ile Gly Met Ala Leu Pro Ala Ala Leu Gly
465                 470                 475                 480

Val Gly Ile Ala Met Gln Asp Tyr Pro Lys Ala His Ile Thr Gly Gly
                485                 490                 495

Gly Ile Lys Glu Asp Tyr Lys Pro Arg Leu Ile Leu Phe Glu Gly Asp
            500                 505                 510

Gly Ala Ala Gln Met Thr Ile Gln Glu Leu Ser Thr Ile Leu Lys Ser
        515                 520                 525

Asn Ile Pro Ile Glu Ile Val Ile Trp Asn Asn Asn Gly Tyr Thr Ile
    530                 535                 540

Glu Arg Ala Ile Met Gly Pro Thr Ser Ser Tyr Asn Asp Ile Met Pro
545                 550                 555                 560

Trp Lys Trp Thr Lys Leu Phe Glu Ala Phe Gly Asp Phe Asp Gly Lys
                565                 570                 575

His Thr Asn Asn Thr Val Ile Glu Ser Pro Ser Gln Leu Ser Leu Lys
            580                 585                 590

Leu Lys Glu Leu Lys Asn Ser Lys Thr Arg Asp Arg Ile Glu Leu Leu
        595                 600                 605

Glu Val Lys Leu Gly Val Phe Asp Phe Pro Glu Gln Leu Lys Cys Met
    610                 615                 620

Val Glu Ala Ala Ala Leu Lys Arg Asn Lys Lys
625                 630                 635

<210>  60
<211>  635
<212>  PRT
<213>  Saccharomyces kudriavzevii IFO 1802

<220>
<221>  POLYPEPTIDE
<222>  (1)..(635)
<223>  phenylpyruvate decarboxylase

<300>
<308>  GenBank: EJT42082.1
<309>  2012-08-21
<313>  (1)..(635)

<400>  60

Met Thr Pro Val Thr Ile Asn Thr Phe Val Asn Gln Lys Glu Pro His
1               5                   10                  15

Leu Val Ser Asn Glu Leu Ala Thr Ile Ser Phe Gly Glu Tyr Ile Phe
            20                  25                  30

Arg Arg Leu Leu Ser Thr Gly Thr Lys Ser Val Phe Gly Val Pro Gly
        35                  40                  45

Asp Phe Asn Leu Ser Leu Leu Glu Tyr Leu Tyr Ala Pro Ser Val Glu
    50                  55                  60

Ser Ala Gly Leu Lys Trp Ile Gly Thr Cys Asn Glu Leu Asn Ala Ala
65                  70                  75                  80

Tyr Ala Ala Asp Gly Tyr Ser Arg Tyr Ser Asn Asn Ile Gly Cys Leu
                85                  90                  95

Val Thr Thr Phe Gly Val Gly Glu Leu Ser Ala Leu Asn Gly Val Ala
            100                 105                 110

Gly Ser Phe Ala Glu Asn Val Lys Val Leu His Ile Val Gly Val Ala
        115                 120                 125

Lys Ser Ile Asp Ser Arg Ser Asp Asn Phe Arg Asp Arg Asn Met His
    130                 135                 140

His Leu Val Pro Gln Leu His Gly Ser Asn Phe Lys Gly Pro Asn His
145                 150                 155                 160

Lys Val Tyr His Asp Met Val Lys Asp Arg Val Ala Cys Ser Val Ala
                165                 170                 175

Tyr Leu Glu Asp Ile Glu Thr Ala Cys Asp Gln Val Asp Asn Val Ile
            180                 185                 190

Arg Asp Ile Tyr Lys Tyr Ser Lys Pro Gly Tyr Ile Phe Val Pro Ala
        195                 200                 205

Asp Phe Ala Asp Met Ser Val Thr Cys Glu Asn Leu Ile Asn Val Pro
    210                 215                 220

His Ile Ser Gln Gln Asp Cys Ile Thr Tyr Pro Ser Gln Ile Gln Leu
225                 230                 235                 240

Phe Asp Ile Leu Asp Arg Ile Thr Ser Trp Met Tyr Ser Ser Lys Thr
                245                 250                 255

Pro Ala Ile Leu Gly Asp Val Leu Thr Asp Arg Tyr Gly Val Ser Glu
            260                 265                 270

Ile Leu Asn Lys Leu Ile Tyr Ala Thr Gly Ile Trp Asn Phe Ser Thr
        275                 280                 285

Val Met Gly Lys Ser Val Ile Asp Glu Ala Asn Pro Lys Tyr Met Gly
    290                 295                 300

Gln Tyr Asn Gly Lys Glu Gly Leu Arg Gln Val Tyr Glu His Phe Glu
305                 310                 315                 320

Ser Cys Asp Leu Val Leu His Phe Gly Val Asp Ile Asn Glu Ile Asn
                325                 330                 335

Asn Gly His Tyr Thr Phe Thr Tyr Lys Pro Asn Ala Lys Ile Ile Gln
            340                 345                 350

Phe His Pro Asn Tyr Ile Arg Phe Val Asp Thr Lys Tyr Gly His Glu
        355                 360                 365

Gln Met Phe Glu Gly Ile Asn Phe Ala Pro Val Leu Met Glu Leu Tyr
    370                 375                 380

Lys Gly Ile Asp Val Ser Arg Leu Ser Leu Gln Tyr Asp Ser Gly Val
385                 390                 395                 400

Thr Ser Tyr Thr Asn Glu Thr Met Gln Leu Glu Asp Pro Thr Asn Asp
                405                 410                 415

Gln Ser Asn Asn Ile Thr Gln Val His Leu Gln Lys Val Met Pro Lys
            420                 425                 430

Phe Leu Ser Pro Gly Asp Val Val Val Cys Glu Thr Gly Ser Phe Gln
        435                 440                 445

Phe Ser Val Arg Asp Phe Ala Phe Pro Thr Gln Leu Lys Tyr Ile Ser
    450                 455                 460

Gln Gly Phe Phe Leu Ser Ile Gly Met Ala Leu Pro Ala Ala Leu Gly
465                 470                 475                 480

Val Gly Ile Ala Met Gln Asp Phe Pro Asn Ala His Ile Ser Asp Glu
                485                 490                 495

Asn Ile Gly Glu Gly Tyr Lys Pro Arg Leu Ile Leu Phe Glu Gly Asp
            500                 505                 510

Gly Ala Ala Gln Met Thr Ile Gln Glu Leu Ser Thr Ile Leu Lys Cys
        515                 520                 525

His Ile Thr Ile Asp Ile Phe Ile Trp Asn Asn Asn Gly Tyr Thr Ile
    530                 535                 540

Glu Arg Ala Ile Met Gly Pro Thr Arg Ala Tyr Asn Asp Val Met Ser
545                 550                 555                 560

Trp Lys Trp Thr Lys Leu Leu Glu Ala Phe Gly Asp Phe Asp Arg Lys
                565                 570                 575

His Thr Asn Ser Thr Val Ile Glu Tyr Pro Ser Lys Leu Ala Met Lys
            580                 585                 590

Leu Glu Glu Leu Lys Ser Ser Asn Thr Arg Asp Gly Ile Glu Leu Val
        595                 600                 605

Glu Val Lys Leu Gly Val Leu Asp Phe Pro Glu Gln Leu Lys Cys Met
    610                 615                 620

Val Asp Ala Ala Ala Leu Lys Arg Asn Lys Lys
625                 630                 635

<210>  61
<211>  1692
<212>  DNA
<213>  Saccharomyces cerevisiae

<220>
<221>  CDS
<222>  (1)..(1692)
<223>  pyruvate decarboxylase

<300>
<308>  NCBI Reference Sequence: NM_001182021.1
<309>  2018-04-04
<313>  (1)..(1692)

<400>  61
atg tct gaa ata acc tta ggt aaa tat tta ttt gaa aga ttg agc caa      48
Met Ser Glu Ile Thr Leu Gly Lys Tyr Leu Phe Glu Arg Leu Ser Gln
1               5                   10                  15

gtc aac tgt aac acc gtc ttc ggt ttg cca ggt gac ttt aac ttg tct      96
Val Asn Cys Asn Thr Val Phe Gly Leu Pro Gly Asp Phe Asn Leu Ser
            20                  25                  30

ctt ttg gat aag ctt tat gaa gtc aaa ggt atg aga tgg gct ggt aac      144
Leu Leu Asp Lys Leu Tyr Glu Val Lys Gly Met Arg Trp Ala Gly Asn
        35                  40                  45

gct aac gaa ttg aac gct gcc tat gct gct gat ggt tac gct cgt atc      192
Ala Asn Glu Leu Asn Ala Ala Tyr Ala Ala Asp Gly Tyr Ala Arg Ile
    50                  55                  60

aag ggt atg tcc tgt att att acc acc ttc ggt gtt ggt gaa ttg tct      240
Lys Gly Met Ser Cys Ile Ile Thr Thr Phe Gly Val Gly Glu Leu Ser
65                  70                  75                  80

gct ttg aat ggt att gcc ggt tct tac gct gaa cat gtc ggt gtt ttg      288
Ala Leu Asn Gly Ile Ala Gly Ser Tyr Ala Glu His Val Gly Val Leu
                85                  90                  95

cac gtt gtt ggt gtt cca tcc atc tct tct caa gct aag caa ttg ttg      336
His Val Val Gly Val Pro Ser Ile Ser Ser Gln Ala Lys Gln Leu Leu
            100                 105                 110

ttg cat cat acc ttg ggt aac ggt gac ttc act gtt ttc cac aga atg      384
Leu His His Thr Leu Gly Asn Gly Asp Phe Thr Val Phe His Arg Met
        115                 120                 125

tct gcc aac att tct gaa acc act gcc atg atc act gat att gct aac      432
Ser Ala Asn Ile Ser Glu Thr Thr Ala Met Ile Thr Asp Ile Ala Asn
    130                 135                 140

gct cca gct gaa att gac aga tgt atc aga acc acc tac act acc caa      480
Ala Pro Ala Glu Ile Asp Arg Cys Ile Arg Thr Thr Tyr Thr Thr Gln
145                 150                 155                 160

aga cca gtc tac ttg ggt ttg cca gct aac ttg gtt gac ttg aac gtc 528
Arg Pro Val Tyr Leu Gly Leu Pro Ala Asn Leu Val Asp Leu Asn Val
                165                 170                 175

cca gcc aag tta ttg gaa act cca att gac ttg tct ttg aag cca aac 576
Pro Ala Lys Leu Leu Glu Thr Pro Ile Asp Leu Ser Leu Lys Pro Asn
            180                 185                 190

gac gct gaa gct gaa gct gaa gtt gtt aga act gtt gtt gaa ttg atc 624
Asp Ala Glu Ala Glu Ala Glu Val Val Arg Thr Val Val Glu Leu Ile
        195                 200                 205

aag gat gct aag aac cca gtt atc ttg gct gat gct tgt gct tct aga 672
Lys Asp Ala Lys Asn Pro Val Ile Leu Ala Asp Ala Cys Ala Ser Arg
    210                 215                 220

cat gat gtc aag gct gaa act aag aag ttg atg gac ttg act caa ttc 720
His Asp Val Lys Ala Glu Thr Lys Lys Leu Met Asp Leu Thr Gln Phe
225                 230                 235                 240

cca gtt tac gtc acc cca atg ggt aag ggt gct att gac gaa caa cac 768
Pro Val Tyr Val Thr Pro Met Gly Lys Gly Ala Ile Asp Glu Gln His
                245                 250                 255

cca aga tac ggt ggt gtt tac gtt ggt acc ttg tct aga cca gaa gtt 816
Pro Arg Tyr Gly Gly Val Tyr Val Gly Thr Leu Ser Arg Pro Glu Val
            260                 265                 270

aag aag gct gta gaa tct gct gat ttg ata ttg tct atc ggt gct ttg 864
Lys Lys Ala Val Glu Ser Ala Asp Leu Ile Leu Ser Ile Gly Ala Leu
        275                 280                 285

ttg tct gat ttc aat acc ggt tct ttc tct tac tcc tac aag acc aaa 912
Leu Ser Asp Phe Asn Thr Gly Ser Phe Ser Tyr Ser Tyr Lys Thr Lys
    290                 295                 300

aat atc gtt gaa ttc cac tct gac cac atc aag atc aga aac gcc acc 960
Asn Ile Val Glu Phe His Ser Asp His Ile Lys Ile Arg Asn Ala Thr
305                 310                 315                 320

ttc cca ggt gtt caa atg aaa ttt gcc ttg caa aaa ttg ttg gat gct 1008
Phe Pro Gly Val Gln Met Lys Phe Ala Leu Gln Lys Leu Leu Asp Ala
                325                 330                 335

att cca gaa gtc gtc aag gac tac aaa cct gtt gct gtc cca gct aga 1056
Ile Pro Glu Val Val Lys Asp Tyr Lys Pro Val Ala Val Pro Ala Arg
            340                 345                 350

gtt cca att acc aag tct act cca gct aac act cca atg aag caa gaa 1104
Val Pro Ile Thr Lys Ser Thr Pro Ala Asn Thr Pro Met Lys Gln Glu
        355                 360                 365

tgg atg tgg aac cat ttg ggt aac ttc ttg aga gaa ggt gat att gtt 1152
Trp Met Trp Asn His Leu Gly Asn Phe Leu Arg Glu Gly Asp Ile Val
    370                 375                 380

att gct gaa acc ggt act tcc gcc ttc ggt att aac caa act act ttc 1200
Ile Ala Glu Thr Gly Thr Ser Ala Phe Gly Ile Asn Gln Thr Thr Phe
385                 390                 395                 400

cca aca gat gta tac gct atc gtc caa gtc ttg tgg ggt tcc att ggt 1248
Pro Thr Asp Val Tyr Ala Ile Val Gln Val Leu Trp Gly Ser Ile Gly
                405                 410                 415

ttc aca gtc ggc gct cta ttg ggt gct act atg gcc gct gaa gaa ctt 1296
Phe Thr Val Gly Ala Leu Leu Gly Ala Thr Met Ala Ala Glu Glu Leu
            420                 425                 430

gat cca aag aag aga gtt att tta ttc att ggt gac ggt tct cta caa 1344
Asp Pro Lys Lys Arg Val Ile Leu Phe Ile Gly Asp Gly Ser Leu Gln
        435                 440                 445

ttg act gtt caa gaa atc tct acc atg att aga tgg ggt ttg aag cca 1392
Leu Thr Val Gln Glu Ile Ser Thr Met Ile Arg Trp Gly Leu Lys Pro
    450                 455                 460

tac att ttt gtc ttg aat aac aac ggt tac acc att gaa aaa ttg att 1440
Tyr Ile Phe Val Leu Asn Asn Asn Gly Tyr Thr Ile Glu Lys Leu Ile
465                 470                 475                 480

cac ggt cct cat gcc gaa tat aat gaa att caa ggt tgg gac cac ttg 1488
His Gly Pro His Ala Glu Tyr Asn Glu Ile Gln Gly Trp Asp His Leu
                485                 490                 495

gcc tta ttg cca act ttt ggt gct aga aac tac gaa acc cac aga gtt 1536
Ala Leu Leu Pro Thr Phe Gly Ala Arg Asn Tyr Glu Thr His Arg Val
            500                 505                 510

gct acc act ggt gaa tgg gaa aag ttg act caa gac aag gac ttc caa 1584
Ala Thr Thr Gly Glu Trp Glu Lys Leu Thr Gln Asp Lys Asp Phe Gln
        515                 520                 525

gac aac tct aag att aga atg att gaa gtt atg ttg cca gtc ttt gat 1632
Asp Asn Ser Lys Ile Arg Met Ile Glu Val Met Leu Pro Val Phe Asp
    530                 535                 540

gct cca caa aac ttg gtt aaa caa gct caa ttg act gcc gct act aac 1680
Ala Pro Gln Asn Leu Val Lys Gln Ala Gln Leu Thr Ala Ala Thr Asn
545                 550                 555                 560

gct aaa caa taa 1692
Ala Lys Gln

<210> 62
<211> 563
<212> PRT
<213> Saccharomyces cerevisiae

<400> 62

Met Ser Glu Ile Thr Leu Gly Lys Tyr Leu Phe Glu Arg Leu Ser Gln
1               5                   10                  15

Val Asn Cys Asn Thr Val Phe Gly Leu Pro Gly Asp Phe Asn Leu Ser
            20                  25                  30

Leu Leu Asp Lys Leu Tyr Glu Val Lys Gly Met Arg Trp Ala Gly Asn
        35                  40                  45

Ala Asn Glu Leu Asn Ala Ala Tyr Ala Ala Asp Gly Tyr Ala Arg Ile
    50                  55                  60

Lys Gly Met Ser Cys Ile Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Ile Ser Ser Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ala Met Ile Thr Asp Ile Ala Asn
    130                 135                 140

Ala Pro Ala Glu Ile Asp Arg Cys Ile Arg Thr Thr Tyr Thr Thr Gln
145                 150                 155                 160

Arg Pro Val Tyr Leu Gly Leu Pro Ala Asn Leu Val Asp Leu Asn Val
                165                 170                 175

Pro Ala Lys Leu Leu Glu Thr Pro Ile Asp Leu Ser Leu Lys Pro Asn
            180                 185                 190

Asp Ala Glu Ala Glu Ala Glu Val Val Arg Thr Val Val Glu Leu Ile
        195                 200                 205

Lys Asp Ala Lys Asn Pro Val Ile Leu Ala Asp Ala Cys Ala Ser Arg
    210                 215                 220

His Asp Val Lys Ala Glu Thr Lys Lys Leu Met Asp Leu Thr Gln Phe
225                 230                 235                 240

Pro Val Tyr Val Thr Pro Met Gly Lys Gly Ala Ile Asp Glu Gln His
                245                 250                 255

Pro Arg Tyr Gly Gly Val Tyr Val Gly Thr Leu Ser Arg Pro Glu Val
            260                 265                 270

Lys Lys Ala Val Glu Ser Ala Asp Leu Ile Leu Ser Ile Gly Ala Leu
        275                 280                 285

Leu Ser Asp Phe Asn Thr Gly Ser Phe Ser Tyr Ser Tyr Lys Thr Lys
    290                 295                 300

Asn Ile Val Glu Phe His Ser Asp His Ile Lys Ile Arg Asn Ala Thr

305                 310                 315                 320

Phe Pro Gly Val Gln Met Lys Phe Ala Leu Gln Lys Leu Leu Asp Ala
                325                 330                 335

Ile Pro Glu Val Val Lys Asp Tyr Lys Pro Val Ala Val Pro Ala Arg
            340                 345                 350

Val Pro Ile Thr Lys Ser Thr Pro Ala Asn Thr Pro Met Lys Gln Glu
        355                 360                 365

Trp Met Trp Asn His Leu Gly Asn Phe Leu Arg Glu Gly Asp Ile Val
    370                 375                 380

Ile Ala Glu Thr Gly Thr Ser Ala Phe Gly Ile Asn Gln Thr Thr Phe
385                 390                 395                 400

Pro Thr Asp Val Tyr Ala Ile Val Gln Val Leu Trp Gly Ser Ile Gly
                405                 410                 415

Phe Thr Val Gly Ala Leu Leu Gly Ala Thr Met Ala Ala Glu Glu Leu
            420                 425                 430

Asp Pro Lys Lys Arg Val Ile Leu Phe Ile Gly Asp Gly Ser Leu Gln
        435                 440                 445

Leu Thr Val Gln Glu Ile Ser Thr Met Ile Arg Trp Gly Leu Lys Pro
    450                 455                 460

Tyr Ile Phe Val Leu Asn Asn Asn Gly Tyr Thr Ile Glu Lys Leu Ile
465                 470                 475                 480

His Gly Pro His Ala Glu Tyr Asn Glu Ile Gln Gly Trp Asp His Leu
                485                 490                 495

Ala Leu Leu Pro Thr Phe Gly Ala Arg Asn Tyr Glu Thr His Arg Val
            500                 505                 510

Ala Thr Thr Gly Glu Trp Glu Lys Leu Thr Gln Asp Lys Asp Phe Gln
        515                 520                 525

Asp Asn Ser Lys Ile Arg Met Ile Glu Val Met Leu Pro Val Phe Asp
    530                 535                 540

Ala Pro Gln Asn Leu Val Lys Gln Ala Gln Leu Thr Ala Ala Thr Asn
545                 550                 555                 560

Ala Lys Gln

<210> 63
<211> 564
<212> PRT
<213> Hanseniaspora guilliermondii

<220>
<221> POLYPEPTIDE
<222> (1)..(564)
<223> Pyruvate decarboxylase

<300>
<308> GenBank: SGZ40454.1
<309> 2017-02-02
<313> (1)..(564)

<400> 63

Met Ser Glu Ile Thr Leu Gly Arg Tyr Val Phe Glu Arg Ile Lys Gln
1               5                   10                  15

Val Gly Val Asn Thr Ile Phe Gly Leu Pro Gly Asp Phe Asn Leu Ser
            20                  25                  30

Leu Leu Asp Lys Ile Tyr Glu Val Glu Gly Leu Arg Trp Ala Ala Ser
        35                  40                  45

Leu Asn Glu Leu Asn Ala Ser Tyr Ala Ala Asp Gly Tyr Ser Arg Ile
    50                  55                  60

Lys Gly Leu Gly Val Ile Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Ile Val Gly Val Pro Ser Leu Ala Ser Gln Ala Lys Gln Leu Leu
            100                 105                 110

Leu His His Thr Leu Gly Asn Gly Asp Phe Asp Val Phe His Arg Met
        115                 120                 125

Ser Ala Asn Ile Ser Glu Thr Thr Ala Met Ile Thr Asp Leu Ala Ala
    130                 135                 140

Ala Pro Ala Glu Ile Asp Arg Cys Ile Arg Thr Ala Tyr Ile Ala Gln
145                 150                 155                 160

Arg Pro Val Tyr Leu Gly Leu Pro Ala Asn Leu Val Asp Leu Asn Val

165                 170                 175

Pro Ala Lys Leu Leu Asp Thr Lys Ile Asp Leu Ser Leu Lys Ala Asn
            180                 185                 190

Asp Ala Glu Ala Glu Glu Glu Val Val Glu Thr Ile Leu Ser Met Ile
        195                 200                 205

Ala Asn Ala Lys Asn Pro Val Ile Leu Ser Asp Ala Cys Ala Ser Arg
    210                 215                 220

His Asn Val Lys Thr Glu Val Lys Lys Leu Ile Asp Ala Thr Gln Phe
225                 230                 235                 240

Pro Ala Phe Val Thr Pro Leu Gly Lys Gly Ser Ile Asp Glu Lys His
                245                 250                 255

Pro Arg Phe Gly Gly Val Tyr Val Gly Thr Leu Ser Ser Pro Glu Val
            260                 265                 270

Lys Lys Ala Val Glu Gly Ala Asp Leu Val Leu Ser Val Gly Ala Leu
        275                 280                 285

Leu Ser Asp Phe Asn Thr Gly Ser Phe Ser Tyr Ser Tyr Gln Thr Lys
    290                 295                 300

Asn Ile Val Glu Phe His Ser Asp Tyr Ile Lys Ile Lys Asn Ala Ser
305                 310                 315                 320

Phe Pro Gly Val Gln Met Lys Phe Val Leu Glu Lys Leu Ile Ala Lys
                325                 330                 335

Val Gly Ala Lys Ile Ser Gly Tyr Ser Pro Ala Pro Val Pro Ser Ala
            340                 345                 350

Lys Pro Lys Asn Ala Pro Val Ala Asp Ser Thr Pro Leu Ala Gln Glu
        355                 360                 365

Trp Leu Trp Asn Glu Leu Ser Glu Phe Leu Glu Glu Gly Asp Ile Val
    370                 375                 380

Val Thr Glu Thr Gly Thr Ser Ala Phe Gly Ile Asn Gln Thr Arg Phe
385                 390                 395                 400

Pro Thr Asp Ala Tyr Gly Ile Ser Gln Val Leu Trp Gly Ser Ile Gly
                405                 410                 415

Tyr Ser Val Gly Ala Met Val Gly Ala Thr Phe Ala Ala Glu Glu Leu
            420                 425                 430

Asp Lys Ser Lys Arg Val Ile Leu Phe Val Gly Asp Gly Ser Leu Gln
        435                 440                 445

Leu Thr Val Gln Glu Ile Ala Cys Leu Ile Arg Trp Gly Leu Lys Pro
    450                 455                 460

Tyr Ile Phe Val Leu Asn Asn Asn Gly Tyr Thr Ile Glu Lys Leu Ile
465                 470                 475                 480

His Gly Pro Thr Ala Gln Tyr Asn Met Ile Gln Asn Trp Lys His Leu
                485                 490                 495

Asp Ile Leu Pro Ala Phe Gly Ala Thr Asp Tyr Glu Ala Ile Pro Val
            500                 505                 510

Lys Thr Val Gly Glu Trp Lys Lys Leu Thr Asn Asp Pro Ala Phe Lys
        515                 520                 525

Lys Asn Ser Thr Ile Arg Leu Ile Glu Val Phe Leu Pro Glu Met Asp
    530                 535                 540

Ala Pro Ser Ser Leu Val Ala Gln Ala Asn Leu Thr Ala Ala Ile Asn
545                 550                 555                 560

Ala Lys Gln Asp

<210>  64
<211>  563
<212>  PRT
<213>  Candida] glabrata

<220>
<221>  POLYPEPTIDE
<222>  (1)..(563)
<223>  pyruvate decarboxylase

<300>
<308>  GenBank: AAN77243.1
<309>  2005-03-28
<313>  (1)..(563)

<400>  64

Met Ser Glu Ile Thr Leu Gly Arg Tyr Leu Phe Glu Arg Leu Asn Gln
1               5                   10                  15

Val Asp Val Lys Thr Ile Phe Gly Leu Pro Gly Asp Phe Asn Leu Ser

20                  25                  30

Leu Leu Asp Lys Ile Tyr Glu Val Glu Gly Met Arg Trp Ala Gly Asn
        35                  40                  45

Ala Asn Glu Leu Asn Ala Ala Tyr Ala Ala Asp Gly Tyr Ala Arg Ile
    50                  55                  60

Lys Gly Met Ser Cys Ile Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Ile Ser Ser Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ala Met Val Thr Asp Ile Ala Thr
    130                 135                 140

Ala Pro Ala Glu Ile Asp Arg Cys Ile Arg Thr Thr Tyr Ile Thr Gln
145                 150                 155                 160

Arg Pro Val Tyr Leu Gly Leu Pro Ala Asn Leu Val Asp Leu Lys Val
                165                 170                 175

Pro Ala Lys Leu Leu Glu Thr Pro Ile Asp Leu Ser Leu Lys Pro Asn
            180                 185                 190

Asp Pro Glu Ala Glu Thr Glu Val Val Asp Thr Val Leu Glu Leu Ile
        195                 200                 205

Lys Ala Ala Lys Asn Pro Val Ile Leu Ala Asp Ala Cys Ala Ser Arg
    210                 215                 220

His Asp Val Lys Ala Glu Thr Lys Lys Leu Ile Asp Ala Thr Gln Phe
225                 230                 235                 240

Pro Ser Phe Val Thr Pro Met Gly Lys Gly Ser Ile Asp Glu Gln His
                245                 250                 255

Pro Arg Phe Gly Gly Val Tyr Val Gly Thr Leu Ser Arg Pro Glu Val
            260                 265                 270

Lys Glu Ala Val Glu Ser Ala Asp Leu Ile Leu Ser Val Gly Ala Leu
        275                 280                 285

Leu Ser Asp Phe Asn Thr Gly Ser Phe Ser Tyr Ser Tyr Lys Thr Lys
    290                 295                 300

Asn Ile Val Glu Phe His Ser Asp Tyr Ile Lys Ile Arg Asn Ala Thr
305                 310                 315                 320

Phe Pro Gly Val Gln Met Lys Phe Ala Leu Gln Lys Leu Leu Asn Ala
                325                 330                 335

Val Pro Glu Ala Ile Lys Gly Tyr Lys Pro Val Pro Val Pro Ala Arg
            340                 345                 350

Val Pro Glu Asn Lys Ser Cys Asp Pro Ala Thr Pro Leu Lys Gln Glu
        355                 360                 365

Trp Met Trp Asn Gln Val Ser Lys Phe Leu Gln Glu Gly Asp Val Val
    370                 375                 380

Ile Thr Glu Thr Gly Thr Ser Ala Phe Gly Ile Asn Gln Thr Pro Phe
385                 390                 395                 400

Pro Asn Asn Ala Tyr Gly Ile Ser Gln Val Leu Trp Gly Ser Ile Gly
                405                 410                 415

Phe Thr Thr Gly Ala Cys Leu Gly Ala Ala Phe Ala Ala Glu Glu Ile
            420                 425                 430

Asp Pro Lys Lys Arg Val Ile Leu Phe Ile Gly Asp Gly Ser Leu Gln
        435                 440                 445

Leu Thr Val Gln Glu Ile Ser Thr Met Ile Arg Trp Gly Leu Lys Pro
    450                 455                 460

Tyr Leu Phe Val Leu Asn Asn Asp Gly Tyr Thr Ile Glu Arg Leu Ile
465                 470                 475                 480

His Gly Glu Lys Ala Gly Tyr Asn Asp Ile Gln Asn Trp Asp His Leu
                485                 490                 495

Ala Leu Leu Pro Thr Phe Gly Ala Lys Asp Tyr Glu Asn His Arg Val
            500                 505                 510

Ala Thr Thr Gly Glu Trp Asp Lys Leu Thr Gln Asp Lys Glu Phe Asn
        515                 520                 525

Lys Asn Ser Lys Ile Arg Met Ile Glu Val Met Leu Pro Val Met Asp
    530                 535                 540

Ala Pro Thr Ser Leu Ile Glu Gln Ala Lys Leu Thr Ala Ser Thr Asn
545                 550                 555                 560

Ala Lys Gln

<210>  65
<211>  564
<212>  PRT
<213>  Kluyveromyces marxianus DMKU3-1042

<220>
<221>  POLYPEPTIDE
<222>  (1)..(564)
<223>  pyruvate decarboxylase

<300>
<308>  NCBI Reference Sequence: XP_022677160.1
<309>  2017-10-23
<313>  (1)..(564)

<400>  65

Met Ser Glu Ile Thr Leu Gly Arg Tyr Leu Phe Glu Arg Leu Lys Gln
1               5                   10                  15

Val Glu Val Gln Thr Ile Phe Gly Leu Pro Gly Asp Phe Asn Leu Ser
            20                  25                  30

Leu Leu Asp Lys Ile Tyr Glu Val Pro Gly Met Arg Trp Ala Gly Asn
        35                  40                  45

Ala Asn Glu Leu Asn Ala Ala Tyr Ala Ala Asp Gly Tyr Ala Arg Leu
    50                  55                  60

Lys Gly Met Ala Cys Val Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Ile Ser Ser Gln Ala Lys Gln Leu Leu
            100                 105                 110

Leu His His Thr Leu Gly Asn Gly Asp Phe Thr Val Phe His Arg Met
        115                 120                 125

Ser Ser Asn Ile Ser Glu Thr Thr Ala Met Ile Thr Asp Ile Asn Ser
    130                 135                 140

Ala Pro Ser Glu Ile Asp Arg Cys Ile Arg Thr Thr Tyr Ile Ser Gln
145                 150                 155                 160

Arg Pro Val Tyr Leu Gly Leu Pro Ala Asn Leu Val Asp Leu Lys Val
                165                 170                 175

Pro Ala Ser Leu Leu Glu Thr Pro Ile Asp Leu Ser Leu Lys Pro Asn
            180                 185                 190

Asp Pro Glu Ala Glu Asn Glu Val Leu Glu Thr Val Leu Glu Leu Ile
        195                 200                 205

Lys Asp Ala Lys Asn Pro Val Ile Leu Ala Asp Ala Cys Cys Ser Arg
    210                 215                 220

His Asn Val Lys Ala Glu Thr Lys Lys Leu Ile Asp Ile Thr Gln Phe
225                 230                 235                 240

Pro Ala Phe Val Thr Pro Met Gly Lys Gly Ser Ile Asp Glu Gln His
                245                 250                 255

Pro Arg Phe Gly Gly Val Tyr Val Gly Thr Leu Ser Ser Pro Glu Val
            260                 265                 270

Lys Glu Ala Val Glu Ser Ala Asp Leu Val Leu Ser Val Gly Ala Leu
        275                 280                 285

Leu Ser Asp Phe Asn Thr Gly Ser Phe Ser Tyr Ser Tyr Lys Thr Lys
    290                 295                 300

Asn Ile Val Glu Phe His Ser Asp Tyr Ile Lys Val Arg Asn Ala Thr
305                 310                 315                 320

Phe Pro Gly Val Gln Met Lys Phe Val Leu Gln Lys Leu Leu Thr Lys
                325                 330                 335

Val Lys Asp Ala Ala Lys Gly Tyr Lys Pro Val Pro Val Pro His Ala
            340                 345                 350

Pro Arg Asp Asn Lys Pro Val Ala Asp Ser Thr Pro Leu Lys Gln Glu
        355                 360                 365

Trp Val Trp Thr Gln Val Gly Lys Phe Leu Gln Glu Gly Asp Val Val
    370                 375                 380

Leu Thr Glu Thr Gly Thr Ser Ala Phe Gly Ile Asn Gln Thr His Phe
385                 390                 395                 400

Pro Asn Asp Thr Tyr Gly Ile Ser Gln Val Leu Trp Gly Ser Ile Gly
                405                 410                 415

Phe Thr Gly Gly Ala Thr Leu Gly Ala Ala Phe Ala Ala Glu Glu Ile
            420                 425                 430

Asp Pro Lys Lys Arg Val Ile Leu Phe Ile Gly Asp Gly Ser Leu Gln
        435                 440                 445

Leu Thr Val Gln Glu Ile Ser Thr Met Ile Arg Trp Gly Leu Lys Pro
    450                 455                 460

Tyr Leu Phe Val Leu Asn Asn Asp Gly Tyr Thr Ile Glu Arg Leu Ile
465                 470                 475                 480

His Gly Glu Thr Ala Gln Tyr Asn Cys Ile Gln Ser Trp Lys His Leu
                485                 490                 495

Asp Leu Leu Pro Thr Phe Gly Ala Lys Asp Tyr Glu Ala Val Arg Val
            500                 505                 510

Ala Thr Thr Gly Glu Trp Asn Lys Leu Thr Thr Asp Lys Lys Phe Gln
        515                 520                 525

Glu Asn Ser Lys Ile Arg Leu Ile Glu Val Met Leu Pro Val Met Asp
    530                 535                 540

Ala Pro Ser Asn Leu Val Lys Gln Ala Gln Leu Thr Ala Ser Ile Asn
545                 550                 555                 560

Ala Lys Gln Glu

<210>  66
<211>  525
<212>  DNA
<213>  Escherichia coli

<220>
<221>  CDS
<222>  (1)..(525)
<223>  Gene encoding shikimate kinase

<300>
<308>  NCBI Reference Sequence: WP_113388075.1
<309>  2018-07-14

<313>  (1)..(525)

<400>  66
atg aca caa cct ctt ttt ctg atc ggg cct cgg ggc tgt ggt aaa aca      48
Met Thr Gln Pro Leu Phe Leu Ile Gly Pro Arg Gly Cys Gly Lys Thr
1               5                   10                  15

acg gtc gga atg gcc ctt gcc gat tcg ctt aac cgt cgg ttt gtc gat      96
Thr Val Gly Met Ala Leu Ala Asp Ser Leu Asn Arg Arg Phe Val Asp
            20                  25                  30

acc gat cag tgg ttg caa tca cag ctc aat atg acg gtc gcg gag atc      144
Thr Asp Gln Trp Leu Gln Ser Gln Leu Asn Met Thr Val Ala Glu Ile
        35                  40                  45

gtc gaa agg gaa gag tgg gcg gga ttt cgc gcc aga gaa acg gcg gcg      192
Val Glu Arg Glu Glu Trp Ala Gly Phe Arg Ala Arg Glu Thr Ala Ala
    50                  55                  60

ctg gaa gcg gta act gcg cca tcc acc gtt atc gct aca ggc ggc ggc      240
Leu Glu Ala Val Thr Ala Pro Ser Thr Val Ile Ala Thr Gly Gly Gly
65                  70                  75                  80

att att ctg acg gaa ttt aat cgt cac ttc atg caa aat aac ggg atc      288
Ile Ile Leu Thr Glu Phe Asn Arg His Phe Met Gln Asn Asn Gly Ile
                85                  90                  95

gtg gtt tat ttg tgt gcg cca gta tca gtc ctg gtt aac cga ctg caa      336
Val Val Tyr Leu Cys Ala Pro Val Ser Val Leu Val Asn Arg Leu Gln
            100                 105                 110

gct gca ccg gaa gaa gat tta cgg cca acc tta acg gga aaa ccg ctg      384
Ala Ala Pro Glu Glu Asp Leu Arg Pro Thr Leu Thr Gly Lys Pro Leu
        115                 120                 125

agc gaa gaa gtt cag gaa gtg ctg gaa gaa cgc gat gcg cta tat cgc      432
Ser Glu Glu Val Gln Glu Val Leu Glu Glu Arg Asp Ala Leu Tyr Arg
    130                 135                 140

gaa gtt gcg cat att atc atc gac gca aca aac gaa ccc agc cag gtg      480
Glu Val Ala His Ile Ile Ile Asp Ala Thr Asn Glu Pro Ser Gln Val
145                 150                 155                 160

att tct gaa att cgc agc gcc ctg gca cag acg atc aat tgt tga          525
Ile Ser Glu Ile Arg Ser Ala Leu Ala Gln Thr Ile Asn Cys
                165                 170


<210>  67
<211>  174
<212>  PRT
<213>  Escherichia coli

<400>  67

Met Thr Gln Pro Leu Phe Leu Ile Gly Pro Arg Gly Cys Gly Lys Thr
1               5                   10                  15


Thr Val Gly Met Ala Leu Ala Asp Ser Leu Asn Arg Arg Phe Val Asp
            20                  25                  30

Thr Asp Gln Trp Leu Gln Ser Gln Leu Asn Met Thr Val Ala Glu Ile
        35                  40                  45

Val Glu Arg Glu Glu Trp Ala Gly Phe Arg Ala Arg Glu Thr Ala Ala
    50                  55                  60

Leu Glu Ala Val Thr Ala Pro Ser Thr Val Ile Ala Thr Gly Gly Gly
65                  70                  75                  80

Ile Ile Leu Thr Glu Phe Asn Arg His Phe Met Gln Asn Asn Gly Ile
                85                  90                  95

Val Val Tyr Leu Cys Ala Pro Val Ser Val Leu Val Asn Arg Leu Gln
            100                 105                 110

Ala Ala Pro Glu Glu Asp Leu Arg Pro Thr Leu Thr Gly Lys Pro Leu
        115                 120                 125

Ser Glu Glu Val Gln Glu Val Leu Glu Glu Arg Asp Ala Leu Tyr Arg
    130                 135                 140

Glu Val Ala His Ile Ile Ile Asp Ala Thr Asn Glu Pro Ser Gln Val
145                 150                 155                 160

Ile Ser Glu Ile Arg Ser Ala Leu Ala Gln Thr Ile Asn Cys
                165                 170

<210>  68
<211>  578
<212>  DNA
<213>  synthetic

<220>
<221>  promoter
<222>  (1)..(578)

<220>
<221>  promoter
<222>  (1)..(578)
<223>  TPI promoter

<400>  68
gtttaaagat tacggatatt taacttactt agaataatgc catttttttg agttataata      60

atcctacgtt agtgtgagcg ggatttaaac tgtgaggacc ttaatacatt cagacacttc      120

tgcggtatca ccctacttat tcccttcgag attatatcta ggaacccatc aggttggtgg      180

aagattaccc gttctaagac ttttcagctt cctctattga tgttacacct ggacacccct      240

tttctggcat ccagttttta atcttcagtg gcatgtgaga ttctccgaaa ttaattaaag      300

caatcacaca attctctcgg ataccacctc ggttgaaact gacaggtggt ttgttacgca      360

tgctaatgca aaggagccta tatacctttg gctcggctgc tgtaacaggg aatataaagg 420

gcagcataat ttaggagttt agtgaacttg caacatttac tattttccct tcttacgtaa 480

atatttttct ttttaattct aaatcaatct ttttcaattt tttgtttgta ttcttttctt 540

gcttaaatct ataactacaa aaaacacata cataaact 578

<210> 69
<211> 559
<212> DNA
<213> synthetic

<220>
<221> terminator
<222> (1)..(559)
<223> pYX212 terminator

<400> 69
tagggcccac aagcttacgc gtcgacccgg gtatccgtat gatgtgcctg actacgcatg 60

atatctcgag ctcagctagc taactgaata aggaacaatg aacgtttttc ctttctcttg 120

ttcctagtat taatgactga ccgatacatc cctttttttt ttgtctttgt ctagctccaa 180

ttcgccctat agtgagtcgt attacaattc actggccgtc gttttacaac gtcgtgactg 240

ggaaaaccct ggcgttaccc aacttaatcg ccttgcagca catccccctt tcgccagctg 300

gcgtaatagc gaagaggccc gcaccgatcg cccttcccaa cagttgcgca gcctgaatgg 360

cgaatggacg cgccctgtag cggcgcatta agcgcggcgg gtgtggtggt tacgcgcagc 420

gtgaccgcta cacttgccag cgccctagcg cccgctcctt tcgctttctt cccttccttt 480

ctcgccacgt tcgccggctt tccccgtcaa gctctaaatc gggggctccc tttagggttc 540

cgatttagtg gtttacggc 559

<210> 70
<211> 771
<212> DNA
<213> Saccharomyces cerevisiae

<220>
<221> CDS
<222> (1)..(771)
<223> Gene encoding feedback resistant chorismate mutase ARO7(G141S)

<400> 70
atg gat ttc aca aaa cca gaa act gtt tta aat cta caa aat att aga 48
Met Asp Phe Thr Lys Pro Glu Thr Val Leu Asn Leu Gln Asn Ile Arg
1               5                   10                  15

gat gaa tta gtt aga atg gag gat tcg atc atc ttc aaa ttt att gag 96
Asp Glu Leu Val Arg Met Glu Asp Ser Ile Ile Phe Lys Phe Ile Glu
            20                  25                  30

agg tcg cat ttc gcc aca tgt cct tca gtt tat gag gca aac cat cca          144
Arg Ser His Phe Ala Thr Cys Pro Ser Val Tyr Glu Ala Asn His Pro
        35                  40                  45

ggt tta gaa att ccg aat ttt aaa gga tct ttc ttg gat tgg gct ctt          192
Gly Leu Glu Ile Pro Asn Phe Lys Gly Ser Phe Leu Asp Trp Ala Leu
    50                  55                  60

tca aat ctt gaa att gcg cat tct cgc atc aga aga ttc gaa tca cct          240
Ser Asn Leu Glu Ile Ala His Ser Arg Ile Arg Arg Phe Glu Ser Pro
65                  70                  75                  80

gat gaa act ccc ttc ttt cct gac aag att cag aaa tca ttc tta ccg          288
Asp Glu Thr Pro Phe Phe Pro Asp Lys Ile Gln Lys Ser Phe Leu Pro
                85                  90                  95

agc att aac tac cca caa att ttg gcg cct tat gcc cca gaa gtt aat          336
Ser Ile Asn Tyr Pro Gln Ile Leu Ala Pro Tyr Ala Pro Glu Val Asn
            100                 105                 110

tac aat gat aaa ata aaa aaa gtt tat att gaa aag att ata cca tta          384
Tyr Asn Asp Lys Ile Lys Lys Val Tyr Ile Glu Lys Ile Ile Pro Leu
        115                 120                 125

att tcg aaa aga gat ggt gat gat aag aat aac ttc agt tct gtt gcc          432
Ile Ser Lys Arg Asp Gly Asp Asp Lys Asn Asn Phe Ser Ser Val Ala
    130                 135                 140

act aga gat ata gaa tgt ttg caa agc ttg agt agg aga atc cac ttt          480
Thr Arg Asp Ile Glu Cys Leu Gln Ser Leu Ser Arg Arg Ile His Phe
145                 150                 155                 160

ggc aag ttt gtt gct gaa gcc aag ttc caa tcg gat atc ccg cta tac          528
Gly Lys Phe Val Ala Glu Ala Lys Phe Gln Ser Asp Ile Pro Leu Tyr
                165                 170                 175

aca aag ctg atc aaa agt aaa gat gtc gag ggg ata atg aag aat atc          576
Thr Lys Leu Ile Lys Ser Lys Asp Val Glu Gly Ile Met Lys Asn Ile
            180                 185                 190

acc aat tct gcc gtt gaa gaa aag att cta gaa aga tta act aag aag          624
Thr Asn Ser Ala Val Glu Glu Lys Ile Leu Glu Arg Leu Thr Lys Lys
        195                 200                 205

gct gaa gtc tat ggt gtg gac cct acc aac gag tca ggt gaa aga agg          672
Ala Glu Val Tyr Gly Val Asp Pro Thr Asn Glu Ser Gly Glu Arg Arg
    210                 215                 220

att act cca gaa tat ttg gta aaa att tat aag gaa att gtt ata cct          720
Ile Thr Pro Glu Tyr Leu Val Lys Ile Tyr Lys Glu Ile Val Ile Pro
225                 230                 235                 240

atc act aag gaa gtt gag gtg gaa tac ttg cta aga agg ttg gaa gag          768
Ile Thr Lys Glu Val Glu Val Glu Tyr Leu Leu Arg Arg Leu Glu Glu
                245                 250                 255

taa                                                                      771

<210>  71
<211>  256
<212>  PRT
<213>  Saccharomyces cerevisiae

<400>  71

Met Asp Phe Thr Lys Pro Glu Thr Val Leu Asn Leu Gln Asn Ile Arg
1               5                   10                  15

Asp Glu Leu Val Arg Met Glu Asp Ser Ile Ile Phe Lys Phe Ile Glu
            20                  25                  30

Arg Ser His Phe Ala Thr Cys Pro Ser Val Tyr Glu Ala Asn His Pro
        35                  40                  45

Gly Leu Glu Ile Pro Asn Phe Lys Gly Ser Phe Leu Asp Trp Ala Leu
    50                  55                  60

Ser Asn Leu Glu Ile Ala His Ser Arg Ile Arg Arg Phe Glu Ser Pro
65                  70                  75                  80

Asp Glu Thr Pro Phe Phe Pro Asp Lys Ile Gln Lys Ser Phe Leu Pro
                85                  90                  95

Ser Ile Asn Tyr Pro Gln Ile Leu Ala Pro Tyr Ala Pro Glu Val Asn
            100                 105                 110

Tyr Asn Asp Lys Ile Lys Lys Val Tyr Ile Glu Lys Ile Ile Pro Leu
        115                 120                 125

Ile Ser Lys Arg Asp Gly Asp Asp Lys Asn Asn Phe Ser Ser Val Ala
    130                 135                 140

Thr Arg Asp Ile Glu Cys Leu Gln Ser Leu Ser Arg Arg Ile His Phe
145                 150                 155                 160

Gly Lys Phe Val Ala Glu Ala Lys Phe Gln Ser Asp Ile Pro Leu Tyr
                165                 170                 175

Thr Lys Leu Ile Lys Ser Lys Asp Val Glu Gly Ile Met Lys Asn Ile
            180                 185                 190

Thr Asn Ser Ala Val Glu Glu Lys Ile Leu Glu Arg Leu Thr Lys Lys
        195                 200                 205

Ala Glu Val Tyr Gly Val Asp Pro Thr Asn Glu Ser Gly Glu Arg Arg
    210                 215                 220

Ile Thr Pro Glu Tyr Leu Val Lys Ile Tyr Lys Glu Ile Val Ile Pro
225                 230                 235                 240

Ile Thr Lys Glu Val Glu Val Glu Tyr Leu Leu Arg Arg Leu Glu Glu
                245                 250                 255

<210>  72
<211>  408
<212>  DNA
<213>  synthetic

<220>
<221>  promoter
<222>  (1)..(408)
<223>  TEF1 promoter

<400>  72
atagcttcaa aatgtttcta ctcctttttt actcttccag attttctcgg actccgcgca      60

tcgccgtacc acttcaaaac acccaagcac agcatactaa atttcccctc tttcttcctc     120

tagggtgtcg ttaattaccc gtactaaagg tttggaaaag aaaaaagaga ccgcctcgtt     180

tctttttctt cgtcgaaaaa ggcaataaaa atttttatca cgtttctttt tcttgaaaat     240

tttttttttt gatttttttc tctttcgatg acctcccatt gatatttaag ttaataaacg     300

gtcttcaatt tctcaagttt cagtttcatt tttcttgttc tattacaact ttttttactt     360

cttgctcatt agaaagaaag catagcaatc taatctaagt tttaatta                  408

<210>  73
<211>  243
<212>  DNA
<213>  synthetic

<220>
<221>  terminator
<222>  (1)..(243)
<223>  ADH1 terminator

<400>  73
gcgaatttct tatgatttat gatttttatt attaaataag ttataaaaaa aataagtgta      60

tacaaatttt aaagtgactc ttaggtttta aaacgaaaat tcttattctt gagtaactct     120

ttcctgtagg tcaggttgct ttctcaggta tagcatgagg tcgctcttat tgaccacacc     180

tctaccggca tgccgagcaa atgcctgcaa atcgctcccc atttcaccca attgtagata     240

tgc                                                                   243

<210>  74
<211>  1113
<212>  DNA
<213>  Saccharomyces cerevisiae

<220>
<221>  CDS
<222>  (1)..(1113)

<223>  Gene encoding feedback resistant DAHP synthase ARO4(K229L)

<400>  74
atg agt gaa tct cca atg ttc gct gcc aac ggc atg cca aag gta aat      48
Met Ser Glu Ser Pro Met Phe Ala Ala Asn Gly Met Pro Lys Val Asn
1               5                   10                  15

caa ggt gct gaa gaa gat gtc aga att tta ggt tac gac cca tta gct      96
Gln Gly Ala Glu Glu Asp Val Arg Ile Leu Gly Tyr Asp Pro Leu Ala
            20                  25                  30

tct cca gct ctc ctt caa gtg caa atc cca gcc aca cca act tct ttg      144
Ser Pro Ala Leu Leu Gln Val Gln Ile Pro Ala Thr Pro Thr Ser Leu
        35                  40                  45

gaa act gcc aag aga ggt aga aga gaa gct ata gat att att acc ggt      192
Glu Thr Ala Lys Arg Gly Arg Arg Glu Ala Ile Asp Ile Ile Thr Gly
    50                  55                  60

aaa gac gac aga gtt ctt gtc att gtc ggt cct tgt tcc atc cat gat      240
Lys Asp Asp Arg Val Leu Val Ile Val Gly Pro Cys Ser Ile His Asp
65                  70                  75                  80

cta gaa gcc gct caa gaa tac gct ttg aga tta aag aaa ttg tca gat      288
Leu Glu Ala Ala Gln Glu Tyr Ala Leu Arg Leu Lys Lys Leu Ser Asp
                85                  90                  95

gaa tta aaa ggt gat tta tcc atc att atg aga gca tac ttg gag aag      336
Glu Leu Lys Gly Asp Leu Ser Ile Ile Met Arg Ala Tyr Leu Glu Lys
            100                 105                 110

cca aga aca acc gtc ggc tgg aaa ggt cta att aat gac cct gat gtt      384
Pro Arg Thr Thr Val Gly Trp Lys Gly Leu Ile Asn Asp Pro Asp Val
        115                 120                 125

aac aac act ttc aac atc aac aag ggt ttg caa tcc gct aga caa ttg      432
Asn Asn Thr Phe Asn Ile Asn Lys Gly Leu Gln Ser Ala Arg Gln Leu
    130                 135                 140

ttt gtc aac ttg aca aat atc ggt ttg cca att ggt tct gaa atg ctt      480
Phe Val Asn Leu Thr Asn Ile Gly Leu Pro Ile Gly Ser Glu Met Leu
145                 150                 155                 160

gat acc att tct cct caa tac ttg gct gat ttg gtc tcc ttc ggt gcc      528
Asp Thr Ile Ser Pro Gln Tyr Leu Ala Asp Leu Val Ser Phe Gly Ala
                165                 170                 175

att ggt gcc aga acc acc gaa tct caa ctg cac aga gaa ttg gcc tcc      576
Ile Gly Ala Arg Thr Thr Glu Ser Gln Leu His Arg Glu Leu Ala Ser
            180                 185                 190

ggt ttg tct ttc cca gtt ggt ttc aag aac ggt acc gat ggt acc tta      624
Gly Leu Ser Phe Pro Val Gly Phe Lys Asn Gly Thr Asp Gly Thr Leu
        195                 200                 205

aat gtt gct gtg gat gct tgt caa gcc gct gct cat tct cac cat ttc      672
Asn Val Ala Val Asp Ala Cys Gln Ala Ala Ala His Ser His His Phe
    210                 215                 220

atg ggt gtt act ttg cat ggt gtt gct gct atc acc act act aag ggt      720
Met Gly Val Thr Leu His Gly Val Ala Ala Ile Thr Thr Thr Lys Gly
225                 230                 235                 240

aac gaa cac tgc ttc gtt att cta aga ggt ggt aaa aag ggt acc aac 768
Asn Glu His Cys Phe Val Ile Leu Arg Gly Gly Lys Lys Gly Thr Asn
                245                 250                 255

tac gac gct aag tcc gtt gca gaa gct aag gct caa ttg cct gcc ggt 816
Tyr Asp Ala Lys Ser Val Ala Glu Ala Lys Ala Gln Leu Pro Ala Gly
            260                 265                 270

tcc aac ggt cta atg att gac tac tct cac ggt aac tcc aat aag gat 864
Ser Asn Gly Leu Met Ile Asp Tyr Ser His Gly Asn Ser Asn Lys Asp
        275                 280                 285

ttc aga aac caa cca aag gtc aat gac gtt gtt tgt gag caa atc gct 912
Phe Arg Asn Gln Pro Lys Val Asn Asp Val Val Cys Glu Gln Ile Ala
    290                 295                 300

aac ggt gaa aac gcc att acc ggt gtc atg att gaa tca aac atc aac 960
Asn Gly Glu Asn Ala Ile Thr Gly Val Met Ile Glu Ser Asn Ile Asn
305                 310                 315                 320

gaa ggt aac caa ggc atc cca gcc gaa ggt aaa gcc ggc ttg aaa tat 1008
Glu Gly Asn Gln Gly Ile Pro Ala Glu Gly Lys Ala Gly Leu Lys Tyr
                325                 330                 335

ggt gtt tcc atc act gat gct tgt ata ggt tgg gaa act act gaa gac 1056
Gly Val Ser Ile Thr Asp Ala Cys Ile Gly Trp Glu Thr Thr Glu Asp
            340                 345                 350

gtc ttg agg aaa ttg gct gct gct gtc aga caa aga aga gaa gtt aac 1104
Val Leu Arg Lys Leu Ala Ala Ala Val Arg Gln Arg Arg Glu Val Asn
        355                 360                 365

aag aaa tag 1113
Lys Lys
    370

<210> 75
<211> 370
<212> PRT
<213> Saccharomyces cerevisiae

<400> 75

Met Ser Glu Ser Pro Met Phe Ala Ala Asn Gly Met Pro Lys Val Asn
1               5                   10                  15

Gln Gly Ala Glu Glu Asp Val Arg Ile Leu Gly Tyr Asp Pro Leu Ala
            20                  25                  30

Ser Pro Ala Leu Leu Gln Val Gln Ile Pro Ala Thr Pro Thr Ser Leu
        35                  40                  45

Glu Thr Ala Lys Arg Gly Arg Arg Glu Ala Ile Asp Ile Ile Thr Gly
    50                  55                  60

Lys Asp Asp Arg Val Leu Val Ile Val Gly Pro Cys Ser Ile His Asp
65                  70                  75                  80

Leu Glu Ala Ala Gln Glu Tyr Ala Leu Arg Leu Lys Lys Leu Ser Asp
                85                  90                  95

Glu Leu Lys Gly Asp Leu Ser Ile Ile Met Arg Ala Tyr Leu Glu Lys
            100                 105                 110

Pro Arg Thr Thr Val Gly Trp Lys Gly Leu Ile Asn Asp Pro Asp Val
        115                 120                 125

Asn Asn Thr Phe Asn Ile Asn Lys Gly Leu Gln Ser Ala Arg Gln Leu
    130                 135                 140

Phe Val Asn Leu Thr Asn Ile Gly Leu Pro Ile Gly Ser Glu Met Leu
145                 150                 155                 160

Asp Thr Ile Ser Pro Gln Tyr Leu Ala Asp Leu Val Ser Phe Gly Ala
                165                 170                 175

Ile Gly Ala Arg Thr Thr Glu Ser Gln Leu His Arg Glu Leu Ala Ser
            180                 185                 190

Gly Leu Ser Phe Pro Val Gly Phe Lys Asn Gly Thr Asp Gly Thr Leu
        195                 200                 205

Asn Val Ala Val Asp Ala Cys Gln Ala Ala Ala His Ser His His Phe
    210                 215                 220

Met Gly Val Thr Leu His Gly Val Ala Ala Ile Thr Thr Thr Lys Gly
225                 230                 235                 240

Asn Glu His Cys Phe Val Ile Leu Arg Gly Gly Lys Lys Gly Thr Asn
                245                 250                 255

Tyr Asp Ala Lys Ser Val Ala Glu Ala Lys Ala Gln Leu Pro Ala Gly
            260                 265                 270

Ser Asn Gly Leu Met Ile Asp Tyr Ser His Gly Asn Ser Asn Lys Asp
        275                 280                 285

Phe Arg Asn Gln Pro Lys Val Asn Asp Val Val Cys Glu Gln Ile Ala
    290                 295                 300

Asn Gly Glu Asn Ala Ile Thr Gly Val Met Ile Glu Ser Asn Ile Asn
305                 310                 315                 320

Glu Gly Asn Gln Gly Ile Pro Ala Glu Gly Lys Ala Gly Leu Lys Tyr
                325                 330                 335

Gly Val Ser Ile Thr Asp Ala Cys Ile Gly Trp Glu Thr Thr Glu Asp
            340                 345                 350

Val Leu Arg Lys Leu Ala Ala Ala Val Arg Gln Arg Arg Glu Val Asn
        355                 360                 365

Lys Lys
    370

<210>  76
<211>  700
<212>  DNA
<213>  synthetic

<220>
<221>  promoter
<222>  (1)..(700)
<223>  PGK1 promoter

<400>  76
acgcacagat attataacat ctgcacaata ggcatttgca agaattactc gtgagtaagg      60

aaagagtgag gaactatcgc atacctgcat ttaaagatgc cgatttgggc gcgaatcctt     120

tattttggct tcaccctcat actattatca gggccagaaa aaggaagtgt ttccctcctt     180

cttgaattga tgttaccctc ataaagcacg tggcctctta tcgagaaaga aattaccgtc     240

gctcgtgatt tgtttgcaaa aagaacaaaa ctgaaaaaac ccagacacgc tcgacttcct     300

gtcttcctat tgattgcagc ttccaatttc gtcacacaac aaggtcctag cgacggctca     360

caggttttgt aacaagcaat cgaaggttct ggaatggcgg gaaagggttt agtaccacat     420

gctatgatgc ccactgtgat ctccagagca aagttcgttc gatcgtactg ttactctctc     480

tctttcaaac agaattgtcc gaatcgtgtg acaacaacag cctgttctca cacactcttt     540

tcttctaacc aagggggtgg tttagtttag tagaacctcg tgaaacttac atttacatat     600

atataaactt gcataaattg gtcaatgcaa gaaatacata tttggtcttt tctaattcgt     660

agtttttcaa gttcttagat gctttctttt tctctttttt                           700

<210>  77
<211>  277
<212>  DNA
<213>  synthetic

<220>
<221>  terminator
<222>  (1)..(277)
<223>  CYC1 terminator

<400>  77

gataccgtcg acctcgagtc atgtaattag ttatgtcacg cttacattca cgccctcccc 60

ccacatccgc tctaaccgaa aaggaaggag ttagacaacc tgaagtctag gtccctattt 120

atttttttat agttatgtta gtattaagaa cgttatttat atttcaaatt tttctttttt 180

ttctgtacag acgcgtgtac gcatgtaaca ttatactgaa aaccttgctt gagaaggttt 240

tgggacgctc gaaggcttta atttgcggcc ggtaccc 277

<210> 78
<211> 60
<212> DNA
<213> synthetic

<220>
<221> misc_feature
<222> (1)..(60)
<223> Saccharomyces cerevisiae pdc5 upstream region

<400> 78
acttatttca cataatcaat ctcaaagaga acaacacaat acaataacaa gaagaacaaa 60

<210> 79
<211> 60
<212> DNA
<213> synthetic

<220>
<221> misc_feature
<222> (1)..(60)
<223> S. cerevisiae pdc5 downstream region

<400> 79
gctaattaac ataaaactca tgattcaacg tttgtgtatt tttttacttt tgaaggttat 60

<210> 80
<211> 60
<212> DNA
<213> synthetic

<220>
<221> misc_feature
<222> (1)..(60)
<223> S. cerevisiae aro10 upstream region

<400> 80
acaagttgac gcgacttctg taaagtttat ttacaagata acaaagaaac tcccttaagc 60

<210> 81
<211> 60
<212> DNA
<213> synthetic

<220>

<221>  misc_feature
<222>  (1)..(60)
<223>  S. cerevisiae aro10 downstream region

<400>  81
aaacttgtgg gcgcaattat aaaacactgc taccaattgt tcgttttctg ttcattaaca      60


<210>  82
<211>  760
<212>  DNA
<213>  synthetic


<220>
<221>  promoter
<222>  (1)..(760)
<223>  GPM1 promoter

<400>  82
tagtcgtgca atgtatgact ttaagatttg tgagcaggaa gaaaagggag aatcttctaa      60

cgataaaccc ttgaaaaact gggtagacta cgctatgttg agttgctacg caggctgcac     120

aattacacga gaatgctccc gcctaggatt taaggctaag ggacgtgcaa tgcagacgac     180

agatctaaat gaccgtgtcg gtgaagtgtt cgccaaactt ttcggttaac acatgcagtg     240

atgcacgcgc gatggtgcta agttacatat atatatatat atatatatat atatatatat     300

agccatagtg atgtctaagt aacctttatg gtatatttct taatgtggaa agatactagc     360

gcgcgcaccc acacacaagc ttcgtctttt cttgaagaaa agaggaagct cgctaaatgg     420

gattccactt tccgttccct gccagctgat ggaaaaaggt tagtggaacg atgaagaata     480

aaaagagaga tccactgagg tgaaatttca gctgacagcg agtttcatga tcgtgatgaa     540

caatggtaac gagttgtggc tgttgccagg gagggtggtt ctcaactttt aatgtatggc     600

caaatcgcta cttgggtttg ttatataaca aagaagaaat aatgaactga ttctcttcct     660

ccttcttgtc ctttcttaat tctgttgtaa ttaccttcct ttgtaatttt ttttgtaatt     720

attcttctta ataatccaaa caaacacaca tattacaata                           760


<210>  83
<211>  401
<212>  DNA
<213>  synthetic


<220>
<221>  terminator
<222>  (1)..(401)
<223>  FBA1 terminator

<400>  83
gttaattcaa attaattgat atagtttttt aatgagtatt gaatctgttt agaaataatg      60

gaatattatt tttatttatt tatttatatt attggtcggc tcttttcttc tgaaggtcaa     120

tgacaaaatg atatgaagga aataatgatt tctaaaattt tacaacgtaa gatattttta      180

caaaagccta gctcatcttt tgtcatgcac tattttactc acgcttgaaa ttaacggcca      240

gtccactgcg gagtcatttc aaagtcatcc taatcgatct atcgtttttg atagctcatt      300

ttggagttcg cgattgtctt ctgttattca caactgtttt aatttttatt tcattctgga      360

actcttcgag ttctttgtaa agtctttcat agtagcttac t                          401

<210>  84
<211>  671
<212>  DNA
<213>  synthetic

<220>
<221>  promoter
<222>  (1)..(671)
<223>  TDH3 promoter

<400>  84
tcgagtttat cattatcaat actgccattt caaagaatac gtaaataatt aatagtagtg       60

attttcctaa ctttatttag tcaaaaaatt agccttttaa ttctgctgta acccgtacat      120

gccaaaatag ggggcgggtt acacagaata tataacactg atggtgcttg ggtgaacagg      180

tttattcctg gcatccacta aatataatgg agcccgcttt ttaagctggc atccagaaaa      240

aaaaagaatc ccagcaccaa aatattgttt tcttcaccaa ccatcagttc ataggtccat      300

tctcttagcg caactacaga gaacagggca caaacaggca aaaaacgggc acaacctcaa      360

tggagtgatg caacctgcct ggagtaaatg atgacacaag gcaattgacc cacgcatgta      420

tctatctcat tttcttacac cttctattac cttctgctct ctctgatttg gaaaaagctg      480

aaaaaaaagg tttaaaccag ttccctgaaa ttattcccct acttgactaa taagtatata      540

aagacggtag gtattgattg taattctgta aatctatttc ttaaacttct taaattctac      600

ttttatagtt agtctttttt ttagttttaa aacaccaaga acttagtttc gaataaacac      660

acataaacaa a                                                           671

<210>  85
<211>  387
<212>  DNA
<213>  synthetic

<220>
<221>  promoter
<222>  (1)..(387)
<223>  tHXT7 pomoter

<400>  85
ctcgtaggaa caatttcggg cccctgcgtg ttcttctgag gttcatcttt tacatttgct       60

tctgctggat aattttcaga ggcaacaagg aaaaattaga tggcaaaaag tcgtctttca      120

aggaaaaatc cccaccatct ttcgagatcc cctgtaactt attggcaact gaaagaatga 180

aaaggaggaa aatacaaaat atactagaac tgaaaaaaaa aaagtataaa tagagacgat 240

atatgccaat acttcacaat gttcgaatct attcttcatt tgcagctatt gtaaaataat 300

aaaacatcaa gaacaaacaa gctcaacttg tcttttctaa gaacaaagaa taaacacaaa 360

aacaaaaagt ttttttaatt ttaatca 387

<210> 86
<211> 535
<212> DNA
<213> syntheic

<220>
<221> misc_feature
<222> (1)..(535)
<223> upstream sequence of Cas9-targeting locus XII-2

<400> 86
cggcatgcaa acatctacac aattagcaag ggcaatccat attttgtctt ttcgcgccct 60

ggaaaggcct aagtaatgtc gtaaacgcat tctatctgta cttcaactct cctctgtgca 120

ttggtttgtg caaatcacat tttacgatac tgccagatat atgcaaaaag agaaaaccaa 180

gggaccagaa caaagcaaaa ttacgatatt cttcgaattc cttcgtgctt gactaagaca 240

aagggatgga cgtagcgatt tttagcgggc caagaactgg ttccgaaaaa gcacaggtac 300

accgaaccct cagctaagga gggacagcac cgatgcggaa ggacaaactt tctttttgcc 360

tatcacagta tcttatcgag ctaactattt tcgacacaca tgaaaaagca gaaatattaa 420

cgaaaaagaa aagaaagacc atgtcatgta cgggcaatca gaatctgtaa caagcgccat 480

ttttttttct gtatcgggcc ctccttactg ctctccttcc gtgtaacgcg ttatg 535

<210> 87
<211> 559
<212> DNA
<213> synthetic

<220>
<221> misc_feature
<222> (1)..(559)
<223> downstream sequence of Cas9-targeting locus XII-2

<400> 87
ctactatcgg cgactctctc gaaatttttc ttaacgcgtc cttgtactgc gtctaacgct 60

tttgccactt ggatttctat tataggaaat agtctcactt actgggcgac gaattttcgc 120

gttttgatga agcacaggaa gaatttcttt tttttttggc ttcttctggt tccgtttttt 180

acgcgcacaa atctaaaaaa agaaataatt ataacctagt ctcgaaaatt ttcatcgatc 240

cattcgttcc tttttttcga ttttttcaga tcaaaattct tgtttctttc tttgtcttag 300

tttatattaa aagatatttt gattttactc ctgaactatt tattctttct aagaaggcca 360

gaacactaca gctgttttaa ccgactacga agttctccat tctcgaacac tagccttcat 420

ttaccaaaca ggaactagcg tatatcatta gtccttattc gaaaagagat tggtagatat 480

ttattgtagt ttgtgagaag gagaaaatac tgtcattgga ctgatagtta gaggacatta 540

acctctctta cgttcgctc 559

<210> 88
<211> 400
<212> DNA
<213> synthetic

<220>
<221> terminator
<222> (1)..(400)
<223> TDH2 teminator

<400> 88
atttaactcc ttaagttact ttaatgattt agtttttatt attaataatt catgctcatg 60

acatctcata tacacgttta taaaacttaa atagattgaa aatgtattaa agattcctca 120

gggattcgat ttttttggaa gtttttgttt ttttttcctt gagatgctgt agtatttggg 180

aacaattata caatcgaaag atatatgctt acattcgacc gttttagccg tgatcattat 240

cctatagtaa cataacctga agcataactg acactactat catcaatact tgtcacatga 300

gaactctgtg aataattagg ccactgaaat ttgatgcctg aaggaccggc atcacggatt 360

ttcgataaag cacttagtat cacactaatt ggcttttcgc 400

<210> 89
<211> 575
<212> DNA
<213> synthetic

<220>
<221> misc_feature
<222> (1)..(575)
<223> upstream sequence of Cas9-targeting locus XI-3

<400> 89
agttacttgc tctatgcgtt tgcgcatcct ctttttactt tttttttttc agtaaagcct 60

aagcataaat cgttttatac gtacgacacg ttcaactttt cttggttagt agtggcaatc 120

tctgcaatac atacagggag tcatggtcta tcatcttgtc caatcaaaga agcatcggtt 180

cagatcgagc aaactgtagg gagaaaggaa agtagaaatg cagagtgtgc tatatgtcca 240

atctcggttt tgtagtttgg atgtcattag agatctacca cccaaccggc tgctttcatg 300

tggaacagaa aagaaatcgg ggcgcttcct cttctgtatt cctttaatta acgtttttat 360

tcagccatct aaccatcata cccccatacg gtaacaaaac ctcttctaag aaaagaagtc 420

tctgctcctc cgccatctta tttttattcg ctgcgcgcgt ttattgtcgc atcgctagcc 480

agcaaaaagt tggttgcctt tttttaccta aaaaagacac atctaactga ttagttttcc 540

gttttaggat attgacgcca agcgtgcgtc tgatt 575

<210> 90
<211> 536
<212> DNA
<213> synthetic

<220>
<221> misc_feature
<222> (1)..(536)
<223> downstream sequence of Cas9-targeting locus XI-3

<400> 90
ttacgtggat tgagccagca atacagatca ttattaaact gttttgtaca tgatgttagt 60

atataatcgt aaagcttttc taatatgtat accttataca tggaactcca cagaacttgc 120

aaacatacca aaaatccttt attcttgttc actcatttta catcaaaaaa taatatttca 180

gttattaagg aaaataaaaa aatagattag agaagcattt tgaagaaata gtatattctt 240

ttattgaacc taagagcgtg atatttttac tcgaaataaa atacgaaaaa tctatacact 300

catctttccg actactattg gctcctgctc aaaaaaagag ggaaaaaaag ctccaaaatt 360

ctatcttttc ctatcgctcc tgtcctatcc ttattacgtt cattactatt ttaatactat 420

ccattctttt attttcagtc taaaaaaaac atttctcata acgggaaaag caaaaaaatg 480

tcaagcttat acatcaaaac accactgcat gcattatctg ctggtccgga ttctca 536

<210> 91
<211> 564
<212> DNA
<213> syntheic

<220>
<221> misc_feature
<222> (1)..(564)
<223> upstream sequence of Cas9-targeting locus X-3

<400> 91
cgagatcttt gtgttcggtt acccggctca gatcctaact tggtatgttt attcgtataa 60

gttactgttg tccacaggca atactctgca gaaaattaaa acggcattaa tgctaggaca 120

accagaattg ttactactgt atgtgcgata gttgataact gcaacattat gcccggtata 180

ttctcaaaaa accctattac tgcatacgaa gaaatcgcaa gagaaatctt tcggtttgga 240

aaagctcact gtgaggttcc ttggagccaa tagtaataca gcacaatcca aggaaaaatc 300

tggcctatat gcaaggaagg agagatagtc aaaagcattc tttcccctag aagttggtgc 360

atatatggca tcgttaaaac atattacccc caaaatttct tctctaaacg atgtgcttgg 420

cctttgtttt ggtttttgat gtcggtcgtt tgaggcccct tgcggaaaat cgagatcgcc 480

gaatggcacg cgagggaagg gaaataaggt ttaaaggcac tgaaacaata ggcaagaagt 540

aggcgagagc cgacatacga gact 564

<210> 92
<211> 560
<212> DNA
<213> synthetic

<220>
<221> misc_feature
<222> (1)..(560)
<223> downstream sequence of Cas9-targeting locus X-3

<400> 92
tgtgtccgcg tttctaaggc cacttttcaa tgaaacggat attgatatgc tagtaaaagg 60

acgagctcaa gagcgaaaat ataagtaaag aattcgagtg cacttgtctc catgcagcaa 120

gatttcatat gagtcttttt tatcttttta ctttttacat tacacgatat gcactttatg 180

aaaatttaac gaggttggaa gccggataat caaccaaaat caggcacgaa ggcacactcg 240

tatatgcatg ttgttgaaac tctgttacgc tgaactaaca atcacacatg tagaggtcac 300

cgggaaaagt tgcgacccca tggaaggtcg atctcttcgt ttggctttgc ttggctggcg 360

gcattgcgct tcttcgctta tacccgtctc ttgacgctcg agctcgttca ttgagatacc 420

tttattcttg cacattttct ggcttttttc gctactcggg tacatgtaat catgcacaca 480

gaaggtgctg tagggtgaaa gttcctttgt gctgtcgttt gtttttaatg ccaaactttc 540

cggtgatcaa taaccacctc 560

<210> 93
<211> 547
<212> DNA
<213> synthetic

<220>
<221> misc_feature
<222> (1)..(547)
<223> upstream sequence of Cas9-targeting locus XII-5

<400> 93
gtagtgatca ttggcttaac gaaacgggca gctggtctgc tgtaagcagc agcacgggtg 60

gccaaagcaa aggttctctt agcagtgatg acacgggagt tgcagatcaa tctggcggct 120

tgagttctca acatgtttta ttttttactt atattgctgg tagggtaaaa aaatataact 180

cctaggaata ggttgtctat atgtttttgt cttgcttcta taattgtaac aaacaaggaa 240

agggaaaata ctgggtgtaa aagccattga gtcaagttag gtcatccctt ttatacaaaa     300

tttttcaatt ttttttccaa gattcttgta cgattaatta tttttttttt gcgtcctaca     360

gcgtgatgaa aatttcgcct gctgcaagat gagcgggaac gggcgaaatg tgcacgcgca     420

caacttacga aacgcggatg agtcactgac agccaccgca gaggttctga ctcctactga     480

gctctattgg aggtggcaga accggtacgg gaggagaccg ctataaccgg tttgaattta     540

ttgtcac                                                               547

<210>  94
<211>  552
<212>  DNA
<213>  synthetic

<220>
<221>  misc_feature
<222>  (1)..(552)
<223>  downstream sequence of Cas9-targeting locus XII-5

<400>  94
caactcagaa gtttgacagc aagcaagttc atcattcgaa ctagccttat tgttttagtt      60

cagtgacagc gaactgccgt actcgatgct ttatttctca cggtagagcg gaagaacaga     120

taggggcagc gtgagaagag ttagaaagta aatttttatc acgtctgaag tattcttatt     180

cataggaaat tttgcaaggt tttttagctc aataacgggc taagttatat aaggtgttca     240

cgcgattttc ttgttatgta tacctcttct ctgaggaatg gtactactgt cctgatgtag     300

gctccttaaa ttggtgggca agaataactt atcgatattt tgtatattgg tcttggagtt     360

caccacgtaa tgcctgttta agaccatcag ttaactctag tattatttgg tcttggctac     420

tggccgtttg ctattattca agtcttttgt gccttcccgt cgggtaaggg agttatttag     480

ggatacagaa tctaacgaaa actaaatctc aatgattaac tctatttaat ccttttttga     540

aaggcaaaag ag                                                         552

<210>  95
<211>  2043
<212>  DNA
<213>  Saccharomyces cerevisiae

<220>
<221>  CDS
<222>  (1)..(2043)
<223>  Gene encoding transketolase KL1

<400>  95
atg act caa ttc act gac att gat aag cta gcc gtc tcc acc ata aga      48
Met Thr Gln Phe Thr Asp Ile Asp Lys Leu Ala Val Ser Thr Ile Arg
1               5                   10                  15

att ttg gct gtg gac acc gta tcc aag gcc aac tca ggt cac cca ggt     96
Ile Leu Ala Val Asp Thr Val Ser Lys Ala Asn Ser Gly His Pro Gly
            20                  25                  30

gct cca ttg ggt atg gca cca gct gca cac gtt cta tgg agt caa atg     144
Ala Pro Leu Gly Met Ala Pro Ala Ala His Val Leu Trp Ser Gln Met
        35                  40                  45

cgc atg aac cca acc aac cca gac tgg atc aac aga gat aga ttt gtc     192
Arg Met Asn Pro Thr Asn Pro Asp Trp Ile Asn Arg Asp Arg Phe Val
    50                  55                  60

ttg tct aac ggt cac gcg gtc gct ttg ttg tat tct atg cta cat ttg     240
Leu Ser Asn Gly His Ala Val Ala Leu Leu Tyr Ser Met Leu His Leu
65                  70                  75                  80

act ggt tac gat ctg tct att gaa gac ttg aaa cag ttc aga cag ttg     288
Thr Gly Tyr Asp Leu Ser Ile Glu Asp Leu Lys Gln Phe Arg Gln Leu
                85                  90                  95

ggt tcc aga aca cca ggt cat cct gaa ttt gag ttg cca ggt gtt gaa     336
Gly Ser Arg Thr Pro Gly His Pro Glu Phe Glu Leu Pro Gly Val Glu
            100                 105                 110

gtt act acc ggt cca tta ggt caa ggt atc tcc aac gct gtt ggt atg     384
Val Thr Thr Gly Pro Leu Gly Gln Gly Ile Ser Asn Ala Val Gly Met
        115                 120                 125

gcc atg gct caa gct aac ctg gct gcc act tac aac aag ccg ggc ttt     432
Ala Met Ala Gln Ala Asn Leu Ala Ala Thr Tyr Asn Lys Pro Gly Phe
    130                 135                 140

acc ttg tct gac aac tac acc tat gtt ttc ttg ggt gac ggt tgt ttg     480
Thr Leu Ser Asp Asn Tyr Thr Tyr Val Phe Leu Gly Asp Gly Cys Leu
145                 150                 155                 160

caa gaa ggt att tct tca gaa gct tcc tcc ttg gct ggt cat ttg aaa     528
Gln Glu Gly Ile Ser Ser Glu Ala Ser Ser Leu Ala Gly His Leu Lys
                165                 170                 175

ttg ggt aac ttg att gcc atc tac gat gac aac aag atc act atc gat     576
Leu Gly Asn Leu Ile Ala Ile Tyr Asp Asp Asn Lys Ile Thr Ile Asp
            180                 185                 190

ggt gct acc agt atc tca ttc gat gaa gat gtt gct aag aga tac gaa     624
Gly Ala Thr Ser Ile Ser Phe Asp Glu Asp Val Ala Lys Arg Tyr Glu
        195                 200                 205

gcc tac ggt tgg gaa gtt ttg tac gta gaa aat ggt aac gaa gat cta     672
Ala Tyr Gly Trp Glu Val Leu Tyr Val Glu Asn Gly Asn Glu Asp Leu
    210                 215                 220

gcc ggt att gcc aag gct att gct caa gct aag tta tcc aag gac aaa     720
Ala Gly Ile Ala Lys Ala Ile Ala Gln Ala Lys Leu Ser Lys Asp Lys
225                 230                 235                 240

cca act ttg atc aaa atg acc aca acc att ggt tac ggt tcc ttg cat     768
Pro Thr Leu Ile Lys Met Thr Thr Thr Ile Gly Tyr Gly Ser Leu His
                245                 250                 255

gcc ggc tct cac tct gtg cac ggt gcc cca ttg aaa gca gat gat gtt     816
Ala Gly Ser His Ser Val His Gly Ala Pro Leu Lys Ala Asp Asp Val
            260                 265                 270

aaa caa cta aag agc aaa ttc ggt ttc aac cca gac aag tcc ttt gtt 864
Lys Gln Leu Lys Ser Lys Phe Gly Phe Asn Pro Asp Lys Ser Phe Val
        275                 280                 285

gtt cca caa gaa gtt tac gac cac tac caa aag aca att tta aag cca 912
Val Pro Gln Glu Val Tyr Asp His Tyr Gln Lys Thr Ile Leu Lys Pro
    290                 295                 300

ggt gtc gaa gcc aac aac aag tgg aac aag ttg ttc agc gaa tac caa 960
Gly Val Glu Ala Asn Asn Lys Trp Asn Lys Leu Phe Ser Glu Tyr Gln
305                 310                 315                 320

aag aaa ttc cca gaa tta ggt gct gaa ttg gct aga aga ttg agc ggc 1008
Lys Lys Phe Pro Glu Leu Gly Ala Glu Leu Ala Arg Arg Leu Ser Gly
                325                 330                 335

caa cta ccc gca aat tgg gaa tct aag ttg cca act tac acc gcc aag 1056
Gln Leu Pro Ala Asn Trp Glu Ser Lys Leu Pro Thr Tyr Thr Ala Lys
            340                 345                 350

gac tct gcc gtg gcc act aga aaa tta tca gaa act gtt ctt gag gat 1104
Asp Ser Ala Val Ala Thr Arg Lys Leu Ser Glu Thr Val Leu Glu Asp
        355                 360                 365

gtt tac aat caa ttg cca gag ttg att ggt ggt tct gcc gat tta aca 1152
Val Tyr Asn Gln Leu Pro Glu Leu Ile Gly Gly Ser Ala Asp Leu Thr
    370                 375                 380

cct tct aac ttg acc aga tgg aag gaa gcc ctt gac ttc caa cct cct 1200
Pro Ser Asn Leu Thr Arg Trp Lys Glu Ala Leu Asp Phe Gln Pro Pro
385                 390                 395                 400

tct tcc ggt tca ggt aac tac tct ggt aga tac att agg tac ggt att 1248
Ser Ser Gly Ser Gly Asn Tyr Ser Gly Arg Tyr Ile Arg Tyr Gly Ile
                405                 410                 415

aga gaa cac gct atg ggt gcc ata atg aac ggt att tca gct ttc ggt 1296
Arg Glu His Ala Met Gly Ala Ile Met Asn Gly Ile Ser Ala Phe Gly
            420                 425                 430

gcc aac tac aaa cca tac ggt ggt act ttc ttg aac ttc gtt tct tat 1344
Ala Asn Tyr Lys Pro Tyr Gly Gly Thr Phe Leu Asn Phe Val Ser Tyr
        435                 440                 445

gct gct ggt gcc gtt aga ttg tcc gct ttg tct ggc cac cca gtt att 1392
Ala Ala Gly Ala Val Arg Leu Ser Ala Leu Ser Gly His Pro Val Ile
    450                 455                 460

tgg gtt gct aca cat gac tct atc ggt gtc ggt gaa gat ggt cca aca 1440
Trp Val Ala Thr His Asp Ser Ile Gly Val Gly Glu Asp Gly Pro Thr
465                 470                 475                 480

cat caa cct att gaa act tta gca cac ttc aga tcc cta cca aac att 1488
His Gln Pro Ile Glu Thr Leu Ala His Phe Arg Ser Leu Pro Asn Ile
                485                 490                 495

caa gtt tgg aga cca gct gat ggt aac gaa gtt tct gcc gcc tac aag 1536
Gln Val Trp Arg Pro Ala Asp Gly Asn Glu Val Ser Ala Ala Tyr Lys
            500                 505                 510

aac tct tta gaa tcc aag cat act cca agt atc att gct ttg tcc aga 1584
Asn Ser Leu Glu Ser Lys His Thr Pro Ser Ile Ile Ala Leu Ser Arg

515                 520                 525

caa aac ttg cca caa ttg gaa ggt agc tct att gaa agc gct tct aag      1632
Gln Asn Leu Pro Gln Leu Glu Gly Ser Ser Ile Glu Ser Ala Ser Lys
    530                 535                 540

ggt ggt tac gta cta caa gat gtt gct aac cca gat att att tta gtg      1680
Gly Gly Tyr Val Leu Gln Asp Val Ala Asn Pro Asp Ile Ile Leu Val
545                 550                 555                 560

gct act ggt tcc gaa gtg tct ttg agt gtt gaa gct gct aag act ttg      1728
Ala Thr Gly Ser Glu Val Ser Leu Ser Val Glu Ala Ala Lys Thr Leu
                565                 570                 575

gcc gca aag aac atc aag gct cgt gtt gtt tct cta cca gat ttc ttc      1776
Ala Ala Lys Asn Ile Lys Ala Arg Val Val Ser Leu Pro Asp Phe Phe
            580                 585                 590

act ttt gac aaa caa ccc cta gaa tac aga cta tca gtc tta cca gac      1824
Thr Phe Asp Lys Gln Pro Leu Glu Tyr Arg Leu Ser Val Leu Pro Asp
        595                 600                 605

aac gtt cca atc atg tct gtt gaa gtt ttg gct acc aca tgt tgg ggc      1872
Asn Val Pro Ile Met Ser Val Glu Val Leu Ala Thr Thr Cys Trp Gly
    610                 615                 620

aaa tac gct cat caa tcc ttc ggt att gac aga ttt ggt gcc tcc ggt      1920
Lys Tyr Ala His Gln Ser Phe Gly Ile Asp Arg Phe Gly Ala Ser Gly
625                 630                 635                 640

aag gca cca gaa gtc ttc aag ttc ttc ggt ttc acc cca gaa ggt gtt      1968
Lys Ala Pro Glu Val Phe Lys Phe Phe Gly Phe Thr Pro Glu Gly Val
                645                 650                 655

gct gaa aga gct caa aag acc att gca ttc tat aag ggt gac aag cta      2016
Ala Glu Arg Ala Gln Lys Thr Ile Ala Phe Tyr Lys Gly Asp Lys Leu
            660                 665                 670

att tct cct ttg aaa aaa gct ttc taa                                  2043
Ile Ser Pro Leu Lys Lys Ala Phe
        675                 680


<210>  96
<211>  680
<212>  PRT
<213>  Saccharomyces cerevisiae

<400>  96

Met Thr Gln Phe Thr Asp Ile Asp Lys Leu Ala Val Ser Thr Ile Arg
1               5                   10                  15


Ile Leu Ala Val Asp Thr Val Ser Lys Ala Asn Ser Gly His Pro Gly
            20                  25                  30


Ala Pro Leu Gly Met Ala Pro Ala Ala His Val Leu Trp Ser Gln Met
        35                  40                  45


Arg Met Asn Pro Thr Asn Pro Asp Trp Ile Asn Arg Asp Arg Phe Val

50                  55                  60

Leu Ser Asn Gly His Ala Val Ala Leu Leu Tyr Ser Met Leu His Leu
65                  70                  75                  80

Thr Gly Tyr Asp Leu Ser Ile Glu Asp Leu Lys Gln Phe Arg Gln Leu
                85                  90                  95

Gly Ser Arg Thr Pro Gly His Pro Glu Phe Glu Leu Pro Gly Val Glu
            100                 105                 110

Val Thr Thr Gly Pro Leu Gly Gln Gly Ile Ser Asn Ala Val Gly Met
        115                 120                 125

Ala Met Ala Gln Ala Asn Leu Ala Ala Thr Tyr Asn Lys Pro Gly Phe
    130                 135                 140

Thr Leu Ser Asp Asn Tyr Thr Tyr Val Phe Leu Gly Asp Gly Cys Leu
145                 150                 155                 160

Gln Glu Gly Ile Ser Ser Glu Ala Ser Ser Leu Ala Gly His Leu Lys
                165                 170                 175

Leu Gly Asn Leu Ile Ala Ile Tyr Asp Asp Asn Lys Ile Thr Ile Asp
            180                 185                 190

Gly Ala Thr Ser Ile Ser Phe Asp Glu Asp Val Ala Lys Arg Tyr Glu
        195                 200                 205

Ala Tyr Gly Trp Glu Val Leu Tyr Val Glu Asn Gly Asn Glu Asp Leu
    210                 215                 220

Ala Gly Ile Ala Lys Ala Ile Ala Gln Ala Lys Leu Ser Lys Asp Lys
225                 230                 235                 240

Pro Thr Leu Ile Lys Met Thr Thr Thr Ile Gly Tyr Gly Ser Leu His
                245                 250                 255

Ala Gly Ser His Ser Val His Gly Ala Pro Leu Lys Ala Asp Asp Val
            260                 265                 270

Lys Gln Leu Lys Ser Lys Phe Gly Phe Asn Pro Asp Lys Ser Phe Val
        275                 280                 285

Val Pro Gln Glu Val Tyr Asp His Tyr Gln Lys Thr Ile Leu Lys Pro
    290                 295                 300

Gly Val Glu Ala Asn Asn Lys Trp Asn Lys Leu Phe Ser Glu Tyr Gln
305                 310                 315                 320

Lys Lys Phe Pro Glu Leu Gly Ala Glu Leu Ala Arg Arg Leu Ser Gly
                325                 330                 335

Gln Leu Pro Ala Asn Trp Glu Ser Lys Leu Pro Thr Tyr Thr Ala Lys
            340                 345                 350

Asp Ser Ala Val Ala Thr Arg Lys Leu Ser Glu Thr Val Leu Glu Asp
        355                 360                 365

Val Tyr Asn Gln Leu Pro Glu Leu Ile Gly Gly Ser Ala Asp Leu Thr
    370                 375                 380

Pro Ser Asn Leu Thr Arg Trp Lys Glu Ala Leu Asp Phe Gln Pro Pro
385                 390                 395                 400

Ser Ser Gly Ser Gly Asn Tyr Ser Gly Arg Tyr Ile Arg Tyr Gly Ile
                405                 410                 415

Arg Glu His Ala Met Gly Ala Ile Met Asn Gly Ile Ser Ala Phe Gly
            420                 425                 430

Ala Asn Tyr Lys Pro Tyr Gly Gly Thr Phe Leu Asn Phe Val Ser Tyr
        435                 440                 445

Ala Ala Gly Ala Val Arg Leu Ser Ala Leu Ser Gly His Pro Val Ile
    450                 455                 460

Trp Val Ala Thr His Asp Ser Ile Gly Val Gly Glu Asp Gly Pro Thr
465                 470                 475                 480

His Gln Pro Ile Glu Thr Leu Ala His Phe Arg Ser Leu Pro Asn Ile
                485                 490                 495

Gln Val Trp Arg Pro Ala Asp Gly Asn Glu Val Ser Ala Ala Tyr Lys
            500                 505                 510

Asn Ser Leu Glu Ser Lys His Thr Pro Ser Ile Ile Ala Leu Ser Arg
        515                 520                 525

Gln Asn Leu Pro Gln Leu Glu Gly Ser Ser Ile Glu Ser Ala Ser Lys
    530                 535                 540

Gly Gly Tyr Val Leu Gln Asp Val Ala Asn Pro Asp Ile Ile Leu Val
545                 550                 555                 560

Ala Thr Gly Ser Glu Val Ser Leu Ser Val Glu Ala Ala Lys Thr Leu
                565                 570                 575

Ala Ala Lys Asn Ile Lys Ala Arg Val Val Ser Leu Pro Asp Phe Phe
            580                 585                 590

Thr Phe Asp Lys Gln Pro Leu Glu Tyr Arg Leu Ser Val Leu Pro Asp
        595                 600                 605

Asn Val Pro Ile Met Ser Val Glu Val Leu Ala Thr Thr Cys Trp Gly
    610                 615                 620

Lys Tyr Ala His Gln Ser Phe Gly Ile Asp Arg Phe Gly Ala Ser Gly
625                 630                 635                 640

Lys Ala Pro Glu Val Phe Lys Phe Phe Gly Phe Thr Pro Glu Gly Val
                645                 650                 655

Ala Glu Arg Ala Gln Lys Thr Ile Ala Phe Tyr Lys Gly Asp Lys Leu
            660                 665                 670

Ile Ser Pro Leu Lys Lys Ala Phe
        675                 680

<210>  97
<211>  60
<212>  DNA
<213>  synthetic

<220>
<221>  misc_feature
<222>  (1)..(60)
<223>  S. cerevisiae gpp1 upstream region

<400>  97
aaacgtttca atgttttaaa atatatcaga acaacaaaag caaatataca aaccatcgca      60

<210>  98
<211>  60
<212>  DNA
<213>  synthetic

<220>
<221>  misc_feature
<222>  (1)..(60)
<223>  S. cerevisiae gpp1 downstream region

<400>  98
ttttctttta tttttttgat aaaactacta cgctaaaaat aaaataaaaa tgtatgattt      60

<210>  99
<211>  1959
<212>  DNA
<213>  Salmonella enterica

<220>
<221>  CDS
<222>  (1)..(1959)
<223>  Gene encoding acetyl-CoA synthease fom Salmonella enterica codon
       optimized for yeast

<400>  99
atg tca caa aca cac aaa cat gct att cct gcg aat atc gct gac agg        48
Met Ser Gln Thr His Lys His Ala Ile Pro Ala Asn Ile Ala Asp Arg
1               5                   10                  15

tgc tta atc aac cct gaa caa tac gaa acg aag tac aag cag tct atc        96
Cys Leu Ile Asn Pro Glu Gln Tyr Glu Thr Lys Tyr Lys Gln Ser Ile
            20                  25                  30

aac gat cct gat act ttc tgg ggc gag caa ggt aag ata ctc gat tgg       144
Asn Asp Pro Asp Thr Phe Trp Gly Glu Gln Gly Lys Ile Leu Asp Trp
        35                  40                  45

att act cca tat caa aag gtc aaa aac aca tcc ttt gct cct gga aat       192
Ile Thr Pro Tyr Gln Lys Val Lys Asn Thr Ser Phe Ala Pro Gly Asn
    50                  55                  60

gtg tca atc aag tgg tac gag gac ggc act cta aac cta gct gct aat       240
Val Ser Ile Lys Trp Tyr Glu Asp Gly Thr Leu Asn Leu Ala Ala Asn
65                  70                  75                  80

tgc ttg gat cga cac ctc cag gaa aat ggt gac aga acg gca atc att       288
Cys Leu Asp Arg His Leu Gln Glu Asn Gly Asp Arg Thr Ala Ile Ile
                85                  90                  95

tgg gaa ggt gat gat act tct caa tct aag cac atc tcc tac aga gag       336
Trp Glu Gly Asp Asp Thr Ser Gln Ser Lys His Ile Ser Tyr Arg Glu
            100                 105                 110

tta cac aga gat gtt tgc aga ttc gcg aat act tta ctg gac ctg ggt       384
Leu His Arg Asp Val Cys Arg Phe Ala Asn Thr Leu Leu Asp Leu Gly
        115                 120                 125

atc aaa aag ggc gat gtt gtg gca atc tac atg cct atg gtc cca gag       432
Ile Lys Lys Gly Asp Val Val Ala Ile Tyr Met Pro Met Val Pro Glu
    130                 135                 140

gca gct gtg gca atg ttg gcc tgt gcc aga ata gga gca gtc cat agc       480
Ala Ala Val Ala Met Leu Ala Cys Ala Arg Ile Gly Ala Val His Ser
145                 150                 155                 160

gtt atc ttt ggc gga ttc tcc cct gaa gcc gtt gct ggg aga atc att       528
Val Ile Phe Gly Gly Phe Ser Pro Glu Ala Val Ala Gly Arg Ile Ile
                165                 170                 175

gac tca tca agt aga tta gtt atc act gcc gac gaa ggt gtt aga gca       576
Asp Ser Ser Ser Arg Leu Val Ile Thr Ala Asp Glu Gly Val Arg Ala
            180                 185                 190

ggt aga tcc atc cca ttg aag aaa aac gtt gat gac gcg ttg aaa aac       624
Gly Arg Ser Ile Pro Leu Lys Lys Asn Val Asp Asp Ala Leu Lys Asn

195                 200                 205

cca aac gtt acg agt gtg gag cat gta att gta cta aag cgt acc ggc         672
Pro Asn Val Thr Ser Val Glu His Val Ile Val Leu Lys Arg Thr Gly
    210                 215                 220

tct gat ata gac tgg cag gaa ggt agg gat ttg tgg tgg aga gat ctt         720
Ser Asp Ile Asp Trp Gln Glu Gly Arg Asp Leu Trp Trp Arg Asp Leu
225                 230                 235                 240

att gag aaa gca agt cca gaa cac caa cca gaa gca atg aat gcg gaa         768
Ile Glu Lys Ala Ser Pro Glu His Gln Pro Glu Ala Met Asn Ala Glu
                245                 250                 255

gat cca ttg ttc atc ttg tat aca tct ggg tca act ggc aaa cca aaa         816
Asp Pro Leu Phe Ile Leu Tyr Thr Ser Gly Ser Thr Gly Lys Pro Lys
            260                 265                 270

ggt gtt ttg cat aca aca ggt ggt tat ctc gta tac gcc gca aca acc         864
Gly Val Leu His Thr Thr Gly Gly Tyr Leu Val Tyr Ala Ala Thr Thr
        275                 280                 285

ttt aag tac gtt ttt gat tac cat cca ggt gat atc tac tgg tgt acc         912
Phe Lys Tyr Val Phe Asp Tyr His Pro Gly Asp Ile Tyr Trp Cys Thr
    290                 295                 300

gct gat gtc ggt tgg gtt act ggt cat agt tac ctg ctt tac ggt cca         960
Ala Asp Val Gly Trp Val Thr Gly His Ser Tyr Leu Leu Tyr Gly Pro
305                 310                 315                 320

ctg gca tgc ggc gca acc act ttg atg ttt gaa gga gta cca aac tgg        1008
Leu Ala Cys Gly Ala Thr Thr Leu Met Phe Glu Gly Val Pro Asn Trp
                325                 330                 335

cca acc cca gcc agg atg tgt caa gtg gtc gat aaa cac caa gtg aac        1056
Pro Thr Pro Ala Arg Met Cys Gln Val Val Asp Lys His Gln Val Asn
            340                 345                 350

ata ttg tac aca gcc cca acc gcc att aga gcg cta atg gcc gaa gga        1104
Ile Leu Tyr Thr Ala Pro Thr Ala Ile Arg Ala Leu Met Ala Glu Gly
        355                 360                 365

gat aag gcg att gag gga aca gat aga agt agc cta cgt atc tta gga        1152
Asp Lys Ala Ile Glu Gly Thr Asp Arg Ser Ser Leu Arg Ile Leu Gly
    370                 375                 380

tcc gtt ggc gag cca atc aat cca gaa gct tgg gaa tgg tat tgg aaa        1200
Ser Val Gly Glu Pro Ile Asn Pro Glu Ala Trp Glu Trp Tyr Trp Lys
385                 390                 395                 400

aag att ggt aag gaa aag tgt cca gta gtg gat aca tgg tgg caa act        1248
Lys Ile Gly Lys Glu Lys Cys Pro Val Val Asp Thr Trp Trp Gln Thr
                405                 410                 415

gaa aca ggt gga ttc atg att aca cct ctt cca ggt gca ata gaa ttg        1296
Glu Thr Gly Gly Phe Met Ile Thr Pro Leu Pro Gly Ala Ile Glu Leu
            420                 425                 430

aag gct ggg tct gct act agg cct ttc ttc ggc gtc caa cct gct tta        1344
Lys Ala Gly Ser Ala Thr Arg Pro Phe Phe Gly Val Gln Pro Ala Leu
        435                 440                 445

gta gac aac gaa ggg cat cca caa gag ggg gca aca gaa ggc aat cta        1392

Val Asp Asn Glu Gly His Pro Gln Glu Gly Ala Thr Glu Gly Asn Leu
    450                 455                 460

gtg ata act gat tcc tgg cct ggt cag gct aga aca ttg ttt ggt gat      1440
Val Ile Thr Asp Ser Trp Pro Gly Gln Ala Arg Thr Leu Phe Gly Asp
465                 470                 475                 480

cac gaa aga ttc gaa caa acc tat ttc tca act ttc aaa aac atg tat      1488
His Glu Arg Phe Glu Gln Thr Tyr Phe Ser Thr Phe Lys Asn Met Tyr
                485                 490                 495

ttc agc ggt gac ggt gcg aga aga gac gaa gat ggg tac tac tgg att      1536
Phe Ser Gly Asp Gly Ala Arg Arg Asp Glu Asp Gly Tyr Tyr Trp Ile
            500                 505                 510

acc ggc aga gta gat gac gtc ctt aac gta tct gga cat cgt ctg ggt      1584
Thr Gly Arg Val Asp Asp Val Leu Asn Val Ser Gly His Arg Leu Gly
        515                 520                 525

aca gct gag att gag tca gct tta gtt gct cat cct aag att gct gaa      1632
Thr Ala Glu Ile Glu Ser Ala Leu Val Ala His Pro Lys Ile Ala Glu
    530                 535                 540

gct gca gtc gtt ggc atc cca cac gct atc aag ggt caa gcc ata tac      1680
Ala Ala Val Val Gly Ile Pro His Ala Ile Lys Gly Gln Ala Ile Tyr
545                 550                 555                 560

gca tat gtt aca ctc aac cat ggt gag gaa cca tct cca gag cta tac      1728
Ala Tyr Val Thr Leu Asn His Gly Glu Glu Pro Ser Pro Glu Leu Tyr
                565                 570                 575

gca gag gtc aga aat tgg gtt cga aag gaa ata ggg cct tta gcc aca      1776
Ala Glu Val Arg Asn Trp Val Arg Lys Glu Ile Gly Pro Leu Ala Thr
            580                 585                 590

cca gat gtt ttg cat tgg aca gat tca ttg cct aag aca aga tct gga      1824
Pro Asp Val Leu His Trp Thr Asp Ser Leu Pro Lys Thr Arg Ser Gly
        595                 600                 605

aag att atg aga cgt ata ctt aga aag atc gcc gcc gga gat acg tct      1872
Lys Ile Met Arg Arg Ile Leu Arg Lys Ile Ala Ala Gly Asp Thr Ser
    610                 615                 620

aac tta ggt gat act tct act ctt gcc gat cca ggc gtg gtc gaa aaa      1920
Asn Leu Gly Asp Thr Ser Thr Leu Ala Asp Pro Gly Val Val Glu Lys
625                 630                 635                 640

cct tta gag gaa aaa caa gct att gct atg cca tca tga                  1959
Pro Leu Glu Glu Lys Gln Ala Ile Ala Met Pro Ser
                645                 650


<210>  100
<211>  652
<212>  PRT
<213>  Salmonella enterica

<400>  100

Met Ser Gln Thr His Lys His Ala Ile Pro Ala Asn Ile Ala Asp Arg
1               5                   10                  15

Cys Leu Ile Asn Pro Glu Gln Tyr Glu Thr Lys Tyr Lys Gln Ser Ile
            20                  25                  30

Asn Asp Pro Asp Thr Phe Trp Gly Glu Gln Gly Lys Ile Leu Asp Trp
        35                  40                  45

Ile Thr Pro Tyr Gln Lys Val Lys Asn Thr Ser Phe Ala Pro Gly Asn
    50                  55                  60

Val Ser Ile Lys Trp Tyr Glu Asp Gly Thr Leu Asn Leu Ala Ala Asn
65                  70                  75                  80

Cys Leu Asp Arg His Leu Gln Glu Asn Gly Asp Arg Thr Ala Ile Ile
                85                  90                  95

Trp Glu Gly Asp Asp Thr Ser Gln Ser Lys His Ile Ser Tyr Arg Glu
            100                 105                 110

Leu His Arg Asp Val Cys Arg Phe Ala Asn Thr Leu Leu Asp Leu Gly
        115                 120                 125

Ile Lys Lys Gly Asp Val Val Ala Ile Tyr Met Pro Met Val Pro Glu
    130                 135                 140

Ala Ala Val Ala Met Leu Ala Cys Ala Arg Ile Gly Ala Val His Ser
145                 150                 155                 160

Val Ile Phe Gly Gly Phe Ser Pro Glu Ala Val Ala Gly Arg Ile Ile
                165                 170                 175

Asp Ser Ser Ser Arg Leu Val Ile Thr Ala Asp Glu Gly Val Arg Ala
            180                 185                 190

Gly Arg Ser Ile Pro Leu Lys Lys Asn Val Asp Asp Ala Leu Lys Asn
        195                 200                 205

Pro Asn Val Thr Ser Val Glu His Val Ile Val Leu Lys Arg Thr Gly
    210                 215                 220

Ser Asp Ile Asp Trp Gln Glu Gly Arg Asp Leu Trp Trp Arg Asp Leu
225                 230                 235                 240

Ile Glu Lys Ala Ser Pro Glu His Gln Pro Glu Ala Met Asn Ala Glu
                245                 250                 255

Asp Pro Leu Phe Ile Leu Tyr Thr Ser Gly Ser Thr Gly Lys Pro Lys
            260                 265                 270

Gly Val Leu His Thr Thr Gly Gly Tyr Leu Val Tyr Ala Ala Thr Thr
        275                 280                 285

Phe Lys Tyr Val Phe Asp Tyr His Pro Gly Asp Ile Tyr Trp Cys Thr
    290                 295                 300

Ala Asp Val Gly Trp Val Thr Gly His Ser Tyr Leu Leu Tyr Gly Pro
305                 310                 315                 320

Leu Ala Cys Gly Ala Thr Thr Leu Met Phe Glu Gly Val Pro Asn Trp
                325                 330                 335

Pro Thr Pro Ala Arg Met Cys Gln Val Val Asp Lys His Gln Val Asn
            340                 345                 350

Ile Leu Tyr Thr Ala Pro Thr Ala Ile Arg Ala Leu Met Ala Glu Gly
        355                 360                 365

Asp Lys Ala Ile Glu Gly Thr Asp Arg Ser Ser Leu Arg Ile Leu Gly
    370                 375                 380

Ser Val Gly Glu Pro Ile Asn Pro Glu Ala Trp Glu Trp Tyr Trp Lys
385                 390                 395                 400

Lys Ile Gly Lys Glu Lys Cys Pro Val Val Asp Thr Trp Trp Gln Thr
                405                 410                 415

Glu Thr Gly Gly Phe Met Ile Thr Pro Leu Pro Gly Ala Ile Glu Leu
            420                 425                 430

Lys Ala Gly Ser Ala Thr Arg Pro Phe Phe Gly Val Gln Pro Ala Leu
        435                 440                 445

Val Asp Asn Glu Gly His Pro Gln Glu Gly Ala Thr Glu Gly Asn Leu
    450                 455                 460

Val Ile Thr Asp Ser Trp Pro Gly Gln Ala Arg Thr Leu Phe Gly Asp
465                 470                 475                 480

His Glu Arg Phe Glu Gln Thr Tyr Phe Ser Thr Phe Lys Asn Met Tyr
                485                 490                 495

Phe Ser Gly Asp Gly Ala Arg Arg Asp Glu Asp Gly Tyr Tyr Trp Ile
            500                 505                 510

Thr Gly Arg Val Asp Asp Val Leu Asn Val Ser Gly His Arg Leu Gly
        515                 520                 525

Thr Ala Glu Ile Glu Ser Ala Leu Val Ala His Pro Lys Ile Ala Glu
    530                 535                 540

Ala Ala Val Val Gly Ile Pro His Ala Ile Lys Gly Gln Ala Ile Tyr
545                 550                 555                 560

Ala Tyr Val Thr Leu Asn His Gly Glu Glu Pro Ser Pro Glu Leu Tyr
                565                 570                 575

Ala Glu Val Arg Asn Trp Val Arg Lys Glu Ile Gly Pro Leu Ala Thr
            580                 585                 590

Pro Asp Val Leu His Trp Thr Asp Ser Leu Pro Lys Thr Arg Ser Gly
        595                 600                 605

Lys Ile Met Arg Arg Ile Leu Arg Lys Ile Ala Ala Gly Asp Thr Ser
    610                 615                 620

Asn Leu Gly Asp Thr Ser Thr Leu Ala Asp Pro Gly Val Val Glu Lys
625                 630                 635                 640

Pro Leu Glu Glu Lys Gln Ala Ile Ala Met Pro Ser
                645                 650

<210>  101
<211>  536
<212>  DNA
<213>  synthetic

<220>
<221>  misc_feature
<222>  (1)..(536)
<223>  upstream sequence of Cas9-targeting locus XI-2

<400>  101
taactcttcg tatgaggatt ttcgatggag caggatgagg agaaatagta ccacatgtat      60

atatccatta caaaaaggtt tatatacaat tacaatagac ccttgttggg gtttctgaaa      120

aaagaagtag tcgatgccat cggcaataat acggaattac gagaaacaca atcccgatcc      180

ttttttgggt aattacttca ccgattctac cgatttatca tgccaaaaaa aattcaccgt      240

gggttctaga agtgcccttt gaggattgta gccactctaa cccacacggc ctccttacta      300

gctgactaag gtgacaaaac cgcaaggact ggaaagtcgc cactcatctg aaaattctca      360

agtttttcac tactgagttt atgctttcga atttttttgt tcggtaatag cacggcggtt      420

cgattcaatt ccgccgctcc gagcgatgct ccgcaaaact cagtaataag ctttctgatg      480

gttcacccct tttttagcac gcggggtgta actcaacaga aaaatgtgcc atagaa      536

<210>  102
<211>  483
<212>  DNA
<213>  synhetic

<220>
<221>  misc_feature
<222>  (1)..(483)
<223>  downstream sequence of Cas9-targeting locus XI-2

<400>  102
ccacaagtaa agctcgttga ccagttgatc agttgagggg ggtacacacg actagcgctt      60

tcagatatta aaaagtttag atgtaggttt tagcggtaac agttatataa atcgtgtttc     120

ttctcttgat gaaacaaaaa aatgctagaa aaactttgtc gtttcttact tttggtgcgc     180

tttgcagttt tcgtggctag acttagaatc atttctcctc agatttcttg attaaagttt     240

ggtgcgaagc cctactctaa cattggtgtt cttcttttca ttcacgcaag ttaagtccag     300

gaaggtgagc aaatgctcat ccttctgttc atgcgtgacg gctgaattat ccttatctgg     360

cgtacccgtg cagccgtttc cgtgcctcgg ttcctccgag atatccttag ggaccgccag     420

ggaccatgat tgcgtcaact gttgtcaccg ctccagagga tcctctgtaa ccttttcaac     480

cat                                                                   483

<210>  103
<211>  442
<212>  DNA
<213>  synthetic

<220>
<221>  promoter
<222>  (1)..(442)
<223>  GAL1 promoter

<400>  103
cggattagaa gccgccgagc gggtgacagc cctccgaagg aagactctcc tccgtgcgtc      60

ctcgtcttca ccggtcgcgt tcctgaaacg cagatgtgcc tcgcgccgca ctgctccgaa     120

caataaagat tctacaatac tagcttttat ggttatgaag aggaaaaatt ggcagtaacc     180

tggccccaca aaccttcaaa tgaacgaatc aaattaacaa ccataggatg ataatgcgat     240

tagtttttta gccttatttc tggggtaatt aatcagcgaa gcgatgattt ttgatctatt     300

aacagatata taaatgcaaa aactgcataa ccactttaac taatactttc aacattttcg     360

gtttgtatta cttcttattc aaatgtaata aaagtatcaa caaaaaattg ttaatatacc     420

tctatacttt aacgtcaagg ag                                              442

<210>  104

<211>  743
<212>  DNA
<213>  synthetic

<220>
<221>  promoter
<222>  (1)..(743)
<223>  GAL2 promoter

<400>  104
cagaaggcac atctattaca tttactgagc ataacgggct gtactaatcc aaggaggttt      60

acggaccaga ggaactttcc agattcagat cacagcaata taggactgga aaacatcagg     120

tagccgcact caacttgtaa ctggcaacta ctttgcatta aactccaatt aaatgcggta     180

gaatcttttc agaaaaggta ttcaacgtca attcggaaag cttccttccg gaatggctta     240

agtaggttgc aatttctttt tctattagta gctaaaaatg ggtcacgtga tctatattcg     300

aaaggggcgg ttgcctcagg aaggcaccgg cggtctttcg tccgtgcgga gatatctgcg     360

ccgttcaggg gtccatgtgc cttggacgat attaaggcag aaggcagtat cggggcggat     420

cactccgaac cgagattagt taagcccttc ccatctcaag atggggagca aatggcatta     480

tactcctgct agaaagttaa ctgtgcacat attcttaaat tatacaatgt tctggagagc     540

tattgtttaa aaaacaaaca tttcgcaggc taaaatgtgg agataggatt agttttgtag     600

acatatataa acaatcagta attggattga aaatttggtg ttgtgaattg ctcttcatta     660

tgcaccttat tcaattatca tcaagaatag caatagttaa gtaaacacaa gattaacata     720

ataaaaaaaa taattctttc ata                                             743

<210>  105
<211>  527
<212>  DNA
<213>  synthetic

<220>
<221>  misc_feature
<222>  (1)..(527)
<223>  upstream sequence of Cas9-targeting locus X-2

<400>  105
cgtctatgag gagactgtta gttggatatc agtaatgaga cgaaaaagct cgaaatgaat      60

ggatatattc tttttgctac tggcaactgt tgaatattta atgttaaaac aaactaactg     120

aggtatattc gtatctgtat gtacacatat actatataca ggaaaagata agcaagagag     180

aggatatcaa ctacgagagc gatcgattat atatcaaaag ctgtccgctt tgccacccat     240

aatcggcgct tagtttcgga gttcaatcat aattctacca ccttacactc aacttactct     300

ttaactccta tagtataata tcgccactga ccccatatta aaaaattttt ttgctcgatc     360

ttctatcctc tttaggttaa ttgtcgctgt tattgtctag attttttctc ggagatggcg     420

catctatttg ccgtcaaaag atcctctcat accatattaa gtaaattgcc tccatttctt 480

tttcctcggg cagagaaact cgcaggcaac ttgctctcga agtggtc 527

<210> 106
<211> 490
<212> DNA
<213> synthetic

<220>
<221> misc_feature
<222> (1)..(490)
<223> downstream sequence of Cas9-targeting locus X-2

<400> 106
cctgcataat cggcctcaca gagggatccc gttacccatc tatgctgaag atttatcata 60

ctattcctcc gctcgtttct tttttcagtg aggtgtgtcg tgaaagaaaa cccacaatta 120

aactttcaac aaccgggcga ctaggaagag agtagtgggc gcggatgacg aaggctaagg 180

tcacttcttc gtttccttta ttggggtttc cgtgtagcct tcccctgaat agtgtgggac 240

gttttatgag aagccgtaag aaataggcaa attgagttat gacaagtaga catgatgccg 300

cagccttgcc tgactttacg tctccttcat gaataagttt ttctatcgag ttcttttcct 360

tttttcgcct taattagctc aattaagcct gtcctcacta cttttctttt tcttatcggc 420

tttgtgccac acctaacctt cgaatgctgt tttattccgt tcttacatgg gatggtaatg 480

ccttggcgag 490

<210> 107
<211> 1635
<212> DNA
<213> Scutellaria baicalensis

<220>
<221> CDS
<222> (1)..(1635)
<223> Gene encoding 4-coumaroyl-CoA-like ligase

<300>
<308> GenBank: KT963458.1
<309> 2016-05-11
<313> (1)..(1635)

<400> 107
atg gag aaa tcg ggc tat ggc cgt gat gga ata tac aga tca ttg agg 48
Met Glu Lys Ser Gly Tyr Gly Arg Asp Gly Ile Tyr Arg Ser Leu Arg
1               5                   10                  15

cca ccg ttg tat ctt ccc aaa aat ccc aat ctt tca ctt gtt tcg ttt 96
Pro Pro Leu Tyr Leu Pro Lys Asn Pro Asn Leu Ser Leu Val Ser Phe
            20                  25                  30

ctc ttc aga aac gtt tcg tct ttt gct gat aac cga gct ctt gtg gat 144

Leu Phe Arg Asn Val Ser Ser Phe Ala Asp Asn Arg Ala Leu Val Asp
        35                  40                  45

gca cat act ggg gaa acc tta acc ttc gcg cag ttt aaa tcc acg gtt 192
Ala His Thr Gly Glu Thr Leu Thr Phe Ala Gln Phe Lys Ser Thr Val
    50                  55                  60

tca aaa gtc gcc cat ggt tta ctt caa ctg ggc atc aag aaa aac gac 240
Ser Lys Val Ala His Gly Leu Leu Gln Leu Gly Ile Lys Lys Asn Asp
65                  70                  75                  80

gtc gtg ctc atc ttc gct ccc aat tcg atc gag ttc cca ata tct ttc 288
Val Val Leu Ile Phe Ala Pro Asn Ser Ile Glu Phe Pro Ile Ser Phe
                85                  90                  95

ttt ggg gtt atc gcc act gga gcc gtc gcc acc acg gtc aac ccc atg 336
Phe Gly Val Ile Ala Thr Gly Ala Val Ala Thr Thr Val Asn Pro Met
            100                 105                 110

tat act gtc tcg gag cta tcc aaa cag gtt caa gat tct act gct aag 384
Tyr Thr Val Ser Glu Leu Ser Lys Gln Val Gln Asp Ser Thr Ala Lys
        115                 120                 125

ctt ata att act gtt gaa ggt ctg ttg cct aaa gtt aag gat ttt ggt 432
Leu Ile Ile Thr Val Glu Gly Leu Leu Pro Lys Val Lys Asp Phe Gly
    130                 135                 140

ttg cct gtt att ctt ctg gga aat gaa aag aaa tca gtt ttg ccc att 480
Leu Pro Val Ile Leu Leu Gly Asn Glu Lys Lys Ser Val Leu Pro Ile
145                 150                 155                 160

ggg aga atc cct tca atc act tca ttt tct gag ctt gtg aac aat gaa 528
Gly Arg Ile Pro Ser Ile Thr Ser Phe Ser Glu Leu Val Asn Asn Glu
                165                 170                 175

ggg ttg gat tta gat agt aat gtt tat gga atc aag caa gat gat aca 576
Gly Leu Asp Leu Asp Ser Asn Val Tyr Gly Ile Lys Gln Asp Asp Thr
            180                 185                 190

gct gct ttg ttg tac tca tcc ggc acc acg ggt atg agc aag ggc gtt 624
Ala Ala Leu Leu Tyr Ser Ser Gly Thr Thr Gly Met Ser Lys Gly Val
        195                 200                 205

ata ctt act cat agg aac ttc ata gct tcc tcg ttg atg atc act gct 672
Ile Leu Thr His Arg Asn Phe Ile Ala Ser Ser Leu Met Ile Thr Ala
    210                 215                 220

gat caa gaa tcg gct ggt gaa atg cac aat gtg ttt ctc tgt gtt ctg 720
Asp Gln Glu Ser Ala Gly Glu Met His Asn Val Phe Leu Cys Val Leu
225                 230                 235                 240

cct atg ttt cat gtt ttt ggt ctt gca gtg atc atg tta tca cag ctg 768
Pro Met Phe His Val Phe Gly Leu Ala Val Ile Met Leu Ser Gln Leu
                245                 250                 255

caa cgt gga aac acc att gtt tcg atg tcg aag ttt gat ttt gaa atg 816
Gln Arg Gly Asn Thr Ile Val Ser Met Ser Lys Phe Asp Phe Glu Met
            260                 265                 270

att ttg aag acg gtg gag aag tat ggg atc acg cat atg tgg gtt gtg 864
Ile Leu Lys Thr Val Glu Lys Tyr Gly Ile Thr His Met Trp Val Val
        275                 280                 285

ccg cca att ata tta gct ttg gcc aaa aac aat gtg gtc aag aag tat 912
Pro Pro Ile Ile Leu Ala Leu Ala Lys Asn Asn Val Val Lys Lys Tyr
    290                 295                 300

aat ttg tca tcc ttg cag cag att gga tcg ggg gcg gct cca ctt ggg 960
Asn Leu Ser Ser Leu Gln Gln Ile Gly Ser Gly Ala Ala Pro Leu Gly
305                 310                 315                 320

aga gaa ttg atg cag gaa tgt gca aag aat ttc cct caa gct gtt gtc 1008
Arg Glu Leu Met Gln Glu Cys Ala Lys Asn Phe Pro Gln Ala Val Val
                325                 330                 335

gtt caa ggt tat gga atg aca gaa acc tgt gga att gtc tcc gtc gag 1056
Val Gln Gly Tyr Gly Met Thr Glu Thr Cys Gly Ile Val Ser Val Glu
            340                 345                 350

aat gtg tat gca ggg cca cga cat tct ggt tct gct ggt aca ctt gtt 1104
Asn Val Tyr Ala Gly Pro Arg His Ser Gly Ser Ala Gly Thr Leu Val
        355                 360                 365

cct ggt gtg gag tgt caa ata gtt agt gtt gat aaa ttg aag cct ctt 1152
Pro Gly Val Glu Cys Gln Ile Val Ser Val Asp Lys Leu Lys Pro Leu
    370                 375                 380

cct cct gga cag ttg ggt gaa ata tgc gtt cga gga ccc aat atg atg 1200
Pro Pro Gly Gln Leu Gly Glu Ile Cys Val Arg Gly Pro Asn Met Met
385                 390                 395                 400

caa ggt tat ttt aag aat cca cag gct acg aag ctt aca ata gat aaa 1248
Gln Gly Tyr Phe Lys Asn Pro Gln Ala Thr Lys Leu Thr Ile Asp Lys
                405                 410                 415

cag ggt tgg gtc cat act gga gat ctt gga tac ttc gat gat gag ggt 1296
Gln Gly Trp Val His Thr Gly Asp Leu Gly Tyr Phe Asp Asp Glu Gly
            420                 425                 430

cag ctc ttt gtt gtt gac cgt att aaa gag ctc att aag tac aaa ggt 1344
Gln Leu Phe Val Val Asp Arg Ile Lys Glu Leu Ile Lys Tyr Lys Gly
        435                 440                 445

ttt cag gtt gct cca gcg gag ctt gaa ggg cta ctc gtc tct cat ccc 1392
Phe Gln Val Ala Pro Ala Glu Leu Glu Gly Leu Leu Val Ser His Pro
    450                 455                 460

gag ata tca gat gct gtt gtc atc ccg ttt cct gat gct gaa gct ggt 1440
Glu Ile Ser Asp Ala Val Val Ile Pro Phe Pro Asp Ala Glu Ala Gly
465                 470                 475                 480

gag gtc ccg att gct tat gtt gtc cgc gct cct aat agc tca ctg tct 1488
Glu Val Pro Ile Ala Tyr Val Val Arg Ala Pro Asn Ser Ser Leu Ser
                485                 490                 495

gaa gaa gat atc aag aac ttc att gct gag cag gtg gct cct ttt aaa 1536
Glu Glu Asp Ile Lys Asn Phe Ile Ala Glu Gln Val Ala Pro Phe Lys
            500                 505                 510

aga ttg aga aga gtt aca ttc ata aac agt gtc ccg aaa tcc gca gct 1584
Arg Leu Arg Arg Val Thr Phe Ile Asn Ser Val Pro Lys Ser Ala Ala
        515                 520                 525

ggg aaa ata ctt aga aga gaa ctc atc gaa aag gtc cga tcg aag tta 1632
Gly Lys Ile Leu Arg Arg Glu Leu Ile Glu Lys Val Arg Ser Lys Leu
    530                 535                 540

taa                                                                    1635

<210>  108
<211>  544
<212>  PRT
<213>  Scutellaria baicalensis

<400>  108

Met Glu Lys Ser Gly Tyr Gly Arg Asp Gly Ile Tyr Arg Ser Leu Arg
1               5                   10                  15

Pro Pro Leu Tyr Leu Pro Lys Asn Pro Asn Leu Ser Leu Val Ser Phe
            20                  25                  30

Leu Phe Arg Asn Val Ser Ser Phe Ala Asp Asn Arg Ala Leu Val Asp
        35                  40                  45

Ala His Thr Gly Glu Thr Leu Thr Phe Ala Gln Phe Lys Ser Thr Val
    50                  55                  60

Ser Lys Val Ala His Gly Leu Leu Gln Leu Gly Ile Lys Lys Asn Asp
65                  70                  75                  80

Val Val Leu Ile Phe Ala Pro Asn Ser Ile Glu Phe Pro Ile Ser Phe
                85                  90                  95

Phe Gly Val Ile Ala Thr Gly Ala Val Ala Thr Thr Val Asn Pro Met
            100                 105                 110

Tyr Thr Val Ser Glu Leu Ser Lys Gln Val Gln Asp Ser Thr Ala Lys
        115                 120                 125

Leu Ile Ile Thr Val Glu Gly Leu Leu Pro Lys Val Lys Asp Phe Gly
    130                 135                 140

Leu Pro Val Ile Leu Leu Gly Asn Glu Lys Lys Ser Val Leu Pro Ile
145                 150                 155                 160

Gly Arg Ile Pro Ser Ile Thr Ser Phe Ser Glu Leu Val Asn Asn Glu
                165                 170                 175

Gly Leu Asp Leu Asp Ser Asn Val Tyr Gly Ile Lys Gln Asp Asp Thr
            180                 185                 190

Ala Ala Leu Leu Tyr Ser Ser Gly Thr Thr Gly Met Ser Lys Gly Val
        195                 200                 205

Ile Leu Thr His Arg Asn Phe Ile Ala Ser Ser Leu Met Ile Thr Ala
    210                 215                 220

Asp Gln Glu Ser Ala Gly Glu Met His Asn Val Phe Leu Cys Val Leu
225                 230                 235                 240

Pro Met Phe His Val Phe Gly Leu Ala Val Ile Met Leu Ser Gln Leu
                245                 250                 255

Gln Arg Gly Asn Thr Ile Val Ser Met Ser Lys Phe Asp Phe Glu Met
            260                 265                 270

Ile Leu Lys Thr Val Glu Lys Tyr Gly Ile Thr His Met Trp Val Val
        275                 280                 285

Pro Pro Ile Ile Leu Ala Leu Ala Lys Asn Asn Val Val Lys Lys Tyr
    290                 295                 300

Asn Leu Ser Ser Leu Gln Gln Ile Gly Ser Gly Ala Ala Pro Leu Gly
305                 310                 315                 320

Arg Glu Leu Met Gln Glu Cys Ala Lys Asn Phe Pro Gln Ala Val Val
                325                 330                 335

Val Gln Gly Tyr Gly Met Thr Glu Thr Cys Gly Ile Val Ser Val Glu
            340                 345                 350

Asn Val Tyr Ala Gly Pro Arg His Ser Gly Ser Ala Gly Thr Leu Val
        355                 360                 365

Pro Gly Val Glu Cys Gln Ile Val Ser Val Asp Lys Leu Lys Pro Leu
    370                 375                 380

Pro Pro Gly Gln Leu Gly Glu Ile Cys Val Arg Gly Pro Asn Met Met
385                 390                 395                 400

Gln Gly Tyr Phe Lys Asn Pro Gln Ala Thr Lys Leu Thr Ile Asp Lys
                405                 410                 415

Gln Gly Trp Val His Thr Gly Asp Leu Gly Tyr Phe Asp Asp Glu Gly
            420                 425                 430

Gln Leu Phe Val Val Asp Arg Ile Lys Glu Leu Ile Lys Tyr Lys Gly
        435                 440                 445

Phe Gln Val Ala Pro Ala Glu Leu Glu Gly Leu Leu Val Ser His Pro
    450                 455                 460

Glu Ile Ser Asp Ala Val Val Ile Pro Phe Pro Asp Ala Glu Ala Gly
465                 470                 475                 480

Glu Val Pro Ile Ala Tyr Val Val Arg Ala Pro Asn Ser Ser Leu Ser
                485                 490                 495

Glu Glu Asp Ile Lys Asn Phe Ile Ala Glu Gln Val Ala Pro Phe Lys
            500                 505                 510

Arg Leu Arg Arg Val Thr Phe Ile Asn Ser Val Pro Lys Ser Ala Ala
        515                 520                 525

Gly Lys Ile Leu Arg Arg Glu Leu Ile Glu Lys Val Arg Ser Lys Leu
    530                 535                 540

<210>  109
<211>  1173
<212>  DNA
<213>  Scutellaria baicalensis

<220>
<221>  CDS
<222>  (1)..(1173)
<223>  Gene encoding chalcone synthase

<300>
<308>  GenBank: KT963461.1
<309>  2016-05-11
<313>  (1)..(1173)

<400>  109
atg gtg aca gtt gaa gaa ttc cac cgg gca aca cgg gca gag ggc ccc      48
Met Val Thr Val Glu Glu Phe His Arg Ala Thr Arg Ala Glu Gly Pro
1               5                   10                  15

gcc acc gtc ttg gcc atc ggc acg gcc aac ccc cca aac tgc gtt gag      96
Ala Thr Val Leu Ala Ile Gly Thr Ala Asn Pro Pro Asn Cys Val Glu
            20                  25                  30

cag tct act tat gct gat tac tac ttt cgc att tgt aaa agt gaa cac      144
Gln Ser Thr Tyr Ala Asp Tyr Tyr Phe Arg Ile Cys Lys Ser Glu His
        35                  40                  45

ttg act gat ctt aaa aag aag ttt agc cgc atg tgc gaa aag tct ggc      192
Leu Thr Asp Leu Lys Lys Lys Phe Ser Arg Met Cys Glu Lys Ser Gly
    50                  55                  60

atc aag aaa cgt tac atg cat tta acg gag gag ttt ctg aag gag aac      240
Ile Lys Lys Arg Tyr Met His Leu Thr Glu Glu Phe Leu Lys Glu Asn
65                  70                  75                  80

gac aac ttc acg gcg tac gag gct ccg tca ctg gat gcc cgg cag gac      288
Asp Asn Phe Thr Ala Tyr Glu Ala Pro Ser Leu Asp Ala Arg Gln Asp
                85                  90                  95

ata gtg gtg gtg gag atc ccg aag ctg ggg aag gag gct gcc cag aag      336

Ile Val Val Val Glu Ile Pro Lys Leu Gly Lys Glu Ala Ala Gln Lys
            100                 105                 110

gcg atc aag gaa tgg ggg cag ccc aag tcc aag atc acc cac gtc atc      384
Ala Ile Lys Glu Trp Gly Gln Pro Lys Ser Lys Ile Thr His Val Ile
        115                 120                 125

ttc tgc act acc agc ggc gtc gac atg ccc ggc gca gac tac cag atc      432
Phe Cys Thr Thr Ser Gly Val Asp Met Pro Gly Ala Asp Tyr Gln Ile
    130                 135                 140

acc aag ctc ctc ggc ctc cgc ccc tcc gtc aag cgc ttc atg atg tac      480
Thr Lys Leu Leu Gly Leu Arg Pro Ser Val Lys Arg Phe Met Met Tyr
145                 150                 155                 160

cag cag ggc tgc ttc gcc ggc ggc acg gtg ctc cgc atg gcc aag gac      528
Gln Gln Gly Cys Phe Ala Gly Gly Thr Val Leu Arg Met Ala Lys Asp
                165                 170                 175

ctg gct gag aac aac gcc gga gct agg gtt ctt gtc gtc tgc tcc gag      576
Leu Ala Glu Asn Asn Ala Gly Ala Arg Val Leu Val Val Cys Ser Glu
            180                 185                 190

atc acc gcc atc act ttc cgg ggg ccc agc gac acc cac ctc gac agc      624
Ile Thr Ala Ile Thr Phe Arg Gly Pro Ser Asp Thr His Leu Asp Ser
        195                 200                 205

ctg gtc ggc cag gcc ctg ttc ggc gac ggc gcc ggg gcg gtc atc gtt      672
Leu Val Gly Gln Ala Leu Phe Gly Asp Gly Ala Gly Ala Val Ile Val
    210                 215                 220

gga tcc gac ccc atc gtg ggg gtg gag cgg ccg ctc ttc cag ctg gtc      720
Gly Ser Asp Pro Ile Val Gly Val Glu Arg Pro Leu Phe Gln Leu Val
225                 230                 235                 240

tcg gcg gcg cag acg att ctc cct gac agt gag ggc gcc att gac ggc      768
Ser Ala Ala Gln Thr Ile Leu Pro Asp Ser Glu Gly Ala Ile Asp Gly
                245                 250                 255

cac gtc cgc gag gtc ggg ctg acc ttc cac ctc ctc aag gac gtc ccc      816
His Val Arg Glu Val Gly Leu Thr Phe His Leu Leu Lys Asp Val Pro
            260                 265                 270

ggg ctg atc tcc aag aac atc gag aag agc ctc aag gag gcc ttc gcg      864
Gly Leu Ile Ser Lys Asn Ile Glu Lys Ser Leu Lys Glu Ala Phe Ala
        275                 280                 285

ccg ctg ggc atc tcc gac tgg aac tcc ctc ttc tgg atc gtg cac ccc      912
Pro Leu Gly Ile Ser Asp Trp Asn Ser Leu Phe Trp Ile Val His Pro
    290                 295                 300

gga ggt ccc gcc att ctc gac cag gtg gag gag aag ctc ggg ctc aag      960
Gly Gly Pro Ala Ile Leu Asp Gln Val Glu Glu Lys Leu Gly Leu Lys
305                 310                 315                 320

ccc gag atc atg gtc cca acc agg cac gtg ctt agc gaa tac ggg aac     1008
Pro Glu Ile Met Val Pro Thr Arg His Val Leu Ser Glu Tyr Gly Asn
                325                 330                 335

atg tcc agc gcc tgc gtt ctc ttc gtg atg gat gag atg agg aag gcc     1056
Met Ser Ser Ala Cys Val Leu Phe Val Met Asp Glu Met Arg Lys Ala
            340                 345                 350

tcc gcc aag gac ggc tgc acc acc acc gga gaa ggg aag gac tgg ggg 1104
Ser Ala Lys Asp Gly Cys Thr Thr Thr Gly Glu Gly Lys Asp Trp Gly
        355                 360                 365

gtt ctt ttc ggc ttc ggc ccg ggc ctc acc gtt gag act gta gtt ctg 1152
Val Leu Phe Gly Phe Gly Pro Gly Leu Thr Val Glu Thr Val Val Leu
    370                 375                 380

cat agt gtg cct ctc aat tga 1173
His Ser Val Pro Leu Asn
385                 390

<210> 110
<211> 390
<212> PRT
<213> Scutellaria baicalensis

<400> 110

Met Val Thr Val Glu Glu Phe His Arg Ala Thr Arg Ala Glu Gly Pro
1               5                   10                  15

Ala Thr Val Leu Ala Ile Gly Thr Ala Asn Pro Pro Asn Cys Val Glu
            20                  25                  30

Gln Ser Thr Tyr Ala Asp Tyr Tyr Phe Arg Ile Cys Lys Ser Glu His
        35                  40                  45

Leu Thr Asp Leu Lys Lys Lys Phe Ser Arg Met Cys Glu Lys Ser Gly
    50                  55                  60

Ile Lys Lys Arg Tyr Met His Leu Thr Glu Glu Phe Leu Lys Glu Asn
65                  70                  75                  80

Asp Asn Phe Thr Ala Tyr Glu Ala Pro Ser Leu Asp Ala Arg Gln Asp
                85                  90                  95

Ile Val Val Val Glu Ile Pro Lys Leu Gly Lys Glu Ala Ala Gln Lys
            100                 105                 110

Ala Ile Lys Glu Trp Gly Gln Pro Lys Ser Lys Ile Thr His Val Ile
        115                 120                 125

Phe Cys Thr Thr Ser Gly Val Asp Met Pro Gly Ala Asp Tyr Gln Ile
    130                 135                 140

Thr Lys Leu Leu Gly Leu Arg Pro Ser Val Lys Arg Phe Met Met Tyr
145                 150                 155                 160

Gln Gln Gly Cys Phe Ala Gly Gly Thr Val Leu Arg Met Ala Lys Asp
                165                 170                 175

Leu Ala Glu Asn Asn Ala Gly Ala Arg Val Leu Val Val Cys Ser Glu
          180                 185                 190

Ile Thr Ala Ile Thr Phe Arg Gly Pro Ser Asp Thr His Leu Asp Ser
        195                 200                 205

Leu Val Gly Gln Ala Leu Phe Gly Asp Gly Ala Gly Ala Val Ile Val
    210                 215                 220

Gly Ser Asp Pro Ile Val Gly Val Glu Arg Pro Leu Phe Gln Leu Val
225                 230                 235                 240

Ser Ala Ala Gln Thr Ile Leu Pro Asp Ser Glu Gly Ala Ile Asp Gly
                245                 250                 255

His Val Arg Glu Val Gly Leu Thr Phe His Leu Leu Lys Asp Val Pro
            260                 265                 270

Gly Leu Ile Ser Lys Asn Ile Glu Lys Ser Leu Lys Glu Ala Phe Ala
        275                 280                 285

Pro Leu Gly Ile Ser Asp Trp Asn Ser Leu Phe Trp Ile Val His Pro
    290                 295                 300

Gly Gly Pro Ala Ile Leu Asp Gln Val Glu Glu Lys Leu Gly Leu Lys
305                 310                 315                 320

Pro Glu Ile Met Val Pro Thr Arg His Val Leu Ser Glu Tyr Gly Asn
                325                 330                 335

Met Ser Ser Ala Cys Val Leu Phe Val Met Asp Glu Met Arg Lys Ala
            340                 345                 350

Ser Ala Lys Asp Gly Cys Thr Thr Thr Gly Glu Gly Lys Asp Trp Gly
        355                 360                 365

Val Leu Phe Gly Phe Gly Pro Gly Leu Thr Val Glu Thr Val Val Leu
    370                 375                 380

His Ser Val Pro Leu Asn
385                 390

<210>  111
<211>  648
<212>  DNA
<213>  Scutellaria baicalensis

<220>
<221>  CDS
<222>  (1)..(648)
<223>  Gene encoding chalcone isomerase

<300>
<308>  GenBank: KT963462.1
<309>  2016-05-11
<313>  (1)..(648)

<400>  111
atg tct gct tcg cca tcc gtc acc aaa gtc cta gtc gaa tcc atc gag      48
Met Ser Ala Ser Pro Ser Val Thr Lys Val Leu Val Glu Ser Ile Glu
1               5                   10                  15

ttc tcg ccg gcc gca aag ccg ccg gga tcc tcc aac acc tta ttc ctc      96
Phe Ser Pro Ala Ala Lys Pro Pro Gly Ser Ser Asn Thr Leu Phe Leu
            20                  25                  30

ggc ggc gcc ggg gtg aga gga atg gag atc cag ggg aac ttc gtg aag      144
Gly Gly Ala Gly Val Arg Gly Met Glu Ile Gln Gly Asn Phe Val Lys
        35                  40                  45

ttc acg gcg atc ggt gtt tac ttg gaa gac agc gcc gtt ccg tct ctc      192
Phe Thr Ala Ile Gly Val Tyr Leu Glu Asp Ser Ala Val Pro Ser Leu
    50                  55                  60

gcc gtt aac tgg aag ggc aag acc gcc gag gag ttg acg gag tca gac      240
Ala Val Asn Trp Lys Gly Lys Thr Ala Glu Glu Leu Thr Glu Ser Asp
65                  70                  75                  80

gac ttt ttc agg gaa atc gtg tct ggt cct ttc gag aaa ttc acc aag      288
Asp Phe Phe Arg Glu Ile Val Ser Gly Pro Phe Glu Lys Phe Thr Lys
                85                  90                  95

gtg acg atg att ctg cca ttg acg ggg aag caa tac tcg gag aaa gtt      336
Val Thr Met Ile Leu Pro Leu Thr Gly Lys Gln Tyr Ser Glu Lys Val
            100                 105                 110

gca gag aac tgc gtt gcg tac tgg aaa gcc gta ggg aaa tac acg gat      384
Ala Glu Asn Cys Val Ala Tyr Trp Lys Ala Val Gly Lys Tyr Thr Asp
        115                 120                 125

gct gaa tcg gaa gca atc gac aag ttt ctc caa gtg ttc aag gac gaa      432
Ala Glu Ser Glu Ala Ile Asp Lys Phe Leu Gln Val Phe Lys Asp Glu
    130                 135                 140

acg ttc gcg cct gga gct tcc atc ctc ttc acc caa tcg ccg gcc ggc      480
Thr Phe Ala Pro Gly Ala Ser Ile Leu Phe Thr Gln Ser Pro Ala Gly
145                 150                 155                 160

tcc ttg acg att agc ttc tcg aaa gac gga tcg att cca gag caa ggg      528
Ser Leu Thr Ile Ser Phe Ser Lys Asp Gly Ser Ile Pro Glu Gln Gly
                165                 170                 175

aag gca gta ata gag aac aaa cag ctg tcg gag gca gtg ctg gag tcg      576
Lys Ala Val Ile Glu Asn Lys Gln Leu Ser Glu Ala Val Leu Glu Ser
            180                 185                 190

atc atc gga aag cat ggt gtg tcg ccg tcg gcg aag cag agt ttg gcg      624
Ile Ile Gly Lys His Gly Val Ser Pro Ser Ala Lys Gln Ser Leu Ala
        195                 200                 205

gca aga cta tcg gag ttg ttt taa                                          648
Ala Arg Leu Ser Glu Leu Phe
    210                 215

<210>  112
<211>  215
<212>  PRT
<213>  Scutellaria baicalensis

<400>  112

Met Ser Ala Ser Pro Ser Val Thr Lys Val Leu Val Glu Ser Ile Glu
1               5                   10                  15

Phe Ser Pro Ala Ala Lys Pro Pro Gly Ser Ser Asn Thr Leu Phe Leu
            20                  25                  30

Gly Gly Ala Gly Val Arg Gly Met Glu Ile Gln Gly Asn Phe Val Lys
        35                  40                  45

Phe Thr Ala Ile Gly Val Tyr Leu Glu Asp Ser Ala Val Pro Ser Leu
    50                  55                  60

Ala Val Asn Trp Lys Gly Lys Thr Ala Glu Glu Leu Thr Glu Ser Asp
65                  70                  75                  80

Asp Phe Phe Arg Glu Ile Val Ser Gly Pro Phe Glu Lys Phe Thr Lys
                85                  90                  95

Val Thr Met Ile Leu Pro Leu Thr Gly Lys Gln Tyr Ser Glu Lys Val
            100                 105                 110

Ala Glu Asn Cys Val Ala Tyr Trp Lys Ala Val Gly Lys Tyr Thr Asp
        115                 120                 125

Ala Glu Ser Glu Ala Ile Asp Lys Phe Leu Gln Val Phe Lys Asp Glu
    130                 135                 140

Thr Phe Ala Pro Gly Ala Ser Ile Leu Phe Thr Gln Ser Pro Ala Gly
145                 150                 155                 160

Ser Leu Thr Ile Ser Phe Ser Lys Asp Gly Ser Ile Pro Glu Gln Gly
                165                 170                 175

Lys Ala Val Ile Glu Asn Lys Gln Leu Ser Glu Ala Val Leu Glu Ser
            180                 185                 190

Ile Ile Gly Lys His Gly Val Ser Pro Ser Ala Lys Gln Ser Leu Ala
        195                 200                 205

Ala Arg Leu Ser Glu Leu Phe
    210                 215

<210>  113
<211>  1509
<212>  DNA
<213>  Scutellaria baicalensis

<220>
<221>  CDS
<222>  (1)..(1509)
<223>  Gene encoding flavone synthase II

<300>
<308>  GenBank: KT963453.1
<309>  2016-05-11
<313>  (1)..(1509)

<400>  113
atg gac tta gta gaa gtc aca ctc tac gcc gcc ctc ttc ctc ctc tcc        48
Met Asp Leu Val Glu Val Thr Leu Tyr Ala Ala Leu Phe Leu Leu Ser
1               5                   10                  15

gcc gcc ttc ctc ctc cta atc ttc gcc gga gac cgc tcc tcc cca ccg        96
Ala Ala Phe Leu Leu Leu Ile Phe Ala Gly Asp Arg Ser Ser Pro Pro
            20                  25                  30

ggc ccg ttc cct ctt ccg atc atc ggg cac ctc cac ctc ctc ggc ccg       144
Gly Pro Phe Pro Leu Pro Ile Ile Gly His Leu His Leu Leu Gly Pro
        35                  40                  45

aaa ctc cac caa agc ttc cac ggg ctg tcc caa cgg cac ggc ccg ctg       192
Lys Leu His Gln Ser Phe His Gly Leu Ser Gln Arg His Gly Pro Leu
    50                  55                  60

atg caa atc cgg ctg ggc tcc atc aac tgc gtg gtg gcg tcg acg ccg       240
Met Gln Ile Arg Leu Gly Ser Ile Asn Cys Val Val Ala Ser Thr Pro
65                  70                  75                  80

gag ctg gct aaa gag ttc ctc aaa acc aac gag ctg gtg ttc tcc tcg       288
Glu Leu Ala Lys Glu Phe Leu Lys Thr Asn Glu Leu Val Phe Ser Ser
                85                  90                  95

cgc aaa cac tcg acc gcc att gat atc gtc acc tac aat tcc tcc ttc       336
Arg Lys His Ser Thr Ala Ile Asp Ile Val Thr Tyr Asn Ser Ser Phe
            100                 105                 110

gcc ttc tcc cca tac ggg ccc tac tgg aag tac atc aag aag ctc tgc       384
Ala Phe Ser Pro Tyr Gly Pro Tyr Trp Lys Tyr Ile Lys Lys Leu Cys
        115                 120                 125

acc tac gag ctg ctc gga gcg aga aac ctc cac cat ttc cag ccc atc       432
Thr Tyr Glu Leu Leu Gly Ala Arg Asn Leu His His Phe Gln Pro Ile
    130                 135                 140

agg acc ttc gag gtc cac act ttt ctc cgg ctt ctc atg gag aag agc       480
Arg Thr Phe Glu Val His Thr Phe Leu Arg Leu Leu Met Glu Lys Ser
145                 150                 155                 160

gaa tcc ggg gag agc ttt aat gtc acc gag gag ctc att aag ctc acc       528

Glu Ser Gly Glu Ser Phe Asn Val Thr Glu Glu Leu Ile Lys Leu Thr
                165                 170                 175

agc aac gtc atg tcc aat atg atg ctc ggc acc agg tgc tcc gcc aca      576
Ser Asn Val Met Ser Asn Met Met Leu Gly Thr Arg Cys Ser Ala Thr
            180                 185                 190

gac ggc gag gcg gag gcg gct agg acg gtg atc cgg gag gtg acg gag      624
Asp Gly Glu Ala Glu Ala Ala Arg Thr Val Ile Arg Glu Val Thr Glu
        195                 200                 205

atc ttc ggg gag ttc gat gct gct gat atc atc tgg ttc tgt aag aac      672
Ile Phe Gly Glu Phe Asp Ala Ala Asp Ile Ile Trp Phe Cys Lys Asn
    210                 215                 220

ttc gat ttg cag gga ata agg aag agg tcg gag gat att cag aga agg      720
Phe Asp Leu Gln Gly Ile Arg Lys Arg Ser Glu Asp Ile Gln Arg Arg
225                 230                 235                 240

tat gat gct ttg ctg gaa aag att atc acc gac aga gag aag ctc cgg      768
Tyr Asp Ala Leu Leu Glu Lys Ile Ile Thr Asp Arg Glu Lys Leu Arg
                245                 250                 255

cgg agc cac cgc ggc ggc gag gcc aag gat ttt ctt gat atc ttt ctg      816
Arg Ser His Arg Gly Gly Glu Ala Lys Asp Phe Leu Asp Ile Phe Leu
            260                 265                 270

gat ata atg gac agt ggc aac tct gag gtg aaa ttc agc aga gaa cat      864
Asp Ile Met Asp Ser Gly Asn Ser Glu Val Lys Phe Ser Arg Glu His
        275                 280                 285

ctc aaa gct ttg att ttg gat ttc ttc acc gct ggt aca gac aca acg      912
Leu Lys Ala Leu Ile Leu Asp Phe Phe Thr Ala Gly Thr Asp Thr Thr
    290                 295                 300

gcg atc agc aca gag tgg gca ata gca gaa ctg atg aac aac cca aag      960
Ala Ile Ser Thr Glu Trp Ala Ile Ala Glu Leu Met Asn Asn Pro Lys
305                 310                 315                 320

gta cta aag aaa gca caa gaa gag atc cag aaa gtg gtg gga tct tgt     1008
Val Leu Lys Lys Ala Gln Glu Glu Ile Gln Lys Val Val Gly Ser Cys
                325                 330                 335

aga ttg atg gat gaa tca gac gcc cct aat ctc cca tac ctt gag gcc     1056
Arg Leu Met Asp Glu Ser Asp Ala Pro Asn Leu Pro Tyr Leu Glu Ala
            340                 345                 350

atc atc aag gag acc ttc agg ctc cac cct ccg atc ccg atg ctt gcc     1104
Ile Ile Lys Glu Thr Phe Arg Leu His Pro Pro Ile Pro Met Leu Ala
        355                 360                 365

cgg aaa tcc gtc tcc gat tgc gtc atc gac ggc tac aac atc ccg gcg     1152
Arg Lys Ser Val Ser Asp Cys Val Ile Asp Gly Tyr Asn Ile Pro Ala
    370                 375                 380

agc act ctc ctc ttc gtc aac att tgg tcc att ggt cgg aac ccc gag     1200
Ser Thr Leu Leu Phe Val Asn Ile Trp Ser Ile Gly Arg Asn Pro Glu
385                 390                 395                 400

tgt tgg gac agc ccc ttc tcc ttc cgg ccc gaa cgc ttc ttc gag aag     1248
Cys Trp Asp Ser Pro Phe Ser Phe Arg Pro Glu Arg Phe Phe Glu Lys
                405                 410                 415

gac aac gct tcg atc gat atc aag ggg cag cac ttc cag ctg ctg cct 1296
Asp Asn Ala Ser Ile Asp Ile Lys Gly Gln His Phe Gln Leu Leu Pro
            420                 425                 430

ttt gga acg ggc agg agg ggc tgc ccg gga atg ctt ttg gcc ata cag 1344
Phe Gly Thr Gly Arg Arg Gly Cys Pro Gly Met Leu Leu Ala Ile Gln
        435                 440                 445

gag ctg cta ctt atc ata ggc act atg att cag tgc ttt gat tgg gaa 1392
Glu Leu Leu Leu Ile Ile Gly Thr Met Ile Gln Cys Phe Asp Trp Glu
    450                 455                 460

ttg cct gag ggt tcg ggc cct gtc gac atg acc gaa cgg gcc ggg ttg 1440
Leu Pro Glu Gly Ser Gly Pro Val Asp Met Thr Glu Arg Ala Gly Leu
465                 470                 475                 480

act gct cca cgg gct gaa gat ttg att tgt cgg gtc tcc tgc cgg gtt 1488
Thr Ala Pro Arg Ala Glu Asp Leu Ile Cys Arg Val Ser Cys Arg Val
                485                 490                 495

gac ccg aaa att gtt ttc tag 1509
Asp Pro Lys Ile Val Phe
            500

<210> 114
<211> 502
<212> PRT
<213> Scutellaria baicalensis

<400> 114

Met Asp Leu Val Glu Val Thr Leu Tyr Ala Ala Leu Phe Leu Leu Ser
1               5                   10                  15

Ala Ala Phe Leu Leu Leu Ile Phe Ala Gly Asp Arg Ser Ser Pro Pro
            20                  25                  30

Gly Pro Phe Pro Leu Pro Ile Ile Gly His Leu His Leu Leu Gly Pro
        35                  40                  45

Lys Leu His Gln Ser Phe His Gly Leu Ser Gln Arg His Gly Pro Leu
    50                  55                  60

Met Gln Ile Arg Leu Gly Ser Ile Asn Cys Val Val Ala Ser Thr Pro
65                  70                  75                  80

Glu Leu Ala Lys Glu Phe Leu Lys Thr Asn Glu Leu Val Phe Ser Ser
                85                  90                  95

Arg Lys His Ser Thr Ala Ile Asp Ile Val Thr Tyr Asn Ser Ser Phe
            100                 105                 110

Ala Phe Ser Pro Tyr Gly Pro Tyr Trp Lys Tyr Ile Lys Lys Leu Cys
        115                 120                 125

Thr Tyr Glu Leu Leu Gly Ala Arg Asn Leu His His Phe Gln Pro Ile
    130                 135                 140

Arg Thr Phe Glu Val His Thr Phe Leu Arg Leu Leu Met Glu Lys Ser
145                 150                 155                 160

Glu Ser Gly Glu Ser Phe Asn Val Thr Glu Glu Leu Ile Lys Leu Thr
                165                 170                 175

Ser Asn Val Met Ser Asn Met Met Leu Gly Thr Arg Cys Ser Ala Thr
            180                 185                 190

Asp Gly Glu Ala Glu Ala Ala Arg Thr Val Ile Arg Glu Val Thr Glu
        195                 200                 205

Ile Phe Gly Glu Phe Asp Ala Ala Asp Ile Ile Trp Phe Cys Lys Asn
    210                 215                 220

Phe Asp Leu Gln Gly Ile Arg Lys Arg Ser Glu Asp Ile Gln Arg Arg
225                 230                 235                 240

Tyr Asp Ala Leu Leu Glu Lys Ile Ile Thr Asp Arg Glu Lys Leu Arg
                245                 250                 255

Arg Ser His Arg Gly Gly Glu Ala Lys Asp Phe Leu Asp Ile Phe Leu
            260                 265                 270

Asp Ile Met Asp Ser Gly Asn Ser Glu Val Lys Phe Ser Arg Glu His
        275                 280                 285

Leu Lys Ala Leu Ile Leu Asp Phe Phe Thr Ala Gly Thr Asp Thr Thr
    290                 295                 300

Ala Ile Ser Thr Glu Trp Ala Ile Ala Glu Leu Met Asn Asn Pro Lys
305                 310                 315                 320

Val Leu Lys Lys Ala Gln Glu Glu Ile Gln Lys Val Val Gly Ser Cys
                325                 330                 335

Arg Leu Met Asp Glu Ser Asp Ala Pro Asn Leu Pro Tyr Leu Glu Ala
            340                 345                 350

Ile Ile Lys Glu Thr Phe Arg Leu His Pro Pro Ile Pro Met Leu Ala
        355                 360                 365

Arg Lys Ser Val Ser Asp Cys Val Ile Asp Gly Tyr Asn Ile Pro Ala
    370                 375                 380

Ser Thr Leu Leu Phe Val Asn Ile Trp Ser Ile Gly Arg Asn Pro Glu
385                 390                 395                 400

Cys Trp Asp Ser Pro Phe Ser Phe Arg Pro Glu Arg Phe Phe Glu Lys
                405                 410                 415

Asp Asn Ala Ser Ile Asp Ile Lys Gly Gln His Phe Gln Leu Leu Pro
            420                 425                 430

Phe Gly Thr Gly Arg Arg Gly Cys Pro Gly Met Leu Leu Ala Ile Gln
        435                 440                 445

Glu Leu Leu Leu Ile Ile Gly Thr Met Ile Gln Cys Phe Asp Trp Glu
    450                 455                 460

Leu Pro Glu Gly Ser Gly Pro Val Asp Met Thr Glu Arg Ala Gly Leu
465                 470                 475                 480

Thr Ala Pro Arg Ala Glu Asp Leu Ile Cys Arg Val Ser Cys Arg Val
                485                 490                 495

Asp Pro Lys Ile Val Phe
            500

<210>  115
<211>  1554
<212>  DNA
<213>  Scutellaria baicalensis

<220>
<221>  CDS
<222>  (1)..(1554)
<223>  Gene encoding flavone 6-hydroxylase

<300>
<308>  GenBank: MF363006.1
<309>  2017-09-05
<313>  (1)..(1554)

<400>  115
atg gag ttg agc tct gtc atc tat ggc gcc atc gcc ttg ctc tct ctt      48
Met Glu Leu Ser Ser Val Ile Tyr Gly Ala Ile Ala Leu Leu Ser Leu
1               5                   10                  15

ttc tac tgt tac cta cac ttc tcc aag cca aaa aag agt tcc ctg aac      96
Phe Tyr Cys Tyr Leu His Phe Ser Lys Pro Lys Lys Ser Ser Leu Asn
            20                  25                  30

gcg ccg ccg gag gct ggc ggc gcg cgc ttc atc acc ggc cac ctc cac      144
Ala Pro Pro Glu Ala Gly Gly Ala Arg Phe Ile Thr Gly His Leu His
        35                  40                  45

ctg atg gac ggc cgc tcc gcc tcc gac aaa ctt cct cac ata aac ctg 192
Leu Met Asp Gly Arg Ser Ala Ser Asp Lys Leu Pro His Ile Asn Leu
    50                  55                  60

ggt ttg ctt gca gac cag cac ggc cca atc ttc acg atc agg ctg ggc 240
Gly Leu Leu Ala Asp Gln His Gly Pro Ile Phe Thr Ile Arg Leu Gly
65                  70                  75                  80

gtg cac cga gcc gtg gtg gtg agc agc tgg gag cta gcc aag gag atc 288
Val His Arg Ala Val Val Val Ser Ser Trp Glu Leu Ala Lys Glu Ile
                85                  90                  95

ttc acc acc cac gac acg gcg gtc atg gct cgg ccc cgg ctc ata gcc 336
Phe Thr Thr His Asp Thr Ala Val Met Ala Arg Pro Arg Leu Ile Ala
            100                 105                 110

gac gac tac ctg agc tac gac gga gcc tcg ctt ggg ttc tca ccc tac 384
Asp Asp Tyr Leu Ser Tyr Asp Gly Ala Ser Leu Gly Phe Ser Pro Tyr
        115                 120                 125

gga cca tac tgg cgc gaa atc cgc aag ctg gtc acc acc gag ctg ctc 432
Gly Pro Tyr Trp Arg Glu Ile Arg Lys Leu Val Thr Thr Glu Leu Leu
    130                 135                 140

tcg gct cgc cgg atc gag ctg cag cga gcc acg cgt gtg cgt gag atc 480
Ser Ala Arg Arg Ile Glu Leu Gln Arg Ala Thr Arg Val Arg Glu Ile
145                 150                 155                 160

acg cag ttc acc ggc gag ctc tac aag ctg tgg gag gag aag aaa gac 528
Thr Gln Phe Thr Gly Glu Leu Tyr Lys Leu Trp Glu Glu Lys Lys Asp
                165                 170                 175

ggg tct ggg agg gtt ttg gtg gac atg aag cag tgg ctg ggg aac ttg 576
Gly Ser Gly Arg Val Leu Val Asp Met Lys Gln Trp Leu Gly Asn Leu
            180                 185                 190

agc ctc aat ctg gtg tcg agg atg gtg gtg ggc aag agg ttc tac ggc 624
Ser Leu Asn Leu Val Ser Arg Met Val Val Gly Lys Arg Phe Tyr Gly
        195                 200                 205

ggc gac gac tcg gag acg acc aag agg tgg cgg ggc gtc atg agg gag 672
Gly Asp Asp Ser Glu Thr Thr Lys Arg Trp Arg Gly Val Met Arg Glu
    210                 215                 220

ttc ttc cag ctc atc gga cag ttc atc ccc ggc gac ggg ctt ccg ttt 720
Phe Phe Gln Leu Ile Gly Gln Phe Ile Pro Gly Asp Gly Leu Pro Phe
225                 230                 235                 240

ctc cgg tgg ctc gac ttg ggt ggg ttc gag aag agg acg agg gac act 768
Leu Arg Trp Leu Asp Leu Gly Gly Phe Glu Lys Arg Thr Arg Asp Thr
                245                 250                 255

gcc tat gag ctg gat aag atc atc gca atg tgg ctg gcg gag tat cgg 816
Ala Tyr Glu Leu Asp Lys Ile Ile Ala Met Trp Leu Ala Glu Tyr Arg
            260                 265                 270

aaa agg gaa tat tcc ggc gac gac aag gag cag tgc ttc atg gcc ctc 864
Lys Arg Glu Tyr Ser Gly Asp Asp Lys Glu Gln Cys Phe Met Ala Leu
        275                 280                 285

atg ctc tca ctc gtg caa gct aat ccc act ctg caa ctc cac tac gat 912
Met Leu Ser Leu Val Gln Ala Asn Pro Thr Leu Gln Leu His Tyr Asp
    290                 295                 300

gct gat acc atc atc aaa gct act tgc cag gtt ttg ata tcg gcg gca 960
Ala Asp Thr Ile Ile Lys Ala Thr Cys Gln Val Leu Ile Ser Ala Ala
305                 310                 315                 320

agt gac acg acg acg gtg atc cta atc tgg gta att tcc ctc cta cta 1008
Ser Asp Thr Thr Thr Val Ile Leu Ile Trp Val Ile Ser Leu Leu Leu
                325                 330                 335

aac aat gct gat gtc cta aaa aag gtt caa gaa gaa ctg gac gaa caa 1056
Asn Asn Ala Asp Val Leu Lys Lys Val Gln Glu Glu Leu Asp Glu Gln
            340                 345                 350

gtg ggg aga gaa aga cga gtg gaa gaa tcg gac ata agc aat tta ccc 1104
Val Gly Arg Glu Arg Arg Val Glu Glu Ser Asp Ile Ser Asn Leu Pro
        355                 360                 365

tac ctc caa gcg gtg gtg aag gag aca atg agg cta tac cct ccg gcc 1152
Tyr Leu Gln Ala Val Val Lys Glu Thr Met Arg Leu Tyr Pro Pro Ala
    370                 375                 380

ccc ttc gcc gga gta cga gcc ttc agc gaa gac tgc acc gtg gga ggc 1200
Pro Phe Ala Gly Val Arg Ala Phe Ser Glu Asp Cys Thr Val Gly Gly
385                 390                 395                 400

tac cac atc cag aaa ggc acg ttt ttg ata gtc aat ctg tgg aag ctg 1248
Tyr His Ile Gln Lys Gly Thr Phe Leu Ile Val Asn Leu Trp Lys Leu
                405                 410                 415

cat cga gac cct cgt gtg tgg tcc gat gat gcc ttg gag ttc aaa cca 1296
His Arg Asp Pro Arg Val Trp Ser Asp Asp Ala Leu Glu Phe Lys Pro
            420                 425                 430

caa cgg ttt ttt gac aaa aag gtg gag gtt aag ggt cag gac ttc gag 1344
Gln Arg Phe Phe Asp Lys Lys Val Glu Val Lys Gly Gln Asp Phe Glu
        435                 440                 445

ttg atg cca ttt ggt ggt ggt cga aga atg tgt ccc ggc tcg aac ctg 1392
Leu Met Pro Phe Gly Gly Gly Arg Arg Met Cys Pro Gly Ser Asn Leu
    450                 455                 460

ggc atg cac atg gtg cac ttt gtg ctg gct aac ata ttg cag gcc ttt 1440
Gly Met His Met Val His Phe Val Leu Ala Asn Ile Leu Gln Ala Phe
465                 470                 475                 480

gac ata acc act ggg tcc act gtg gat atg acc gag agt gtt ggg ttg 1488
Asp Ile Thr Thr Gly Ser Thr Val Asp Met Thr Glu Ser Val Gly Leu
                485                 490                 495

acc aac atg aaa gcc aca cca ctt gat gct att ctc act cca agg ttg 1536
Thr Asn Met Lys Ala Thr Pro Leu Asp Ala Ile Leu Thr Pro Arg Leu
            500                 505                 510

tca cct act cta tat taa 1554
Ser Pro Thr Leu Tyr
        515

<210> 116
<211> 517
<212> PRT
<213> Scutellaria baicalensis

<400>  116
   Met Glu Leu Ser Ser Val Ile Tyr Gly Ala Ile Ala Leu Leu Ser Leu
   1               5                   10                  15
   Phe Tyr Cys Tyr Leu His Phe Ser Lys Pro Lys Lys Ser Ser Leu Asn
               20                  25                  30
   Ala Pro Pro Glu Ala Gly Gly Ala Arg Phe Ile Thr Gly His Leu His
           35                  40                  45
   Leu Met Asp Gly Arg Ser Ala Ser Asp Lys Leu Pro His Ile Asn Leu
       50                  55                  60
   Gly Leu Leu Ala Asp Gln His Gly Pro Ile Phe Thr Ile Arg Leu Gly
   65                  70                  75                  80
   Val His Arg Ala Val Val Val Ser Ser Trp Glu Leu Ala Lys Glu Ile
                   85                  90                  95
   Phe Thr Thr His Asp Thr Ala Val Met Ala Arg Pro Arg Leu Ile Ala
               100                 105                 110
   Asp Asp Tyr Leu Ser Tyr Asp Gly Ala Ser Leu Gly Phe Ser Pro Tyr
           115                 120                 125
   Gly Pro Tyr Trp Arg Glu Ile Arg Lys Leu Val Thr Thr Glu Leu Leu
       130                 135                 140
   Ser Ala Arg Arg Ile Glu Leu Gln Arg Ala Thr Arg Val Arg Glu Ile
   145                 150                 155                 160
   Thr Gln Phe Thr Gly Glu Leu Tyr Lys Leu Trp Glu Glu Lys Lys Asp
                   165                 170                 175
   Gly Ser Gly Arg Val Leu Val Asp Met Lys Gln Trp Leu Gly Asn Leu
               180                 185                 190
   Ser Leu Asn Leu Val Ser Arg Met Val Val Gly Lys Arg Phe Tyr Gly
           195                 200                 205
   Gly Asp Asp Ser Glu Thr Thr Lys Arg Trp Arg Gly Val Met Arg Glu
       210                 215                 220
   Phe Phe Gln Leu Ile Gly Gln Phe Ile Pro Gly Asp Gly Leu Pro Phe
   225                 230                 235                 240
   Leu Arg Trp Leu Asp Leu Gly Gly Phe Glu Lys Arg Thr Arg Asp Thr
   245 250 255
   Ala Tyr Glu Leu Asp Lys Ile Ile Ala Met Trp Leu Ala Glu Tyr Arg
   260 265 270
   Lys Arg Glu Tyr Ser Gly Asp Asp Lys Glu Gln Cys Phe Met Ala Leu
   275 280 285
   Met Leu Ser Leu Val Gln Ala Asn Pro Thr Leu Gln Leu His Tyr Asp
   290 295 300
   Ala Asp Thr Ile Ile Lys Ala Thr Cys Gln Val Leu Ile Ser Ala Ala
   305 310 315 320
   Ser Asp Thr Thr Thr Val Ile Leu Ile Trp Val Ile Ser Leu Leu Leu
   325 330 335
   Asn Asn Ala Asp Val Leu Lys Lys Val Gln Glu Glu Leu Asp Glu Gln
   340 345 350
   Val Gly Arg Glu Arg Arg Val Glu Glu Ser Asp Ile Ser Asn Leu Pro
   355 360 365
   Tyr Leu Gln Ala Val Val Lys Glu Thr Met Arg Leu Tyr Pro Pro Ala
   370 375 380
   Pro Phe Ala Gly Val Arg Ala Phe Ser Glu Asp Cys Thr Val Gly Gly
   385 390 395 400
   Tyr His Ile Gln Lys Gly Thr Phe Leu Ile Val Asn Leu Trp Lys Leu
   405 410 415
   His Arg Asp Pro Arg Val Trp Ser Asp Asp Ala Leu Glu Phe Lys Pro
   420 425 430
   Gln Arg Phe Phe Asp Lys Lys Val Glu Val Lys Gly Gln Asp Phe Glu
   435 440 445
   Leu Met Pro Phe Gly Gly Gly Arg Arg Met Cys Pro Gly Ser Asn Leu
   450 455 460
   Gly Met His Met Val His Phe Val Leu Ala Asn Ile Leu Gln Ala Phe
   465 470 475 480
   Asp Ile Thr Thr Gly Ser Thr Val Asp Met Thr Glu Ser Val Gly Leu
   485 490 495
   Thr Asn Met Lys Ala Thr Pro Leu Asp Ala Ile Leu Thr Pro Arg Leu
               500                 505                 510
   Ser Pro Thr Leu Tyr
           515

<210>  117
<211>  1446
<212>  DNA
<213>  Arabidopsis thaliana

<220>
<221>  CDS
<222>  (1)..(1446)
<223>  Gene encoding uridine diphosphate (UDP)-glucose dehydrogenase

<300>
<308>  NCBI Reference Sequence: NM_102419.4
<309>  2017-03-20
<313>  (1)..(1446)

<400>  117
atg gtg aag ata tgc tgc ata gga gct ggt tat gtg ggt ggt cca acc        48
Met Val Lys Ile Cys Cys Ile Gly Ala Gly Tyr Val Gly Gly Pro Thr
1               5                   10                  15

atg gcg gtg atg gct ctt aag tgt cct gag att gaa gta gtc gtt gtg        96
Met Ala Val Met Ala Leu Lys Cys Pro Glu Ile Glu Val Val Val Val
            20                  25                  30

gat atc tct gaa cca agg atc aat gct tgg aac agt gat agg ctt cct       144
Asp Ile Ser Glu Pro Arg Ile Asn Ala Trp Asn Ser Asp Arg Leu Pro
        35                  40                  45

att tac gag ccg gga ttg gaa gat gtg gtg aaa caa tgc aga ggg aaa       192
Ile Tyr Glu Pro Gly Leu Glu Asp Val Val Lys Gln Cys Arg Gly Lys
    50                  55                  60

aac ctc ttc ttt agc aca gac gtg gag aaa cat gta ttt gag agt gat       240
Asn Leu Phe Phe Ser Thr Asp Val Glu Lys His Val Phe Glu Ser Asp
65                  70                  75                  80

att gta ttt gtc tca gtt aac act cca acc aaa aca caa ggt ctt ggt       288
Ile Val Phe Val Ser Val Asn Thr Pro Thr Lys Thr Gln Gly Leu Gly
                85                  90                  95

gct ggc aaa gct gct gat ctt act tac tgg gag agt gct gct cgg atg       336
Ala Gly Lys Ala Ala Asp Leu Thr Tyr Trp Glu Ser Ala Ala Arg Met
            100                 105                 110

atc gct gat gtc tcc aaa tct agc aaa atc gtt gtt gag aaa tcc acg       384
Ile Ala Asp Val Ser Lys Ser Ser Lys Ile Val Val Glu Lys Ser Thr
        115                 120                 125

gtt cct gtg agg aca gca gag gct att gaa aag ata ctg aca cat aac       432
Val Pro Val Arg Thr Ala Glu Ala Ile Glu Lys Ile Leu Thr His Asn
    130                 135                 140

agc aaa ggc ata gag ttt cag att ctc tct aac cct gaa ttt ctt gct       480
Ser Lys Gly Ile Glu Phe Gln Ile Leu Ser Asn Pro Glu Phe Leu Ala

145                 150                 155                 160

gag ggt act gca att aag gat ctt tat aac cca gac cgt gtg ttg att      528
Glu Gly Thr Ala Ile Lys Asp Leu Tyr Asn Pro Asp Arg Val Leu Ile
                165                 170                 175

ggt ggt agg gat act gca gca ggg caa aag gct att aaa gct tta aga      576
Gly Gly Arg Asp Thr Ala Ala Gly Gln Lys Ala Ile Lys Ala Leu Arg
            180                 185                 190

gat gtt tat gct cat tgg gtt cca gtg gaa caa atc att tgc acg aac      624
Asp Val Tyr Ala His Trp Val Pro Val Glu Gln Ile Ile Cys Thr Asn
        195                 200                 205

ctg tgg tcc gct gag ctc tct aag ctt gca gca aat gca ttc tta gct      672
Leu Trp Ser Ala Glu Leu Ser Lys Leu Ala Ala Asn Ala Phe Leu Ala
    210                 215                 220

cag agg ata tca tct gtc aat gcc atg tca gct cta tgt gag gca act      720
Gln Arg Ile Ser Ser Val Asn Ala Met Ser Ala Leu Cys Glu Ala Thr
225                 230                 235                 240

ggc gct gat gtt aca caa gtt gcg cat gcc gtg ggt aca gat act aga      768
Gly Ala Asp Val Thr Gln Val Ala His Ala Val Gly Thr Asp Thr Arg
                245                 250                 255

att ggt cca aag ttc ttg aat gct agt gtt ggt ttt ggt gga tca tgt      816
Ile Gly Pro Lys Phe Leu Asn Ala Ser Val Gly Phe Gly Gly Ser Cys
            260                 265                 270

ttc caa aag gac atc cta aat ctt atc tat att tgt gaa tgc aac ggc      864
Phe Gln Lys Asp Ile Leu Asn Leu Ile Tyr Ile Cys Glu Cys Asn Gly
        275                 280                 285

ttg ccc gaa gca gct aat tac tgg aaa caa gtc gta aag gtg aac gac      912
Leu Pro Glu Ala Ala Asn Tyr Trp Lys Gln Val Val Lys Val Asn Asp
    290                 295                 300

tat cag aaa ata cgg ttt gca aac cgg gtt gtt tct tca atg ttt aac      960
Tyr Gln Lys Ile Arg Phe Ala Asn Arg Val Val Ser Ser Met Phe Asn
305                 310                 315                 320

aca gtc tcg ggc aag aaa atc gcg atc ctc ggt ttt gcc ttc aag aag     1008
Thr Val Ser Gly Lys Lys Ile Ala Ile Leu Gly Phe Ala Phe Lys Lys
                325                 330                 335

gac aca ggt gac acg aga gag act cca gcg att gat gtt tgt aac aga     1056
Asp Thr Gly Asp Thr Arg Glu Thr Pro Ala Ile Asp Val Cys Asn Arg
            340                 345                 350

tta gtt gca gac aag gcc aag ctg agc ata tac gac cca caa gtt ctt     1104
Leu Val Ala Asp Lys Ala Lys Leu Ser Ile Tyr Asp Pro Gln Val Leu
        355                 360                 365

gaa gaa cag atc aga aga gat ctt tcc atg gct agg ttt gac tgg gac     1152
Glu Glu Gln Ile Arg Arg Asp Leu Ser Met Ala Arg Phe Asp Trp Asp
    370                 375                 380

cac cct gtt cct ctt cag cag att aaa gct gaa ggt atc tca gag caa     1200
His Pro Val Pro Leu Gln Gln Ile Lys Ala Glu Gly Ile Ser Glu Gln
385                 390                 395                 400

gtg aat gtc gtc tca gat gct tac gag gca act aaa gat gcg cac ggc     1248

Val Asn Val Val Ser Asp Ala Tyr Glu Ala Thr Lys Asp Ala His Gly
                405                 410                 415

cta tgt gtc tta acc gaa tgg gat gag ttt aaa tcc ttg gac ttc aag      1296
Leu Cys Val Leu Thr Glu Trp Asp Glu Phe Lys Ser Leu Asp Phe Lys
            420                 425                 430

aaa atc ttt gac aat atg cag aaa cca gct ttt gtg ttc gat ggt agg      1344
Lys Ile Phe Asp Asn Met Gln Lys Pro Ala Phe Val Phe Asp Gly Arg
        435                 440                 445

aat gtt gtt gat gca gtg aag ctg cgt gag atc ggt ttc atc gtc tac      1392
Asn Val Val Asp Ala Val Lys Leu Arg Glu Ile Gly Phe Ile Val Tyr
    450                 455                 460

tcc att ggt aaa ccg ctt gat tca tgg ctc aag gat atg cct gct gtg      1440
Ser Ile Gly Lys Pro Leu Asp Ser Trp Leu Lys Asp Met Pro Ala Val
465                 470                 475                 480

gca tga                                                              1446
Ala

<210>  118
<211>  481
<212>  PRT
<213>  Arabidopsis thaliana

<400>  118

Met Val Lys Ile Cys Cys Ile Gly Ala Gly Tyr Val Gly Gly Pro Thr
1               5                   10                  15

Met Ala Val Met Ala Leu Lys Cys Pro Glu Ile Glu Val Val Val Val
            20                  25                  30

Asp Ile Ser Glu Pro Arg Ile Asn Ala Trp Asn Ser Asp Arg Leu Pro
        35                  40                  45

Ile Tyr Glu Pro Gly Leu Glu Asp Val Val Lys Gln Cys Arg Gly Lys
    50                  55                  60

Asn Leu Phe Phe Ser Thr Asp Val Glu Lys His Val Phe Glu Ser Asp
65                  70                  75                  80

Ile Val Phe Val Ser Val Asn Thr Pro Thr Lys Thr Gln Gly Leu Gly
                85                  90                  95

Ala Gly Lys Ala Ala Asp Leu Thr Tyr Trp Glu Ser Ala Ala Arg Met
            100                 105                 110

Ile Ala Asp Val Ser Lys Ser Ser Lys Ile Val Val Glu Lys Ser Thr
        115                 120                 125

Val Pro Val Arg Thr Ala Glu Ala Ile Glu Lys Ile Leu Thr His Asn
    130                 135                 140

Ser Lys Gly Ile Glu Phe Gln Ile Leu Ser Asn Pro Glu Phe Leu Ala
145                 150                 155                 160

Glu Gly Thr Ala Ile Lys Asp Leu Tyr Asn Pro Asp Arg Val Leu Ile
                165                 170                 175

Gly Gly Arg Asp Thr Ala Ala Gly Gln Lys Ala Ile Lys Ala Leu Arg
            180                 185                 190

Asp Val Tyr Ala His Trp Val Pro Val Glu Gln Ile Ile Cys Thr Asn
        195                 200                 205

Leu Trp Ser Ala Glu Leu Ser Lys Leu Ala Ala Asn Ala Phe Leu Ala
    210                 215                 220

Gln Arg Ile Ser Ser Val Asn Ala Met Ser Ala Leu Cys Glu Ala Thr
225                 230                 235                 240

Gly Ala Asp Val Thr Gln Val Ala His Ala Val Gly Thr Asp Thr Arg
                245                 250                 255

Ile Gly Pro Lys Phe Leu Asn Ala Ser Val Gly Phe Gly Gly Ser Cys
            260                 265                 270

Phe Gln Lys Asp Ile Leu Asn Leu Ile Tyr Ile Cys Glu Cys Asn Gly
        275                 280                 285

Leu Pro Glu Ala Ala Asn Tyr Trp Lys Gln Val Val Lys Val Asn Asp
    290                 295                 300

Tyr Gln Lys Ile Arg Phe Ala Asn Arg Val Val Ser Ser Met Phe Asn
305                 310                 315                 320

Thr Val Ser Gly Lys Lys Ile Ala Ile Leu Gly Phe Ala Phe Lys Lys
                325                 330                 335

Asp Thr Gly Asp Thr Arg Glu Thr Pro Ala Ile Asp Val Cys Asn Arg
            340                 345                 350

Leu Val Ala Asp Lys Ala Lys Leu Ser Ile Tyr Asp Pro Gln Val Leu
        355                 360                 365

Glu Glu Gln Ile Arg Arg Asp Leu Ser Met Ala Arg Phe Asp Trp Asp
    370                 375                 380

His Pro Val Pro Leu Gln Gln Ile Lys Ala Glu Gly Ile Ser Glu Gln
385                 390                 395                 400

Val Asn Val Val Ser Asp Ala Tyr Glu Ala Thr Lys Asp Ala His Gly
                405                 410                 415

Leu Cys Val Leu Thr Glu Trp Asp Glu Phe Lys Ser Leu Asp Phe Lys
            420                 425                 430

Lys Ile Phe Asp Asn Met Gln Lys Pro Ala Phe Val Phe Asp Gly Arg
        435                 440                 445

Asn Val Val Asp Ala Val Lys Leu Arg Glu Ile Gly Phe Ile Val Tyr
    450                 455                 460

Ser Ile Gly Lys Pro Leu Asp Ser Trp Leu Lys Asp Met Pro Ala Val
465                 470                 475                 480

Ala

<210>  119
<211>  1374
<212>  DNA
<213>  Antirrhinum majus

<220>
<221>  CDS
<222>  (1)..(1374)
<223>  Gene encoding UDP-dependent glycosyltransferase

<300>
<308>  GenBank: AB362988.1
<309>  2010-04-09
<313>  (1)..(1374)

<400>  119
atg gag gac act atc gtt ctc tac gct tca gca gag cac ctt aac tcc      48
Met Glu Asp Thr Ile Val Leu Tyr Ala Ser Ala Glu His Leu Asn Ser
1               5                   10                  15

atg cta cta ctc ggc aaa ctc atc aac aaa cac cac ccc aca atc tcc      96
Met Leu Leu Leu Gly Lys Leu Ile Asn Lys His His Pro Thr Ile Ser
            20                  25                  30

gtc gcc att atc agc acc gcc cca aac gcc gcc gct agt tcc gtc gcc      144
Val Ala Ile Ile Ser Thr Ala Pro Asn Ala Ala Ala Ser Ser Val Ala
        35                  40                  45

gac gtg gcg gcc ata tct tat cag caa ctc aaa ccg gcc act ctc cct      192
Asp Val Ala Ala Ile Ser Tyr Gln Gln Leu Lys Pro Ala Thr Leu Pro
    50                  55                  60

tcg gat cta acc aaa aac cca atc gag ctc ttc ttc gaa atc cca cgt      240

Ser Asp Leu Thr Lys Asn Pro Ile Glu Leu Phe Phe Glu Ile Pro Arg
65                  70                  75                  80

cta cat aat cct aac ttg ctc gaa gcg ctg gaa gaa ctg tca cta aaa      288
Leu His Asn Pro Asn Leu Leu Glu Ala Leu Glu Glu Leu Ser Leu Lys
                85                  90                  95

tca aaa gta agg gca ttt gtg ata gat ttc ttt tgc aat ccc gca ttt      336
Ser Lys Val Arg Ala Phe Val Ile Asp Phe Phe Cys Asn Pro Ala Phe
            100                 105                 110

gag gtt tcg act agc ttg aac ata ccc act tac ttc tat gtc agc agc      384
Glu Val Ser Thr Ser Leu Asn Ile Pro Thr Tyr Phe Tyr Val Ser Ser
        115                 120                 125

ggc gcg ttt ggg cta tgc ggg ttc ttg cat ttt ccg aca atc gac gaa      432
Gly Ala Phe Gly Leu Cys Gly Phe Leu His Phe Pro Thr Ile Asp Glu
    130                 135                 140

act gtc gaa aaa gac atc ggt gaa ctg aac gat atc ttg gag atc ccg      480
Thr Val Glu Lys Asp Ile Gly Glu Leu Asn Asp Ile Leu Glu Ile Pro
145                 150                 155                 160

ggt tgc ccc ccg gtt ttg tcc tcg gat ttt ccg aaa ggt atg ttc ttt      528
Gly Cys Pro Pro Val Leu Ser Ser Asp Phe Pro Lys Gly Met Phe Phe
                165                 170                 175

cgc aag agt aac act tac aag cat ttt tta gac acg gcg aaa aac atg      576
Arg Lys Ser Asn Thr Tyr Lys His Phe Leu Asp Thr Ala Lys Asn Met
            180                 185                 190

agg aga gcg aaa ggg atc gtg gtg aac gcc ttc gac gcg atg gag ttc      624
Arg Arg Ala Lys Gly Ile Val Val Asn Ala Phe Asp Ala Met Glu Phe
        195                 200                 205

cga gct aaa gaa gcc ctc gtc aac aat ctg tgc gta ccc aat tcg cca      672
Arg Ala Lys Glu Ala Leu Val Asn Asn Leu Cys Val Pro Asn Ser Pro
    210                 215                 220

act ccc cca gtt ttc tta gtc ggc cca ttg gtc gga gca agc aca act      720
Thr Pro Pro Val Phe Leu Val Gly Pro Leu Val Gly Ala Ser Thr Thr
225                 230                 235                 240

acg aaa acc aca aac gaa cag cac gaa tgc ttg aaa tgg ctg gac gtg      768
Thr Lys Thr Thr Asn Glu Gln His Glu Cys Leu Lys Trp Leu Asp Val
                245                 250                 255

cag cca gac aga agc gtg atc ttc tta tgt ttc ggt agg agg ggt ttg      816
Gln Pro Asp Arg Ser Val Ile Phe Leu Cys Phe Gly Arg Arg Gly Leu
            260                 265                 270

ttc tcc gca gac caa ttg aag gaa atc gca att ggt ctg gag aac agc      864
Phe Ser Ala Asp Gln Leu Lys Glu Ile Ala Ile Gly Leu Glu Asn Ser
        275                 280                 285

ggc cac agg ttc ctg tgg tcc gtg cgt tgc cca cca agt aag cct aac      912
Gly His Arg Phe Leu Trp Ser Val Arg Cys Pro Pro Ser Lys Pro Asn
    290                 295                 300

tct tat aac act gat ccg gac ctg gac gag ctc ctg ccc gag ggg ttt      960
Ser Tyr Asn Thr Asp Pro Asp Leu Asp Glu Leu Leu Pro Glu Gly Phe
305                 310                 315                 320

ttg tcc agg acc gag acc cgg ggt ttc gtg atc aag tcg tgg gcg cct 1008
Leu Ser Arg Thr Glu Thr Arg Gly Phe Val Ile Lys Ser Trp Ala Pro
                325                 330                 335

cag aag gag gtg ctg agc cat ggc gcg gtt gga ggg ttc gtg acg cac 1056
Gln Lys Glu Val Leu Ser His Gly Ala Val Gly Gly Phe Val Thr His
            340                 345                 350

tgt ggg agg agt tcg ata ttg gaa gcg gtg tcg ttt ggg gtg ccg atg 1104
Cys Gly Arg Ser Ser Ile Leu Glu Ala Val Ser Phe Gly Val Pro Met
        355                 360                 365

atc ggg tgg ccg ata tac gcg gag cag agg atg aat agg gtg ttc atg 1152
Ile Gly Trp Pro Ile Tyr Ala Glu Gln Arg Met Asn Arg Val Phe Met
    370                 375                 380

gtg gag gag atg aag gtg gcg ttg cag ttg gat gag gtg gag gaa ggg 1200
Val Glu Glu Met Lys Val Ala Leu Gln Leu Asp Glu Val Glu Glu Gly
385                 390                 395                 400

ttc gtg gcg gcg gtg gaa ttg gag aag aga gtg aag gag ttg atg gat 1248
Phe Val Ala Ala Val Glu Leu Glu Lys Arg Val Lys Glu Leu Met Asp
                405                 410                 415

tcg aag aat ggg aga gcg gtt agg cag aga gtg aag gag atg aaa gtg 1296
Ser Lys Asn Gly Arg Ala Val Arg Gln Arg Val Lys Glu Met Lys Val
            420                 425                 430

gcg gct gag gtg gcg gtt gaa aag ggt ggt tcg tca gtt gtg gcg ttg 1344
Ala Ala Glu Val Ala Val Glu Lys Gly Gly Ser Ser Val Val Ala Leu
        435                 440                 445

caa cgc ttt gtt gat atg gtg gtt tct taa 1374
Gln Arg Phe Val Asp Met Val Val Ser
    450                 455

<210> 120
<211> 457
<212> PRT
<213> Antirrhinum majus

<400> 120

Met Glu Asp Thr Ile Val Leu Tyr Ala Ser Ala Glu His Leu Asn Ser
1               5                   10                  15

Met Leu Leu Leu Gly Lys Leu Ile Asn Lys His His Pro Thr Ile Ser
            20                  25                  30

Val Ala Ile Ile Ser Thr Ala Pro Asn Ala Ala Ala Ser Ser Val Ala
        35                  40                  45

Asp Val Ala Ala Ile Ser Tyr Gln Gln Leu Lys Pro Ala Thr Leu Pro
    50                  55                  60

Ser Asp Leu Thr Lys Asn Pro Ile Glu Leu Phe Phe Glu Ile Pro Arg
65                  70                  75                  80

Leu His Asn Pro Asn Leu Leu Glu Ala Leu Glu Glu Leu Ser Leu Lys
                85                  90                  95

Ser Lys Val Arg Ala Phe Val Ile Asp Phe Phe Cys Asn Pro Ala Phe
            100                 105                 110

Glu Val Ser Thr Ser Leu Asn Ile Pro Thr Tyr Phe Tyr Val Ser Ser
        115                 120                 125

Gly Ala Phe Gly Leu Cys Gly Phe Leu His Phe Pro Thr Ile Asp Glu
    130                 135                 140

Thr Val Glu Lys Asp Ile Gly Glu Leu Asn Asp Ile Leu Glu Ile Pro
145                 150                 155                 160

Gly Cys Pro Pro Val Leu Ser Ser Asp Phe Pro Lys Gly Met Phe Phe
                165                 170                 175

Arg Lys Ser Asn Thr Tyr Lys His Phe Leu Asp Thr Ala Lys Asn Met
            180                 185                 190

Arg Arg Ala Lys Gly Ile Val Val Asn Ala Phe Asp Ala Met Glu Phe
        195                 200                 205

Arg Ala Lys Glu Ala Leu Val Asn Asn Leu Cys Val Pro Asn Ser Pro
    210                 215                 220

Thr Pro Pro Val Phe Leu Val Gly Pro Leu Val Gly Ala Ser Thr Thr
225                 230                 235                 240

Thr Lys Thr Thr Asn Glu Gln His Glu Cys Leu Lys Trp Leu Asp Val
                245                 250                 255

Gln Pro Asp Arg Ser Val Ile Phe Leu Cys Phe Gly Arg Arg Gly Leu
            260                 265                 270

Phe Ser Ala Asp Gln Leu Lys Glu Ile Ala Ile Gly Leu Glu Asn Ser
        275                 280                 285

Gly His Arg Phe Leu Trp Ser Val Arg Cys Pro Pro Ser Lys Pro Asn
    290                 295                 300

Ser Tyr Asn Thr Asp Pro Asp Leu Asp Glu Leu Leu Pro Glu Gly Phe
305                 310                 315                 320

Leu Ser Arg Thr Glu Thr Arg Gly Phe Val Ile Lys Ser Trp Ala Pro
                325                 330                 335

Gln Lys Glu Val Leu Ser His Gly Ala Val Gly Gly Phe Val Thr His
            340                 345                 350

Cys Gly Arg Ser Ser Ile Leu Glu Ala Val Ser Phe Gly Val Pro Met
        355                 360                 365

Ile Gly Trp Pro Ile Tyr Ala Glu Gln Arg Met Asn Arg Val Phe Met
    370                 375                 380

Val Glu Glu Met Lys Val Ala Leu Gln Leu Asp Glu Val Glu Glu Gly
385                 390                 395                 400

Phe Val Ala Ala Val Glu Leu Glu Lys Arg Val Lys Glu Leu Met Asp
                405                 410                 415

Ser Lys Asn Gly Arg Ala Val Arg Gln Arg Val Lys Glu Met Lys Val
            420                 425                 430

Ala Ala Glu Val Ala Val Glu Lys Gly Gly Ser Ser Val Val Ala Leu
        435                 440                 445

Gln Arg Phe Val Asp Met Val Val Ser
    450                 455

<210>  121
<211>  1581
<212>  DNA
<213>  Scutellaria baicalensis

<220>
<221>  CDS
<222>  (1)..(1581)
<223>  Gene encoding flavone 8-hydroxylase

<300>
<308>  GenBank: MF363008.1
<309>  2017-09-05
<313>  (1)..(1581)

<400>  121
atg cta tat gac aac agc caa tac tta gcc atg gaa ttc agt tct gcc      48
Met Leu Tyr Asp Asn Ser Gln Tyr Leu Ala Met Glu Phe Ser Ser Ala
1               5                   10                  15

att tat gga gcc att gcc ttc ttt ctt ttc tta tac tat tgc ctg ctc      96
Ile Tyr Gly Ala Ile Ala Phe Phe Leu Phe Leu Tyr Tyr Cys Leu Leu
            20                  25                  30

tat aca aca tct tcc aag cca aat acc aga gct tac aaa gca ccg ccg      144
Tyr Thr Thr Ser Ser Lys Pro Asn Thr Arg Ala Tyr Lys Ala Pro Pro
        35                  40                  45

gag gct ggc ggg gcg cgc ctc ttt tcc ggc cat ctc cat ctc atg gct 192
Glu Ala Gly Gly Ala Arg Leu Phe Ser Gly His Leu His Leu Met Ala
    50                  55                  60

ggt ggc acc acc ggt gaa ctt ccc cac atc aac ttg gct aac tta gca 240
Gly Gly Thr Thr Gly Glu Leu Pro His Ile Asn Leu Ala Asn Leu Ala
65                  70                  75                  80

gat aaa cac ggc cca gtt ttc aca atc cgg ctg ggc gta aag cga gcc 288
Asp Lys His Gly Pro Val Phe Thr Ile Arg Leu Gly Val Lys Arg Ala
                85                  90                  95

ttg gtg gtg agc agt tgg gaa tca gcc aaa gaa ctg ttc act acc tgt 336
Leu Val Val Ser Ser Trp Glu Ser Ala Lys Glu Leu Phe Thr Thr Cys
            100                 105                 110

gac gtg gcg gtg tcg tcc cgt cca cgt atg aag gcg gcg aag ctc tta 384
Asp Val Ala Val Ser Ser Arg Pro Arg Met Lys Ala Ala Lys Leu Leu
        115                 120                 125

ggc tat gat ttc gcc atg ttt ggg ttc gcc cca tac ggt gca tac tgg 432
Gly Tyr Asp Phe Ala Met Phe Gly Phe Ala Pro Tyr Gly Ala Tyr Trp
    130                 135                 140

cgt gag ctg cga aaa ctg atc tca gtt gag ctt ctc tca act cgt aga 480
Arg Glu Leu Arg Lys Leu Ile Ser Val Glu Leu Leu Ser Thr Arg Arg
145                 150                 155                 160

atg gag ttg cag aaa gag gtt cga gat tca gaa act agg gag tcc att 528
Met Glu Leu Gln Lys Glu Val Arg Asp Ser Glu Thr Arg Glu Ser Ile
                165                 170                 175

aaa gag ctt tat aag ctt tgg gaa gag aga agt gag ggt tcg gac agt 576
Lys Glu Leu Tyr Lys Leu Trp Glu Glu Arg Ser Glu Gly Ser Asp Ser
            180                 185                 190

gtg ttg gtg gac atg aag cag tgg ttt ggg gac ttg aac ttg aat gtt 624
Val Leu Val Asp Met Lys Gln Trp Phe Gly Asp Leu Asn Leu Asn Val
        195                 200                 205

gtc ctg aga atg gtg gcg ggg aag aga tgg atc ggc ccg gag acg ggg 672
Val Leu Arg Met Val Ala Gly Lys Arg Trp Ile Gly Pro Glu Thr Gly
    210                 215                 220

cgg tgg agg gag gtg ctg agg gat ttc ttc tac ttg gcg ggg atg ttt 720
Arg Trp Arg Glu Val Leu Arg Asp Phe Phe Tyr Leu Ala Gly Met Phe
225                 230                 235                 240

gtt ccg gcc gat gcc ttt ccg ttt ctt ggg tgg ttg gat tta ggc gga 768
Val Pro Ala Asp Ala Phe Pro Phe Leu Gly Trp Leu Asp Leu Gly Gly
                245                 250                 255

cat gag aag aga atg agg caa act gct aaa gaa ttg gac gga att gtt 816
His Glu Lys Arg Met Arg Gln Thr Ala Lys Glu Leu Asp Gly Ile Val
            260                 265                 270

gga gga tgg gtg gcg gag cat cgg gaa aag gaa tat tcc ggc gaa gat 864
Gly Gly Trp Val Ala Glu His Arg Glu Lys Glu Tyr Ser Gly Glu Asp
        275                 280                 285

aag cca aag gat ttc gtc gat gtt atg ttg tct gtt gta caa ggt tca 912
Lys Pro Lys Asp Phe Val Asp Val Met Leu Ser Val Val Gln Gly Ser
    290                 295                 300

agt ctt cga gct gac tat gat gct gat acc atc atc aaa gca aca tgc 960
Ser Leu Arg Ala Asp Tyr Asp Ala Asp Thr Ile Ile Lys Ala Thr Cys
305                 310                 315                 320

gag gct ttg att gtg ggt gga agt gac act aca aca gtg atg cta ata 1008
Glu Ala Leu Ile Val Gly Gly Ser Asp Thr Thr Thr Val Met Leu Ile
                325                 330                 335

tgg aca ctt tct ttc cta ttg aat aat cgg cat gtt ctg aga aag gct 1056
Trp Thr Leu Ser Phe Leu Leu Asn Asn Arg His Val Leu Arg Lys Ala
            340                 345                 350

caa gaa gag ctg gac aag cat gta gga agg gaa aga cgt gta aac caa 1104
Gln Glu Glu Leu Asp Lys His Val Gly Arg Glu Arg Arg Val Asn Gln
        355                 360                 365

tca gac atc aac aac tta gtc tat ctt cag gca ata gtt aaa gag act 1152
Ser Asp Ile Asn Asn Leu Val Tyr Leu Gln Ala Ile Val Lys Glu Thr
    370                 375                 380

ttg aga tta tac cct gct ggc ccc att ggt ggg ata cga gag ttc acc 1200
Leu Arg Leu Tyr Pro Ala Gly Pro Ile Gly Gly Ile Arg Glu Phe Thr
385                 390                 395                 400

caa gat tgc caa gtc gga ggc tac cac gtt cct aaa ggc acg tgg ctg 1248
Gln Asp Cys Gln Val Gly Gly Tyr His Val Pro Lys Gly Thr Trp Leu
                405                 410                 415

atc gtg aac ttg tgg aag ttg cat cga gat ccc aag gtt tgg tca gaa 1296
Ile Val Asn Leu Trp Lys Leu His Arg Asp Pro Lys Val Trp Ser Glu
            420                 425                 430

gat tgt ttg gaa ttt agg cca gaa cga ttt cta aat aaa aac ata gat 1344
Asp Cys Leu Glu Phe Arg Pro Glu Arg Phe Leu Asn Lys Asn Ile Asp
        435                 440                 445

gtc agg ggt caa gat ttc gag ttg atc cca ttt ggt ggt ggt cgg agg 1392
Val Arg Gly Gln Asp Phe Glu Leu Ile Pro Phe Gly Gly Gly Arg Arg
    450                 455                 460

att tgc ccc ggg gct aat ttt ggg ctg cat atg ctg cat ttg gtg ttg 1440
Ile Cys Pro Gly Ala Asn Phe Gly Leu His Met Leu His Leu Val Leu
465                 470                 475                 480

gct aac ttg ctg caa gct ttc gag ctc acc act gtg tcc gat caa gtg 1488
Ala Asn Leu Leu Gln Ala Phe Glu Leu Thr Thr Val Ser Asp Gln Val
                485                 490                 495

att gat atg acg gag agt gct gga atg aca aat atg aaa gcc aca cca 1536
Ile Asp Met Thr Glu Ser Ala Gly Met Thr Asn Met Lys Ala Thr Pro
            500                 505                 510

ctt aat gtt ctt gtt gcc cca agg ttg tct ccc act ctt tac tag 1581
Leu Asn Val Leu Val Ala Pro Arg Leu Ser Pro Thr Leu Tyr
        515                 520                 525

<210> 122
<211> 526
<212> PRT
<213> Scutellaria baicalensis

<400>  122

Met Leu Tyr Asp Asn Ser Gln Tyr Leu Ala Met Glu Phe Ser Ser Ala
1               5                   10                  15

Ile Tyr Gly Ala Ile Ala Phe Phe Leu Phe Leu Tyr Tyr Cys Leu Leu
            20                  25                  30

Tyr Thr Thr Ser Ser Lys Pro Asn Thr Arg Ala Tyr Lys Ala Pro Pro
        35                  40                  45

Glu Ala Gly Gly Ala Arg Leu Phe Ser Gly His Leu His Leu Met Ala
    50                  55                  60

Gly Gly Thr Thr Gly Glu Leu Pro His Ile Asn Leu Ala Asn Leu Ala
65                  70                  75                  80

Asp Lys His Gly Pro Val Phe Thr Ile Arg Leu Gly Val Lys Arg Ala
                85                  90                  95

Leu Val Val Ser Ser Trp Glu Ser Ala Lys Glu Leu Phe Thr Thr Cys
            100                 105                 110

Asp Val Ala Val Ser Ser Arg Pro Arg Met Lys Ala Ala Lys Leu Leu
        115                 120                 125

Gly Tyr Asp Phe Ala Met Phe Gly Phe Ala Pro Tyr Gly Ala Tyr Trp
    130                 135                 140

Arg Glu Leu Arg Lys Leu Ile Ser Val Glu Leu Leu Ser Thr Arg Arg
145                 150                 155                 160

Met Glu Leu Gln Lys Glu Val Arg Asp Ser Glu Thr Arg Glu Ser Ile
                165                 170                 175

Lys Glu Leu Tyr Lys Leu Trp Glu Glu Arg Ser Glu Gly Ser Asp Ser
            180                 185                 190

Val Leu Val Asp Met Lys Gln Trp Phe Gly Asp Leu Asn Leu Asn Val
        195                 200                 205

Val Leu Arg Met Val Ala Gly Lys Arg Trp Ile Gly Pro Glu Thr Gly
    210                 215                 220

Arg Trp Arg Glu Val Leu Arg Asp Phe Phe Tyr Leu Ala Gly Met Phe
225                 230                 235                 240

Val Pro Ala Asp Ala Phe Pro Phe Leu Gly Trp Leu Asp Leu Gly Gly

245                 250                 255

His Glu Lys Arg Met Arg Gln Thr Ala Lys Glu Leu Asp Gly Ile Val
            260                 265                 270

Gly Gly Trp Val Ala Glu His Arg Glu Lys Glu Tyr Ser Gly Glu Asp
        275                 280                 285

Lys Pro Lys Asp Phe Val Asp Val Met Leu Ser Val Val Gln Gly Ser
    290                 295                 300

Ser Leu Arg Ala Asp Tyr Asp Ala Asp Thr Ile Ile Lys Ala Thr Cys
305                 310                 315                 320

Glu Ala Leu Ile Val Gly Gly Ser Asp Thr Thr Thr Val Met Leu Ile
                325                 330                 335

Trp Thr Leu Ser Phe Leu Leu Asn Asn Arg His Val Leu Arg Lys Ala
            340                 345                 350

Gln Glu Glu Leu Asp Lys His Val Gly Arg Glu Arg Arg Val Asn Gln
        355                 360                 365

Ser Asp Ile Asn Asn Leu Val Tyr Leu Gln Ala Ile Val Lys Glu Thr
    370                 375                 380

Leu Arg Leu Tyr Pro Ala Gly Pro Ile Gly Gly Ile Arg Glu Phe Thr
385                 390                 395                 400

Gln Asp Cys Gln Val Gly Gly Tyr His Val Pro Lys Gly Thr Trp Leu
                405                 410                 415

Ile Val Asn Leu Trp Lys Leu His Arg Asp Pro Lys Val Trp Ser Glu
            420                 425                 430

Asp Cys Leu Glu Phe Arg Pro Glu Arg Phe Leu Asn Lys Asn Ile Asp
        435                 440                 445

Val Arg Gly Gln Asp Phe Glu Leu Ile Pro Phe Gly Gly Gly Arg Arg
    450                 455                 460

Ile Cys Pro Gly Ala Asn Phe Gly Leu His Met Leu His Leu Val Leu
465                 470                 475                 480

Ala Asn Leu Leu Gln Ala Phe Glu Leu Thr Thr Val Ser Asp Gln Val
                485                 490                 495

Ile Asp Met Thr Glu Ser Ala Gly Met Thr Asn Met Lys Ala Thr Pro
            500                 505                 510

Leu Asn Val Leu Val Ala Pro Arg Leu Ser Pro Thr Leu Tyr
        515                 520                 525

<210>  123
<211>  363
<212>  DNA
<213>  Cucurbita maxima

<220>
<221>  CDS
<222>  (1)..(363)
<223>  Gene encoding 8-O-methyltransferase

<300>
<308>  NCBI Reference Sequence: XM_023130435.1
<309>  2017-11-29
<313>  (1)..(363)

<400>  123
atg gaa ggg aag ctg gat gag ttg ctt caa tct caa gct cat gtt tgg      48
Met Glu Gly Lys Leu Asp Glu Leu Leu Gln Ser Gln Ala His Val Trp
1               5                   10                  15

aac cat gtt ttg aaa ttt atg aac tcc atg gct ctt aaa tgt gct gtt      96
Asn His Val Leu Lys Phe Met Asn Ser Met Ala Leu Lys Cys Ala Val
            20                  25                  30

gaa ttg ggc ata ccg gat gtt atc cat agc cac gcc caa ccc atg acc      144
Glu Leu Gly Ile Pro Asp Val Ile His Ser His Ala Gln Pro Met Thr
        35                  40                  45

ctc tcc gat tta gtg gcg gcg ctc cgt att caa cct tca aag gct caa      192
Leu Ser Asp Leu Val Ala Ala Leu Arg Ile Gln Pro Ser Lys Ala Gln
    50                  55                  60

tac ctt ggc cgc ctc atg cgc ctc ctc gtc cat tct ggc ttc ttt gat      240
Tyr Leu Gly Arg Leu Met Arg Leu Leu Val His Ser Gly Phe Phe Asp
65                  70                  75                  80

gcc agc gag gaa gaa gac gtg aaa tac aga ctc act cca tcc tct cgc      288
Ala Ser Glu Glu Glu Asp Val Lys Tyr Arg Leu Thr Pro Ser Ser Arg
                85                  90                  95

ctt ctc ctc cgc cac acc cac acc act ttc caa atc aca ccc ttc ttg      336
Leu Leu Leu Arg His Thr His Thr Thr Phe Gln Ile Thr Pro Phe Leu
            100                 105                 110

ttt ctt agt ttg gat aaa act gct taa                                  363
Phe Leu Ser Leu Asp Lys Thr Ala
        115                 120

<210>  124
<211>  120
<212>  PRT
<213>  Cucurbita maxima

<400>  124

Met Glu Gly Lys Leu Asp Glu Leu Leu Gln Ser Gln Ala His Val Trp
1               5                   10                  15

Asn His Val Leu Lys Phe Met Asn Ser Met Ala Leu Lys Cys Ala Val
            20                  25                  30

Glu Leu Gly Ile Pro Asp Val Ile His Ser His Ala Gln Pro Met Thr
        35                  40                  45

Leu Ser Asp Leu Val Ala Ala Leu Arg Ile Gln Pro Ser Lys Ala Gln
    50                  55                  60

Tyr Leu Gly Arg Leu Met Arg Leu Leu Val His Ser Gly Phe Phe Asp
65                  70                  75                  80

Ala Ser Glu Glu Glu Asp Val Lys Tyr Arg Leu Thr Pro Ser Ser Arg
                85                  90                  95

Leu Leu Leu Arg His Thr His Thr Thr Phe Gln Ile Thr Pro Phe Leu
            100                 105                 110

Phe Leu Ser Leu Asp Lys Thr Ala
        115                 120

<210>  125
<211>  1704
<212>  DNA
<213>  Pueraria montana var. lobata

<220>
<221>  CDS
<222>  (1)..(1704)
<223>  Gene encoding 4-coumaroyl-CoA ligase

<300>
<308>  GenBank: KF233530.1
<309>  2014-05-05
<313>  (1)..(1704)

<400>  125
atg cta acg gta gct cct tct gtc gat gcc aaa gag gtg tct acc cca      48
Met Leu Thr Val Ala Pro Ser Val Asp Ala Lys Glu Val Ser Thr Pro
1               5                   10                  15

aaa act gag caa aac caa gtg tgt gat ccc caa act acc cat gtc ttc      96
Lys Thr Glu Gln Asn Gln Val Cys Asp Pro Gln Thr Thr His Val Phe
            20                  25                  30

aaa tca aaa tta cca gat ata cca atc tcc aac cac atc cct ctc cac      144
Lys Ser Lys Leu Pro Asp Ile Pro Ile Ser Asn His Ile Pro Leu His
        35                  40                  45

gcc tac tgc ttc gag aaa ctc tcc gac ttc gct gac cgc ccc tgt ctc     192
Ala Tyr Cys Phe Glu Lys Leu Ser Asp Phe Ala Asp Arg Pro Cys Leu
    50                  55                  60

atc gtc ggc gcc gcc tcc aaa acc tac acc tac gcc gag acg cac ctc     240
Ile Val Gly Ala Ala Ser Lys Thr Tyr Thr Tyr Ala Glu Thr His Leu
65                  70                  75                  80

att tcg cgc aag atc gcc gcc gga ttg tcc aac ctc gga atc cgc aaa     288
Ile Ser Arg Lys Ile Ala Ala Gly Leu Ser Asn Leu Gly Ile Arg Lys
                85                  90                  95

ggc gac gtg gtc atg atc ctc ctc cag aac tcc gcc gag ttt gtt ttc     336
Gly Asp Val Val Met Ile Leu Leu Gln Asn Ser Ala Glu Phe Val Phe
            100                 105                 110

tcc ttc ctc gcc gcc tcc atg atc ggc gcc gtc ggc acc acc gcg aac     384
Ser Phe Leu Ala Ala Ser Met Ile Gly Ala Val Gly Thr Thr Ala Asn
        115                 120                 125

ccg ttc tac act gcc gcg gag atc ttc aag cag ttc acc gtc tcc aag     432
Pro Phe Tyr Thr Ala Ala Glu Ile Phe Lys Gln Phe Thr Val Ser Lys
    130                 135                 140

gcg aag ctg atc ata acg caa gca atg tac gtg gac aag ctc cgc cac     480
Ala Lys Leu Ile Ile Thr Gln Ala Met Tyr Val Asp Lys Leu Arg His
145                 150                 155                 160

cac gac ggt acg aac ctc ggc gag gac ttc aag gtg gtg acc gtg gac     528
His Asp Gly Thr Asn Leu Gly Glu Asp Phe Lys Val Val Thr Val Asp
                165                 170                 175

gat ccg ccg gag aat tgc ctc cac ttc tcg gtg ctc tcg gag gcg aac     576
Asp Pro Pro Glu Asn Cys Leu His Phe Ser Val Leu Ser Glu Ala Asn
            180                 185                 190

gag agc gag gtg ccg gag gtg gag atc cac ccg gac gac gcg gtg gcg     624
Glu Ser Glu Val Pro Glu Val Glu Ile His Pro Asp Asp Ala Val Ala
        195                 200                 205

atg ccg ttc tcc tcc ggc acg acg ggt tta cct aaa gga gtg gtt ctc     672
Met Pro Phe Ser Ser Gly Thr Thr Gly Leu Pro Lys Gly Val Val Leu
    210                 215                 220

acg cac aag agt tta acg acg agc gtg gcg cag cag gtg gac gga gag     720
Thr His Lys Ser Leu Thr Thr Ser Val Ala Gln Gln Val Asp Gly Glu
225                 230                 235                 240

aac cct aac ctc tac ctc acc acc gaa gac gtg ctc ctc tgc gtg ctt     768
Asn Pro Asn Leu Tyr Leu Thr Thr Glu Asp Val Leu Leu Cys Val Leu
                245                 250                 255

ccg ctg ttt cac ata ttc tcg ctc aac agt gtg cta ttg tgc gcg ctc     816
Pro Leu Phe His Ile Phe Ser Leu Asn Ser Val Leu Leu Cys Ala Leu
            260                 265                 270

agg gcc ggg agt ggc gtt ttg ctg atg cag aag ttc gag att ggg acg     864
Arg Ala Gly Ser Gly Val Leu Leu Met Gln Lys Phe Glu Ile Gly Thr
        275                 280                 285

ctg ctg gag ctc ata cag cgg cac cgc gta tcg gtg gcg atg gtg gtg     912
Leu Leu Glu Leu Ile Gln Arg His Arg Val Ser Val Ala Met Val Val
    290                 295                 300

ccg ccg ctg gtg ctg gcg ctg gcc aag aat ccg atg gtg gcg gat ttt 960
Pro Pro Leu Val Leu Ala Leu Ala Lys Asn Pro Met Val Ala Asp Phe
305                 310                 315                 320

gac ctc agt tca ata cgg cta gtg ctg tcc gga gcg gct ccc ttg ggg 1008
Asp Leu Ser Ser Ile Arg Leu Val Leu Ser Gly Ala Ala Pro Leu Gly
                325                 330                 335

aag gaa ctc gag gag gct ctc cgg aac agg gtg cct caa gct gtt ttg 1056
Lys Glu Leu Glu Glu Ala Leu Arg Asn Arg Val Pro Gln Ala Val Leu
            340                 345                 350

gga cag ggg tac ggg atg act gaa gca gga cca gtg ctg tcc atg tgc 1104
Gly Gln Gly Tyr Gly Met Thr Glu Ala Gly Pro Val Leu Ser Met Cys
        355                 360                 365

ttg ggc ttt gca aag caa cca ttc cca aca aaa tca ggc tct tgt ggt 1152
Leu Gly Phe Ala Lys Gln Pro Phe Pro Thr Lys Ser Gly Ser Cys Gly
    370                 375                 380

acc gta gtc aga aat gca gaa ctc aag gtt gtt gac ccc gaa act ggt 1200
Thr Val Val Arg Asn Ala Glu Leu Lys Val Val Asp Pro Glu Thr Gly
385                 390                 395                 400

cgt tct ctt ggc tac aat caa ccc ggt gaa att tgc atc cga ggg caa 1248
Arg Ser Leu Gly Tyr Asn Gln Pro Gly Glu Ile Cys Ile Arg Gly Gln
                405                 410                 415

cag atc atg aaa gga tat ctg aac gat gag aaa gcg aca gca ttg acc 1296
Gln Ile Met Lys Gly Tyr Leu Asn Asp Glu Lys Ala Thr Ala Leu Thr
            420                 425                 430

ata gat tca gag ggt tgg ctt cac acc ggt gat gtt ggc tat gta gat 1344
Ile Asp Ser Glu Gly Trp Leu His Thr Gly Asp Val Gly Tyr Val Asp
        435                 440                 445

gat gat gat gaa att ttc att gtt gac agg gtg aaa gaa ctc atc aaa 1392
Asp Asp Asp Glu Ile Phe Ile Val Asp Arg Val Lys Glu Leu Ile Lys
    450                 455                 460

ttt aaa ggc ttc cag gtg ccc cct gca gaa ctt gaa ggg ctt ctt gta 1440
Phe Lys Gly Phe Gln Val Pro Pro Ala Glu Leu Glu Gly Leu Leu Val
465                 470                 475                 480

agc cat ccc tcc att gca gac gca gct gtt gtc cca caa aag gat gtt 1488
Ser His Pro Ser Ile Ala Asp Ala Ala Val Val Pro Gln Lys Asp Val
                485                 490                 495

gct gct ggt gaa gtt cct gtt gcc ttc gtt gtg aga tca aac ggc ttt 1536
Ala Ala Gly Glu Val Pro Val Ala Phe Val Val Arg Ser Asn Gly Phe
            500                 505                 510

gat cta act gaa gag gct gta aaa gag ttt ata gct aaa cag gtg gtg 1584
Asp Leu Thr Glu Glu Ala Val Lys Glu Phe Ile Ala Lys Gln Val Val
        515                 520                 525

ttc tac aaa aga ctg cac aaa gtt tat ttt gtt cat gct att ccc aag 1632
Phe Tyr Lys Arg Leu His Lys Val Tyr Phe Val His Ala Ile Pro Lys
    530                 535                 540

tct cca tca gga aag ata tta agg aaa gac ctc aga gca aag cta gaa 1680
Ser Pro Ser Gly Lys Ile Leu Arg Lys Asp Leu Arg Ala Lys Leu Glu

545                 550                 555                 560

agc gcc gcc act cag acg cct taa                                   1704
Ser Ala Ala Thr Gln Thr Pro
                565

<210>  126
<211>  567
<212>  PRT
<213>  Pueraria montana var. lobata

<400>  126

Met Leu Thr Val Ala Pro Ser Val Asp Ala Lys Glu Val Ser Thr Pro
1               5                   10                  15

Lys Thr Glu Gln Asn Gln Val Cys Asp Pro Gln Thr Thr His Val Phe
            20                  25                  30

Lys Ser Lys Leu Pro Asp Ile Pro Ile Ser Asn His Ile Pro Leu His
        35                  40                  45

Ala Tyr Cys Phe Glu Lys Leu Ser Asp Phe Ala Asp Arg Pro Cys Leu
    50                  55                  60

Ile Val Gly Ala Ala Ser Lys Thr Tyr Thr Tyr Ala Glu Thr His Leu
65                  70                  75                  80

Ile Ser Arg Lys Ile Ala Ala Gly Leu Ser Asn Leu Gly Ile Arg Lys
                85                  90                  95

Gly Asp Val Val Met Ile Leu Leu Gln Asn Ser Ala Glu Phe Val Phe
            100                 105                 110

Ser Phe Leu Ala Ala Ser Met Ile Gly Ala Val Gly Thr Thr Ala Asn
        115                 120                 125

Pro Phe Tyr Thr Ala Ala Glu Ile Phe Lys Gln Phe Thr Val Ser Lys
    130                 135                 140

Ala Lys Leu Ile Ile Thr Gln Ala Met Tyr Val Asp Lys Leu Arg His
145                 150                 155                 160

His Asp Gly Thr Asn Leu Gly Glu Asp Phe Lys Val Val Thr Val Asp
                165                 170                 175

Asp Pro Pro Glu Asn Cys Leu His Phe Ser Val Leu Ser Glu Ala Asn
            180                 185                 190

Glu Ser Glu Val Pro Glu Val Glu Ile His Pro Asp Asp Ala Val Ala

195                 200                 205

Met Pro Phe Ser Ser Gly Thr Thr Gly Leu Pro Lys Gly Val Val Leu
    210                 215                 220

Thr His Lys Ser Leu Thr Thr Ser Val Ala Gln Gln Val Asp Gly Glu
225                 230                 235                 240

Asn Pro Asn Leu Tyr Leu Thr Thr Glu Asp Val Leu Leu Cys Val Leu
                245                 250                 255

Pro Leu Phe His Ile Phe Ser Leu Asn Ser Val Leu Leu Cys Ala Leu
            260                 265                 270

Arg Ala Gly Ser Gly Val Leu Leu Met Gln Lys Phe Glu Ile Gly Thr
        275                 280                 285

Leu Leu Glu Leu Ile Gln Arg His Arg Val Ser Val Ala Met Val Val
    290                 295                 300

Pro Pro Leu Val Leu Ala Leu Ala Lys Asn Pro Met Val Ala Asp Phe
305                 310                 315                 320

Asp Leu Ser Ser Ile Arg Leu Val Leu Ser Gly Ala Ala Pro Leu Gly
                325                 330                 335

Lys Glu Leu Glu Glu Ala Leu Arg Asn Arg Val Pro Gln Ala Val Leu
            340                 345                 350

Gly Gln Gly Tyr Gly Met Thr Glu Ala Gly Pro Val Leu Ser Met Cys
        355                 360                 365

Leu Gly Phe Ala Lys Gln Pro Phe Pro Thr Lys Ser Gly Ser Cys Gly
    370                 375                 380

Thr Val Val Arg Asn Ala Glu Leu Lys Val Val Asp Pro Glu Thr Gly
385                 390                 395                 400

Arg Ser Leu Gly Tyr Asn Gln Pro Gly Glu Ile Cys Ile Arg Gly Gln
                405                 410                 415

Gln Ile Met Lys Gly Tyr Leu Asn Asp Glu Lys Ala Thr Ala Leu Thr
            420                 425                 430

Ile Asp Ser Glu Gly Trp Leu His Thr Gly Asp Val Gly Tyr Val Asp
        435                 440                 445

Asp Asp Asp Glu Ile Phe Ile Val Asp Arg Val Lys Glu Leu Ile Lys
    450                 455                 460

Phe Lys Gly Phe Gln Val Pro Pro Ala Glu Leu Glu Gly Leu Leu Val
465                 470                 475                 480

Ser His Pro Ser Ile Ala Asp Ala Ala Val Val Pro Gln Lys Asp Val
                485                 490                 495

Ala Ala Gly Glu Val Pro Val Ala Phe Val Val Arg Ser Asn Gly Phe
            500                 505                 510

Asp Leu Thr Glu Glu Ala Val Lys Glu Phe Ile Ala Lys Gln Val Val
        515                 520                 525

Phe Tyr Lys Arg Leu His Lys Val Tyr Phe Val His Ala Ile Pro Lys
    530                 535                 540

Ser Pro Ser Gly Lys Ile Leu Arg Lys Asp Leu Arg Ala Lys Leu Glu
545                 550                 555                 560

Ser Ala Ala Thr Gln Thr Pro
                565

<210>  127
<211>  1170
<212>  DNA
<213>  Pueraria montana var. lobata

<220>
<221>  CDS
<222>  (1)..(1170)
<223>  Gene encoding chalcone synthase

<300>
<308>  GenBank: D10223.1
<309>  2007-12-11
<313>  (1)..(1170)

<400>  127
atg gtg agc gta gct gag atc cgc cag gca caa agg gca gaa ggc cca        48
Met Val Ser Val Ala Glu Ile Arg Gln Ala Gln Arg Ala Glu Gly Pro
1               5                   10                  15

gca acc atc ctt gcc att gga act gca aac cca cca aac tgt gtt gat        96
Ala Thr Ile Leu Ala Ile Gly Thr Ala Asn Pro Pro Asn Cys Val Asp
            20                  25                  30

cag agc acc tat cct gat tac tac ttc aga atc acc aac agt gag cac       144
Gln Ser Thr Tyr Pro Asp Tyr Tyr Phe Arg Ile Thr Asn Ser Glu His
        35                  40                  45

atg acc gag ctc aaa gag aaa ttc cag cgc atg tgt gac aag tct atg       192
Met Thr Glu Leu Lys Glu Lys Phe Gln Arg Met Cys Asp Lys Ser Met

50                  55                  60

atc aag aag aga tac atg tac tta acc gaa gag atc ttg aaa gag aat        240
Ile Lys Lys Arg Tyr Met Tyr Leu Thr Glu Glu Ile Leu Lys Glu Asn
65                  70                  75                  80

cca aac atg tgt gct tac atg gca cct tct ttg gat gct agg caa gac        288
Pro Asn Met Cys Ala Tyr Met Ala Pro Ser Leu Asp Ala Arg Gln Asp
                85                  90                  95

atg gtg gtg gtg gag gta cca aaa cta ggg aaa gag gct gca aca aag        336
Met Val Val Val Glu Val Pro Lys Leu Gly Lys Glu Ala Ala Thr Lys
            100                 105                 110

gcc ata aag gag tgg ggc cag cca aag tca aag att acc cac ttg atc        384
Ala Ile Lys Glu Trp Gly Gln Pro Lys Ser Lys Ile Thr His Leu Ile
        115                 120                 125

ttt tgc acc aca agt ggt gtg gac atg cct ggt gct gat tac caa ctc        432
Phe Cys Thr Thr Ser Gly Val Asp Met Pro Gly Ala Asp Tyr Gln Leu
    130                 135                 140

acc aaa caa ttg ggc ctt cgc ccc tat gtg aag agg tac atg atg tac        480
Thr Lys Gln Leu Gly Leu Arg Pro Tyr Val Lys Arg Tyr Met Met Tyr
145                 150                 155                 160

caa caa ggt tgc ttt gca ggt ggc acg gtg ctt cgt ttg gcc aag gat        528
Gln Gln Gly Cys Phe Ala Gly Gly Thr Val Leu Arg Leu Ala Lys Asp
                165                 170                 175

ttg gct gag aac aac aag ggt gca cgt gtg tta gtt gtc tgt tct gag        576
Leu Ala Glu Asn Asn Lys Gly Ala Arg Val Leu Val Val Cys Ser Glu
            180                 185                 190

atc act gca gtc aca ttc cgt ggc cca agt gac act cac ctt gat agt        624
Ile Thr Ala Val Thr Phe Arg Gly Pro Ser Asp Thr His Leu Asp Ser
        195                 200                 205

ctt gtg ggc caa gca ttg ttt gga gat gga gcg gct gca gta att gtt        672
Leu Val Gly Gln Ala Leu Phe Gly Asp Gly Ala Ala Ala Val Ile Val
    210                 215                 220

ggt tct gac cca att cca cag gtt gag aag cct ttg tat gag ctg gtt        720
Gly Ser Asp Pro Ile Pro Gln Val Glu Lys Pro Leu Tyr Glu Leu Val
225                 230                 235                 240

tgg act gca caa aca att gct cca gac agt gaa ggg gct att gat gga        768
Trp Thr Ala Gln Thr Ile Ala Pro Asp Ser Glu Gly Ala Ile Asp Gly
                245                 250                 255

cac ctt cgt gaa gtt ggc ctc aca ttt cat ctc ctc aag gat gtt cct        816
His Leu Arg Glu Val Gly Leu Thr Phe His Leu Leu Lys Asp Val Pro
            260                 265                 270

ggg att gtc tca aag aac att gat aag gca ctt ttt gag gca ttc aac        864
Gly Ile Val Ser Lys Asn Ile Asp Lys Ala Leu Phe Glu Ala Phe Asn
        275                 280                 285

cca ctg aac atc tct gat tac aac tcc atc ttt tgg att gca cac cct        912
Pro Leu Asn Ile Ser Asp Tyr Asn Ser Ile Phe Trp Ile Ala His Pro
    290                 295                 300

ggt ggg cct gca att ttg gac caa gtt gag cag aag ttg ggt ctc aaa        960

Gly Gly Pro Ala Ile Leu Asp Gln Val Glu Gln Lys Leu Gly Leu Lys
305                 310                 315                 320

cct gag aag atg aag gcc act aga gat gtg ctt agt gac tat ggt aac       1008
Pro Glu Lys Met Lys Ala Thr Arg Asp Val Leu Ser Asp Tyr Gly Asn
                325                 330                 335

atg tca agt gct tgt gtt ctt ttc atc ttg gat gag atg agg agg aaa       1056
Met Ser Ser Ala Cys Val Leu Phe Ile Leu Asp Glu Met Arg Arg Lys
            340                 345                 350

tca gct gaa aac gga ctt aaa acc aca ggt gaa gga ctt gaa tgg ggt       1104
Ser Ala Glu Asn Gly Leu Lys Thr Thr Gly Glu Gly Leu Glu Trp Gly
        355                 360                 365

gtg ttg ttc ggt ttt gga cct gga ctt act att gag act gtt gtt ctg       1152
Val Leu Phe Gly Phe Gly Pro Gly Leu Thr Ile Glu Thr Val Val Leu
    370                 375                 380

cgt agt gtg gcc atc tga                                               1170
Arg Ser Val Ala Ile
385

<210>  128
<211>  389
<212>  PRT
<213>  Pueraria montana var. lobata

<400>  128

Met Val Ser Val Ala Glu Ile Arg Gln Ala Gln Arg Ala Glu Gly Pro
1               5                   10                  15

Ala Thr Ile Leu Ala Ile Gly Thr Ala Asn Pro Pro Asn Cys Val Asp
            20                  25                  30

Gln Ser Thr Tyr Pro Asp Tyr Tyr Phe Arg Ile Thr Asn Ser Glu His
        35                  40                  45

Met Thr Glu Leu Lys Glu Lys Phe Gln Arg Met Cys Asp Lys Ser Met
    50                  55                  60

Ile Lys Lys Arg Tyr Met Tyr Leu Thr Glu Glu Ile Leu Lys Glu Asn
65                  70                  75                  80

Pro Asn Met Cys Ala Tyr Met Ala Pro Ser Leu Asp Ala Arg Gln Asp
                85                  90                  95

Met Val Val Val Glu Val Pro Lys Leu Gly Lys Glu Ala Ala Thr Lys
            100                 105                 110

Ala Ile Lys Glu Trp Gly Gln Pro Lys Ser Lys Ile Thr His Leu Ile
        115                 120                 125

Phe Cys Thr Thr Ser Gly Val Asp Met Pro Gly Ala Asp Tyr Gln Leu
    130                 135                 140

Thr Lys Gln Leu Gly Leu Arg Pro Tyr Val Lys Arg Tyr Met Met Tyr
145                 150                 155                 160

Gln Gln Gly Cys Phe Ala Gly Gly Thr Val Leu Arg Leu Ala Lys Asp
                165                 170                 175

Leu Ala Glu Asn Asn Lys Gly Ala Arg Val Leu Val Val Cys Ser Glu
            180                 185                 190

Ile Thr Ala Val Thr Phe Arg Gly Pro Ser Asp Thr His Leu Asp Ser
        195                 200                 205

Leu Val Gly Gln Ala Leu Phe Gly Asp Gly Ala Ala Ala Val Ile Val
    210                 215                 220

Gly Ser Asp Pro Ile Pro Gln Val Glu Lys Pro Leu Tyr Glu Leu Val
225                 230                 235                 240

Trp Thr Ala Gln Thr Ile Ala Pro Asp Ser Glu Gly Ala Ile Asp Gly
                245                 250                 255

His Leu Arg Glu Val Gly Leu Thr Phe His Leu Leu Lys Asp Val Pro
            260                 265                 270

Gly Ile Val Ser Lys Asn Ile Asp Lys Ala Leu Phe Glu Ala Phe Asn
        275                 280                 285

Pro Leu Asn Ile Ser Asp Tyr Asn Ser Ile Phe Trp Ile Ala His Pro
    290                 295                 300

Gly Gly Pro Ala Ile Leu Asp Gln Val Glu Gln Lys Leu Gly Leu Lys
305                 310                 315                 320

Pro Glu Lys Met Lys Ala Thr Arg Asp Val Leu Ser Asp Tyr Gly Asn
                325                 330                 335

Met Ser Ser Ala Cys Val Leu Phe Ile Leu Asp Glu Met Arg Arg Lys
            340                 345                 350

Ser Ala Glu Asn Gly Leu Lys Thr Thr Gly Glu Gly Leu Glu Trp Gly
        355                 360                 365

Val Leu Phe Gly Phe Gly Pro Gly Leu Thr Ile Glu Thr Val Val Leu
    370                 375                 380

Arg Ser Val Ala Ile
385

<210> 129
<211> 948
<212> DNA
<213> Glycine max

<220>
<221> CDS
<222> (1)..(948)

<300>
<308> NCBI Reference Sequence: NM_001367006.1
<309> 2018-11-01
<313> (1)..(948)

<400> 129
atg gct gcc acc acc tta gtc tct gaa gtc act ctc ccc tcc tcc tcc 48
Met Ala Ala Thr Thr Leu Val Ser Glu Val Thr Leu Pro Ser Ser Ser
1               5                   10                  15

ggc cag cgg aag atg ccg ctg atg ggc ctc ggc acc gcg ccg gaa gca 96
Gly Gln Arg Lys Met Pro Leu Met Gly Leu Gly Thr Ala Pro Glu Ala
            20                  25                  30

acc agc gcg gtt acc aca aaa gat gct gtt ctt gag gcc atc aag cag 144
Thr Ser Ala Val Thr Thr Lys Asp Ala Val Leu Glu Ala Ile Lys Gln
        35                  40                  45

ggc tac agg cac ttt gat gct gct tct gct tat ggt gtg gaa cag tct 192
Gly Tyr Arg His Phe Asp Ala Ala Ser Ala Tyr Gly Val Glu Gln Ser
    50                  55                  60

gtg gga gaa gcc ata gct gaa gca ctt aag caa gga cta att gca tcc 240
Val Gly Glu Ala Ile Ala Glu Ala Leu Lys Gln Gly Leu Ile Ala Ser
65                  70                  75                  80

aga gat caa ctc ttc atc act acc aaa ttg tgg gtc act gat aac cat 288
Arg Asp Gln Leu Phe Ile Thr Thr Lys Leu Trp Val Thr Asp Asn His
                85                  90                  95

gcc cat act att ctt cct gcc ctg cag aaa tct ctc agg aca ctt caa 336
Ala His Thr Ile Leu Pro Ala Leu Gln Lys Ser Leu Arg Thr Leu Gln
            100                 105                 110

cta gaa tac ata gat ctc ttt ctg atc cac tgg ccc att gct act aag 384
Leu Glu Tyr Ile Asp Leu Phe Leu Ile His Trp Pro Ile Ala Thr Lys
        115                 120                 125

cct gga aaa gtt gtg tac ccc att gaa gta tca gag ata gtg gaa ttt 432
Pro Gly Lys Val Val Tyr Pro Ile Glu Val Ser Glu Ile Val Glu Phe
    130                 135                 140

gac atg aag ggt gtg tgg gga tcc atg gag gag tgc cag aga ctt ggc 480
Asp Met Lys Gly Val Trp Gly Ser Met Glu Glu Cys Gln Arg Leu Gly
145                 150                 155                 160

ctc acc aaa gcc att gga gtc agc aac ttc tcc atc aag aag ctt gaa 528
Leu Thr Lys Ala Ile Gly Val Ser Asn Phe Ser Ile Lys Lys Leu Glu

165                 170                 175

aaa ttg ctc tct ttt gct acc atc cct ccc gca gtg aat caa gta gaa       576
Lys Leu Leu Ser Phe Ala Thr Ile Pro Pro Ala Val Asn Gln Val Glu
            180                 185                 190

gtt aac ctt ggg tgg caa caa cag aag ctt aga gat ttc tgc aag gaa       624
Val Asn Leu Gly Trp Gln Gln Gln Lys Leu Arg Asp Phe Cys Lys Glu
        195                 200                 205

aag ggt att act gta act gcc ttc tca ccc ctg agg aag ggt gct agt       672
Lys Gly Ile Thr Val Thr Ala Phe Ser Pro Leu Arg Lys Gly Ala Ser
    210                 215                 220

agg ggt gct aat ttt gtg ctg gac aat gat gta atc aaa gaa ttg gca       720
Arg Gly Ala Asn Phe Val Leu Asp Asn Asp Val Ile Lys Glu Leu Ala
225                 230                 235                 240

gat gct cat ggc aag act gca gcc cag att tgt ctt cga tgg cta tat       768
Asp Ala His Gly Lys Thr Ala Ala Gln Ile Cys Leu Arg Trp Leu Tyr
                245                 250                 255

gaa caa ggg ttg act ttt gtt gtt aag agc tat gac aaa gag agg atg       816
Glu Gln Gly Leu Thr Phe Val Val Lys Ser Tyr Asp Lys Glu Arg Met
            260                 265                 270

aag caa aac ttg ggg att ttt gat tgg tca ctg act gag gat gat tac       864
Lys Gln Asn Leu Gly Ile Phe Asp Trp Ser Leu Thr Glu Asp Asp Tyr
        275                 280                 285

aag aaa ata agt gaa atc cac caa gag agg ctc atc aaa gga cca acc       912
Lys Lys Ile Ser Glu Ile His Gln Glu Arg Leu Ile Lys Gly Pro Thr
    290                 295                 300

aag cct ctt ctt gat gat cta tgg gat gaa gaa tga                       948
Lys Pro Leu Leu Asp Asp Leu Trp Asp Glu Glu
305                 310                 315


<210>  130
<211>  315
<212>  PRT
<213>  Glycine max

<400>  130

Met Ala Ala Thr Thr Leu Val Ser Glu Val Thr Leu Pro Ser Ser Ser
1               5                   10                  15


Gly Gln Arg Lys Met Pro Leu Met Gly Leu Gly Thr Ala Pro Glu Ala
            20                  25                  30


Thr Ser Ala Val Thr Thr Lys Asp Ala Val Leu Glu Ala Ile Lys Gln
        35                  40                  45


Gly Tyr Arg His Phe Asp Ala Ala Ser Ala Tyr Gly Val Glu Gln Ser
    50                  55                  60


Val Gly Glu Ala Ile Ala Glu Ala Leu Lys Gln Gly Leu Ile Ala Ser

65                  70                  75                  80

Arg Asp Gln Leu Phe Ile Thr Thr Lys Leu Trp Val Thr Asp Asn His
                85                  90                  95

Ala His Thr Ile Leu Pro Ala Leu Gln Lys Ser Leu Arg Thr Leu Gln
            100                 105                 110

Leu Glu Tyr Ile Asp Leu Phe Leu Ile His Trp Pro Ile Ala Thr Lys
        115                 120                 125

Pro Gly Lys Val Val Tyr Pro Ile Glu Val Ser Glu Ile Val Glu Phe
    130                 135                 140

Asp Met Lys Gly Val Trp Gly Ser Met Glu Glu Cys Gln Arg Leu Gly
145                 150                 155                 160

Leu Thr Lys Ala Ile Gly Val Ser Asn Phe Ser Ile Lys Lys Leu Glu
                165                 170                 175

Lys Leu Leu Ser Phe Ala Thr Ile Pro Pro Ala Val Asn Gln Val Glu
            180                 185                 190

Val Asn Leu Gly Trp Gln Gln Gln Lys Leu Arg Asp Phe Cys Lys Glu
        195                 200                 205

Lys Gly Ile Thr Val Thr Ala Phe Ser Pro Leu Arg Lys Gly Ala Ser
    210                 215                 220

Arg Gly Ala Asn Phe Val Leu Asp Asn Asp Val Ile Lys Glu Leu Ala
225                 230                 235                 240

Asp Ala His Gly Lys Thr Ala Ala Gln Ile Cys Leu Arg Trp Leu Tyr
                245                 250                 255

Glu Gln Gly Leu Thr Phe Val Val Lys Ser Tyr Asp Lys Glu Arg Met
            260                 265                 270

Lys Gln Asn Leu Gly Ile Phe Asp Trp Ser Leu Thr Glu Asp Asp Tyr
        275                 280                 285

Lys Lys Ile Ser Glu Ile His Gln Glu Arg Leu Ile Lys Gly Pro Thr
    290                 295                 300

Lys Pro Leu Leu Asp Asp Leu Trp Asp Glu Glu
305                 310                 315

<210>  131
<211>  675
<212>  DNA
<213>  Pueraria montana var. lobata

<220>
<221>  CDS
<222>  (1)..(675)
<223>  Gene encoding chalcone isomerase

<300>
<308>  GenBank: D63577.1
<309>  2003-01-15
<313>  (1)..(675)

<400>  131
atg gcg gca gca gca gca gta gca acc atc agc gct gtg cag gtg gag      48
Met Ala Ala Ala Ala Ala Val Ala Thr Ile Ser Ala Val Gln Val Glu
1               5                   10                  15

ttc ctt gag ttc cca gcg gtg gtt act tca cca gcc tcc ggc agg acc      96
Phe Leu Glu Phe Pro Ala Val Val Thr Ser Pro Ala Ser Gly Arg Thr
            20                  25                  30

tat ttc ctc ggc ggc gca ggg gag aga gga ttg acg att gag ggg aag      144
Tyr Phe Leu Gly Gly Ala Gly Glu Arg Gly Leu Thr Ile Glu Gly Lys
        35                  40                  45

ttc ata aag ttc aca ggc ata gga gta tac ctg gag gat aaa gcg gtg      192
Phe Ile Lys Phe Thr Gly Ile Gly Val Tyr Leu Glu Asp Lys Ala Val
    50                  55                  60

tca tca ctc gcc gct aag tgg aag ggt aag cct tcc gag gag ctg gtt      240
Ser Ser Leu Ala Ala Lys Trp Lys Gly Lys Pro Ser Glu Glu Leu Val
65                  70                  75                  80

gag acc ctt gac ttc tac agg gat atc att tca ggg ccg ttt gaa aag      288
Glu Thr Leu Asp Phe Tyr Arg Asp Ile Ile Ser Gly Pro Phe Glu Lys
                85                  90                  95

cta att aga ggg tcg aag att ctg cca ttg tct ggg gtg gaa tac tca      336
Leu Ile Arg Gly Ser Lys Ile Leu Pro Leu Ser Gly Val Glu Tyr Ser
            100                 105                 110

aag aag gtg atg gaa aac tgc gtg gca cac atg aag tct gtt ggg act      384
Lys Lys Val Met Glu Asn Cys Val Ala His Met Lys Ser Val Gly Thr
        115                 120                 125

tac ggt gat gct gaa gcc gca gcc att gaa aag ttt gct gaa gcc ttc      432
Tyr Gly Asp Ala Glu Ala Ala Ala Ile Glu Lys Phe Ala Glu Ala Phe
    130                 135                 140

aag aat gtg aat ttc caa ccc ggt gcc act gtt ttc tac agg caa tca      480
Lys Asn Val Asn Phe Gln Pro Gly Ala Thr Val Phe Tyr Arg Gln Ser
145                 150                 155                 160

ccc gat gga gta ttg ggg ctt agt ttc tct gaa gat gta aca ata cca      528
Pro Asp Gly Val Leu Gly Leu Ser Phe Ser Glu Asp Val Thr Ile Pro
                165                 170                 175

gac aat gag gct gca gtg ata gag aac aag gct gtg tca gcg gca gtg      576
Asp Asn Glu Ala Ala Val Ile Glu Asn Lys Ala Val Ser Ala Ala Val

180                 185                 190

ttg gag acc atg att ggc gaa cac gct gtt tcc cct gac tta aaa cgc       624
Leu Glu Thr Met Ile Gly Glu His Ala Val Ser Pro Asp Leu Lys Arg
        195                 200                 205

agt ttg gct tca cga ttg cct gct gta ttg agc cac ggc att ata gtc       672
Ser Leu Ala Ser Arg Leu Pro Ala Val Leu Ser His Gly Ile Ile Val
    210                 215                 220

tga                                                                   675


<210>  132
<211>  224
<212>  PRT
<213>  Pueraria montana var. lobata

<400>  132

Met Ala Ala Ala Ala Ala Val Ala Thr Ile Ser Ala Val Gln Val Glu
1               5                   10                  15


Phe Leu Glu Phe Pro Ala Val Val Thr Ser Pro Ala Ser Gly Arg Thr
            20                  25                  30


Tyr Phe Leu Gly Gly Ala Gly Glu Arg Gly Leu Thr Ile Glu Gly Lys
        35                  40                  45


Phe Ile Lys Phe Thr Gly Ile Gly Val Tyr Leu Glu Asp Lys Ala Val
    50                  55                  60


Ser Ser Leu Ala Ala Lys Trp Lys Gly Lys Pro Ser Glu Glu Leu Val
65                  70                  75                  80


Glu Thr Leu Asp Phe Tyr Arg Asp Ile Ile Ser Gly Pro Phe Glu Lys
                85                  90                  95


Leu Ile Arg Gly Ser Lys Ile Leu Pro Leu Ser Gly Val Glu Tyr Ser
            100                 105                 110


Lys Lys Val Met Glu Asn Cys Val Ala His Met Lys Ser Val Gly Thr
        115                 120                 125


Tyr Gly Asp Ala Glu Ala Ala Ala Ile Glu Lys Phe Ala Glu Ala Phe
    130                 135                 140


Lys Asn Val Asn Phe Gln Pro Gly Ala Thr Val Phe Tyr Arg Gln Ser
145                 150                 155                 160


Pro Asp Gly Val Leu Gly Leu Ser Phe Ser Glu Asp Val Thr Ile Pro
                165                 170                 175

Asp Asn Glu Ala Ala Val Ile Glu Asn Lys Ala Val Ser Ala Ala Val
            180                 185                 190

Leu Glu Thr Met Ile Gly Glu His Ala Val Ser Pro Asp Leu Lys Arg
        195                 200                 205

Ser Leu Ala Ser Arg Leu Pro Ala Val Leu Ser His Gly Ile Ile Val
    210                 215                 220

<210>  133
<211>  1566
<212>  DNA
<213>  Pueraria montana var. lobata

<220>
<221>  CDS
<222>  (1)..(1566)
<223>  Gene encoding 2-hydroxyisoflavanone synthase

<300>
<308>  GenBank: HQ219053.1
<309>  2011-06-09
<313>  (1)..(1566)

<400>  133
atg ttg ctg gaa ctt gca ctt ggt tta ttg gtg tta gct ttg ttt ctg       48
Met Leu Leu Glu Leu Ala Leu Gly Leu Leu Val Leu Ala Leu Phe Leu
1               5                   10                  15

cac ttg cgt ccc aca ccg act gca aaa tcc aaa gcc ctt cgc cac ctt       96
His Leu Arg Pro Thr Pro Thr Ala Lys Ser Lys Ala Leu Arg His Leu
            20                  25                  30

cta aac cct cct agt cca aag cct cgt ctt ccc ttc att ggt cac ctt      144
Leu Asn Pro Pro Ser Pro Lys Pro Arg Leu Pro Phe Ile Gly His Leu
        35                  40                  45

cac ctt ttg aaa gat caa ctt ctc cac cac gcc ctc atc gat ctc tcc      192
His Leu Leu Lys Asp Gln Leu Leu His His Ala Leu Ile Asp Leu Ser
    50                  55                  60

aaa aaa tat ggg ccc tta tac tct ctc tac ttt ggg tcc atg ccc act      240
Lys Lys Tyr Gly Pro Leu Tyr Ser Leu Tyr Phe Gly Ser Met Pro Thr
65                  70                  75                  80

gtt gtt gcc tcc acc cct gag ttg ttc aaa ctc ttt ctt caa acc cat      288
Val Val Ala Ser Thr Pro Glu Leu Phe Lys Leu Phe Leu Gln Thr His
                85                  90                  95

gag gcc act tcc ttc aac aca agg ttc caa acc tca gcc ata agg cgc      336
Glu Ala Thr Ser Phe Asn Thr Arg Phe Gln Thr Ser Ala Ile Arg Arg
            100                 105                 110

ctc act tac gac aac tct gtg gcc atg gtt ccc ttc gga cct tac tgg      384
Leu Thr Tyr Asp Asn Ser Val Ala Met Val Pro Phe Gly Pro Tyr Trp
        115                 120                 125

aag ttc atc agg aag ctc atc atg aac gac ctc ctc aac gcc acc act      432

Lys Phe Ile Arg Lys Leu Ile Met Asn Asp Leu Leu Asn Ala Thr Thr
    130                 135                 140

gtt aac aag ttg agg ccc ttg agg aca gaa cag atc cgc aag ttc ctc      480
Val Asn Lys Leu Arg Pro Leu Arg Thr Glu Gln Ile Arg Lys Phe Leu
145                 150                 155                 160

agg gtt atg gcc caa agt gca gag gcc caa cag ccc ctt aac gtt acc      528
Arg Val Met Ala Gln Ser Ala Glu Ala Gln Gln Pro Leu Asn Val Thr
                165                 170                 175

gag gag ctt cta aag tgg acc aac agc acc atc tcg atg atg atg ttg      576
Glu Glu Leu Leu Lys Trp Thr Asn Ser Thr Ile Ser Met Met Met Leu
            180                 185                 190

ggt gag gcc gaa gag atc aga gac atc gct cgt gag gtg ctt aag atc      624
Gly Glu Ala Glu Glu Ile Arg Asp Ile Ala Arg Glu Val Leu Lys Ile
        195                 200                 205

ttt ggg gaa tac agt gtt act aac ttc atc tgg ccc ttg atg tat ctc      672
Phe Gly Glu Tyr Ser Val Thr Asn Phe Ile Trp Pro Leu Met Tyr Leu
    210                 215                 220

aag gtt gga aag tat gag aag agg att gat gat ata ttc aac aag ttt      720
Lys Val Gly Lys Tyr Glu Lys Arg Ile Asp Asp Ile Phe Asn Lys Phe
225                 230                 235                 240

gac cct gtc att gaa agg gtt atc aag aag cgc cga gag atc gtc aga      768
Asp Pro Val Ile Glu Arg Val Ile Lys Lys Arg Arg Glu Ile Val Arg
                245                 250                 255

agg aga aag aac gga gaa gtt gtc gag ggt gag gcc agt ggg gtt ttc      816
Arg Arg Lys Asn Gly Glu Val Val Glu Gly Glu Ala Ser Gly Val Phe
            260                 265                 270

ctc gat act ttg ctt gaa ttc gct gag gac gag acc atg gag atc aaa      864
Leu Asp Thr Leu Leu Glu Phe Ala Glu Asp Glu Thr Met Glu Ile Lys
        275                 280                 285

att acc aag gag cag atc aag ggt ctt gtt gtc gac ttc ttc tca gca      912
Ile Thr Lys Glu Gln Ile Lys Gly Leu Val Val Asp Phe Phe Ser Ala
    290                 295                 300

ggg aca gat tca aca gcc gtg gca aca gag tgg gct ttg gca gag ctc      960
Gly Thr Asp Ser Thr Ala Val Ala Thr Glu Trp Ala Leu Ala Glu Leu
305                 310                 315                 320

atc aac aat cct agg gtg ttg caa aag gct cgg gat gag gtc tac agt     1008
Ile Asn Asn Pro Arg Val Leu Gln Lys Ala Arg Asp Glu Val Tyr Ser
                325                 330                 335

gtt gtg gga aaa gat aga ctt gtt gac gaa gtt gat act caa aac ctt     1056
Val Val Gly Lys Asp Arg Leu Val Asp Glu Val Asp Thr Gln Asn Leu
            340                 345                 350

cct tac att aga gcc att gtg aag gag aca ttc cgt atg cac cca cca     1104
Pro Tyr Ile Arg Ala Ile Val Lys Glu Thr Phe Arg Met His Pro Pro
        355                 360                 365

ctc cct gtg gtc aaa aga aag tgt gtg gaa gag tgt gag gtt aac ggc     1152
Leu Pro Val Val Lys Arg Lys Cys Val Glu Glu Cys Glu Val Asn Gly
    370                 375                 380

ttt gtg atc cct gag gga gct ttg att ctt ttc aat gtt tgg gct gta 1200
Phe Val Ile Pro Glu Gly Ala Leu Ile Leu Phe Asn Val Trp Ala Val
385                 390                 395                 400

gga aga gac cca aaa tac tgg gac aga cca ttg gaa ttt cgt cct gag 1248
Gly Arg Asp Pro Lys Tyr Trp Asp Arg Pro Leu Glu Phe Arg Pro Glu
                405                 410                 415

agg ttc tta gaa agt ggt gct gaa ggg gaa gta gga cct att gat ctt 1296
Arg Phe Leu Glu Ser Gly Ala Glu Gly Glu Val Gly Pro Ile Asp Leu
            420                 425                 430

agg ggc caa cat ttc caa ctt ctc cca ttt gga tct gga agg aga atg 1344
Arg Gly Gln His Phe Gln Leu Leu Pro Phe Gly Ser Gly Arg Arg Met
        435                 440                 445

tgc cct gga gtg aat ttg gct act tca gga atg gca aca ctg ctt gca 1392
Cys Pro Gly Val Asn Leu Ala Thr Ser Gly Met Ala Thr Leu Leu Ala
    450                 455                 460

tct ctt atc cag tgc ttt gac ctg caa gta gtg ggc cca aaa gga gaa 1440
Ser Leu Ile Gln Cys Phe Asp Leu Gln Val Val Gly Pro Lys Gly Glu
465                 470                 475                 480

ata ttg aaa ggt aaa gac gcc aaa gtg agc atg gaa gag agt gct ggc 1488
Ile Leu Lys Gly Lys Asp Ala Lys Val Ser Met Glu Glu Ser Ala Gly
                485                 490                 495

ctc acc gtt cca agg gca cat aat ctc agg tgt gtt cca ctt gca agg 1536
Leu Thr Val Pro Arg Ala His Asn Leu Arg Cys Val Pro Leu Ala Arg
            500                 505                 510

aca agc gtt gca gct aaa gtc ctt tct tga 1566
Thr Ser Val Ala Ala Lys Val Leu Ser
        515                 520


<210> 134
<211> 521
<212> PRT
<213> Pueraria montana var. lobata

<400> 134

Met Leu Leu Glu Leu Ala Leu Gly Leu Leu Val Leu Ala Leu Phe Leu
1               5                   10                  15


His Leu Arg Pro Thr Pro Thr Ala Lys Ser Lys Ala Leu Arg His Leu
            20                  25                  30


Leu Asn Pro Pro Ser Pro Lys Pro Arg Leu Pro Phe Ile Gly His Leu
        35                  40                  45


His Leu Leu Lys Asp Gln Leu Leu His His Ala Leu Ile Asp Leu Ser
    50                  55                  60


Lys Lys Tyr Gly Pro Leu Tyr Ser Leu Tyr Phe Gly Ser Met Pro Thr
65                  70                  75                  80

Val Val Ala Ser Thr Pro Glu Leu Phe Lys Leu Phe Leu Gln Thr His
                85                  90                  95

Glu Ala Thr Ser Phe Asn Thr Arg Phe Gln Thr Ser Ala Ile Arg Arg
            100                 105                 110

Leu Thr Tyr Asp Asn Ser Val Ala Met Val Pro Phe Gly Pro Tyr Trp
        115                 120                 125

Lys Phe Ile Arg Lys Leu Ile Met Asn Asp Leu Leu Asn Ala Thr Thr
    130                 135                 140

Val Asn Lys Leu Arg Pro Leu Arg Thr Glu Gln Ile Arg Lys Phe Leu
145                 150                 155                 160

Arg Val Met Ala Gln Ser Ala Glu Ala Gln Gln Pro Leu Asn Val Thr
                165                 170                 175

Glu Glu Leu Leu Lys Trp Thr Asn Ser Thr Ile Ser Met Met Met Leu
            180                 185                 190

Gly Glu Ala Glu Glu Ile Arg Asp Ile Ala Arg Glu Val Leu Lys Ile
        195                 200                 205

Phe Gly Glu Tyr Ser Val Thr Asn Phe Ile Trp Pro Leu Met Tyr Leu
    210                 215                 220

Lys Val Gly Lys Tyr Glu Lys Arg Ile Asp Asp Ile Phe Asn Lys Phe
225                 230                 235                 240

Asp Pro Val Ile Glu Arg Val Ile Lys Lys Arg Arg Glu Ile Val Arg
                245                 250                 255

Arg Arg Lys Asn Gly Glu Val Val Glu Gly Glu Ala Ser Gly Val Phe
            260                 265                 270

Leu Asp Thr Leu Leu Glu Phe Ala Glu Asp Glu Thr Met Glu Ile Lys
        275                 280                 285

Ile Thr Lys Glu Gln Ile Lys Gly Leu Val Val Asp Phe Phe Ser Ala
    290                 295                 300

Gly Thr Asp Ser Thr Ala Val Ala Thr Glu Trp Ala Leu Ala Glu Leu
305                 310                 315                 320

Ile Asn Asn Pro Arg Val Leu Gln Lys Ala Arg Asp Glu Val Tyr Ser
                325                 330                 335

Val Val Gly Lys Asp Arg Leu Val Asp Glu Val Asp Thr Gln Asn Leu
            340                 345                 350

Pro Tyr Ile Arg Ala Ile Val Lys Glu Thr Phe Arg Met His Pro Pro
        355                 360                 365

Leu Pro Val Val Lys Arg Lys Cys Val Glu Glu Cys Glu Val Asn Gly
    370                 375                 380

Phe Val Ile Pro Glu Gly Ala Leu Ile Leu Phe Asn Val Trp Ala Val
385                 390                 395                 400

Gly Arg Asp Pro Lys Tyr Trp Asp Arg Pro Leu Glu Phe Arg Pro Glu
                405                 410                 415

Arg Phe Leu Glu Ser Gly Ala Glu Gly Glu Val Gly Pro Ile Asp Leu
            420                 425                 430

Arg Gly Gln His Phe Gln Leu Leu Pro Phe Gly Ser Gly Arg Arg Met
        435                 440                 445

Cys Pro Gly Val Asn Leu Ala Thr Ser Gly Met Ala Thr Leu Leu Ala
    450                 455                 460

Ser Leu Ile Gln Cys Phe Asp Leu Gln Val Val Gly Pro Lys Gly Glu
465                 470                 475                 480

Ile Leu Lys Gly Lys Asp Ala Lys Val Ser Met Glu Glu Ser Ala Gly
                485                 490                 495

Leu Thr Val Pro Arg Ala His Asn Leu Arg Cys Val Pro Leu Ala Arg
            500                 505                 510

Thr Ser Val Ala Ala Lys Val Leu Ser
        515                 520

<210>  135
<211>  978
<212>  DNA
<213>  Pueraria montana var. lobata

<220>
<221>  CDS
<222>  (1)..(978)
<223>  Gene encoding 2-hydroxyisoflavanone dehydratase

<300>
<308>  GenBank: HQ219054.1

<309>  2011-06-09
<313>  (1)..(978)

<400>  135
atg gcg aac gag aat tcg aac aag gaa ata gtg aaa gag gtt ctt cct      48
Met Ala Asn Glu Asn Ser Asn Lys Glu Ile Val Lys Glu Val Leu Pro
1               5                   10                  15

ctt att cga gtg tac aag gat ggc acc gtg gag cgt ctt ctg agc tct      96
Leu Ile Arg Val Tyr Lys Asp Gly Thr Val Glu Arg Leu Leu Ser Ser
            20                  25                  30

cca aac gtg gca gcc tcc cct gaa gac ccc gaa aca gga gtc tca tcc      144
Pro Asn Val Ala Ala Ser Pro Glu Asp Pro Glu Thr Gly Val Ser Ser
        35                  40                  45

aaa gac ata gtc atc gca cac aac ccc tac gtc tcc gct cgc att ttc      192
Lys Asp Ile Val Ile Ala His Asn Pro Tyr Val Ser Ala Arg Ile Phe
    50                  55                  60

ctt cct aat att aat aaa tcc cac aac aaa ctc ccc atc ttc gtt tac      240
Leu Pro Asn Ile Asn Lys Ser His Asn Lys Leu Pro Ile Phe Val Tyr
65                  70                  75                  80

ttc cac ggt ggc gcc ttc tgc gtc gaa tca gct ttc tcc ttt ttc gtc      288
Phe His Gly Gly Ala Phe Cys Val Glu Ser Ala Phe Ser Phe Phe Val
                85                  90                  95

cac cgc tat ctc aac atc tta gcc tcc caa gcc aac ata ata gcc gtg      336
His Arg Tyr Leu Asn Ile Leu Ala Ser Gln Ala Asn Ile Ile Ala Val
            100                 105                 110

tcc gtc gac ttc aga ctc ctt cca cac cac cct ctc ccc gca gcc tac      384
Ser Val Asp Phe Arg Leu Leu Pro His His Pro Leu Pro Ala Ala Tyr
        115                 120                 125

gaa gac ggt tgg acc act ctc cag tgg att gct tcc cac gcc aac aac      432
Glu Asp Gly Trp Thr Thr Leu Gln Trp Ile Ala Ser His Ala Asn Asn
    130                 135                 140

acc gcc aca aac ccc gag cca tgg tta ctc aac cac gcc gac ttc aac      480
Thr Ala Thr Asn Pro Glu Pro Trp Leu Leu Asn His Ala Asp Phe Asn
145                 150                 155                 160

aaa ctc tac gta gga ggt gaa acc agc ggt gct aac ctt gca cac aac      528
Lys Leu Tyr Val Gly Gly Glu Thr Ser Gly Ala Asn Leu Ala His Asn
                165                 170                 175

ttg ctc ctc cgt gcc ggg aac ggg aac caa tcc ctc cct ggg gat ctc      576
Leu Leu Leu Arg Ala Gly Asn Gly Asn Gln Ser Leu Pro Gly Asp Leu
            180                 185                 190

aaa ata ttg ggt gga tta ctc tgc tgt ccc ttc ttc tgg ggc tcg aag      624
Lys Ile Leu Gly Gly Leu Leu Cys Cys Pro Phe Phe Trp Gly Ser Lys
        195                 200                 205

cca att ggg tca gag cct gtg gat gag cac gag cag agt ttg gcc atg      672
Pro Ile Gly Ser Glu Pro Val Asp Glu His Glu Gln Ser Leu Ala Met
    210                 215                 220

aag gtg tgg aac ttg gcc tgc cct gat gca ccc ggt gga atc gat aac      720
Lys Val Trp Asn Leu Ala Cys Pro Asp Ala Pro Gly Gly Ile Asp Asn
225                 230                 235                 240

cca tgg atc aac cca tgt gtt gct ggg gca cct tcc ttg gct acg ctt 768
Pro Trp Ile Asn Pro Cys Val Ala Gly Ala Pro Ser Leu Ala Thr Leu
                245                 250                 255

ggc tgc tcc aag ttg ctg gtt act atc act ggg agg gac gag ttc aga 816
Gly Cys Ser Lys Leu Leu Val Thr Ile Thr Gly Arg Asp Glu Phe Arg
            260                 265                 270

gac aga gat att ctg tac cac gac act gtt aag aaa agt ggg tgg gaa 864
Asp Arg Asp Ile Leu Tyr His Asp Thr Val Lys Lys Ser Gly Trp Glu
        275                 280                 285

ggc caa ctg gaa ctc ttt gat gct ggg gat gag gag cat gct ttc cag 912
Gly Gln Leu Glu Leu Phe Asp Ala Gly Asp Glu Glu His Ala Phe Gln
    290                 295                 300

ctc ttc aag cct gaa act gat acg gct aaa gcc atg atc aaa cgc ttg 960
Leu Phe Lys Pro Glu Thr Asp Thr Ala Lys Ala Met Ile Lys Arg Leu
305                 310                 315                 320

gct tct ttt ctc gtc tag 978
Ala Ser Phe Leu Val
                325

<210> 136
<211> 325
<212> PRT
<213> Pueraria montana var. lobata

<400> 136

Met Ala Asn Glu Asn Ser Asn Lys Glu Ile Val Lys Glu Val Leu Pro
1               5                   10                  15

Leu Ile Arg Val Tyr Lys Asp Gly Thr Val Glu Arg Leu Leu Ser Ser
            20                  25                  30

Pro Asn Val Ala Ala Ser Pro Glu Asp Pro Glu Thr Gly Val Ser Ser
        35                  40                  45

Lys Asp Ile Val Ile Ala His Asn Pro Tyr Val Ser Ala Arg Ile Phe
    50                  55                  60

Leu Pro Asn Ile Asn Lys Ser His Asn Lys Leu Pro Ile Phe Val Tyr
65                  70                  75                  80

Phe His Gly Gly Ala Phe Cys Val Glu Ser Ala Phe Ser Phe Phe Val
                85                  90                  95

His Arg Tyr Leu Asn Ile Leu Ala Ser Gln Ala Asn Ile Ile Ala Val
            100                 105                 110

Ser Val Asp Phe Arg Leu Leu Pro His His Pro Leu Pro Ala Ala Tyr
        115                 120                 125

Glu Asp Gly Trp Thr Thr Leu Gln Trp Ile Ala Ser His Ala Asn Asn
    130                 135                 140

Thr Ala Thr Asn Pro Glu Pro Trp Leu Leu Asn His Ala Asp Phe Asn
145                 150                 155                 160

Lys Leu Tyr Val Gly Gly Glu Thr Ser Gly Ala Asn Leu Ala His Asn
                165                 170                 175

Leu Leu Leu Arg Ala Gly Asn Gly Asn Gln Ser Leu Pro Gly Asp Leu
            180                 185                 190

Lys Ile Leu Gly Gly Leu Leu Cys Cys Pro Phe Phe Trp Gly Ser Lys
        195                 200                 205

Pro Ile Gly Ser Glu Pro Val Asp Glu His Glu Gln Ser Leu Ala Met
    210                 215                 220

Lys Val Trp Asn Leu Ala Cys Pro Asp Ala Pro Gly Gly Ile Asp Asn
225                 230                 235                 240

Pro Trp Ile Asn Pro Cys Val Ala Gly Ala Pro Ser Leu Ala Thr Leu
                245                 250                 255

Gly Cys Ser Lys Leu Leu Val Thr Ile Thr Gly Arg Asp Glu Phe Arg
            260                 265                 270

Asp Arg Asp Ile Leu Tyr His Asp Thr Val Lys Lys Ser Gly Trp Glu
        275                 280                 285

Gly Gln Leu Glu Leu Phe Asp Ala Gly Asp Glu Glu His Ala Phe Gln
    290                 295                 300

Leu Phe Lys Pro Glu Thr Asp Thr Ala Lys Ala Met Ile Lys Arg Leu
305                 310                 315                 320

Ala Ser Phe Leu Val
                325

<210>  137
<211>  1410
<212>  DNA
<213>  Pueraria montana var. lobata

<220>
<221>  CDS
<222>  (1)..(1410)

<223>  Gene encoding 8-C-glucosyltransferase

<300>
<308>  GenBank: KU317801.2
<309>  2016-08-18
<313>  (1)..(1410)

<400>  137
atg act aga tat gaa gtg gtt ttc atc gct atc cca acc ctt ggc aac      48
Met Thr Arg Tyr Glu Val Val Phe Ile Ala Ile Pro Thr Leu Gly Asn
1               5                   10                  15

ctc gtt cca caa gtg gaa ttc gcc aac ctc cta acc aaa cat gac cct      96
Leu Val Pro Gln Val Glu Phe Ala Asn Leu Leu Thr Lys His Asp Pro
            20                  25                  30

cga ttc tct gca acc att ctc act gtc tcc atg ccg caa cga ccc ctc      144
Arg Phe Ser Ala Thr Ile Leu Thr Val Ser Met Pro Gln Arg Pro Leu
        35                  40                  45

atg aac acc tac gtc caa gcc cgc gct tcc tca gcc gct aat atc aaa      192
Met Asn Thr Tyr Val Gln Ala Arg Ala Ser Ser Ala Ala Asn Ile Lys
    50                  55                  60

ctc ctc cag ctc cct atc gta gac ccg ccc gca ccc gag cag tac caa      240
Leu Leu Gln Leu Pro Ile Val Asp Pro Pro Ala Pro Glu Gln Tyr Gln
65                  70                  75                  80

acc ttg gtc ggc ttc ctc tcc ctc cac atg caa aat cac aaa cac cac      288
Thr Leu Val Gly Phe Leu Ser Leu His Met Gln Asn His Lys His His
                85                  90                  95

gtg aaa cac gca ctc ctc aac ctc atg aaa aca acc gag tcg aac tcg      336
Val Lys His Ala Leu Leu Asn Leu Met Lys Thr Thr Glu Ser Asn Ser
            100                 105                 110

tcc aac tcg gtt cga ctc gcg gcc atc ttc gtc gac atg ttc tct acc      384
Ser Asn Ser Val Arg Leu Ala Ala Ile Phe Val Asp Met Phe Ser Thr
        115                 120                 125

acc ctc atc gac gtc gcc gcc gag ctt gcc gtc ccg tgc tac ctc ttc      432
Thr Leu Ile Asp Val Ala Ala Glu Leu Ala Val Pro Cys Tyr Leu Phe
    130                 135                 140

ttc gcc tcg ccg gcg agc tgc ctc ggc ttc acg ctc gac ctc ccc aga      480
Phe Ala Ser Pro Ala Ser Cys Leu Gly Phe Thr Leu Asp Leu Pro Arg
145                 150                 155                 160

ttc gac ctg gct gag tca aaa tct gag ttc aca gtt ccg tgt ttt aaa      528
Phe Asp Leu Ala Glu Ser Lys Ser Glu Phe Thr Val Pro Cys Phe Lys
                165                 170                 175

aac ctt ttg ccc cgt tcc gtt ttt ccg aac ctc gtg ttg gac gcg aaa      576
Asn Leu Leu Pro Arg Ser Val Phe Pro Asn Leu Val Leu Asp Ala Lys
            180                 185                 190

gac ggg acc ttt tgg ctc tca tac cat gcg aga agg tac aaa gaa aca      624
Asp Gly Thr Phe Trp Leu Ser Tyr His Ala Arg Arg Tyr Lys Glu Thr
        195                 200                 205

aag ggc ata gtc ata aat acg ctt cag gag ctt gaa act cac gcg ctt      672
Lys Gly Ile Val Ile Asn Thr Leu Gln Glu Leu Glu Thr His Ala Leu
    210                 215                 220

caa tca ctc cac aat gat tcg cag ttg cag cgc gtt tac cca att ggg 720
Gln Ser Leu His Asn Asp Ser Gln Leu Gln Arg Val Tyr Pro Ile Gly
225                 230                 235                 240

ccg att ctt gac ctt gtt gga tcg gcc caa tgg gat ccc aac ccg gcc 768
Pro Ile Leu Asp Leu Val Gly Ser Ala Gln Trp Asp Pro Asn Pro Ala
                245                 250                 255

cag tat aaa cga atc atg gag tgg ctt gat cag cag cct ttg tct tcc 816
Gln Tyr Lys Arg Ile Met Glu Trp Leu Asp Gln Gln Pro Leu Ser Ser
            260                 265                 270

gtt gtg ctt ctc tgc ttc ggg agc atg gga agc tta gag gcg aat cag 864
Val Val Leu Leu Cys Phe Gly Ser Met Gly Ser Leu Glu Ala Asn Gln
        275                 280                 285

gtg gag gag atc gca att ggg ctc gaa cgg gca ggg gtt agg ttc ttg 912
Val Glu Glu Ile Ala Ile Gly Leu Glu Arg Ala Gly Val Arg Phe Leu
    290                 295                 300

tgg gct ttg agg gag tct cca aag gcc caa tta gaa tac ccg aga gat 960
Trp Ala Leu Arg Glu Ser Pro Lys Ala Gln Leu Glu Tyr Pro Arg Asp
305                 310                 315                 320

tac gaa aat cac aag gat gtg cta cca gat gga ttt ttg gaa cgt aca 1008
Tyr Glu Asn His Lys Asp Val Leu Pro Asp Gly Phe Leu Glu Arg Thr
                325                 330                 335

aat aac att ggt ttg gta tgt ggg tgg gtc cca caa gcg gtt gtg ttg 1056
Asn Asn Ile Gly Leu Val Cys Gly Trp Val Pro Gln Ala Val Val Leu
            340                 345                 350

gcc cac aag gcc gtt ggg ggg ttc gtg tcg cat tgt ggt tgg aac tcg 1104
Ala His Lys Ala Val Gly Gly Phe Val Ser His Cys Gly Trp Asn Ser
        355                 360                 365

ata tta gag agc ttg tgg cac ggc gtg ccg gtt gcc acg tgg ccg ttg 1152
Ile Leu Glu Ser Leu Trp His Gly Val Pro Val Ala Thr Trp Pro Leu
    370                 375                 380

tac tca gag caa caa atg aac gcg ttt cag atg gtt agg gat ctg gga 1200
Tyr Ser Glu Gln Gln Met Asn Ala Phe Gln Met Val Arg Asp Leu Gly
385                 390                 395                 400

tta gcg gtg gag att agc gtg gat tat agg gtg ggt gcg gat ctg gtg 1248
Leu Ala Val Glu Ile Ser Val Asp Tyr Arg Val Gly Ala Asp Leu Val
                405                 410                 415

cgg gcg gaa gaa gtg gag aat ggt ttg agg tcg ctg atg aag ggt ggt 1296
Arg Ala Glu Glu Val Glu Asn Gly Leu Arg Ser Leu Met Lys Gly Gly
            420                 425                 430

gat gag atc cga aga aag gtg aag gag atg agt gac acg tgt aga ggc 1344
Asp Glu Ile Arg Arg Lys Val Lys Glu Met Ser Asp Thr Cys Arg Gly
        435                 440                 445

gcg ttg ctg gag aat ggg tca tct tac agt aac cta gtg tcc ctg att 1392
Ala Leu Leu Glu Asn Gly Ser Ser Tyr Ser Asn Leu Val Ser Leu Ile
    450                 455                 460

caa gag ttg aca agt taa 1410
Gln Glu Leu Thr Ser

465

<210>  138
<211>  469
<212>  PRT
<213>  Pueraria montana var. lobata

<400>  138

Met Thr Arg Tyr Glu Val Val Phe Ile Ala Ile Pro Thr Leu Gly Asn
1               5                   10                  15

Leu Val Pro Gln Val Glu Phe Ala Asn Leu Leu Thr Lys His Asp Pro
            20                  25                  30

Arg Phe Ser Ala Thr Ile Leu Thr Val Ser Met Pro Gln Arg Pro Leu
        35                  40                  45

Met Asn Thr Tyr Val Gln Ala Arg Ala Ser Ser Ala Ala Asn Ile Lys
    50                  55                  60

Leu Leu Gln Leu Pro Ile Val Asp Pro Pro Ala Pro Glu Gln Tyr Gln
65                  70                  75                  80

Thr Leu Val Gly Phe Leu Ser Leu His Met Gln Asn His Lys His His
                85                  90                  95

Val Lys His Ala Leu Leu Asn Leu Met Lys Thr Thr Glu Ser Asn Ser
            100                 105                 110

Ser Asn Ser Val Arg Leu Ala Ala Ile Phe Val Asp Met Phe Ser Thr
        115                 120                 125

Thr Leu Ile Asp Val Ala Ala Glu Leu Ala Val Pro Cys Tyr Leu Phe
    130                 135                 140

Phe Ala Ser Pro Ala Ser Cys Leu Gly Phe Thr Leu Asp Leu Pro Arg
145                 150                 155                 160

Phe Asp Leu Ala Glu Ser Lys Ser Glu Phe Thr Val Pro Cys Phe Lys
                165                 170                 175

Asn Leu Leu Pro Arg Ser Val Phe Pro Asn Leu Val Leu Asp Ala Lys
            180                 185                 190

Asp Gly Thr Phe Trp Leu Ser Tyr His Ala Arg Arg Tyr Lys Glu Thr
        195                 200                 205

Lys Gly Ile Val Ile Asn Thr Leu Gln Glu Leu Glu Thr His Ala Leu

210                 215                 220

Gln Ser Leu His Asn Asp Ser Gln Leu Gln Arg Val Tyr Pro Ile Gly
225                 230                 235                 240

Pro Ile Leu Asp Leu Val Gly Ser Ala Gln Trp Asp Pro Asn Pro Ala
                245                 250                 255

Gln Tyr Lys Arg Ile Met Glu Trp Leu Asp Gln Gln Pro Leu Ser Ser
            260                 265                 270

Val Val Leu Leu Cys Phe Gly Ser Met Gly Ser Leu Glu Ala Asn Gln
        275                 280                 285

Val Glu Glu Ile Ala Ile Gly Leu Glu Arg Ala Gly Val Arg Phe Leu
    290                 295                 300

Trp Ala Leu Arg Glu Ser Pro Lys Ala Gln Leu Glu Tyr Pro Arg Asp
305                 310                 315                 320

Tyr Glu Asn His Lys Asp Val Leu Pro Asp Gly Phe Leu Glu Arg Thr
                325                 330                 335

Asn Asn Ile Gly Leu Val Cys Gly Trp Val Pro Gln Ala Val Val Leu
            340                 345                 350

Ala His Lys Ala Val Gly Gly Phe Val Ser His Cys Gly Trp Asn Ser
        355                 360                 365

Ile Leu Glu Ser Leu Trp His Gly Val Pro Val Ala Thr Trp Pro Leu
    370                 375                 380

Tyr Ser Glu Gln Gln Met Asn Ala Phe Gln Met Val Arg Asp Leu Gly
385                 390                 395                 400

Leu Ala Val Glu Ile Ser Val Asp Tyr Arg Val Gly Ala Asp Leu Val
                405                 410                 415

Arg Ala Glu Glu Val Glu Asn Gly Leu Arg Ser Leu Met Lys Gly Gly
            420                 425                 430

Asp Glu Ile Arg Arg Lys Val Lys Glu Met Ser Asp Thr Cys Arg Gly
        435                 440                 445

Ala Leu Leu Glu Asn Gly Ser Ser Tyr Ser Asn Leu Val Ser Leu Ile
    450                 455                 460

Gln Glu Leu Thr Ser
465